# EUROPEAN PATENT APPLICATION

(11) **EP 3 718 397 A1**
(43) Date of publication of application: **07.10.2020**
(21) Application number: 19167093.4
(22) Date of filing: 03.04.2019
(51) Int. Cl.: A01H 1/04, A01H 5/10, A01H 6/46, C07K 14/415, C12N 15/82, C12Q 1/6895

(54) **WHEAT CYTOPLASMIC MALE STERILITY RESTORER GENES, MOLECULAR MARKERS AND USES THEREOF**

(71) Applicant: German Seed Alliance GmbH, 51149 Köln (DE); KWS SAAT SE & Co. KGaA, 37574 Einbeck (DE); Saatzucht Bauer GmbH & Co. KG, 93083 Niedertraubling (DE); Saatzucht Streng-Engelen GmbH & Co. KG, 97215 Uffenheim (DE)
(72) Inventor: Geyer, Manuel, 85356 Freising (DE); Mohler, Volker, 80634 Munich (DE); Hartl, Lorenz, 86316 Friedberg (DE); Stein, Nils, 06484 Quedlinburg (DE); Zhou, Ruonan, 12355 Berlin (DE)
(74) Representative: Maiwald Patent- und Rechtsanwaltsgesellschaft mbH

(57) **Abstract**

The present invention provides functional restorer genes for cytoplasmic male sterility. The invention inter alia relates to functional restorer gene alleles localizing to a position corresponding to or referred to 14584864 to 14587134 of chromosome 1A of Chinese Spring wheat; or 19061029 to 19063412 of chromosome 1B of Chinese Spring wheat. The invention also relates to nucleic acids, polypeptides, marker alleles and SNPs.

## Description

### Field of the invention

The present invention *inter alia* provides functional restorer genes for cytoplasmic male sterility, methods for selecting or producing a plant comprising these functional restorer genes and nucleic acids and SNPs for use therein. The present invention thus relates to the field of molecular genetics, molecular biology, plant science and plant breeding.

### Background

Production of hybrid seeds using a cytoplasmic male sterility (CMS) system is successfully established in many crop species. In wheat, a male sterility system based on the cytoplasm of *Triticum timopheevii* is available (so called wheat G-type CMS, hereinafter abbreviated as "CMS"), but effective restorer genes have not been identified clearly so far. Many genes conferring restoration in plants, in particular in cereal plants, belong to the P-class PPR gene family. PPR proteins constitute a large family, with >400 members in *Arabidopsis* and rice that are thought to be RNA-binding proteins involved in post-transcriptional processes (RNA processing and translation) in mitochondria and chloroplasts, but little data exist on the functions of individual proteins in this family. Lurin et al. (2004) The Plant Cell, 16(8), 2089-2103) hypothesized that PPR proteins function as sequence-specific adaptors for a variety of other RNA-associated proteins. This idea was supported by several authors, however, there is only a small number of different genes within this PPR cluster which have been identified as fertility restorers with divergent molecular functions. Analyses of these clusters in other CMS systems are needed for a complete understanding of the evolution and molecular basis of CMS. This is often technically very difficult since the restorer genes belonging to the P-class PPR proteins are present in small clusters of highly homologous gene copies generated by local gene duplication and illegitimate recombination events (Schmitz-Linneweber and Small (2008) Trends in plant science, 13(12), 663-670.; Dahan and Mireau (2013) RNA biology, 10(9), 1469-1476). As a result of their fast evolving structure, restoration loci show an extreme allelic diversity with a highly complex and diverse gene content (Dahan and Mireau (2013); Melonek et al. (2016) Scientific reports, 6, 35152).

Recently published WO2018/015403 A1 and WO2018/015404 A1 describe the identification of at least one P-class PPR gene at the Rf3 locus which is alleged to be the restorer gene Rf3 in wheat. However, there are doubts that this gene is a functional restorer gene, since there are no structural differences between this gene in restorer and non-restorer background which could explain its functionality.

Hence, there is a need for the identification of new P-class PPR genes allowing fertility restoration in the F1 generation. Further, there is a need for molecular markers that allow detection of such restorer genes during breeding cycles.

### Summary of the invention

The present invention *inter alia* provides functional restorer genes for cytoplasmic male sterility. In one embodiment, the invention provides functional restorer genes for cytoplasmic male sterility in cereals. In another embodiment, the invention provides functional restorer genes for cytoplasmic male sterility in wheat.

The present invention provides two functional restorer (Rf) loci and genes for wheat G-type cytoplasmic male sterility (i.e., *T. timopheevii* cytoplasm) as well as markers associated therewith. The identified loci are located on chromosome 1A (short arm 1AS) and on chromosome 1B (short arm IBS), respectively, in the wheat genome.

### Functional restorer genes

In a first aspect, the present invention provides a nucleic acid comprising a functional restorer gene allele localizing to a position referred to position
(i) 14584864 to 14587134 of chromosome 1A of Chinese Spring wheat; and/or
(ii) 19061029 to 19063412 of chromosome 1B of Chinese Spring wheat.

The present invention also provides a nucleic acid comprising a functional restorer gene allele localizing to a position corresponding to position
(i) 14584864 to 14587134 of chromosome 1A of Chinese Spring wheat; and/or
(ii) 19061029 to 19063412 of chromosome 1B of Chinese Spring wheat.

In one embodiment, the nucleic acid comprising the functional restorer gene is derived from a wheat plant. The wheat plant may be selected from *Triticum aestivum* (e.g., Primepi, L19, R3 and R113), *Triticum durum, Triticum spelta* (e.g., Badenkrone) or *Triticale* sp.

The genome of Chinese Spring wheat serves as reference genome. The indicated nucleotide positions indicate the position of the allele in Chinese spring. In other wheat genomes, it is referred to the respective corresponding positions.

In another aspect, the present invention provides a nucleic acid comprising a functional restorer gene allele localizing to a position
(i) on chromosome 1A of a wheat plant flanked by markers according to SEQ ID NO: 109 and SEQ ID NO: 218, wherein preferably the SNP in SEQ ID NO: 109 is a G and/or the SNP in SEQ ID NO: 218 is a G; and/or
(ii) on chromosome 1B of a wheat plant flanked by markers according to SEQ ID NO: 47 and SEQ ID NO: 106, wherein preferably the SNP in SEQ ID NO: 47 is an A and/or the SNP in SEQ ID NO: 106 is an A.

In yet another aspect, the present invention provides a nucleic acid comprising a nucleic acid sequence
(i) having the coding sequence according to SEQ ID NO: 2;
(ii) encoding a protein having an amino acid sequence according to SEQ ID NO: 3;
(iii) having a coding sequence with at least 80%, at least 85%, preferably at least 90%, more preferably at least 95%, at least 96%, at least 97%, at least 98%, and most preferably at least 99% sequence identity with SEQ ID NO: 2, preferably over the entire length of SEQ ID NO: 2;
(iv) encoding a protein having an amino acid sequence having at at least 80%, at least 85%, preferably at least 90%, more preferably at least 95% at least 96%, at least 97%, at least 98%, and most preferably at least 99% sequence identity with SEQ ID NO: 3, preferably over the entire length of SEQ ID NO: 3;
(v) having the coding sequence according to SEQ ID NO: 13;
(vi) encoding a protein having an amino acid sequence according to SEQ ID NO: 14;
(vii) having a coding sequence with at least 80%, at least 85%, preferably at least 90%, more preferably at least 95%, at least 96%, at least 97%, at least 98%, and most preferably at least 99% sequence identity with SEQ ID NO: 13, preferably over the entire length of SEQ ID NO: 13; and/or
(viii) encoding a protein having an amino acid sequence having at least 80%, at least 85%, preferably at least 90%, more preferably at least 95% at least 96%, at least 97%, at least 98%, and most preferably at least 99% sequence identity with SEQ ID NO: 14, preferably over the entire length of SEQ ID NO: 14.

SEQ ID NO: 2 corresponds to the cDNA of Rf3 derived from genotype Primepi. This nucleic acid encodes a protein according to SEQ ID NO: 3.

SEQ ID NO:13 corresponds to the cDNA of Rf1 derived from genotype R3. Rf1 is also represented by the nucleic acid sequence according to SEQ ID NO: 15, or the nucleic acid according to SEQ ID NO: 17. These nucleic acids are identical to SEQ ID NO: 13, but are derived from different wheat genotypes (R113 and L19). These nucleic acids encode a protein according to SEQ ID NO: 16 or 18, respectively, which consequently are identical to SEQ ID NO 14.

In one embodiment, the nucleic acid sequence comprises a functional restorer gene. In one embodiment, the functional restorer gene is capable of restoring fertility in CMS plants, preferably in CMS cereal plants or wheat plants, by itself. In another embodiment, the functional restorer gene allele is capable of restoring fertility in CMS plants, preferably in CMS cereal plants or wheat plants, in combination with further restorer genes.

In a further embodiment, the functional restorer gene encodes a pentatricopeptide repeat (PPR) protein (i.e. a PPR gene). Preferably, the functional restorer gene encodes a pentatricopeptide repeat (PPR) protein with at least 10 P-motifs, at least 15 P-motifs, at least 16 P-motifs or at least 17 P-motifs. More preferably, the functional restorer gene encodes a pentatricopeptide repeat (PPR) protein with 16 P-motifs or 17 P-motifs.

The functional restorer gene may be for restoring cytoplasmic male sterility (CMS). Preferably, the functional restorer gene is for restoring cereal cytoplasmic male sterility or wheat cytoplasmic male sterility. More preferably, the functional restorer gene is for restoring G-type wheat cytoplasmic male sterility.

The functional restorer genes provided and described herein may be used for the identification of or screening for variants of the functional restorer genes of the present invention or further functional restorer genes. Such identification may involve methods established in the art including, but not limited to, sequence analysis, whole genome sequencing, molecular markers, hybridization and DNA amplification. Validation of thus identified variants of the functional restorer genes or further functional restorer genes may involve methods established in the art including, but not limited to stabile or transient transformation of the respective gene into the genome of a CMS plant, preferably in CMS cereal plants or wheat plants, stabile or transient genome editing-mediated integration of the respective gene into the genome of a CMS plant, preferably in CMS cereal plants or wheat plants, or by crossing a plant, preferably a cereal plant or a wheat plant, comprising the respective gene with a CMS plant of the same species.

In yet another aspect, the invention provides a chimeric gene comprising the following operably linked elements
(i) a promoter functional in a plant cell, preferably a cereal plant cell or a wheat plant cell, and
(ii) the nucleic acid according to the present invention,
preferably wherein at least one of said operably linked elements is heterologous with respect to at least one other element, more preferably wherein the promoter is heterologous with respect to the nucleic acid according to the invention.

The invention also provides a recombinant gene comprising the following operably linked elements
(i) a promoter functional in a plant cell, preferably a cereal plant cell or a wheat plant cell, and
(ii) the nucleic acid according to the present invention,
preferably wherein at least one of said operably linked elements is heterologous with respect to at least one other element, more preferably wherein the promoter is heterologous with respect to the nucleic acid according to the invention.

In one embodiment, the chimeric or recombinant gene further comprises a transcription termination and polyadenylation region functional in a plant cell, preferably a cereal plant cell or a wheat plant cell. In one embodiment, the promoter is capable of directing expression of the operably linked nucleic acid at least during early pollen development and/or meiosis. In another embodiment, the promoter is capable of directing expression of the operably linked nucleic acid in anther, tapetum, and/or developing microspores. Further, the promoter may be capable of directing expression of the operably linked nucleic acid at any stage of plant development. Promoters driving expression in plants are established in the art. Such promoters include, but are not limited to, promoters obtained from Agrobacterium, bacteria, viruses and plants.

In another aspect, the invention provides a vector or expression cassette comprising the nucleic acid of the invention. In another aspect, the invention provides a polypeptide, which is encoded by the nucleic acid of the invention.

### Plant cells, plants, part of plants, seeds

In one further aspect, the invention provides a plant cell, preferably a cereal plant cell or a wheat plant cell, comprising one or more of the nucleic acids of the invention, one or more of the vectors of the invention or one or more of the chimeric or recombinant genes of the invention. The nucleic acid(s) may be heterologous with respect to said plant cell or to the genomic location within said plant cell, e.g. as transgene or cisgene. The nucleic acid(s) may be comprised as exogenous or endogenous sequence. The chimeric or recombinant gene(s) may be heterologous with respect to said plant cell regarding the nucleic acid encoding the functional restorer gene. For example, the promoter of the chimeric or recombinant gene may be homologous with respect to said plant cell and the nucleic acid encoding the functional restorer gene may be heterologous with respect to said plant cell.

In one further aspect, the invention provides a plant, preferably a cereal plant or a wheat plant, or a part thereof comprising one or more of the nucleic acids of the invention, one or more of the vectors of the invention or one or more of the chimeric or recombinant genes of the invention. The nucleic acid(s) may be heterologous with respect to said plant or to the genomic location within said plant, e.g. as transgene or cisgene. The nucleic acid(s) may be comprised as exogenous or endogenous sequence. The chimeric or recombinant gene(s) may be heterologous with respect to said plant regarding the nucleic acid encoding the functional restorer gene. For example, the promoter of the chimeric or recombinant gene may be homologous with respect to said plant and the nucleic acid encoding the functional restorer gene may be heterologous with respect to said plant. In one embodiment the plant of the present invention comprises a plant cell of the present invention as described above.

In one aspect, the present invention relates to a part of a plant, preferably of a cereal plant or of a wheat plant, as described in any of the aspects and embodiments, which is or originates from preferably a shoot, root, petiole, bud, hypocotyl, flower or floral organ, seed, pollen, anther, fruit, ovule, embryo, plant tissue or cell.

In another aspect, the invention provides a seed of a plant, preferably of a cereal plant or of a wheat plant, comprising one or more of the nucleic acids of the invention, one or more of the vectors of the invention or one or more of the chimeric or recombinant genes of the invention. The nucleic acid(s) may be heterologous with respect to said seed or to the genomic location with said plant cell, e.g. as transgene or cisgene. The nucleic acid(s) may be comprised as exogenous or endogenous sequence. The chimeric or recombinant gene(s) may be heterologous with respect to said seed regarding the nucleic acid encoding the functional restorer gene. For example, the promoter of the chimeric or recombinant gene may be homologous with respect to said seed and the nucleic acid encoding the functional restorer gene may be heterologous with respect to said seed. In one embodiment the seed of the present invention comprises a plant cell of the present invention as described above.

The plant cell, the plant, the part thereof or the seed may be a cereal plant cell, cereal plant or part thereof or cereal seed, preferably a wheat plant cell, wheat plant or part thereof or wheat seed. The cereal plant cell, cereal plant or part thereof or cereal seed may be from a plant species selected from the group consisting of: *Triticum aestivum, Hordeum vulgare, Secale cereale, Avena sativa, Oryza sativa, Sorghum bicolor, Zea mays, Triticum durum, Triticum spelta, Triticum dicoccon, Triticum timopheevii, Triticum turanicum, Triticum monococcum* or Triticale sp. The wheat plant cell, wheat plant or part thereof or wheat seed may be from a plant species selected from the group consisting of: *Triticum aestivum, Triticum durum, Triticum spelta, Triticum dicoccon, Triticum timopheevii, Triticum turanicum, Triticum monococcum* or Triticale sp. Wheat species include, but are not limited to the genotypes Primepi, L19, R3 and R113.

In one embodiment, the plant cell, plant or part thereof or seed comprises a nucleic acid comprising the nucleic acid sequence of the Rf3 gene or a gene encoding Rf3. In another embodiment, the plant cell, plant or part thereof or seed comprises a nucleic acid comprising the nucleic acid sequence of the Rf1 gene or a gene encoding Rf1. In a further embodiment, the plant cell, plant or part thereof or seed comprises a nucleic acid comprising the nucleic acid sequence of the Rf3 gene or a gene encoding Rf3, and the nucleic acid sequence of the Rf1 gene or a gene encoding Rf1, or comprises the nucleic acid sequence of the Rf3 gene or a gene encoding Rf3, and the nucleic acid sequence of the Rf1 gene or a gene encoding Rf3.

In one embodiment, the plant cell, plant, part thereof or seed comprises a nucleic acid sequence comprising the nucleic acid sequence having the coding sequence according to SEQ ID NO: 2. In another embodiment, the plant cell, plant, part thereof or seed comprises a nucleic acid sequence comprising the nucleic acid sequence having the coding sequence according to SEQ ID NO: 13. In a further embodiment, the plant cell, plant, part thereof or seed comprises the nucleic acid sequence comprising a nucleic acid sequence having the coding sequence according to SEQ ID NO: 2 and the nucleic acid sequence comprising a nucleic acid sequence having the coding sequence according to SEQ ID NO: 13.

In yet another aspect, the invention provides a method for producing a plant cell preferably a cereal plant cell or a wheat plant cell, or a plant, preferably a cereal plant or a wheat plant, or a part thereof or a seed comprising the functional restorer gene allele for restoring cytoplasmic male sterility as described above. In another aspect, the present invention provides a method for increasing restoration capacity of a plant with cytoplasmic male sterility, preferably with G-type cytoplasmic male sterility.

In another aspect, the present invention provides a method for converting a non-restoring plant, preferably a non-restoring cereal plant or a non-restoring wheat plant, into a restoring plant preferably a restoring cereal plant or a restoring wheat plant, respectively, comprising the functional restorer gene allele of the present invention for restoring cytoplasmic male sterility, preferably with G-type cytoplasmic male sterility. In yet another aspect, the present invention provides a method for converting a non-restoring plant, preferably a non-restoring cereal plant or a non-restoring wheat plant, into a restoring plant preferably a restoring cereal plant or a restoring wheat plant, respectively, comprising more than one functional restorer gene alleles for restoring cytoplasmic male sterility, preferably wherein at least one of these functional restorer gene alleles is a functional restorer gene allele of the present invention. More than one functional restorer gene alleles for restoring cytoplasmic male sterility can mean for instances two or more functional restorer gene alleles for restoring cytoplasmic male sterility, three or more functional restorer gene alleles for restoring cytoplasmic male sterility, or four or more functional restorer gene alleles for restoring cytoplasmic male sterility. Particularly, more than one functional restorer gene alleles for restoring cytoplasmic male sterility can mean for instances at least two functional restorer gene alleles for restoring cytoplasmic male sterility, at least three functional restorer gene alleles for restoring cytoplasmic male sterility, at least four functional restorer gene alleles for restoring cytoplasmic male sterility or at least five functional restorer gene alleles for restoring cytoplasmic male sterility.

Moreover, more than one functional restorer gene alleles for restoring cytoplasmic male sterility can mean for instances two functional restorer gene alleles for restoring cytoplasmic male sterility, three functional restorer gene alleles for restoring cytoplasmic male sterility, four functional restorer gene alleles for restoring cytoplasmic male sterility or five functional restorer gene alleles for restoring cytoplasmic male sterility.

Any of these methods may involve a step of introducing the nucleic acid of the invention, the vector of the invention or the chimeric gene of the invention into the plant cell, the plant or a part thereof, or seed by transformation. Any of these methods may involve a step of introducing the nucleic acid of the invention, the vector of the invention or the chimeric gene of the invention into the plant cell, the plant or a part thereof, or seed by genome editing. Any of these methods may involve a step of introducing the nucleic acid of the invention, the vector of the invention or the chimeric gene of the invention into the plant cell, the plant or a part thereof, or seed by mutagenesis, in particular by random mutagenesis (e.g., chemical induced mutagenesis, irradiation induced mutagenesis, TILLING, etc.). Any of these methods may involve a step of introducing the nucleic acid of the invention, the vector of the invention or the chimeric gene of the invention into the plant cell, the plant or a part thereof, or seed by crossing and optionally selection. Further, any of these methods may additionally comprise a step of allowing expression of the polypeptide encoded by the introduced nucleic acid, vector or chimeric gene.

In one embodiment, the present invention provides a method for producing a plant cell, a plant, a part thereof and/or a seed comprising a functional restorer gene, the method comprising the step of crossing a plant comprising a functional restorer gene with a CMS plant, preferably of the same species. In a further embodiment, the present invention provides a method for producing a plant cell, a plant, a part thereof and/or a seed comprising a functional restorer gene, the method comprising the step of crossing a male plant comprising the functional restorer gene with a female CMS plant, preferably of the same species. The method may further comprise the step of collecting, selecting or harvesting the plant cell, plant, a part thereof and/or seed. The functional restorer gene may be any functional restorer gene described herein.

In one aspect, the invention provides a plant cell, a plant, a part thereof, or seed obtained or obtainable by any of the methods according to the present invention.

In a further aspect, the invention provides method for identifying or selecting a plant cell, plant or a part thereof or a seed, preferably a cereal plant cell, cereal plant or a part thereof or cereal seed or a wheat plant cell, wheat plant or a part thereof or wheat seed, comprising the functional restorer gene allele for restoring cytoplasmic male sterility of the present invention. The method may comprise a step of identifying or detecting in said plant or said seed the presence or absence of the nucleic acid of the invention. The method may comprise a step of identifying or detecting in said plant or said seed the presence or absence of the polypeptide of the invention. The method may comprise a step of identifying or detecting in said plant or said seed the presence or absence of a chimeric gene or recombinant gene of the invention. Optionally, the method for identifying or selecting further comprise the step of selecting a plant cell, plant or a part thereof or a seed, preferably a cereal plant cell, cereal plant or a part thereof or cereal seed or a wheat plant cell, wheat plant or a part thereof or wheat seed, comprising the functional restorer gene allele for restoring cytoplasmic male sterility of the present invention. Selected plant or a plant grown or regenerated from selected plant cell or plant part or the plant from which the plant cell or the plant part has been isolated for identifying/detecting step may be used for further breeding, preferably for the breeding of a restorer line, for the breeding of hybrid lines, for backcrossing, for reverse breeding or forward breeding.

### Markers and methods involving markers

In one aspect, the invention provides a method for identifying or selecting a wheat plant cell, wheat plant or a part thereof or a wheat seed comprising the functional restorer gene allele for wheat G-type cytoplasmic male sterility located on chromosome 1A and/or the functional restorer gene allele for wheat G-type cytoplasmic male sterility located on chromosome 1B, comprising the steps of:
(i) detecting in a wheat plant cell, wheat plant or a part thereof or a wheat seed at least one marker allele linked to the functional restorer gene allele for wheat G-type cytoplasmic male sterility located on chromosome 1A (1A restorer marker allele) and/or at least one marker allele linked to the functional restorer gene allele for wheat G-type cytoplasmic male sterility located on chromosome 1B (1B restorer marker allele); and
(ii) identifying and optionally selecting a plant cell, wheat plant or a part thereof or a wheat seed comprising the at least one 1A restorer marker allele and/or the at least one 1B restorer marker allele based on the detection in step (i).

The method may further comprise the step of breeding a restoring wheat plant by crossing a non-restoring plant with the plant selected in (ii) or a progeny thereof, with a plant grown or regenerated from selected plant cell or plant part or with the plant from which the plant cell or the plant part has been isolated for identifying/detecting step, to obtain a restorer plant, wherein the progeny or the grown/regenerated plant comprises the at least one 1A restorer marker allele and/or the at least one 1B restorer marker allele. The method may alternatively comprise the step of breeding a restoring wheat plant by crossing a CMS plant with the plant selected in (ii) or a progeny thereof, with a plant grown or regenerated from selected plant cell or plant part or with the plant from which the plant cell or the plant part has been isolated for identifying/detecting step, to obtain a restored plant, wherein the progeny or the grown/regenerated plant comprises the at least one 1A restorer marker allele and/or the at least one 1B restorer marker allele. Further, the method may comprise the step of breeding a CMS hybrid by crossing a CMS plant with the plant selected in (ii) or a progeny thereof, with a plant grown or regenerated from selected plant cell or plant part or with the plant from which the plant cell or the plant part has been isolated for identifying/detecting step, to obtain a restored plant, wherein the progeny or the grown/regenerated plant comprises the at least one 1A restorer marker allele and/or the at least one 1B restorer marker allele.

In one embodiment, the method is for identifying or selecting a wheat plant comprising the functional restorer gene allele for wheat G-type cytoplasmic male sterility located on chromosome 1A. In another embodiment, the method is for identifying or selecting a wheat plant comprising the functional restorer gene allele for wheat G-type cytoplasmic male sterility located on chromosome 1B. In one embodiment, the functional restorer gene allele for wheat G-type cytoplasmic male sterility located on chromosome 1A is Rf1. In one embodiment, the functional restorer gene allele for wheat G-type cytoplasmic male sterility located on chromosome 1B is Rf3.

In another aspect, the invention provides a method for determining the presence, absence or zygosity status of the functional restorer gene allele for wheat G-type cytoplasmic male sterility located on chromosome 1A and/or on chromosome 1B in a sample derived from a wheat plant, comprising the steps of
(i) providing genomic DNA from said sample, and
(ii) analyzing and/or determining said DNA for the presence, absence or zygosity status of at least one marker allele linked to the functional restorer gene allele for wheat G-type cytoplasmic male sterility located on chromosome 1A (1A restorer marker allele) and/or the presence, absence or zygosity status of at least one marker allele linked to the functional restorer gene allele for wheat G-type cytoplasmic male sterility located on chromosome 1B (1B restorer marker allele).

In one embodiment, the method is for determining the presence, absence or zygosity status of the functional restorer gene allele for wheat G-type cytoplasmic male sterility located on chromosome 1A. In another embodiment, the method is for determining the presence, absence or zygosity status of the functional restorer gene allele for wheat G-type cytoplasmic male sterility located on chromosome 1B. In one embodiment, the functional restorer gene allele for wheat G-type cytoplasmic male sterility located on chromosome 1A is Rf1. In one embodiment, the functional restorer gene allele for wheat G-type cytoplasmic male sterility located on chromosome 1B is Rf3.

In another embodiment, the method for determining the presence, absence or zygosity status of the functional restorer gene allele for wheat G-type cytoplasmic male sterility located on chromosome 1A and/or on chromosome 1B in a sample derived from a wheat plant, comprising the step (iii) of identifying or selecting the wheat plant from which the sample comprising the at least one 1A restorer marker allele and/or the at least one 1B restorer marker allele, optionally in the desired zygosity status, has been isolated, based on the analysis and/or determination in step (ii). Preferably, the zygosity status is selected from the group of heterozygosity, hemizygosity, homozygosity or homoeozygosity.

The method may further comprise the step (iv) of breeding a restoring wheat plant by crossing a non-restoring plant with the plant selected in (iii) or a progeny thereof to obtain a restorer plant, wherein the progeny comprises the at least one 1A restorer marker allele and/or the at least one 1B restorer marker allele. The method may alternatively comprise the step of breeding a restoring wheat plant or a hybrid wheat plant (CMS hybrid) by crossing a CMS plant with the plant selected in (ii) or a progeny thereof to obtain a restored plant, wherein the progeny or the grown/regenerated plant comprises the at least one 1A restorer marker allele and/or the at least one 1B restorer marker allele. Alternatively, selected plant may be used for further breeding, preferably for the breeding of a restorer line, for the breeding of hybrid lines, for backcrossing, for reverse breeding or forward breeding.

The following embodiments apply to both of the above aspects.

In an embodiment, the at least one 1A restorer marker allele localizes within an interval on chromosome 1A comprising and flanked by the markers of SEQ ID NO: 109 and SEQ ID NO: 152 and/or the at least one 1B restorer marker allele localizes within an interval on chromosome 1B comprising and flanked by the markers of SEQ ID NO: 105 and SEQ ID NO: 108.

In a further embodiment, the sequence of the at least one 1A restorer marker allele linked to said functional restorer gene allele may be selected from the group consisting of SEQ ID NOs: 47- 108, wherein the at least one restorer marker allele is a SNP and the nucleotide at the SNP position in SEQ ID NOs: 47-108 is
a. a A at SEQ ID NO: 47
b. a G at SEQ ID NO: 48
c. a G at SEQ ID NO: 49
d. a C at SEQ ID NO: 50
e. a C at SEQ ID NO: 51
f. a T at SEQ ID NO: 52
g. a T at SEQ ID NO: 53
h. a A at SEQ ID NO: 54
i. a A at SEQ ID NO: 55
j. a C at SEQ ID NO: 56
k. a G at SEQ ID NO: 57
1. a G at SEQ ID NO: 58
m. a G at SEQ ID NO: 59
n. a A at SEQ ID NO: 60
o. a C at SEQ ID NO: 61
p. a A at SEQ ID NO: 62
q. a C at SEQ ID NO: 63
r. a C at SEQ ID NO: 64
s. a A at SEQ ID NO: 65
t. a C at SEQ ID NO: 66
u. a G at SEQ ID NO: 67
v. a A at SEQ ID NO: 68
w. a C at SEQ ID NO: 69
x. a C at SEQ ID NO: 70
y. a Cat SEQ ID NO: 71
z. a G at SEQ ID NO: 72
aa. a T at SEQ ID NO: 73
bb. a C at SEQ ID NO: 74
cc. a G at SEQ ID NO: 75
dd. a G at SEQ ID NO: 76
ee. a G at SEQ ID NO: 77
ff. a G at SEQ ID NO: 78
gg. a A at SEQ ID NO: 79
hh. a T at SEQ ID NO: 80
ii. a A at SEQ ID NO: 81
jj. a C at SEQ ID NO: 82
kk. a G at SEQ ID NO: 83
ll. a C at SEQ ID NO: 84
mm. a T at SEQ ID NO: 85
nn. a A at SEQ ID NO: 86
oo. a T at SEQ ID NO: 87
pp. a G at SEQ ID NO: 88
qq. a A at SEQ ID NO: 89
rr. a G at SEQ ID NO: 90
ss. a C at SEQ ID NO: 91
tt. a G at SEQ ID NO: 92
uu. a A at SEQ ID NO: 93
vv. a C at SEQ ID NO: 94
ww. a T at SEQ ID NO: 95
xx. a T at SEQ ID NO: 96
yy. a G at SEQ ID NO: 97
zz. a A at SEQ ID NO: 98
aaa. a T at SEQ ID NO: 99
bbb. a C at SEQ ID NO: 100
ccc. a G at SEQ ID NO: 101
ddd. a A at SEQ ID NO: 102
eee. a C at SEQ ID NO: 103
fff. a T at SEQ ID NO: 104
ggg. a T at SEQ ID NO: 105
hhh. a A at SEQ ID NO: 106
iii. a G at SEQ ID NO: 107, and
jjj. a C at SEQ ID NO: 108; respectively.

In another embodiment, the sequence of the at least one 1B restorer marker allele linked to said functional restorer gene allele is selected from the group consisting of SEQ ID NOs: 109- 152, wherein the at least one restorer marker allele is a SNP and the nucleotide at the SNP position in SEQ ID NOs: 109-152 is
a. a G at SEQ ID NO: 109
b. a T at SEQ ID NO: 110
c. a C at SEQ ID NO: 111
d. a G at SEQ ID NO: 112
e. a T at SEQ ID NO: 113
f. a Cat SEQ ID NO: 114
g. a Cat SEQ ID NO: 115
h. a Cat SEQ ID NO: 116
i. a T at SEQ ID NO: 117
j. a C at SEQ ID NO: 118
k. a A at SEQ ID NO: 119
l. a A at SEQ ID NO: 120
m. a Cat SEQ ID NO: 121
n. a T at SEQ ID NO: 122
o. a G at SEQ ID NO: 123
p. a G at SEQ ID NO: 124
q. a A at SEQ ID NO: 125
r. a G at SEQ ID NO: 126
s. a T at SEQ ID NO: 127
t. a T at SEQ ID NO: 128
u. a T at SEQ ID NO: 129
v. a G at SEQ ID NO: 130
w. a Cat SEQ ID NO: 131
x. a G at SEQ ID NO: 132
y. a C at SEQ ID NO: 133
z. a T at SEQ ID NO: 134
aa. a C at SEQ ID NO: 135
bb. a A at SEQ ID NO: 136
cc. a A at SEQ ID NO: 137
dd. a A at SEQ ID NO: 138
ee. a G at SEQ ID NO: 139
ff. a A at SEQ ID NO: 140
gg. a T at SEQ ID NO: 141
hh. a C at SEQ ID NO: 142
ii. a A at SEQ ID NO: 143
jj. a A at SEQ ID NO: 144
kk. a T at SEQ ID NO: 145
ll. a T at SEQ ID NO: 146
mm. a C at SEQ ID NO: 147
nn. a C at SEQ ID NO: 148
oo. a T at SEQ ID NO: 149
pp. a G at SEQ ID NO: 150
qq. a C at SEQ ID NO: 151, and
rr. a G at SEQ ID NO: 152; respectively.

In another embodiment, the sequence of the at least one 1B restorer marker allele linked to said functional restorer gene allele is selected from the group consisting of SEQ ID NOs: 153- 189, wherein the at least one restorer marker allele is a SNP and the nucleotide at the SNP position in SEQ ID NOs: 153-189 is
a. a G at SEQ ID NO: 153
b. a T at SEQ ID NO: 154
c. a C at SEQ ID NO: 155
d. a Cat SEQ ID NO: 156
e. a C at SEQ ID NO: 157
f. aG atSEQID NO: 158
g. a T at SEQ ID NO: 159
h. a C at SEQ ID NO: 160
i. a T at SEQ ID NO: 161
j. a C at SEQ ID NO: 162
k. a A at SEQ ID NO: 163
l. a A at SEQ ID NO: 164
m. a C at SEQ ID NO: 165
n. a G at SEQ ID NO: 166
o. a G at SEQ ID NO: 167
p. a A at SEQ ID NO: 168
q. a G at SEQ ID NO: 169
r. a T at SEQ ID NO: 170
s. a T at SEQ ID NO: 171
t. a T at SEQ ID NO: 172
u. a G at SEQ ID NO: 173
v. a C at SEQ ID NO: 174
w. a G at SEQ ID NO: 175
x. a C at SEQ ID NO: 176
y. a T at SEQ ID NO: 177
z. a C at SEQ ID NO: 178
aa. a A at SEQ ID NO: 179
bb. a A at SEQ ID NO: 180
cc. a G at SEQ ID NO: 181
dd. a C at SEQ ID NO: 182
ee. a C at SEQ ID NO: 183
ff. a T at SEQ ID NO: 184
gg. a G at SEQ ID NO: 185
hh. a A at SEQ ID NO: 186
ii. a Cat SEQ ID NO: 187
jj. a C at SEQ ID NO: 188, and
kk. a T at SEQ ID NO: 189; respectively.

In another embodiment, the sequence of the at least one 1B restorer marker allele linked to said functional restorer gene allele is selected from the group consisting of SEQ ID NOs: 190- 225, wherein the at least one restorer marker allele is a SNP and the nucleotide at the SNP position in SEQ ID NOs: 190-225 is
a. a G at SEQ ID NO: 190
b. a T at SEQ ID NO: 191
c. a C at SEQ ID NO: 192
d. a G at SEQ ID NO: 193
e. a T at SEQ ID NO: 194
f. a C at SEQ ID NO: 195
g. a C at SEQ ID NO: 196
h. a T at SEQ ID NO: 197
i. aCat SEQID NO: 198
j. a A at SEQ ID NO: 199
k. a A at SEQ ID NO: 200
l. a C at SEQ ID NO: 201
m. a A at SEQ ID NO: 202
n. a G at SEQ ID NO: 203
o. a G at SEQ ID NO: 204
p. a A at SEQ ID NO: 205
q. a G at SEQ ID NO: 206
r. a T at SEQ ID NO: 207
s. a T at SEQ ID NO: 208
t. a T at SEQ ID NO: 209
u. a G at SEQ ID NO: 210
v. a Cat SEQ ID NO: 211
w. a G at SEQ ID NO: 212
x. a C at SEQ ID NO: 213
y. a T at SEQ ID NO: 214
z. a Cat SEQ ID NO: 215
aa. a A at SEQ ID NO: 216
bb. a A at SEQ ID NO: 217
cc. a G at SEQ ID NO: 218
dd. a Cat SEQ ID NO: 219
ee. a T at SEQ ID NO: 220
ff. a G at SEQ ID NO: 221
gg. a A at SEQ ID NO: 222
hh. a C at SEQ ID NO: 223
ii. a C at SEQ ID NO: 224, and
jj. a T at SEQ ID NO: 225; respectively.

In a further embodiment, the sequence of the at least one 1A restorer marker allele is selected from the group consisting of SEQ ID NO: 109, SEQ ID NO: 111 and SEQ ID NO: 218, wherein the at least one restorer marker allele is a SNP and the nucleotide at the SNP position in SEQ ID NO: 109, SEQ ID NO: 111 and SEQ ID NO: 218 is
a. a G at SEQ ID NO: 109,
b. a Cat SEQ ID NO: 111, and
c. a G at SEQ ID NO: 218; respectively.

In another embodiment, the sequence of the at least one 1B restorer marker allele is selected from the group consisting of SEQ ID NOs: 56, 58, 64, 66, 75, 84, 85, 89, 94, 99, 101, 105 and 107, wherein the at least one restorer marker allele is a SNP and the nucleotide at the SNP position in SEQ ID NOs: 56, 58, 64, 66, 75, 84, 85, 89, 94, 99, 101, 105 and 107 is
a. a C at SEQ ID NO: 56,
b. a G at SEQ ID NO: 58,
c. a C at SEQ ID NO: 64,
d. a C at SEQ ID NO: 66,
e. a G at SEQ ID NO: 75,
f. a C at SEQ ID NO: 84,
g. a T at SEQ ID NO: 85,
h. a A at SEQ ID NO: 89,
i. a C at SEQ ID NO: 94,
j. a T at SEQ ID NO: 99,
k. a G at SEQ ID NO: 101,
l. a T at SEQ ID NO: 105, and
m. a G at SEQ ID NO: 107; respectively.

In one embodiment, the at least one marker allele linked to the functional restorer gene allele for wheat G-type cytoplasmic male sterility located on chromosome 1A is a SNP selected from the SNPs shown in tables 4-6.

In one embodiment, the at least one marker allele linked to the functional restorer gene allele for wheat G-type cytoplasmic male sterility located on chromosome 1B is a SNP selected from the SNPs shown in table 3.

In these tables, the nucleotide position of the SNP is shown in column 2 of the respective table, and the nucleotide at this position is shown in column 5 of the respective table.

In one embodiment, the presence of the at least one 1A restorer marker allele and/or the at least one 1B restorer marker allele indicates the presence of the functional restorer gene allele.

The presence or the absence or the zygosity status of the restorer marker allele of the present invention may be detected by any known method, in particular methods for detecting SNPs. In one embodiment, the presence/absence/zygosity status of the marker allele is detected by PCR. In another embodiment, the presence/absence/zygosity status of the marker allele is detected by hybridization. Further detection methods include, but are not limited to DNA / RNA sequencing, mass-spectrometry, DHPLC, DNA chip, pyrosequencing, WGS (whole genome sequencing) or KASP marker technology.

### Markers and uses

In one aspect, the invention provides an oligonucleotide comprising a nucleic acid sequence according to any one of SEQ ID NOs: 47-225, wherein the nucleotide position of the SNP is shown in column 2 of the respective tables 3-6, and the nucleotide at this position is shown in column 5 of the respective tables 3-6.

In one embodiment, the invention provides an oligonucleotide comprising a nucleic acid sequence according to any one of SEQ ID NOs: 109, 111 and 218, wherein the nucleotide position of the SNP is shown in column 2 of table 8, and the nucleotide at this position is shown in column 5 of table 8.

In one embodiment, the invention provides an oligonucleotide comprising a nucleic acid sequence according to any one of SEQ ID NOs: 56, 58, 64, 66, 75, 84, 85, 89, 94, 99, 101, 105 and 107, wherein the nucleotide position of the SNP is shown in column 2 of table 7, and the nucleotide at this position is shown in column 5 of table 7.

In another aspect, the invention provides the use of an oligonucleotide comprising a nucleic acid sequence according to any one of SEQ ID NOs: 47-225 in a method for identifying one or more functional restorer gene alleles in a wheat plant, wherein the nucleotide position of the SNP is shown in column 2 of tables 3-6, and the nucleotide at this position is shown in column 5 of tables 3-6.

In another aspect, the invention provides the use of an oligonucleotide comprising a nucleic acid sequence according to any one of SEQ ID NOs: 47-225 for identification of a wheat plant comprising a functional restorer gene allele for wheat G-type cytoplasmic male sterility, wherein the nucleotide position of the SNP is shown in column 2 of tables 3-6, and the nucleotide at this position is shown in column 5 of tables 3-6.

In yet another aspect, the invention provides the use of an oligonucleotide comprising a nucleic acid sequence according to any one of SEQ ID NOs: 47-225 to identify at least one further marker allele linked to a functional restorer gene for wheat G-type cytoplasmic male sterility, wherein the nucleotide position of the SNP is shown in column 2 of tables 3-6, and the nucleotide at this position is shown in column 5 of tables 3-6.

The following embodiments apply to the uses described in the aspects above.

In one embodiment, the nucleic acid is an oligonucleotide comprising a nucleic acid sequence according to any one of SEQ ID NOs: 109, 111 and 218, wherein the nucleotide position of the SNP is shown in column 2 of table 8, and the nucleotide at this position is shown in column 5 of table 8.

In one embodiment, the nucleic acid is an oligonucleotide comprising a nucleic acid sequence according to any one of SEQ ID NOs: 56, 58, 64, 66, 75, 84, 85, 89, 94, 99, 101, 105 and 107, wherein the nucleotide position of the SNP is shown in column 2 of table 7, and the nucleotide at this position is shown in column 5 of table 7.

In the above described uses, the markers may be present on a chip or array. The markers may be present in combination with further markers or nucleotide sequences. Such arrays or chips allow detection of multiple candidate genes or sequence features.

### SNPs

In one aspect, the present invention provides a SNP selected from the group of SNPs shown in tables 4-6, wherein the nucleotide position of the SNP is shown in column 2 of the respective table, and the nucleotide at this position is shown in column 5 of the respective table.

In another aspect, the present invention provides a SNP selected from the group of SNPs shown in table 3, wherein the nucleotide position of the SNP is shown in column 2 of table 3, and the nucleotide at this position is shown in column 5 of table 3.

In one embodiment, the invention provides a SNP selected from the group of SNPs shown in table 8, wherein the nucleotide position of the SNP is shown in column 2 of table 8, and the nucleotide at this position is shown in column 5 of table 8.

In one embodiment, the invention provides a SNP selected from the group of SNPs shown in table 7, wherein the nucleotide position of the SNP is shown in column 2 of table 7, and the nucleotide at this position is shown in column 5 of table 7.

The invention further provides oligonucleotides for detecting any of the SNPs disclosed herein. The oligonucleotide may comprise nucleic acid sequences (e.g. parts) derived from any one of SEQ ID Nos: 47-225, wherein the nucleotide position of the SNP is shown in column 2 of tables 3-6, and the nucleotide at this position is shown in column 5 of tables 3-6. The oligonucleotide may be of any length (e.g., 21 nt, 31 nt, 41 nt, 51 nt, 61 nt, 71 nt, 81 nt, 91 nt, 101 nt, 121 nt, 131 nt, 141 nt, 151 nt, 161 nt, 171 nt, 181 nt, 191 nt, 201 nt, 211 nt) provided that the nucleotide sequence of the oligonucleotide covers the SNP position. For example, the oligonucleotide may be an oligonucleotide comprising the SNP position and at least 10 nt upstream and at least 10 nt, downstream, or 50 nt upstream and 50 nt downstream thereof. The SNP position is embedded in the oligonucleotide with a length of at least 20 nucleotides, at least 30 nucleotides, at least 40 nucleotides, at least 50 nucleotides, preferably at least 75 nucleotides, at least 100 nucleotides at least 150 nucleotides or at least 200 nucleotides, which is able to detect any of the SNPs disclosed herein.

### Uses of SNPs

In one aspect, the invention provides the use of a SNP selected from the group of SNPs shown in tables 4-6 in a method for identifying one or more functional restorer gene alleles in a wheat plant, wherein the nucleotide position of the SNP is shown in column 2 of the respective table, and the nucleotide at this position is shown in column 5 of the respective table.

In one aspect, the invention provides the use of a SNP selected from the group of SNPs shown in table 3 in a method for identifying one or more functional restorer gene alleles in a wheat plant, wherein the nucleotide position of the SNP is shown in column 2 of table 3, and the nucleotide at this position is shown in column 5 of table 3.

In one aspect, the invention provides the use of a SNP selected from the group of SNPs shown in tables 4-6 in a method for identification of a wheat plant comprising a functional restorer gene allele for wheat G-type cytoplasmic male sterility, wherein the nucleotide position of the SNP is shown in column 2 of the respective table, and the nucleotide at this position is shown in column 5 of the respective table.

In one aspect, the invention provides the use of a SNP selected from the group of SNPs shown in table 3 in a method identification of a wheat plant comprising a functional restorer gene allele for wheat G-type cytoplasmic male sterility, wherein the nucleotide position of the SNP is shown in column 2 of table 3, and the nucleotide at this position is shown in column 5 of table 3.

In one embodiment, the presence or absence of the SNP is detected in a wheat plant and the presence of the SNP indicates the presence a functional restorer gene allele. The presence of the SNP may be detected by any known method. In one embodiment, the presence of the SNP is detected by PCR. In another embodiment, the presence of the SNP is detected by hybridization. Further detection methods include, but are not limited to DNA / RNA sequencing, mass-spectrometry, DHPLC and DNA chip.

In one aspect, the invention provides the use of a SNP selected from the group of SNPs shown in tables 4-6 to identify at least one further marker allele linked to a functional restorer gene for wheat G-type cytoplasmic male sterility, wherein the nucleotide position of the SNP is shown in column 2 of the respective table, and the nucleotide at this position is shown in column 5 of the respective table.

In one aspect, the invention provides the use of a SNP selected from the group of SNPs shown in table 3 to identify at least one further marker allele linked to a functional restorer gene for wheat G-type cytoplasmic male sterility, wherein the nucleotide position of the SNP is shown in column 2 of table 3, and the nucleotide at this position is shown in column 5 of table 3.

In one embodiment, the invention provides the use of a SNP selected from the group of SNPs shown in table 8, wherein the nucleotide position of the SNP is shown in column 2 of table 8, and the nucleotide at this position is shown in column 5 of table 8.

In one embodiment, the invention provides the use of a SNP selected from the group of SNPs shown in table 7, wherein the nucleotide position of the SNP is shown in column 2 of table 7, and the nucleotide at this position is shown in column 5 of table 7.

### Figures

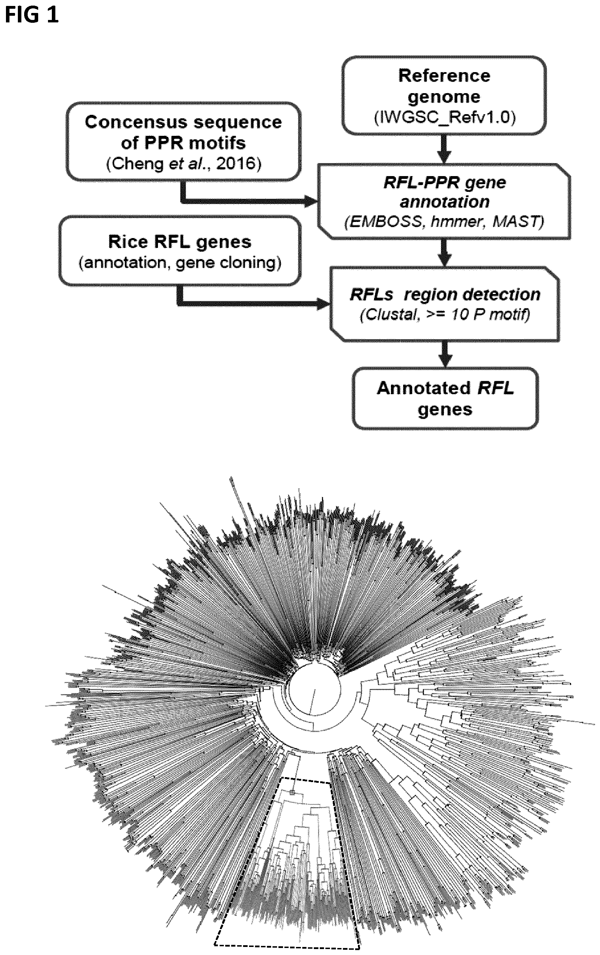

Fig. 1 shows the PPR gene family in wheat. Upper panel: flowchart of analytical steps of PPR gene family analysis in wheat. Below panel: 1659 PPR ORFs were found and sequences were used for performing a phylogenomic gene family analysis. In the section emphasized by dashed lines, all 159 RFL-PPR genes are shown, forming a distinct cluster in the gene family. (references: Cheng, S., Gutmann, B., Zhong, X., Ye, Y., Fischer, M., Bai, F., Castleden, I., Song, Y., Song, B., Huang, J., Liu, X., Xu, X., Lim, B., Bond, C., Yiu, S., Small, I. (2016) Redefining the structural motifs that determine RNA binding and RNA editing by pentatricopeptide repeat proteins in land plants, the Plant Journal 85, p. 532-547.; HMMER 3.1b2 (February 2015); http://hmmer.org/).

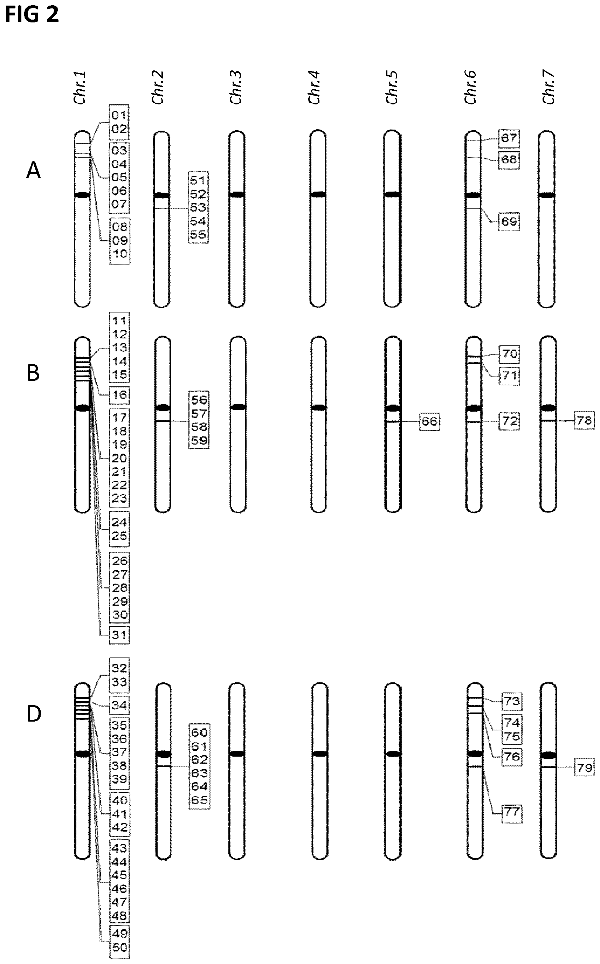

**Fig. 2** shows genome-wide distribution of the found full length RFL-PPR genes in wheat. The 79 full length RFL-PPR gene sequences of Chinese Spring represent the reference dataset for resequencing the same genes in Rf- and non-Rf-genotypes.

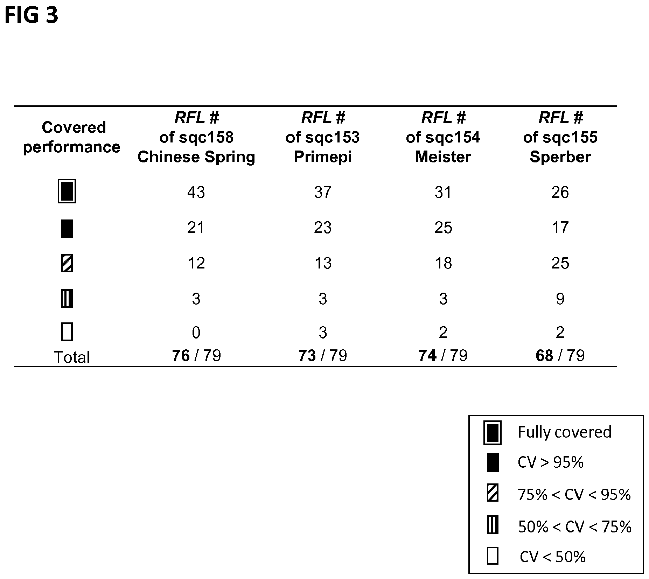

**Fig. 3** shows the summary statistics of resequencing by PPR sequence capture. In total, 76 of the 79 full length RFL genes in Chinese Spring could be captured.

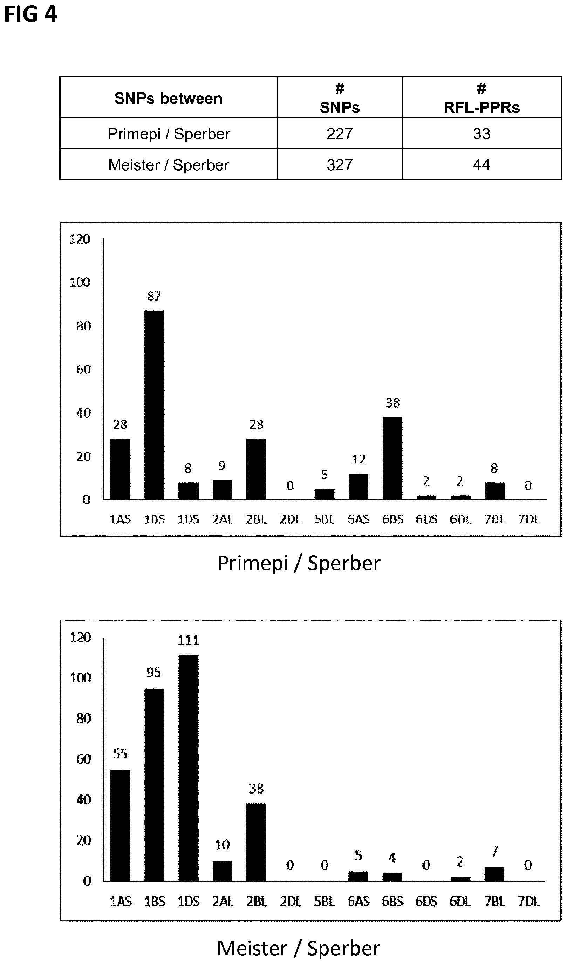

**Fig. 4** shows SNP polymorphism summary between wheat genotypes and their genomic allocation/distribution.

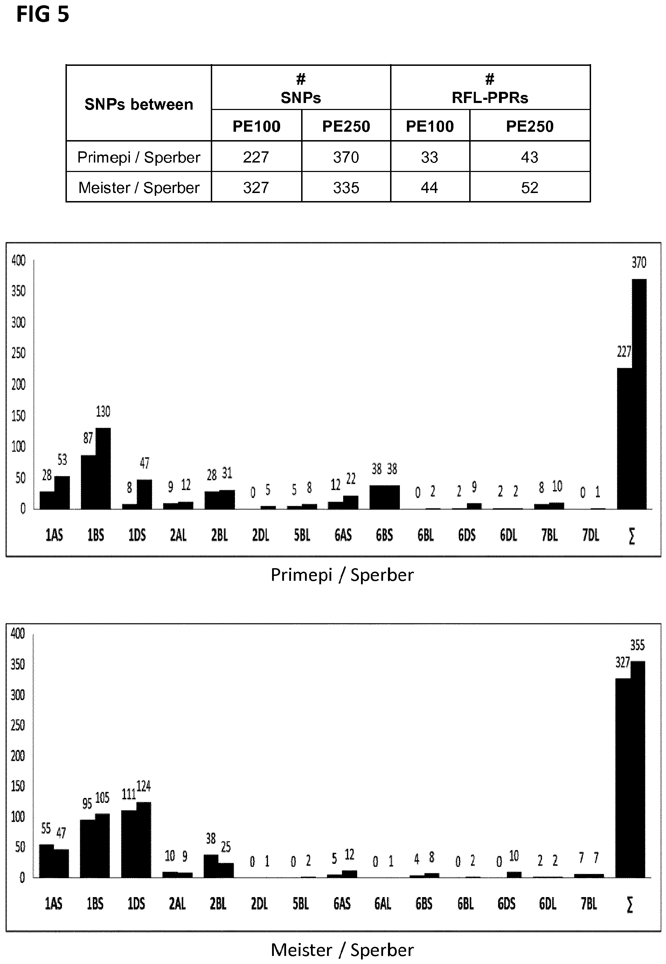

**Fig. 5** represents RFL-PPR sequence capture and SNP identification. The number of RFL-PPR genes exhibiting potential SNPs was increased by approximately 30%.

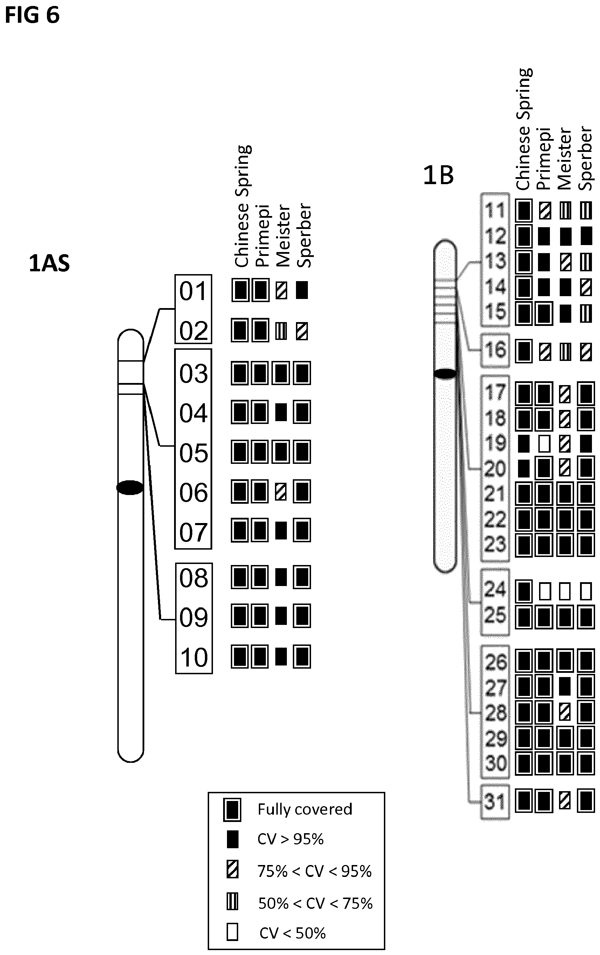

**Fig. 6** shows distribution of resequencing by PPR sequence capture on chromosomes 1A and 1B as summarized in Fig. 3. On the right side of the identified full length RFL-PPR gene sequences the coverage over the different genotypes Chinese Spring, Primepi, Meister and Sperber is indicated.

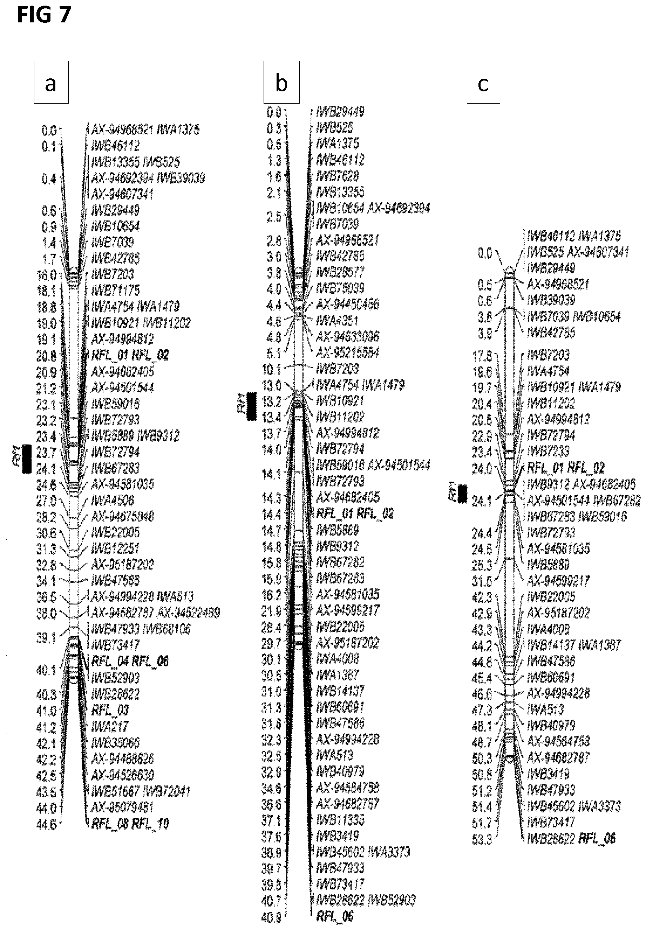

**Fig. 7** shows the mapping of Rf1 based on novel RFL-PPR capture derived SNP markers. A cluster of RFL genes allocates genetically with the Rf1 locus mapped in three BC1 populations derived from the CMS line Sperber and the Rf1 donor lines (a) R3 (N = 197), (b) R113 (N = 201) and (c) L19 (N = 230).

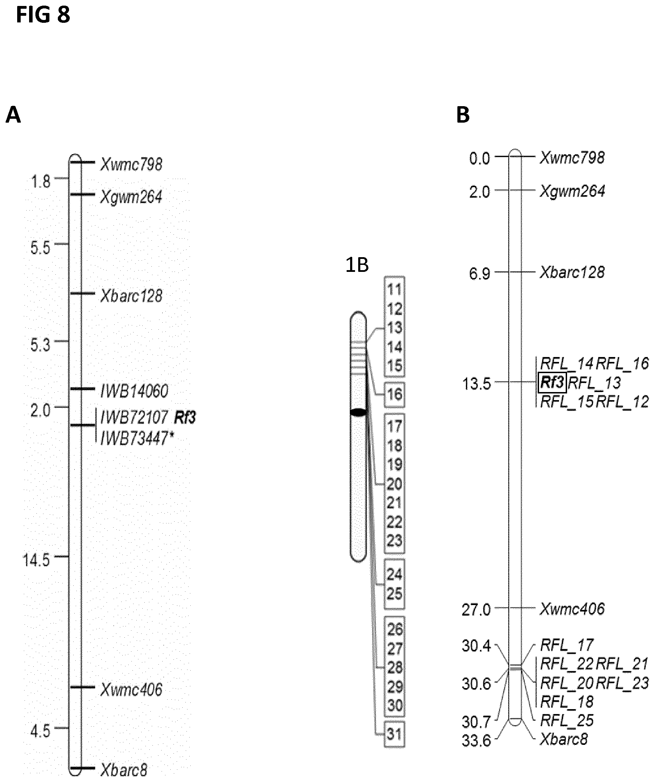

**Fig. 8** represents the mapping of Rf3 based on novel RFL-PPR capture derived SNP markers. A cluster of RFL genes allocates genetically with the Rf3 locus. Left panel: schematic representation of Rf gene loci distribution on wheat chromosome IBS. Right panel: genetic map of chromosome IBS at the Rf3 locus showing 12 novel RFL-PPR capture derived SNP markers, the cluster of RFL_12-16 was cosegregating with Rf3 at the given genetic resolution.

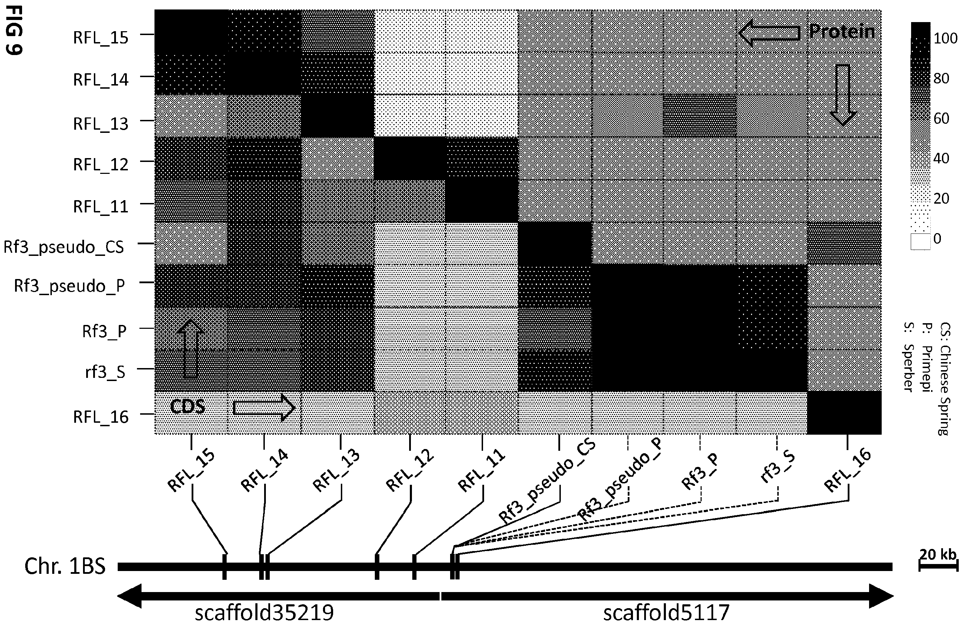

**Fig. 9** shows the heatmap of percent sequence identity matrix for the Rf3 locus. RFL genes at the Rf3 locus are compared for DNA and protein sequence identity. RFL11-16 are RFL genes identified on the basis of the CS reference sequence and were among the 79 RFL loci addressed in the RFL-PPR capture design. After sequence capture additional gene/pseudogene copies were identified that are involved in presence absence (PA) and copy-number variation polymorphisms between Restorer and non-Restorer genotypes carrying the Rf3 locus.

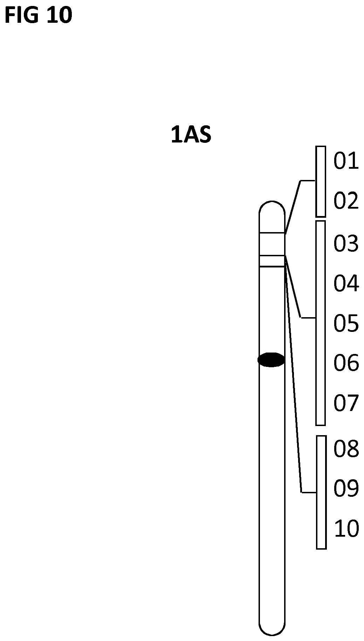

**Fig. 10** shows RFL mapping on wheat chromosome 1AS. From the 193 SNPs located in RFLs on chromosome 1AS, 48 marker assays were developed for genotyping. The RFL genes were mapped in three BC1 populations derived from the CMS line Sperber and the Rf1 donor lines R3 (N = 197), R113 (N = 201) and L19 (N = 230). QTL analyses revealed that RFL_01 and RFL_02 were located in the 1.5-LOD support interval for Rf1 in all three populations and are therefore candidates for Rf1. All other mapped RFL genes can be ruled out as candidates for Rf1.

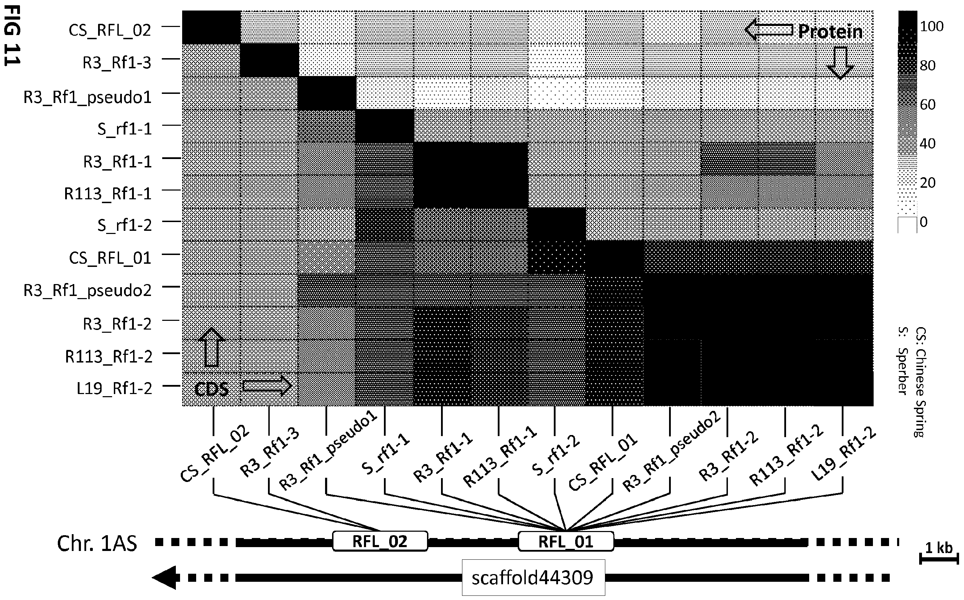

**Fig. 11** shows the heatmap of percent sequence identity matrix for the Rf1 locus. RFL genes at the Rf1 locus are compared for DNA and protein sequence identity. RFL_01 and RFL_02 are present on CS scaffold 44309. Whereas RFL_02 could not be detected in Rf1 containing genotypes, a series of RFL_01 paralogs was detected. Based on these results the best candidate for Rf1 is the Rf1-2 paralog (741 aa).

### Definitions

The present invention may be understood more readily by reference to the following detailed description of the preferred embodiments of the invention and the examples included herein. Unless otherwise noted, the terms used herein are to be understood according to conventional usage by those of ordinary skill in the relevant art.

It is to be understood that as used in the specification and in the claims, "a" or "an" can mean one or more, depending upon the context in which it is used. Therefore, reference to "a nucleic acid" can mean that at least one nucleic acid can be utilized.

It is to be understood that the term "comprising" is not limiting. Herein, the term "consisting of' is considered to be a preferred embodiment of the term "comprising". If hereinafter a group is defined to comprise at least a certain number of embodiments, this is meant to also encompass a group which preferably consists of these embodiments only.

Furthermore, the terms "first", "second", "third" or "(i)", "(ii)", "(iii)" etc. in the are used herein for distinguishing between similar elements and not necessarily for describing a sequential or chronological order. Such terms are interchangeable under appropriate circumstances and the embodiments of the invention described herein are capable of operation in other orders than the ones described herein.

The term "wheat" as used herein refers to any species of wheat, e.g., hexaploid species including but not limited to *Triticum (T.) aestivum,* or *T. spelta,* tetraploid species including but not limited to *T. durum, T. dicoccon, T. timopheevii* or *T. turanicum,* and diploid species including but not limited *to T. monococcum.*

The term "male sterility" in connection with the present invention refers to the failure or partial failure of plants to produce functional pollen or male gametes. This can be due to natural or artificially introduced genetic predispositions or to human intervention on the plant in the field. Male fertility on the other hand relates to plants capable of producing normal functional pollen and male gametes.

The term "male parent" or "pollen parent" refers to a parent plant that provides the male gametes (pollen) for fertilization, while the term "female parent" or "seed parent" refers to a parent plant that provides the female gametes for fertilization, said female plant being the one bearing the seeds.

The term "cytoplasmic male sterility" or "CMS" refers to cytoplasmic-based and maternally-inherited male sterility. CMS is total or partial male sterility in plants as the result of specific nuclear and mitochondrial interactions and is maternally inherited via the cytoplasm. Male sterility is the failure of plants to produce functional anthers, pollen, or male gametes although CMS plants still produce viable female gametes. Cytoplasmic male sterility is used in agriculture to facilitate the production of hybrid seed.

The term "wheat G-type cytoplasmic male sterility" as used herein refers to the cytoplasm of *Triticum timopheevii* that can confer male sterility when introduced into common wheat (i.e. *Triticum aestivum*)*,* thereby resulting in a plant carrying common wheat nuclear genes but cytoplasm from *Triticum timopheevii* that is male sterile. The cytoplasm of *Triticum timopheevii* (G-type) as inducer of male sterility in common wheat has been extensively studied (Kaul, Male sterility in higher plants, Springer Verlag, 1988;Mukai and Tsunewaki, Theor. Appl. Genet. 54, 1979; Tsunewaki, Jpn. Soc. Prom. Sci., 1980; Tsunewaki et al., Genes Genet. Syst. 71, 1996).

The term "a functional restorer gene", "a functional restorer gene of cytoplasmic male sterility", "a functional restorer gene for wheat cytoplasmic male sterility" or "a functional restorer gene allele for wheat G-type cytoplasmic male sterility" refers to an allele that has the capacity to restore fertility in the progeny of a cross of the line comprising the functional restorer gene with a CMS-line, i.e., a line carrying common wheat nuclear genes but cytoplasm from *Triticum timopheevii.* Such restorer genes or alleles are also referred to as Rf genes and Rf alleles.

The term "maintainer" refers to a plant that when crossed with the CMS plant does not restore fertility and maintains sterility in the progeny. The maintainer is used to propagate the CMS line, and may also be referred to as a non- restorer line. Maintainer lines have the same nuclear genes as the sterile one (i.e. do not contain functional Rf genes), but differ in the composition of cytoplasmic factors that cause male sterility in plants i.e. maintainers have "fertile" cytoplasm. Therefore, when a male sterile line is crossed with its maintainer progeny with the same male sterile genotype will be obtained.

The term "molecular marker" or "marker" or "marker allele" as used herein refers to a polymorphic locus, i.e. a polymorphic nucleotide (a so-called single nucleotide polymorphism or SNP) or a polymorphic DNA sequence at a specific locus. A marker refers to a measurable, genetic characteristic with a fixed position in the genome, which is normally inherited in a Mendelian fashion, and which can be used for mapping of a trait of interest or to identify certain individuals with a certain trait of interest. A marker thus refers to a nucleotide sequence that can be used to identify plants having a particular allele, e.g., the presently described Rf alleles on chromosome 1A and 1B, respectively. A marker may be described as a variation at a given genomic locus.

The term "single nucleotide polymorphism" or "SNP" may refer to a DNA sequence variation occurring when a single nucleotide in the genome (or other shared sequence) differs between members of a species or paired chromosomes in an individual. Single nucleotide polymorphisms may fall within coding sequences of genes, non-coding regions of genes, or in the intergenic regions between genes.

SNPs within a coding sequence will not necessarily change the amino acid sequence of the protein that is produced, due to degeneracy of the genetic code. A SNP in which both forms lead to the same polypeptide sequence is termed "synonymous" (sometimes referred to a silent mutation). If a different polypeptide sequence is produced, they are termed "non-synonymous". A non-synonymous change may either be missense or nonsense, where a missense change results in a different amino acid and a nonsense change results in a premature stop codon. SNPs that are not in protein-coding regions may still have consequences for e.g. gene splicing, transcription factor binding, or the sequence of non-coding RNA (e.g. affecting transcript stability, translation). SNPs are usually biallelic and thus easily assayed in plants and animals.

A particularly useful assay for detection of SNP markers is for example a KASP assay. KASP genotyping assays are based on competitive allele-specific PCR and enable biallelic scoring of single nucleotide polymorphisms (SNPs) and insertions and deletions (Indels) at specific loci. For developing the KASP-assay 70-100 base pairs upstream and 70-100 base pairs downstream of the SNP are selected and two allele-specific forward primers and one allele specific reverse primer is designed. See e.g. Allen et al. 2011, Plant Biotechnology J. 9, 1086-1099, especially p1097-1098 for KASP assay method.

The terms "nucleic acid" or "nucleic acid molecule" or nucleic acid sequence" or "nucleotide sequence" are used interchangeable.

The terms "chimeric gene" and "recombinant gene" are used interchangeable in the context of the present invention.

The term "biallelic" means that a polymorphism has two forms. Typically, the first identified allele is designated as the original allele whereas other alleles are designated as alternative alleles.

The terms "linked to" or "linkage", as used herein, refers to a measurable probability that genes or markers located on a given chromosome are being passed on together to individuals in the next generation. Thus, the term "linked" may refer to one or more genes or markers that are passed together with a gene with a probability greater than 0.5 (which is expected from independent assortment where markers/genes are located on different chromosomes). Because the distance between two genes or markers on a chromosome is directly related to the probability that the genes or markers will be passed together to individuals in the next generation, the term "linked" may also refer herein to one or more genes or markers that are located within about 50 centimorgan (cM) or less of one another on the same chromosome. Genetic linkage is usually expressed in terms of cM. Centimorgan is a unit of recombinant frequency for measuring genetic linkage, defined as that distance between genes or markers for which one product of meiosis in 100 is recombinant, or in other words, the centimorgan is equal to a 1% chance that a marker at one genetic locus on a chromosome will be separated from a marker at a second locus due to crossing over in a single generation. It is often used to infer distance along a chromosome. The number of base pairs to which cM correspond varies widely across the genome (different regions of a chromosome have different propensities towards crossover) and the species (i.e. the total size of the genome).

The term "interval" refers to a continuous linear span of chromosomal DNA with termini defined by map position and/or markers. Accordingly, a flanking marker as used herein, is a marker that defines one of the termini of an interval (and is included in that interval). It will be clear that any of such intervals may comprise further markers.

The term "contig", as used herein refers to set of overlapping DNA segments that together represent a consensus region of DNA. In bottom-up sequencing projects, a contig refers to overlapping sequence data (reads); in top-down sequencing projects, contig refers to the overlapping clones that form a physical map of the genome that is used to guide sequencing and assembly. Contigs can thus refer both to overlapping DNA sequence and to overlapping physical segments (fragments) contained in clones depending on the context.

The term "scaffold" as, used herein, refers to overlapping DNA contigs that together represent a consensus region of DNA.

The term "locus", as used herein refers to a certain place or position on the genome, e.g. on a chromosome or chromosome arm, where for example a gene or genetic marker is found.

The term "allele(s)", such as of a gene, means any of one or more alternative forms of a gene at a particular locus. In a diploid cell of an organism, alleles of a given gene are located at a specific location or locus on a chromosome. One allele is present on each chromosome of the pair of homologous chromosomes or possibly on homologous chromosomes.

The term "homologous chromosomes" means chromosomes that contain information for the same biological features and contain the same genes at the same loci but possibly different alleles of those genes. Homologous chromosomes are chromosomes that pair during meiosis. "Non-homologous chromosomes", representing all the biological features of an organism, form a set, and the number of sets in a cell is called ploidy. Diploid organisms contain two sets of non-homologous chromosomes, wherein each homologous chromosome is inherited from a different parent. In tetraploid species, two sets of diploid genomes exist, whereby the chromosomes of the two genomes are referred to as "homoeologous chromosomes" (and similarly, the loci or genes of the two genomes are referred to as homologous loci or genes). Likewise, hexaploid species have three sets of diploid genomes, etc. A diploid, tetraploid or hexaploid plant species may comprise a large number of different alleles at a particular locus. The ploidy levels of domesticated wheat species range from diploid (*Triticum monococcum,* 2n = 14, AA), tetraploid (*T. turgidum,* 2n = 28, AABB) to hexaploid (*T. aestivum*, 2n = 42, AABBDD).

The term "heterozygous" means a genetic condition existing when two different alleles reside at a specific locus, but are positioned individually on corresponding pairs of homologous chromosomes in the cell. Conversely, as used herein, the term "homozygous" means a genetic condition existing when two identical alleles reside at a specific locus, but are positioned individually on corresponding pairs of homologous chromosomes in the cell.

The term "paralog" as used herein refers to genes that are homologs that stem from a duplication event. Hence, paralogs originate from a duplication event in a common ancestor and subsequent mutation. Descendants of the common ancestor may therefore exhibit paralogues (mutated versions of the original gene that was duplicated). For example, in the common ancestor, one gene (gene) may get duplicated to make a separate similar gene (gene'). Gene and gene' may be passed to subsequent generations. At some point, one environment will favor a mutation in the original gene (gene*), producing a new species with gene' and gene*. Another environment will favor a mutation the duplicated gene' (gene") giving rise to a new species with gene and gene". The descendant's gene* and gene" are paralogs.

The term "PPR proteins" as used herein defines proteins which are classified based on their domain architecture. P-class PPR proteins possess the canonical 35 amino acid motif and normally lack additional domains. Members of this class have functions in most aspects of organelle gene expression. PLS-class PPR proteins have three different types of PPR motifs, which vary in length; P (35 amino acids), L (long, 35-36 amino acids) and S (short, ∼31 amino acids), and members of this class are thought to mainly function in RNA editing. Subtypes of the PLS class are categorized based on the additional C-terminal domains they possess (reviewed by Manna et al., 2015, Biochemie 113, p93-99).

The term "cytoplasm" defines the material within a cell, enclosed by the cell membrane, except for the cell nucleus. The main components of the cytoplasm are cytosol, the organelles, and various cytoplasmic inclusions.

The term "introducing" in the meaning of the present invention includes stable integration by means of transformation including Agrobacterium-mediated transformation, transfection, microinjection, biolistic bombardment, insertion using gene editing technology like CRISPR systems (e.g. CRISPR/Cas, in particular CRISPR/Cas9 or CRISPR/Cpf1, CRISPR/CasX, CRISPR/CasY, CRISPR/Csm1 or CRISPR/MAD7), TALENs, zinc finger nucleases or meganucleases, homologous recombination optionally by means of one of the mentioned gene editing technologies including preferably a repair template, modification of endogenous gene using random or targeted mutagenesis like TILLING or below mentioned gene editing technologies, etc. The term "introducing" may or may not encompass the introgression using conventional or non-conventional breeding techniques.

The term "mutation" as used herein refers to any modification of genomic information including deletion, introduction and exchange of any number of nucleotides.

The term "TILLING" as used herein is an abbreviation for "Targeting Induced Local Lesions in Genomes" and describes a well-known reverse genetics technique designed to detect unknown SNPs (single nucleotide polymorphisms) in genes of interest using an enzymatic digestion and is widely employed in plant genomics. The technique allows for the high-throughput identification of an allelic series of mutants with a range of modified functions for a particular gene. TILLING combines mutagenesis (e.g., chemical or via UV-light) with a sensitive DNA screening-technique that identifies single base mutations.

The terms "transgene" or "transgenic" as used herein refer to at least one nucleic acid sequence that is taken from the genome of one organism, or produced synthetically, and which is then introduced into a host cell or organism or tissue of interest and which is subsequently integrated into the host' s genome by means of "stable" transformation or transfection approaches.

The term "genome editing" as used herein refers to strategies and techniques for the targeted, specific modification of any genetic information or genome of a plant cell by means of or involving a double-stranded DNA break inducing enzyme or single-stranded DNA or RNA break inducing enzyme. As such, the terms comprise gene editing, but also the editing of regions other than gene encoding regions of a genome, such as intronic sequences, non-coding RNAs, miRNAs, sequences of regulatory elements like promoter, terminator, transcription activator binding sites, cis or trans acting elements. Additionally, the terms may comprise base editing for targeted replacement of single nucleobases. It can further comprise the editing of the nuclear genome as well as other genetic information of a plant cell, i.e. mitochondrial genome or chloroplast genome as well as miRNA, pre-mRNA or mRNA.

The term "transformation" as used herein refers to the genetic modification of a cell by incorporation of genetic material from the outside. Plant transformation may make use of vectors (e.g. Agrobacterium or virus).

As used herein, a "double-stranded DNA break inducing enzyme" or "DSBI enzyme" is an enzyme capable of inducing a double-stranded DNA break at a particular nucleotide sequence, called the "recognition site" or "predetermined site". Accordingly, a "single-stranded DNA or RNA break inducing enzyme" or "SSBI enzyme" is an enzyme capable of inducing a single-stranded DNA or RNA break at a particular nucleotide sequence, called the "recognition site" or "predetermined site".

In order to enable a break at a predetermined target site, the enzymes preferably include a binding/recognition domain and a cleavage domain. Particular enzymes capable of inducing double or single-stranded breaks are nucleases or nickases as well as variants thereof, including such molecules no longer comprising a nuclease or nickase function but rather operating as recognition molecules in combination with another enzyme. In recent years, many suitable nucleases, especially tailored endonucleases have been developed comprising meganucleases, zinc finger nucleases, TALE nucleases, Argonaute nucleases, derived, for example, from *Natronobacterium gregoryi,* and CRISPR nucleases, comprising, for example, Cas9, Cpf1, CasX, CasY, MAD7 or Csm1 nucleases as part of the Clustered Regularly Interspaced Short Palindromic Repeats (CRISPR) system. Thus, in a preferred aspect of the invention, the genome engineering component comprises a DSB or SSB inducing enzyme or a variant thereof selected from a CRISPR/Cas endonuclease, preferably a CRISPR/Cas9 endonuclease or a CRISPR/Cpf1 endonuclease, a zinc finger nuclease (ZFN), a homing endonuclease, a meganuclease and a TAL effector nuclease.

### Detailed description

The present invention provides several restorer genes. The genes Rf3 and Rf1 are provided as effective genes for achieving restoration. Previous studies located Rf3 on chromosome 1B of spelt wheat and later Primepi. Rf1 has been located on chromosome 1A in common wheat (with introgressions from T. timopheevii). However, the exact position and sequence of Rf3 and Rf1 in common and spelt wheat remained unclear. Further, there is the need for accurate markers to identify and track Rf loci. Such markers are particularly useful for plant breeding, hybrid seed production, and for methods for fertility restoration in wheat *T. timopheevii* cytoplasm.

The present invention provides precise loci of these genes in the chromosome of restorer lines and reveals genetic markers for the identification and detection of these restoration genes and its zygosity status.

Most restorer-of-fertility (Rf) genes come from a small clade of genes encoding pentatricopeptide repeat (PPR) proteins (Fuji et al., 2011, PNAS 108(4), 1723-1728). PPR genes functioning as Rf genes are referred to in Fuji (supra) as Rf-PPR genes. Rf-PPR genes are usually present in clusters of similar Rf-PPR-like (RFL-PPR) genes, which show a number of characteristic features compared with other PPR genes. They are comprised primarily of tandem arrays of 15-20 PPR motifs, each composed of 35 amino acids.

Most RFL-PPR genes belong to the P-class PPR subfamily, although also PLS-class PPR Rf genes have been identified and are characterized by the presence of tandem arrays of 15 to 20 PPR motifs each composed of 35 amino acid residues. High substitution rates observed for particular amino acids within otherwise very conserved PPR motifs, indicating diversifying selection, prompted the conclusion that these residues might be directly involved in binding to RNA targets. This has led to the development of a "PPR code" which allows the prediction of RNA targets of naturally occurring PPR proteins as well as the design of synthetic PPR proteins that can bind RNA molecules of interest, whereby sequence specificity is ensured by distinct patterns of hydrogen bonding between each RNA base and the amino acid side chains at positions 5 and 35 in the aligned PPR motif (Melonek et al., 2016, Nat Sci Report 6:35152, Barkan et al., 2012, PLoS Genet 8(8): e1002910).

Accordingly, a functional allele of a PPR gene, as used herein, refers to an allele of a PPR gene that is a functional restorer gene or gene allele for cytoplasmic male sterility, for instances for cereal cytoplasmic male sterility or wheat cytoplasmic male sterility, preferably G-type cytoplasmic male sterility, as described herein, i.e. that when expressed in a (sexually compatible) plant has the capacity to restore fertility in the progeny of a cross with a cytoplasmic male sterile plant. Such a functional allele of a PPR gene is also referred to as an RFL-PPR gene (or Rf gene), which in turn encodes an RFL-PPR (or Rf) protein.

The present invention is based on studies of resequencing of the PPR gene family of *Triticum aestivum* by sequence-capture for a targeted development of molecular markers for the genetic mapping of Rf-genes (fertility restoration of cytoplasmic male sterility).

Using this resequencing technique, the inventors identified nucleic acid sequences comprising functional restorer gene alleles and flanking markers to these functional restorer gene alleles, respectively.

In their studies, the inventors compared PPR gene sequences derived from different *Triticum aestivum* cultivars. Chinese Spring (CS) is known to have no restoration capacity in G-type CMS. Therefore, throughout the studies, CS serves as a reference genome for RFL-PPR genes. Primepi and Meister are wheat G-type CMS restorer lines comprising restorer genes. Sperber is not containing Rf-activity (non-restorer line) and serves as female parent line for Primepi and Meister in hybrid breeding. Based on elaborate sequencing efforts and novel sequence analysing technology, functional restorer PPR genes have been identified.

For the mapping of the Rf3 gene, sequence analysis was performed for the CS *Rf3*-linked RFL genes RFL12, RFL13, RFL14, RFL15 and RFL16. RFL16 (SEQ ID NO: 10) was identified to be located on CS Refseq scaffold 5117. The same scaffold was identified to further carry a pseudogene (Rf3_pseudo_CS). RFL11-15 are all located on CS Refseq scaffold 35219. Both scaffolds are direct neighbours sitting tail-to-tail on chromosome IBS of the CS RefSeqv1.0. Therefore, RFL11-16, including an additional pseudogene, form one larger RFL-PPR gene cluster that is linked to the Rf3 locus.

The inventors compared this locus in the genotypes of Primepi (restorer), Sperber (non-restorer) and CS (reference non-restorer) and identified that Primepi carries two homologs of Rf3_pseudo_CS, which are Rf3_pseudo_P (SEQ ID NO: 6) and Rf3_P (SEQ ID NO: 2), respectively. Rf3_pseudo_P is a different allele compared to Rf3_pseudo_CS, which carries a 23bp deletion leading to a frameshift with no restoring function. Rf3_P, however, has been found to be a functional restorer gene encoding an RFL-PPR protein of 792 amino acids. This functional 17-P-motif RFL gene is missing in the reference CS line, but is present in the non-restorer line Sperber where it carries, however, a single base pair deletion corresponding to position 669 of SEQ ID NO:2 leading to a frameshift and thus to a non-functional restorer gene.

The identification of the functional restorer gene Rf3 is described in more detail in the Examples.

Further, a functional Rf1 restorer gene has been identified in the present invention. The identification of the Rf1 gene is based on sequencing of the loci for RFL_01 and RFL_02 located on CS Refseq scaffold44309. For these resequencing studies three Rf1 restorer genotypes (R3, R113 and L19) have been tested together with non-restorer line Sperber. It was found, that the RFL_02 gene is not present in the three Rf1 restorer genotypes and is also missing in Sperber. Therefore, the study focused on RFL_01. The RFL_01 locus showed different levels of complexity including putatively functional and non-functional paralogs in restorer and non-restorer genotypes (see also **Fig. 11**). For example, restorer line R3 carries five RFL_01 paralogs (RF1-1, Rf1-2, Rf1-3, Rf1_pseudo1/rf1-1 and Rf1_pseudo2/rf1-2).

RFL_01 alleles and paralogs are present in all three Rf1 genotypes but only one paralog is shared by all of the three restorer lines (R3, R113 and L19) and Chinese Spring (CS), which is Rf1-2 in the restorer lines and RFL_01 in CS. However, sequence homology between the CS RFL_01 allele is slightly lower to the respective alleles from Rf1-2 containing genotypes, which all carry the identical Rfl-2 allele sequence (**Fig. 11**). In case of S_rf1-2 (SEQ ID NO: 35), there is a C to A conversion at position 1471 referenced to the CS sequence (SEQ ID NO: 23) resulting in the creation of a stop codon TGA from nucleotide 1474 to 1476 of SEQ ID NO: 35. Sperber contains two pseudogene paralogs of RFL_01 (S_rf1-1 and S_rf1-2) which carry mutations leading to a pre-stop in translation.

According to the above, the inventors identified the candidate gene for Rf1 to be the allele Rfl-2 (encoding the protein having 741 aa).

For large scale crop growing, e.g. for food production, hybrid plants are commonly used as they often exhibit higher yields compared to inbred lines. Fertility-controlling systems such as CMS are a promising for an efficient hybrid seed production. For this reason, using CMS hybrid plants is highly advantageous. CMS hybrids are for example generated by crossing (i) a CMS line (mother, T. *timopheevi* cytoplasm, no restoring alleles in nuclear genome, sterility prevents self-pollination in hybrid seed production), with (ii) a restorer line (father, usually normal cytoplasm, restoring alleles in nuclear genome). The product is a CMS hybrid which shares the cytoplasm of the mother (maternally inherited) and a nuclear restorer allele of the father. The restoring alleles ensure fertility (grain yield) despite the sterile cytoplasm. The CMS hybrid is the final product on the farmers field.

Moreover, also in order to further develop CMS lines (e.g. introduction of new features by breeding), the fertility of these CMS lines needs to be restored. Fertility restoration can again occur by crossing CMS lines with restorer plants.

Yet, since the location and sequence of the genes responsible for restoration of fertility was not known, successful restoration could only be tested by growing plants and analyzing their fertility. Such tests are therefore time consuming (at least one breeding cycle).

The present invention provides restorer genes as well as markers for detecting their presence. Therefore, the successful restoration of fertility can now be detected directly in the seed, e.g, after harvest. Hence, the provided means considerably simplify the detection of successful fertility restoration.

Further, the restorer genes can be used for converting non-restorer into restorer plants by introducing said restorer gene(s) into plants by genetic modification. Such plants can be used for restoring fertility in CMS hybrids. Hence, the provided means simplify the generation of new plants.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

The invention is also described by the following items:
[01] A nucleic acid comprising a functional restorer gene allele localizing to a position corresponding to or referred to
   (i) 14584864 to 14587134 of chromosome 1A of Chinese Spring wheat; or
   (ii) 19061029 to 19063412 of chromosome 1B of Chinese Spring wheat.
[02] A nucleic acid comprising a functional restorer gene allele localizing to a position
   (i) on chromosome 1A of a wheat plant flanked by markers according to SEQ ID NO: 109 and SEQ ID NO: 218, wherein preferably the SNP in SEQ ID NO: 109 is a G and/or the SNP in SEQ ID NO: 218 is a G; or
   (ii) on chromosome 1B of a wheat plant flanked by markers according to SEQ ID NO: 47 and SEQ ID NO: 106, wherein preferably the SNP in SEQ ID NO: 47 is an A and/or the SNP in SEQ ID NO: 106 is an A.
[03] A nucleic acid comprising a nucleic acid sequence of a functional restorer gene allele
   (i) having the coding sequence according to SEQ ID NO: 2;
   (ii) encoding a protein having an amino acid sequence according to SEQ ID NO: 3;
   (iii) having a coding sequence with at least 80%, at least 85%, preferably at least 90%, more preferably at least 95%, at least 96%, at least 97%, at least 98% and most preferred at least 99% sequence identity with SEQ ID NO: 2, preferably over the entire length of SEQ ID NO: 2;
   (iv) encoding a protein having an amino acid sequence having at least 80%, at least 85%, preferably at least 90%, more preferably at least 95%, at least 96%, at least 97%, at least 98% and most preferred at least 99% sequence identity with SEQ ID NO: 3, preferably over the entire length of SEQ ID NO: 3;
   (v) having the coding sequence according to SEQ ID NO: 13;
   (vi) encoding a protein having an amino acid sequence according to SEQ ID NO: 14;
   (vii) having a coding sequence with at least 80%, at least 85%, preferably at least 90%, more preferably at least 95%, at least 96%, at least 97%, at least 98% and most preferred at least 99% sequence identity with SEQ ID NO: 13, preferably over the entire length of SEQ ID NO: 13;
   (viii) encoding a protein having an amino acid sequence having at least 80%, at least 85%, preferably at least 90%, more preferably at least 95%, at least 96%, at least 97%, at least 98% and most preferred at least 99% sequence identity with SEQ ID NO: 14, preferably over the entire length of SEQ ID NO: 14.
[04] The nucleic acid sequence according to item [03], wherein the nucleic acid sequence comprises a functional restorer gene allele which preferably encodes a pentatricopeptide repeat (PPR) protein with at least 10 P-motifs, at least 15 P-motifs, at least 16 P-motifs or at least 17 P-motifs, more preferably a pentatricopeptide repeat (PPR) protein with 16 P-motifs or 17 P-motifs.
[05] The nucleic acid of any one of items [01]-[04], wherein the functional restorer gene allele is for restoring cytoplasmic male sterility, in particular G-type wheat cytoplasmic male sterility.
[06] A vector or expression cassette comprising the nucleic acid of any one of items [01]-[05] or any one items [08]-[11].
[07] A polypeptide encoded by the nucleic acid of any one of items [01]-[05].
[08] A chimeric gene comprising the following operably linked elements
   (i) a promoter functional in plants, and
   (ii) the nucleic acid according to any one of items [01]-[05],
   preferably wherein at least one of said operably linked elements is heterologous with respect to at least one other element, more preferably wherein the promoter is heterologous with respect to the nucleic acid according to any one of items [01]-[05].
[09] The chimeric gene of item [08], further comprising a transcription termination and polyadenylation region functional in plant cells.
[10]. The chimeric gene of item [08] or [09], wherein the promoter is capable of directing expression of the operably linked nucleic acid at least during early pollen development and/or meiosis.
[11] The chimeric gene of any one of items [08]-[10], wherein the promoter is capable of directing expression of the operably linked nucleic acid in anther, tapetum, and/or developing microspores.
[12] A plant cell, preferably a cereal plant cell or a wheat plant cell, comprising one or more of the nucleic acids according to any one of items [01]-[05] as exogenous or endogenous sequence, one or more of the vectors or expression cassette of item [06] or one or more of the chimeric genes of any one of items [08]-[11], preferably wherein said nucleic acid(s), said expression cassette or said chimeric gene(s) is/are heterologous with respect to said plant cell or to the genomic location within said plant cell.
[13] A plant, preferably a cereal plant or a wheat plant, or a part thereof comprising a plant cell of item [12], one or more of the nucleic acids according to any one of items [01]-[05] as exogenous or endogenous sequence, one or more of the vectors or expression cassette of item [06] or one or more of the chimeric genes of any one of items [08]-[11], preferably wherein said nucleic acid(s), said expression cassette or said chimeric gene(s) is/are heterologous with respect to said plant or to the genomic location within said plant cell.
[14] A seed of a plant, preferably of a cereal plant or of a wheat plant, comprising one or more of the nucleic acids according to any one of items [01]-[05] as exogenous or endogenous sequence, one or more of the vectors or expression cassette of item [06] or one or more of the chimeric genes of any one of items [08]-[11], wherein said nucleic acid(s), said expression cassette or said chimeric gene(s) is/are heterologous with respect to said seed or to the genomic location within said plant cell.
[15] The plant cell of item [12], the plant or part thereof of item [13] or the seed of item [14], wherein the plant cell, plant or seed is a wheat plant cell, a wheat plant or a wheat seed.
[16] A method for producing a plant cell, preferably a cereal plant cell or a wheat plant cell, or a plant, preferably a cereal plant or a wheat plant, comprising the functional restorer gene allele for restoring cytoplasmic male sterility, wherein the method comprises
   a) introducing the nucleic acid of any one of items [01]-[05], the vector or expression cassette of item [06] or the chimeric gene of any one of items [08]-[11] into the plant cell or the plant by transformation, genome editing, mutagenesis, or crossing and optionally subsequent selection, and
   b) optionally regenerating a plant from the plant cell of a).
[17] A method for increasing restoration capacity of a plant, preferably a cereal plant or a wheat plant, wherein the method comprises
   a) introducing the nucleic acid of any one of items [01]-[05], the vector or expression cassette of item [06] or the chimeric gene of any one of items [08]-[11] into a plant cell or the plant by transformation, genome editing, mutagenesis, or crossing and optionally subsequent selection, and
   b) optionally regenerating a plant from the plant cell of a) having increased restoration capacity.
[18] A method for converting a non-restoring plant, preferably a non-restoring cereal plant or a non-restoring wheat plant, into a restoring plant, preferably a restoring cereal plant or a restoring wheat plant, comprising the functional restorer gene allele for restoring cytoplasmic male sterility, wherein the method comprises
   a) introducing the nucleic acid of any one of items [01]-[05], the vector or expression cassette of item [06] or the chimeric gene of any one of items [08]-[11] into a plant cell of a non-restoring plant or the non-restoring plant by transformation, genome editing, mutagenesis, or crossing and optionally subsequent selection, and
   b) optionally regenerating a restoring plant from the plant cell of a).
[19] The method of any one of items [16]-[18] further comprising the step of allowing expression of the polypeptide encoded by the introduced nucleic acid, vector, expression cassette or chimeric gene.
[20] A plant or plant cell obtained by the method of any one of items [16]-[19].
[21] A method for identifying or selecting a plant cell, a plant or a part thereof or a seed comprising a functional restorer gene allele for restoring cytoplasmic male sterility comprising the step of identifying or detecting in said plant cell, in said plant or in said seed the presence or absence of the nucleic acid of any one of items [01]-[05], the polypeptide of item [07] or the chimeric gene of any one of items [08]-[11].
[22] A method for identifying or selecting a wheat plant cell, wheat plant or part thereof or wheat seed comprising the functional restorer gene allele for wheat G-type cytoplasmic male sterility located on chromosome 1A and/or a functional restorer gene allele for wheat G-type cytoplasmic male sterility located on chromosome 1B, comprising the steps of:
   (i) detecting a wheat plant cell, wheat plant or part thereof or wheat seed comprising at least one marker allele linked to the functional restorer gene allele for wheat G-type cytoplasmic male sterility located on chromosome 1A (1A restorer marker allele) and/or at least one marker allele linked to the functional restorer gene allele for wheat G-type cytoplasmic male sterility located on chromosome 1B (1B restorer marker allele); and
   (ii) identifying and optionally selecting the wheat plant cell, wheat plant or part thereof or wheat seed comprising the at least one 1A restorer marker allele and/or the at least one 1B restorer marker allele based on the detection in step (i).
[23] A method for determining the presence, absence or zygosity status of the functional restorer gene allele for wheat G-type cytoplasmic male sterility located on chromosome 1A and/or on chromosome 1B in a sample derived from a wheat plant, comprising the steps of
   (i) providing genomic DNA from said sample, and
   (ii) analyzing said DNA and determining for the presence, absence or zygosity status of at least one marker allele linked to the functional restorer gene allele for wheat G-type cytoplasmic male sterility located on chromosome 1A (1A restorer marker allele) and/or the presence of at least one marker allele linked to the functional restorer gene allele for wheat G-type cytoplasmic male sterility located on chromosome 1B (1B restorer marker allele).
[24] The method of item [22] or item [23], wherein the at least one 1A restorer marker allele localizes within an interval on chromosome 1A comprising and flanked by the markers of SEQ ID NO: 109 and SEQ ID NO: 152 and/or the at least one 1B restorer marker allele localizes within an interval on chromosome 1B comprising and flanked by the markers of SEQ ID NO: 105 and SEQ ID NO: 108.
[25] The method of item [22] or item [23], wherein the sequence of the at least one 1A restorer marker allele linked to said functional restorer gene allele is selected from the group consisting of SEQ ID NOs: 47- 108, wherein the at least one restorer marker allele is a SNP and the nucleotide at the SNP position in SEQ ID NOs: 47- 108 is
   a. a A at SEQ ID NO: 47
   b. a G at SEQ ID NO: 48
   c. a G at SEQ ID NO: 49
   d. a C at SEQ ID NO: 50
   e. a C at SEQ ID NO: 51
   f. a T at SEQ ID NO: 52
   g. a T at SEQ ID NO: 53
   h. a A at SEQ ID NO: 54
   i. a A at SEQ ID NO: 55
   j. a C at SEQ ID NO: 56
   k. a G at SEQ ID NO: 57
   l. a G at SEQ ID NO: 58
   m. a G at SEQ ID NO: 59
   n. a A at SEQ ID NO: 60
   o. a C at SEQ ID NO: 61
   p. a A at SEQ ID NO: 62
   q. a C at SEQ ID NO: 63
   r. a C at SEQ ID NO: 64
   s. a A at SEQ ID NO: 65
   t. a C at SEQ ID NO: 66
   u. a G at SEQ ID NO: 67
   v. a A at SEQ ID NO: 68
   w. a C at SEQ ID NO: 69
   x. a C at SEQ ID NO: 70
   y. a Cat SEQ ID NO: 71
   z. a G at SEQ ID NO: 72
   aa. a T at SEQ ID NO: 73
   bb. a C at SEQ ID NO: 74
   cc. a G at SEQ ID NO: 75
   dd. a G at SEQ ID NO: 76
   ee. a G at SEQ ID NO: 77
   ff. a G at SEQ ID NO: 78
   gg. a A at SEQ ID NO: 79
   hh. a T at SEQ ID NO: 80
   ii. a A at SEQ ID NO: 81
   jj. a C at SEQ ID NO: 82
   kk. a G at SEQ ID NO: 83
   ll. a C at SEQ ID NO: 84
   mm. a T at SEQ ID NO: 85
   nn. a A at SEQ ID NO: 86
   oo. a T at SEQ ID NO: 87
   pp. a G at SEQ ID NO: 88
   qq. a A at SEQ ID NO: 89
   rr. a G at SEQ ID NO: 90
   ss. a C at SEQ ID NO: 91
   tt. a G at SEQ ID NO: 92
   uu. a A at SEQ ID NO: 93
   vv. a C at SEQ ID NO: 94
   ww. a T at SEQ ID NO: 95
   xx. a T at SEQ ID NO: 96
   yy. a G at SEQ ID NO: 97
   zz. a A at SEQ ID NO: 98
   aaa. a T at SEQ ID NO: 99
   bbb. a C at SEQ ID NO: 100
   ccc. a G at SEQ ID NO: 101
   ddd. a A at SEQ ID NO: 102
   eee. a C at SEQ ID NO: 103
   fff. a T at SEQ ID NO: 104
   ggg. a T at SEQ ID NO: 105
   hhh. a A at SEQ ID NO: 106
   iii. a G at SEQ ID NO: 107, and
   jjj. a C at SEQ ID NO: 108, respectively;
   and/or
   wherein the sequence of the at least one 1B restorer marker allele linked to said functional restorer gene allele is selected from the group consisting of SEQ ID NOs: 109-152, wherein the at least one restorer marker allele is a SNP and the nucleotide at the SNP position in SEQ ID NOs: 109-152 is
   a. a G at SEQ ID NO: 109
   b. a T at SEQ ID NO: 110
   c. a C at SEQ ID NO: 111
   d. a G at SEQ ID NO: 112
   e. a T at SEQ ID NO: 113
   f. a Cat SEQ ID NO: 114
   g. a Cat SEQ ID NO: 115
   h. a Cat SEQ ID NO: 116
   i. a T at SEQ ID NO: 117
   j. a C at SEQ ID NO: 118
   k. a A at SEQ ID NO: 119
   l. a A at SEQ ID NO: 120
   m. a Cat SEQ ID NO: 121
   n. a T at SEQ ID NO: 122
   o. a G at SEQ ID NO: 123
   p. a G at SEQ ID NO: 124
   q. a A at SEQ ID NO: 125
   r. a G at SEQ ID NO: 126
   s. a T at SEQ ID NO: 127
   t. a T at SEQ ID NO: 128
   u. a T at SEQ ID NO: 129
   v. a G at SEQ ID NO: 130
   w. a Cat SEQ ID NO: 131
   x. a G at SEQ ID NO: 132
   y. a C at SEQ ID NO: 133
   z. a T at SEQ ID NO: 134
   aa. a C at SEQ ID NO: 135
   bb. a A at SEQ ID NO: 136
   cc. a A at SEQ ID NO: 137
   dd. a A at SEQ ID NO: 138
   ee. a G at SEQ ID NO: 139
   ff. a A at SEQ ID NO: 140
   gg. a T at SEQ ID NO: 141
   hh. a C at SEQ ID NO: 142
   ii. a A at SEQ ID NO: 143
   jj. a A at SEQ ID NO: 144
   kk. a T at SEQ ID NO: 145
   ll. a T at SEQ ID NO: 146
   mm. a C at SEQ ID NO: 147
   nn. a C at SEQ ID NO: 148
   oo. a T at SEQ ID NO: 149
   pp. a G at SEQ ID NO: 150
   qq. a C at SEQ ID NO: 151, and
   rr. a G at SEQ ID NO: 152, respectively;
   wherein the sequence of the at least one 1B restorer marker allele linked to said functional restorer gene allele is selected from the group consisting of SEQ ID NOs: 153189, wherein the at least one restorer marker allele is a SNP and the nucleotide at the SNP position in SEQ ID NOs: 153- 189 is
   a. a G at SEQ ID NO: 153
   b. a T at SEQ ID NO: 154
   c. a C at SEQ ID NO: 155
   d. aCat SEQID NO: 156
   e. a C at SEQ ID NO: 157
   f. a G at SEQ ID NO: 158
   g. a T at SEQ ID NO: 159
   h. a C at SEQ ID NO: 160
   i. a T at SEQ ID NO: 161
   j. a C at SEQ ID NO: 162
   k. a A at SEQ ID NO: 163
   l. a A at SEQ ID NO: 164
   m. a C at SEQ ID NO: 165
   n. a G at SEQ ID NO: 166
   o. a G at SEQ ID NO: 167
   p. a A at SEQ ID NO: 168
   q. a G at SEQ ID NO: 169
   r. a T at SEQ ID NO: 170
   s. a T at SEQ ID NO: 171
   t. a T at SEQ ID NO: 172
   u. a G at SEQ ID NO: 173
   v. a C at SEQ ID NO: 174
   w. a G at SEQ ID NO: 175
   x. a C at SEQ ID NO: 176
   y. a T at SEQ ID NO: 177
   z. a C at SEQ ID NO: 178
   aa. a A at SEQ ID NO: 179
   bb. a A at SEQ ID NO: 180
   cc. a G at SEQ ID NO: 181
   dd. a C at SEQ ID NO: 182
   ee. a C at SEQ ID NO: 183
   ff. a T at SEQ ID NO: 184
   gg. a G at SEQ ID NO: 185
   hh. a A at SEQ ID NO: 186
   ii. a Cat SEQ ID NO: 187
   jj. a C at SEQ ID NO: 188, and
   kk. a T at SEQ ID NO: 189, respectively;
   and/or wherein the sequence of the at least one 1B restorer marker allele linked to said functional restorer gene allele is selected from the group consisting of SEQ ID NOs: 190- 225, wherein the at least one restorer marker allele is a SNP and the nucleotide at the SNP position in SEQ ID NOs: 190- 225 is
   a. a G at SEQ ID NO: 190
   b. a T at SEQ ID NO: 191
   c. a C at SEQ ID NO: 192
   d. a G at SEQ ID NO: 193
   e. a T at SEQ ID NO: 194
   f. a C at SEQ ID NO: 195
   g. a C at SEQ ID NO: 196
   h. a T at SEQ ID NO: 197
   i. a Cat SEQ ID NO: 198
   j. a A at SEQ ID NO: 199
   k. a A at SEQ ID NO: 200
   l. a C at SEQ ID NO: 201
   m. a A at SEQ ID NO: 202
   n. a G at SEQ ID NO: 203
   o. a G at SEQ ID NO: 204
   p. a A at SEQ ID NO: 205
   q. a G at SEQ ID NO: 206
   r. a T at SEQ ID NO: 207
   s. a T at SEQ ID NO: 208
   t. a T at SEQ ID NO: 209
   u. a G at SEQ ID NO: 210
   v. a Cat SEQ ID NO: 211
   w. a G at SEQ ID NO: 212
   x. a C at SEQ ID NO: 213
   y. a T at SEQ ID NO: 214
   z. a Cat SEQ ID NO: 215
   aa. a A at SEQ ID NO: 216
   bb. a A at SEQ ID NO: 217
   cc. a G at SEQ ID NO: 218
   dd. a Cat SEQ ID NO: 219
   ee. a T at SEQ ID NO: 220
   ff. a G at SEQ ID NO: 221
   gg. a A at SEQ ID NO: 222
   hh. a C at SEQ ID NO: 223
   ii. a C at SEQ ID NO: 224, and
   jj. a T at SEQ ID NO: 225, respectively.
[26] The method of any one of items [22]-[25], wherein the sequence of the at least one 1A restorer marker allele is selected from the group consisting of SEQ ID NO: 109, SEQ ID NO: 111 and SEQ ID NO: 218, wherein the at least one restorer marker allele is a SNP and the nucleotide at the SNP position in SEQ ID NO: 109, SEQ ID NO: 111 and SEQ ID NO: 218 is
   a. a G at SEQ ID NOs: 109,
   b. a C at SEQ ID NOs: 111, and
   c. a G at SEQ ID NOs: 218, respectively;
   and/or
   wherein the sequence of the at least one 1B restorer marker allele is selected from the group consisting of SEQ ID NOs: 56, 58, 64, 66, 75, 84, 85, 89, 94, 99, 101, 105 and 107, wherein the at least one restorer marker allele is a SNP and the nucleotide at the SNP position in SEQ ID NOs: 56, 58, 64, 66, 75, 84, 85, 89, 94, 99, 101, 105 and 107 is
   a. a C at SEQ ID NOs: 56,
   b. a G at SEQ ID NOs: 58,
   c. a C at SEQ ID NOs: 64,
   d. a C at SEQ ID NOs: 66,
   e. a G at SEQ ID NOs: 75,
   f. a C at SEQ ID NOs: 84,
   g. a T at SEQ ID NOs: 85,
   h. a A at SEQ ID NOs: 89,
   i. a C at SEQ ID NOs: 94,
   j. a T at SEQ ID NOs: 99,
   k. a G at SEQ ID NOs: 101,
   l. a T at SEQ ID NOs: 105, and
   m. a G at SEQ ID NOs: 107, respectively.
[27] The method of item [22] or [23],
   (i) wherein the marker allele linked to the functional restorer gene allele for wheat G-type cytoplasmic male sterility located on chromosome 1A is a SNP selected from the SNPs shown in tables 4-6, and/or
   (ii) wherein the marker allele linked to the functional restorer gene allele for wheat G-type cytoplasmic male sterility located on chromosome 1B is a SNP selected from the SNPs shown in table 3,
   wherein the nucleotide position of the SNP is shown in column 2 of the respective table, and the nucleotide at this position is shown in column 5 of the respective table.
[28] An oligonucleotide comprising a nucleic acid sequence according to any one of SEQ ID NOs: 47-225, wherein the nucleotide position of the SNP is shown in column 2 of the respective tables 3-6, and the nucleotide at this position is shown in column 5 of the respective tables 3-6.
[29] A SNP selected from the group of SNPs shown in tables 3-6, wherein the nucleotide position of the SNP is shown in column 2 of the respective table, and the nucleotide at this position is shown in column 5 of the respective table.
[30] Use of the oligonucleotide according to item [28] or the SNP according to item [29] in a method for identifying one or more functional restorer gene alleles in a wheat plant.
[31] Use of the oligonucleotide according to item 28 or the SNP according to item [29] for identification of a wheat plant comprising a functional restorer gene allele for wheat G-type cytoplasmic male sterility.
[32] Use of the oligonucleotide according to item [28] or the SNP according to item [29] to identify at least one further marker allele linked to a functional restorer gene for wheat G-type cytoplasmic male sterility in a wheat plant cell, wheat plant or a part thereof or a wheat seed.

### Examples

### Example 1: Design of PPR-capture and RFL-PPR capture

Based on public and own proprietary sequence resources (e.g., RefSeqv1.0: https://www.wheatgenome.org/News/Latest-news/RefSeq-v1.0-URGI) the family of wheat PPR genes was annotated. Based on the phylogenic analysis, among 1659 PPR ORFs a total of 159 PPR genes were similar to restorer-of-fertility-like PPR (RFL-PPR) genes and exhibited a P motif. The P motif is the typical structure of the RFL genes. One P motif can fold into two anti-parallel α helices in 3D structure. In the protein-RNA binding reaction, one pair of α helices is corresponding to one RNA residue. According to previously cloned Rf genes, the number of P motifs is not less than 15. So here, the threshold was set at 10 for full length RFL genes. 79 genes contained more than 10 PPR repeats and were defined as core set of Chinese Spring full length RFL-PPR genes (Fig. 1). These full length RFL-PPRs are the candidates for Rf genes in the reference genome, since they all have more than 10 P motifs and are similar to the cloned Rf genes (belong to the same cluster).

The 79 full length RFL-PPR genes distributed in clusters mainly on the homoeologous linkage groups 1, 2, 6 and 7 (Fig. 2).

### Example 2: Resequencing of RFL-PPR genes and SNP identification

Capture sequencing has been established for PPR gene sequencing. This method has been used in the reported experiments. All 79 full length RFL-PPR genes were resequenced from the following genotypes: Chinese Spring, Primepi, Meister and Sperber (**Figs 3** **and** **6****)**. Chinese Spring is known to carry a restorer gene against the cytoplasm of *Aegilops kotschyi,* but it has shown no restoration capacity in G-type CMS (Ahmed et al. (2001) QTL analysis of fertility-restoration against cytoplasmic male sterility in wheat. Genes Genet Syst, 76, 33-38). Chinese Spring serves as reference genome of the RFL gene annotation and hybrid baits designing. It was included into the experiment to monitor the capture efficiency. Primepi and Meister are wheat restorer lines comprising restorer genes (e.g. Rf3, see the **Table 1**). Sperber does not contain any Rf-activity (non-restorer genotype) and serves as female parent line of Primepi and Meister in hybrid breeding. It will be used in the SNP calling based on the captured reads mapping data.

For the majority of the 79 full length RFL-PPR genes of Chinese Spring sequence information was captured from all of the 4 tested genotypes, where Chinese Spring served as the positive control for calibrating the capture efficiency. On the basis of the accumulated resequencing data, sequence polymorphisms were surveyed in comparison to the non-restorer genotype Sperber. SNPs between Rf and non-Rf genotypes could be determined for about 50% of the full length RFL-PPR genes (**Fig. 4**).

On basis of these results, the initial PPR capture design (general wheat PPR baits library) comprising all the annotated PPR gene sequences of wheat was reduced to represent only the RFL-PPR genes. Additionally, RFL-PPRs of rye and barley, which could be used in other cereal species for RFL gene resequencing have been included. In a next step resequencing took advantage of longer sequence reads which allowed then to capture an even larger number of SNPs in the given genotype combinations (**Fig. 5**).

In a subsequent experiment three additional restorer lines L19, R3 and R113 comprising different restorer genes or combinations thereof (see also **Table 1**)**,** were selected for RFL-PPR capture and resequencing on basis of pre-existing knowledge about Rf3-independent Rf-activity (**Table 1**). R3 and R113 contain two Rf loci resulting in an increase of called SNPs. L19, R3 and R113 comprise other Rf genes (not cloned yet), they also have good fertility restoration performance. From these genotypes an even higher number of SNPs could be determined (**Table 2**).

**Table 1: Overview of Rf gene composition in a selection of wheat genotypes used for RFL-PPR sequence capture.**

| **Genotype** | **Restorer gene** | **Gene location [chromosome]** |
|---|---|---|
| Primepi | *Rf3* | 1BS |
| Meister | unknown | unknown |
| L19 | *Rf1* | 1AS |
| R3 | *Rf1, Rf2* | 1AS, 7D |
| R113 | *Rf1, Rf4* | 1AS, 6BS |

**Table 2: Number of SNPs after RFL-PPR capture in comparison to the non-Rf genotype Sperber.**

| **SNPs** | | **#SNPs** |
|---|---|---|
| Primepi | / Sperber | 370 |
| Meister | / Sperber | 355 |
| L19 | / Sperber | 309 |
| R3 | / Sperber | 630 |
| R113 | / Sperber | 499 |

### Example 3: Marker design and mapping of Rf loci

Based on the resequencing and SNP identification results, a list of candidate SNPs for chromosomes IBS (**Table 3**, *Rf3*) and 1AS (**Tables 4-6,** *Rf1*) was compiled and used for SNP assay development and genetic mapping of RFL-PPR genes. Very closely linked markers were developed for Rf1 (**Fig. 7**) and Rf3 (**Fig. 8** **A** and **B**), providing alternative gene-based SNP assays for marker-assisted breeding (MAS).

**Table 3: Candidate SNPs for chromosome IBS (resistance locus Rf3; P=Primepi and S=Sperber)**

| **Chr.** | **SNP position (RefSeq v1.0)** | **Allocation to RFL ID** | **SNP quality** | **SNP** | | | **Marker sequence** |
|---|---|---|---|---|---|---|---|
| | | | | **P** | **S** | **[P/S]** | |
| 1B | 18869968 | RFL_11 | 999 | A | G | [A/G] | SEQ ID NO: 47 |
| 1B | 18869933 | RFL_11 | 999 | G | A | [G/A] | SEQ ID NO: 48 |
| 1B | 18869867 | RFL_11 | 999 | G | A | [G/A] | SEQ ID NO: 49 |
| 1B | 18869848 | RFL_11 | 153 | C | A | [C/A] | SEQ ID NO: 50 |
| 1B | 18869837 | RFL_11 | 999 | C | G | [C/G] | SEQ ID NO: 51 |
| 1B | 18869284 | RFL_11 | 766 | T | C | [T/C] | SEQ ID NO: 52 |
| 1B | 18869280 | RFL_11 | 102 | T | C | [T/C] | SEQ ID NO: 53 |
| 1B | 18685099 | RFL_12 | 208 | A | G | [A/G] | SEQ ID NO: 54 |
| 1B | 18684968 | RFL_12 | 208 | A | G | [A/G] | SEQ ID NO: 55 |
| 1B | 18684869 | RFL_12 | 999 | C | T | [C/T] | SEQ ID NO: 56 |
| 1B | 18684760 | RFL_12 | 999 | G | A | [G/A] | SEQ ID NO: 57 |
| 1B | 18684739 | RFL_12 | 999 | G | A | [G/A] | SEQ ID NO: 58 |
| 1B | 18684717 | RFL_12 | 999 | G | A | [G/A] | SEQ ID NO: 59 |
| 1B | 18684685 | RFL_12 | 416 | A | G | [A/G] | SEQ ID NO: 60 |
| 1B | 18684680 | RFL_12 | 990 | C | G | [C/G] | SEQ ID NO: 61 |
| 1B | 18684488 | RFL_12 | 688 | A | G | [A/G] | SEQ ID NO: 62 |
| 1B | 18684471 | RFL_12 | 164 | C | T | [C/T] | SEQ ID NO: 63 |
| 1B | 18684440 | RFL_12 | 999 | C | A | [C/A] | SEQ ID NO: 64 |
| 1B | 18684202 | RFL_12 | 999 | A | T | [A/T] | SEQ ID NO: 65 |
| 1B | 18684110 | RFL_12 | 999 | C | G | [C/G] | SEQ ID NO: 66 |
| 1B | 18683870 | RFL_12 | 208 | G | A | [G/A] | SEQ ID NO: 67 |
| 1B | 18683863 | RFL_12 | 999 | A | G | [A/G] | SEQ ID NO: 68 |
| 1B | 18683793 | RFL_12 | 760 | C | G | [C/G] | SEQ ID NO: 69 |
| 1B | 18683770 | RFL_12 | 208 | C | T | [C/T] | SEQ ID NO: 70 |
| 1B | 18683761 | RFL_12 | 999 | C | T | [C/T] | SEQ ID NO: 71 |
| 1B | 18683729 | RFL_12 | 999 | G | A | [G/A] | SEQ ID NO: 72 |
| 1B | 18117730 | RFL_13 | 83 | T | A | [T/A] | SEQ ID NO: 73 |
| 1B | 18117718 | RFL_13 | 177 | C | A | [C/A] | SEQ ID NO: 74 |
| 1B | 18117629 | RFL_13 | 208 | G | A | [G/A] | SEQ ID NO: 75 |
| 1B | 18117595 | RFL_13 | 208 | G | A | [G/A] | SEQ ID NO: 76 |
| 1B | 18117582 | RFL_13 | 955 | G | A | [G/A] | SEQ ID NO: 77 |
| 1B | 18117570 | RFL_13 | 208 | G | A | [G/A] | SEQ ID NO: 78 |
| 1B | 18117566 | RFL_13 | 206 | A | G | [A/G] | SEQ ID NO: 79 |
| 1B | 18117565 | RFL_13 | 202 | T | C | [T/C] | SEQ ID NO: 80 |
| 1B | 18117424 | RFL_13 | 149 | A | G | [A/G] | SEQ ID NO: 81 |
| 1B | 18117423 | RFL_13 | 151 | C | A | [C/A] | SEQ ID NO: 82 |
| 1B | 18117416 | RFL_13 | 112 | G | A | [G/A] | SEQ ID NO: 83 |
| 1B | 18117047 | RFL_13 | 208 | C | T | [C/T] | SEQ ID NO: 84 |
| 1B | 18116721 | RFL_13 | 425 | T | A | [T/A] | SEQ ID NO: 85 |
| 1B | 18116496 | RFL_13 | 885 | A | G | [A/G] | SEQ ID NO: 86 |
| 1B | 18116446 | RFL_13 | 208 | T | G | [T/G] | SEQ ID NO: 87 |
| 1B | 18116441 | RFL_13 | 208 | G | A | [G/A] | SEQ ID NO: 88 |
| 1B | 18116377 | RFL_13 | 208 | A | C | [A/C] | SEQ ID NO: 89 |
| 1B | 18116329 | RFL_13 | 178 | G | T | [G/T] | SEQ ID NO: 90 |
| 1B | 18116303 | RFL_13 | 110 | C | T | [C/T] | SEQ ID NO: 91 |
| 1B | 18116289 | RFL_13 | 138 | G | A | [G/A] | SEQ ID NO: 92 |
| 1B | 18116263 | RFL_13 | 208 | A | G | [A/G] | SEQ ID NO: 93 |
| 1B | 18116227 | RFL_13 | 208 | C | G | [C/G] | SEQ ID NO: 94 |
| 1B | 18116023 | RFL_13 | 80 | T | C | [T/C] | SEQ ID NO: 95 |
| 1B | 18092768 | RFL_14 | 999 | T | C | [T/C] | SEQ ID NO: 96 |
| 1B | 18092488 | RFL_14 | 192 | G | T | [G/T] | SEQ ID NO: 97 |
| 1B | 18092485 | RFL_14 | 999 | A | G | [A/G] | SEQ ID NO: 98 |
| 1B | 18092449 | RFL_14 | 999 | T | G | [T/G] | SEQ ID NO: 99 |
| 1B | 18092433 | RFL_14 | 167 | C | G | [C/G] | SEQ ID NO: 100 |
| 1B | 17895446 | RFL_15 | 999 | G | A | [G/A] | SEQ ID NO: 101 |
| 1B | 17895044 | RFL_15 | 999 | A | G | [A/G] | SEQ ID NO: 102 |
| 1B | 17894980 | RFL_15 | 999 | C | T | [C/T] | SEQ ID NO: 103 |
| 1B | 17894910 | RFL_15 | 139 | T | A | [T/A] | SEQ ID NO: 104 |
| 1B | 17894539 | RFL_15 | 999 | T | C | [T/C] | SEQ ID NO: 105 |
| 1B | 19073938 | RFL_16 | 208 | A | T | [A/T] | SEQ ID NO: 106 |
| 1B | 19073961 | RFL_16 | 208 | G | T | [G/T] | SEQ ID NO: 107 |
| 1B | 19074732 | RFL_16 | 208 | C | T | [C/T] | SEQ ID NO: 108 |

**Table 4: Candidate SNPs for chromosome 1AS of R3 (resistance locus Rf1; S=Sperber)**

| **Chr.** | **SNP position (RefSeq v1.0)** | **Allocation to RFL ID** | **SNP quality** | **SNP** | | | **Marker sequence** |
|---|---|---|---|---|---|---|---|
| | | | | **R3** | **S** | **R3/S** | |
| 1A | 14587250 | RFL_01 | 999 | G | T | [G/T] | SEQ ID NO: 109 |
| 1A | 14587092 | RFL_01 | 999 | T | C | [T/C] | SEQ ID NO: 110 |
| 1A | 14586743 | RFL_01 | 999 | C | G | [C/G] | SEQ ID NO: 111 |
| 1A | 14586682 | RFL_01 | 205.473 | G | A | [G/A] | SEQ ID NO: 112 |
| 1A | 14586435 | RFL_01 | 999 | T | C | [T/C] | SEQ ID NO: 113 |
| 1A | 14586077 | RFL_01 | 999 | C | T | [C/T] | SEQ ID NO: 114 |
| 1A | 14585902 | RFL_01 | 999 | C | T | [C/T] | SEQ ID NO: 115 |
| 1A | 14585866 | RFL_01 | 999 | C | T | [C/T] | SEQ ID NO: 116 |
| 1A | 14585862 | RFL_01 | 999 | T | A | [T/A] | SEQ ID NO: 117 |
| 1A | 14585766 | RFL_01 | 211.551 | C | A | [C/A] | SEQ ID NO: 118 |
| 1A | 14585709 | RFL_01 | 840.012 | A | G | [A/G] | SEQ ID NO: 119 |
| 1A | 14585688 | RFL_01 | 141,26 | A | G | [A/G] | SEQ ID NO: 120 |
| 1A | 14585686 | RFL_01 | 536.025 | C | T | [C/T] | SEQ ID NO: 121 |
| 1A | 14585672 | RFL_01 | 141,2 | T | C | [T/C] | SEQ ID NO: 122 |
| 1A | 14585409 | RFL_01 | 205.541 | G | A | [G/A] | SEQ ID NO: 123 |
| 1A | 14585406 | RFL_01 | 205.608 | G | T | [G/T] | SEQ ID NO: 124 |
| 1A | 14585383 | RFL_01 | 999 | A | T | [A/T] | SEQ ID NO: 125 |
| 1A | 14585379 | RFL_01 | 999 | G | A | [G/A] | SEQ ID NO: 126 |
| 1A | 14585377 | RFL_01 | 999 | T | C | [T/C] | SEQ ID NO: 127 |
| 1A | 14585368 | RFL_01 | 999 | T | C | [T/C] | SEQ ID NO: 128 |
| 1A | 14585358 | RFL_01 | 999 | T | G | [T/G] | SEQ ID NO: 129 |
| 1A | 14585339 | RFL_01 | 999 | G | C | [G/C] | SEQ ID NO: 130 |
| 1A | 14585297 | RFL_01 | 999 | C | T | [C/T] | SEQ ID NO: 131 |
| 1A | 14585268 | RFL_01 | 207.533 | G | C | [G/C] | SEQ ID NO: 132 |
| 1A | 14585246 | RFL_01 | 201.533 | C | T | [C/T] | SEQ ID NO: 133 |
| 1A | 14585244 | RFL_01 | 185.533 | T | C | [T/C] | SEQ ID NO: 134 |
| 1A | 14585242 | RFL_01 | 189.533 | C | T | [C/T] | SEQ ID NO: 135 |
| 1A | 14585199 | RFL_01 | 143.533 | A | G | [A/G] | SEQ ID NO: 136 |
| 1A | 14585183 | RFL_01 | 128.502 | A | G | [A/G] | SEQ ID NO: 137 |
| 1A | 14579414 | RFL_02 | 999 | A | C | [A/C] | SEQ ID NO: 138 |
| 1A | 14579411 | RFL_02 | 999 | G | A | [G/A] | SEQ ID NO: 139 |
| 1A | 14579108 | RFL_02 | 135.849 | A | G | [A/G] | SEQ ID NO: 140 |
| 1A | 14579093 | RFL_02 | 999 | T | G | [T/G] | SEQ ID NO: 141 |
| 1A | 14579090 | RFL_02 | 132.073 | C | G | [C/G] | SEQ ID NO: 142 |
| 1A | 14579089 | RFL_02 | 999 | A | G | [A/G] | SEQ ID NO: 143 |
| 1A | 14579088 | RFL_02 | 999 | A | G | [A/G] | SEQ ID NO: 144 |
| 1A | 14579069 | RFL_02 | 379.353 | T | A | [T/A] | SEQ ID NO: 145 |
| 1A | 14579065 | RFL_02 | 999 | T | C | [T/C] | SEQ ID NO: 146 |
| 1A | 14579060 | RFL_02 | 999 | C | T | [C/T] | SEQ ID NO: 147 |
| 1A | 14579042 | RFL_02 | 205.739 | C | T | [C/T] | SEQ ID NO: 148 |
| 1A | 14578944 | RFL_02 | 999 | T | C | [T/C] | SEQ ID NO: 149 |
| 1A | 14578538 | RFL_02 | 130.717 | G | C | [G/C] | SEQ ID NO: 150 |
| 1A | 14577713 | RFL_02 | 999 | C | A | [C/A] | SEQ ID NO: 151 |
| 1A | 14577638 | RFL_02 | 210.244 | G | A | [G/A] | SEQ ID NO: 152 |

**Table 5: Candidate SNPs for chromosome 1AS of RIB (resistance locus Rf1; S=Sperber)**

| **Chr.** | **SNP position (RefSeq v1.0)** | **Allocation to RFL ID** | **SNP quality** | **SNP** | | | **Marker sequence** |
|---|---|---|---|---|---|---|---|
| | | | | **R113** | **S** | **R113/S** | |
| 1A | 14587250 | RFL_01 | 999 | G | T | [G/T] | SEQ ID NO: 153 |
| 1A | 14587092 | RFL_01 | 999 | T | C | [T/C] | SEQ ID NO: 154 |
| 1A | 14587027 | RFL_01 | 999 | C | T | [C/T] | SEQ ID NO: 155 |
| 1A | 14586980 | RFL_01 | 999 | C | T | [C/T] | SEQ ID NO: 156 |
| 1A | 14586743 | RFL_01 | 999 | C | G | [C/G] | SEQ ID NO: 157 |
| 1A | 14586682 | RFL_01 | 205.473 | G | A | [G/A] | SEQ ID NO: 158 |
| 1A | 14586435 | RFL_01 | 999 | T | C | [T/C] | SEQ ID NO: 159 |
| 1A | 14585984 | RFL_01 | 999 | C | G | [C/G] | SEQ ID NO: 160 |
| 1A | 14585981 | RFL_01 | 999 | T | G | [T/G] | SEQ ID NO: 161 |
| 1A | 14585976 | RFL_01 | 999 | C | T | [C/T] | SEQ ID NO: 162 |
| 1A | 14585961 | RFL_01 | 999 | A | G | [A/G] | SEQ ID NO: 163 |
| 1A | 14585955 | RFL_01 | 999 | A | G | [A/G] | SEQ ID NO: 164 |
| 1A | 14585902 | RFL_01 | 999 | C | T | [C/T] | SEQ ID NO: 165 |
| 1A | 14585409 | RFL_01 | 205.541 | G | A | [G/A] | SEQ ID NO: 166 |
| 1A | 14585406 | RFL_01 | 205.608 | G | T | [G/T] | SEQ ID NO: 167 |
| 1A | 14585383 | RFL_01 | 999 | A | T | [A/T] | SEQ ID NO: 168 |
| 1A | 14585379 | RFL_01 | 999 | G | A | [G/A] | SEQ ID NO: 169 |
| 1A | 14585377 | RFL_01 | 999 | T | C | [T/C] | SEQ ID NO: 170 |
| 1A | 14585368 | RFL_01 | 999 | T | C | [T/C] | SEQ ID NO: 171 |
| 1A | 14585358 | RFL_01 | 999 | T | G | [T/G] | SEQ ID NO: 172 |
| 1A | 14585339 | RFL_01 | 999 | G | C | [G/C] | SEQ ID NO: 173 |
| 1A | 14585297 | RFL_01 | 999 | C | T | [C/T] | SEQ ID NO: 174 |
| 1A | 14585268 | RFL_01 | 207.533 | G | C | [G/C] | SEQ ID NO: 175 |
| 1A | 14585246 | RFL_01 | 201.533 | C | T | [C/T] | SEQ ID NO: 176 |
| 1A | 14585244 | RFL_01 | 185.533 | T | C | [T/C] | SEQ ID NO: 177 |
| 1A | 14585242 | RFL_01 | 189.533 | C | T | [C/T] | SEQ ID NO: 178 |
| 1A | 14585199 | RFL_01 | 143.533 | A | G | [A/G] | SEQ ID NO: 179 |
| 1A | 14585183 | RFL_01 | 128.502 | A | G | [A/G] | SEQ ID NO: 180 |
| 1A | 14579246 | RFL_02 | 155.772 | G | A | [G/A] | SEQ ID NO: 181 |
| 1A | 14579194 | RFL_02 | 154.204 | C | A | [C/A] | SEQ ID NO: 182 |
| 1A | 14578963 | RFL_02 | 999 | C | G | [C/G] | SEQ ID NO: 183 |
| 1A | 14578944 | RFL_02 | 999 | T | C | [T/C] | SEQ ID NO: 184 |
| 1A | 14578929 | RFL_02 | 999 | G | C | [G/C] | SEQ ID NO: 185 |
| 1A | 14578898 | RFL_02 | 999 | A | C | [A/C] | SEQ ID NO: 186 |
| 1A | 14578885 | RFL_02 | 124.772 | C | T | [C/T] | SEQ ID NO: 187 |
| 1A | 14578869 | RFL_02 | 115.771 | C | T | [C/T] | SEQ ID NO: 188 |
| 1A | 14578856 | RFL_02 | 999 | T | C | [T/C] | SEQ ID NO: 189 |

**Table 6: Candidate SNPs for chromosome 1AS of L19 (resistance locus Rf1; S=Sperber)**

| **Chr.** | **SNP position (RefSeq v1.0)** | **Allocation to RFL ID** | **SNP quality** | **SNP** | | | **SNP sequence** |
|---|---|---|---|---|---|---|---|
| | | | | **L19** | **S** | **L19/S** | |
| 1A | 14587250 | RFL_01 | 999 | G | T | [G/T] | SEQ ID NO: 190 |
| 1A | 14587092 | RFL_01 | 999 | T | C | [T/C] | SEQ ID NO: 191 |
| 1A | 14586743 | RFL_01 | 999 | C | G | [C/G] | SEQ ID NO: 192 |
| 1A | 14586682 | RFL_01 | 205.473 | G | A | [G/A] | SEQ ID NO: 193 |
| 1A | 14586435 | RFL_01 | 999 | T | C | [T/C] | SEQ ID NO: 194 |
| 1A | 14586077 | RFL_01 | 999 | C | T | [C/T] | SEQ ID NO: 195 |
| 1A | 14585984 | RFL_01 | 999 | C | G | [C/G] | SEQ ID NO: 196 |
| 1A | 14585981 | RFL_01 | 999 | T | G | [T/G] | SEQ ID NO: 197 |
| 1A | 14585976 | RFL_01 | 999 | C | T | [C/T] | SEQ ID NO: 198 |
| 1A | 14585961 | RFL_01 | 999 | A | G | [A/G] | SEQ ID NO: 199 |
| 1A | 14585955 | RFL_01 | 999 | A | G | [A/G] | SEQ ID NO: 200 |
| 1A | 14585902 | RFL_01 | 999 | C | T | [C/T] | SEQ ID NO: 201 |
| 1A | 14585431 | RFL_01 | 999 | A | G | [A/G] | SEQ ID NO: 202 |
| 1A | 14585409 | RFL_01 | 205.541 | G | A | [G/A] | SEQ ID NO: 203 |
| 1A | 14585406 | RFL_01 | 205.608 | G | T | [G/T] | SEQ ID NO: 204 |
| 1A | 14585383 | RFL_01 | 999 | A | T | [A/T] | SEQ ID NO: 205 |
| 1A | 14585379 | RFL_01 | 999 | G | A | [G/A] | SEQ ID NO: 206 |
| 1A | 14585377 | RFL_01 | 999 | T | C | [T/C] | SEQ ID NO: 207 |
| 1A | 14585368 | RFL_01 | 999 | T | C | [T/C] | SEQ ID NO: 208 |
| 1A | 14585358 | RFL_01 | 999 | T | G | [T/G] | SEQ ID NO: 209 |
| 1A | 14585339 | RFL_01 | 999 | G | C | [G/C] | SEQ ID NO: 210 |
| 1A | 14585297 | RFL_01 | 999 | C | T | [C/T] | SEQ ID NO: 211 |
| 1A | 14585268 | RFL_01 | 207.533 | G | C | [G/C] | SEQ ID NO: 212 |
| 1A | 14585246 | RFL_01 | 201533 | C | T | [C/T] | SEQ ID NO: 213 |
| 1A | 14585244 | RFL_01 | 185533 | T | C | [T/C] | SEQ ID NO: 214 |
| 1A | 14585242 | RFL_01 | 189.533 | C | T | [C/T] | SEQ ID NO: 215 |
| 1A | 14585199 | RFL_01 | 143.533 | A | G | [A/G] | SEQ ID NO: 216 |
| 1A | 14585183 | RFL_01 | 128502 | A | G | [A/G] | SEQ ID NO: 217 |
| 1A | 14579275 | RFL_02 | 999 | G | C | [G/C] | SEQ ID NO: 218 |
| 1A | 14578963 | RFL_02 | 999 | C | G | [C/G] | SEQ ID NO: 219 |
| 1A | 14578944 | RFL_02 | 999 | T | C | [T/C] | SEQ ID NO: 220 |
| 1A | 14578929 | RFL_02 | 999 | G | C | [G/C] | SEQ ID NO: 221 |
| 1A | 14578898 | RFL_02 | 999 | A | C | [A/C] | SEQ ID NO: 222 |
| 1A | 14578885 | RFL_02 | 124772 | C | T | [C/T] | SEQ ID NO: 223 |
| 1A | 14578869 | RFL_02 | 115771 | C | T | [C/T] | SEQ ID NO: 224 |
| 1A | 14578856 | RFL_02 | 999 | T | C | [T/C] | SEQ ID NO: 225 |

### Example 4: Validation of candidate genes using exemplary markers

For genetic mapping of candidate genes, competitive allele specific PCR assays were developed for a subset of the SNPs detected within the RFL-PPR genes on chromosomes 1A and 1B of Example 3. Identical genotypes of markers located within the same RFL-PPR gene were combined to construct a single marker genotype for each candidate gene. Genetic mapping of the detected candidate genes was performed using four biparental BC1 populations derived from the common parent CMS-Sperber and the Rf donor lines Primepi, R3, R113 and L19. The populations comprised 193, 197, 201 and 230 individuals, respectively. The assessment of fertility restoration and mapping of the restorer loci Rf1 and Rf3 in these populations was described in detail by Geyer et al. (Mol Breeding (2016) 36:167 and Mol Genetics and Genomics (2018) 293:451-462). Very closely linked markers were found for RF1 (**Fig. 7** **and** Tables 8-10) and RF3 (**Fig. 8** **A** and **B,** and Table 7), providing alternative gene-based SNP assays for marker-assisted breeding (MAS).

**Table 7: Subset of mapped candidate SNPs for chromosome IBS (resistance locus Rf3; P=Primepi and S=Sperber)**

| **Chr.** | **SNP position (RefSeq v1.0)** | **Allocation to RFL ID** | **SNP quality** | **SNP** | | | **Marker sequence** |
|---|---|---|---|---|---|---|---|
| | | | | **P** | **S** | **P/S** | |
| 1B | 18684869 | RFL_12 | 999 | C | T | [C/T] | SEQ ID NO: 56 |
| 1B | 18684739 | RFL_12 | 999 | G | A | [G/A] | SEQ ID NO: 58 |
| 1B | 18684440 | RFL_12 | 999 | C | A | [C/A] | SEQ ID NO: 64 |
| 1B | 18684110 | RFL_12 | 999 | C | G | [C/G] | SEQ ID NO: 66 |
| 1B | 18117629 | RFL_13 | 208 | G | A | [G/A] | SEQ ID NO: 75 |
| 1B | 18117047 | RFL_13 | 208 | C | T | [C/T] | SEQ ID NO: 84 |
| 1B | 18116721 | RFL_13 | 425 | T | A | [T/A] | SEQ ID NO: 85 |
| 1B | 18116377 | RFL_13 | 208 | A | C | [A/C] | SEQ ID NO: 89 |
| 1B | 18116227 | RFL_13 | 208 | C | G | [C/G] | SEQ ID NO: 94 |
| 1B | 18092449 | RFL_14 | 999 | T | G | [T/G] | SEQ ID NO: 99 |
| 1B | 17895446 | RFL_15 | 999 | G | A | [G/A] | SEQ ID NO: 101 |
| 1B | 19073938 | RFL_15 | 999 | T | C | [T/C] | SEQ ID NO: 105 |
| 1B | 19073961 | RFL_16 | 208 | G | T | [G/T] | SEQ ID NO: 107 |

**Table 8: Subset of mapped candidate SNPs for chromosome 1AS of R3 (resistance locus Rf1; S=Sperber)**

| **Chr.** | **SNP position (RefSeq v1.0)** | **Allocation to RFL ID** | **SNP quality** | **SNP** | | | **Marker sequence** |
|---|---|---|---|---|---|---|---|
| | | | | **R3** | **S** | **R3/S** | |
| 1A | 14587250 | RFL_01 | 999 | G | T | [G/T] | SEQ ID NO: 109 |
| 1A | 14586743 | RFL_01 | 999 | C | G | [C/G] | SEQ ID NO: 111 |
| 1A | 14579275 | RFL_02 | 999 | G | C | [G/C] | SEQ ID NO: 218 |

The SNPs listed in Table 8 have also been identified in the restorer lines R113 and L19 as shown in Tables 9 and 10, respectively.

**Table 9: Subset of mapped candidate SNPs for chromosome 1AS of R113 (resistance locus Rf1; S=Sperber)**

| **Chr.** | **SNP position (RefSeq v1.0)** | **RFL ID** | | | | | |
|---|---|---|---|---|---|---|---|
| | | | **SNP quality** | **R113** | **S** | **R113/ S** | **SNP sequence** |
| 1A | 14587250 | RFL_01 | 999 | G | T | [G/T] | SEQ ID NO: 153 |
| 1A | 14586743 | RFL_01 | 999 | C | G | [C/G] | SEQ ID NO: 157 |
| 1A | 14579275 | RFL_02 | 999 | G | C | [G/C] | SEQ ID NO: 218 |

**Table 10: Subset of mapped candidate SNPs for chromosome 1AS of L19 (resistance locus Rf1; S=Sperber)**

| **Chr.** | **SNP position (RefSeq v1.0)** | **RFL ID** | **SNP quality** | **L19** | **S** | **L19/S** | **SNP sequence** |
|---|---|---|---|---|---|---|---|
| 1A | 14587250 | RFL_01 | 999 | G | T | [G/T] | SEQ ID NO: 190 |
| 1A | 14586743 | RFL_01 | 999 | C | G | [C/G] | SEQ ID NO: 192 |
| 1A | 14579275 | RFL_02 | 999 | G | C | [G/C] | SEQ ID NO: 218 |

### Example 5: Identification of an Rf3 candidate gene

Based on the Rf3 mapping results, a re-analysis of the PACBIO PPR capture sequencing analysis was performed for the Rf3-linked RFL genes RFL12, RFL13, RFL14, RFL15 and RFL16 (see also **Fig. 9**). RFL16 is positioned on scaffold5117 from position 80191 to 82548 on the scaffold. (SEQ ID NO: 10). The same scaffold carries an additional RFL pseudogene, called Rf3_pseudo_CS (see **Fig. 9**). RFL11-15 are all situated on CS RefSeq scaffold35219. RFL11 is located from position 129386 to 131872, RFL12 from position 313723 to 316122, RFL13 from position 880602 to 883172, RFL14 from position 906067 to 908022, RFL15 from position 1102780 to 1105257 on the scaffold. Both scaffolds, scaffold5117 and scaffold35219, are direct neighbors sitting tail-to-tail on chromosome IBS of the CS RefSeqv1.0 chromosome-scale assembly (see **Fig. 9**), i.e. RFL11-16, including additional pseudogenes, form one larger RFL-PPR gene cluster that is linked to the Rf3 locus. Interestingly, RFL16 is missing in the PACBIO resequencing data of the Rf3-carrying genotype Primepi. After de novo assembly of the Primepi (restorer genotype), Sperber (non-restorer genotype) and CS RFL-PPR capture PACBIO resequencing data, it became obvious that Primepi carries two homologs of Rf3_pseudo_CS (Rf3_pseudo_P (SEQ ID NO: 6) and Rf3_P (SEQ ID NO: 2)). One copy of the homologs (Rf3_pseudo_P; SEQ ID NO: 6) is a putative pseudogene, which compared to the CS allele (Rf3_pseudo_CS (SEQ ID NO: 8) as reference) is a different allele. Compared to SEQ ID NO; 2, SEQ ID NO: 6 carries inter alia an independent 23-bp deletion leading to a frameshift. The second copy (Rf3_P; SEQ ID NO: 2) is a functional RFL gene encoding a protein of 792 amino acids (SEQ ID NO:3).

This second gene copy encoding the functional RFL gene is missing in the reference CS. In the non-restorer genotype Sperber this homolog is present (rf3_S), however, in the form of a non-functional allele which carries a single base pair deletion corresponding to position 669 of SEQ ID NO: 2 leading to a frameshift mutation and to a non-functional RFL gene.

**Fig. 9** illustrates the DNA and protein sequence identity of RFL-PPR genes within the identified cluster - RFL11-16 plus the Rf3_pseudo_CS homologs from CS, Primepi and Sperber.

Taking the above into account, the inventors identified the Rf3_P to be the predicted functional Rf3 gene, because Rf3_P has a functional polymorphism (above described single base pair deletion) between the restorer genotype (Primepi) and the corresponding non-restorer line (Sperber).

Another strong indication that Rf3_P is the restorer allele is that this observed group of paralogs is showing the typical pattern of PA (presence/absence) / CNV (copy number variation) polymorphism between restorer/non-restorer genotypes that was reported before also for the Rf1 locus in rice (Komori T, Ohta S, Murai N, Takakura Y, Kuraya Y, Suzuki S, Hiei Y, Imaseki H, Nitta N (2004) Map-based cloning of a fertility restorer gene, Rf-1, in rice (Oryza sativa L.). The Plant Journal 37:315-325).

### Example 6: Identification of a Rf1 candidate gene

Analysis of the Rf1 locus was performed in analogy to the above data presented for Rf3. SNP generated by RFL-PPR capture in combination with Illumina sequencing for CS RFL gene loci RFL_01-10 were mapped (**Fig. 10**).

The genes RFL_01 and RFL_02 were mapped to the Rf1 QTL support interval. RFL-PPR capture in combination with PACBIO SMRT sequencing revealed that RFL_02 is not present in the three tested Rf1 genotypes (R3, R113 and L19) and is also missing in Sperber. Thus, RFL_01 seems to be the more interesting target locus. The RFL_01 locus shows different levels of complexity including putatively functional and non-functional paralogs in restorer and non-restorer genotypes (**Table 11**). For example, restorer line R3 is carrying five RFL_01 paralogs (Rf1-1, Rf1-2, Rf1-3, Rf1_pseudo1 and Rf1_pseudo2).

**Table 11: RFL_01 paralogs in different wheat accessions**

| **Lines** | **Gene** | **Length of the protein [aa]** | **Number of P motif** | **Comments** |
|---|---|---|---|---|
| R3 (restorer) | Rf1-1 | 738 | 16 | - |
| | Rf1-2 | 741 | 17 | - |
| | Rf1-3 | 620 | 15 | Incomplete gene due to low coverage |
| | Rf1_pseudo1 | - | - | - |
| | Rf1_pseudo2 | - | - | - |
| R113 (restorer) | Rf1-1 | 738 | 16 | - |
| | Rf1-2 | 741 | 17 | - |
| L19 (restorer) | Rf1-2 | 741 | 17 | - |
| CS (probably non-restorer) | RFL_01 | 741 | 17 | - |
| | RFL_02 | 784 | 17 | - |
| Sperber (non-restorer) | rf1-1 | - | - | pre-stop due to deletion |
| | rf1-2 | - | - | pre-stop due to the TGC/TGA SNP |

RFL_01 alleles and paralogs are present in all three Rf1 genotypes but only one paralog is shared by all of the three restorer lines (R3, R113 and L19) and Chinese Spring (CS), which is Rf1-2 in the restorer lines and RFL_01 in CS. However, sequence homology between the CS RFL_01 allele is slightly lower to the respective alleles from Rf1-2 containing genotypes, which all carry the identical Rfl-2 allele sequence (**Fig. 11**). Sperber contains two pseudogene paralogs of RFL_01 (rf1-1 and rfl-2) which carry mutations leading to a pre-stop in translation. In case of rf1-1 (SEQ ID NO: 33), there is a deletion of one base pair at position 657 referenced to the CS sequence (SEQ ID NO: 23) resulting in a frame shift and thereby to a premature stop after 225 amino acids (stop codon is from nucleotide 676 to 678 of SEQ ID NO: 33). In case of rf1-2 (SEQ ID NO: 35), there is a C to A conversion at position 1471 referenced to the CS sequence (SEQ ID NO: 23) resulting in the creation of a stop codon TGA from nucleotide 1474 to 1476 of SEQ ID NO: 35.

According to the above, the inventors identified the candidate gene for Rf1 to be the allele Rf1-2 (encoding the protein having 741 aa). However, it cannot be excluded that also Rf1-1, which is present in R3 and R113, contain additional restoration capability.

### Example 7: Functional validation by targeted mutagenesis (RGEN)

The identified genes of the present invention may further be functionally validated by targeted mutagenesis (RGEN).

Site-directed mutagenesis by RNA-guided Cas9 endonuclease is an efficient tool in strategies towards gene function validation (J. A. Doudna, E. Charpentier, The new frontier of genome engineering with CRISPR-Cas9. Science 346 (2014); J. D. Sander, J. K. Joung, CRISPR-Cas systems for editing, regulating and targeting genomes. Nat Biotechnol 32, 347 (2014)). Gene specific guide RNAs can be synthesized for principally any region of a given candidate gene as long as a PAM sequence can be found next to a unique sequence of a minimum of 17-20 nucleotides in length. Only guide RNAs with perfect sequence match will lead to the introduction of site directed mutations, thus even in a complex gene family, paralogous gene copies may be distinguished. Guide RNAs may be synthesized for the identified candidate genes and co-transformed with the Cas9 endonuclease into the respective Rf-gene containing wheat genotype. Gene specific primers may be used for PCR and sequence analysis of the target gene in primary transgenic regenerated plants (To Generation). Plants carrying deletions leading to frameshift mutations are likely to result in non-functional genes/proteins and the loss of restoration of fertility in crosses. Independent events of such mutations will be tested in homozygous mutants for a change in restoration capacity compared to wild type To regenerated plants lacking a mutation in the target gene. Inheritance of the mutation and phenotype will be confirmed in the following generation (T₁).

### Sequence information

**Table 12: SEQ ID NOs**

| **SEQ ID NO:** | **Comment** |
|---|---|
| 1 | scaffold5117 from position 67379 to 69762 |
| 2 | DNA Rf3_P |
| 3 | Prot Rf3_P |
| 4 | DNA rf3_S |
| 5 | Prot rf3_S |
| 6 | DNA Rf3_pseudo_P |
| 7 | Prot Rf3_pseudo_P |
| 8 | DNA Rf3_pseudo_CS |
| 9 | Prot Rf3_pseudo_CS |
| 10 | DNA RFL16 |
| 11 | Prot RFL16 |
| 12 | scaffold44309 from position 647269 to 649539 |
| 13 | DNA R3_Rf1-2 |
| 14 | Prot R3_Rf1-2 |
| 15 | DNA R113_Rf1-2 |
| 16 | Prot R113_Rf1-2 |
| 17 | DNA L19_Rf1-2 |
| 18 | Prot L19_Rf1-2 |
| 19 | DNA R3_Rf1-1 |
| 20 | Prot R3_Rf1-1 |
| 21 | DNA R113_Rf1-1 |
| 22 | Prot R113_Rf1-1 |
| 23 | DNA RFL_01_cs |
| 24 | Prot RFL_01_cs |
| 25 | DNA RFL_02_cs |
| 26 | Prot RFL_02_cs |
| 27 | DNA R3_Rf1-3 |
| 28 | Prot R3_Rf1-3 |
| 29 | DNA R3_Rf1_pseudo1 |
| 30 | Prot R3_Rf1_pseudo1 |
| 31 | DNA R3_Rf1_pseudo2 |
| 32 | Prot R3_Rf1_pseudo2 |
| 33 | DNA S_rf1-1 |
| 34 | Prot S_rf1-1 |
| 35 | DNA S_rf1-2 |
| 36 | Prot S_rf1-2 |
| 37 | DNA RFL11 |
| 38 | Prot RFL11 |
| 39 | DNA RFL12 |
| 40 | Prot RFL12 |
| 41 | DNA RFL13 |
| 42 | Prot RFL13 |
| 43 | DNA RFL14 |
| 44 | Prot RFL14 |
| 45 | DNA RFL15 |
| 46 | Prot RFL15 |
| 47-108 | Molecular marker of Table 3 |
| 109-152 | molecular marker of Table 4 |
| 153-189 | molecular marker of Table 5 |
| 190-225 | molecular marker of Table 6 |

## Claims

1. A nucleic acid comprising a nucleic acid sequence
(i) having the coding sequence according to SEQ ID NO: 2;
(ii) encoding a protein having an amino acid sequence according to SEQ ID NO: 3;
(iii) having a coding sequence with at least 80% sequence identity with SEQ ID NO: 2;
(iv) encoding a protein having an amino acid sequence having at least 80% sequence identity with SEQ ID NO: 3;
(v) having the coding sequence according to SEQ ID NO: 13;
(vi) encoding a protein having an amino acid sequence according to SEQ ID NO: 14;
(vii) having at least 80% sequence identity with SEQ ID NO: 13; and/or
(viii) encoding a protein having an amino acid sequence having at least 80% sequence identity with SEQ ID NO: 14;
preferably wherein the nucleic acid sequence comprises a functional restorer gene allele.

2. A nucleic acid comprising a functional restorer gene allele localizing to a position referred to as position
(i) 14584864 to 14587134 of chromosome 1A of Chinese Spring wheat as reference genome; and/or
(ii) 19061029 to 19063412 of chromosome 1B of Chinese Spring wheat as reference genome.

3. A nucleic acid comprising a functional restorer gene allele localizing to a position
(i) on chromosome 1A of a wheat plant flanked by markers according to SEQ ID NO: 109 and SEQ ID NO: 218, wherein the markers detect SNPs and the SNP in SEQ ID NO: 109 is a G and the SNP in SEQ ID NO: 218 is a G; and/or
(ii) on chromosome 1B of a wheat plant flanked by markers according to SEQ ID NO: 47 and SEQ ID NO: 106, wherein the markers detect SNPs and the SNP in SEQ ID NO: 47 is an A and the SNP in SEQ ID NO: 106 is an A.

4. The nucleic acid of any one of claims 1-3, wherein the functional restorer gene allele is for restoring G-type wheat cytoplasmic male sterility.

5. A vector or expression cassette comprising the nucleic acid of any one of claims 1-4.

6. A polypeptide encoded by the nucleic acid of any one of claims 1-4.

7. A recombinant gene comprising the following operably linked elements
(i) a promoter functional in plants, and
(ii) a nucleic acid according to any one of claims 1-4.

8. A method for producing a plant cell, a plant or part thereof or a seed comprising a functional restorer gene allele for restoring cytoplasmic male sterility or for converting a non-restoring plant into a restoring plant comprising a functional restorer gene allele for restoring cytoplasmic male sterility, wherein the method comprises
a) introducing the nucleic acid of any one of claims 1-4, the vector or the expression cassette of claim 5 or the recombinant gene of claim 7 into the plant cell the plant or the part thereof or the seed, and
b) optionally regenerating a plant from the plant cell of a).

9. A method for identifying or selecting a plant cell, a plant or part thereof or a seed comprising a functional restorer gene allele for restoring cytoplasmic male sterility comprising the step of detecting or identifying in said plant cell, said plant or said part thereof or said seed the presence of the nucleic acid of any one of claims 1-4, the vector or expression cassette of claim 5, the polypeptide of claim 6 or the recombinant gene of claim 7.

10. A method for identifying or selecting a wheat plant cell, a wheat plant or part thereof or a wheat seed comprising a functional restorer gene allele for wheat G-type cytoplasmic male sterility located on chromosome 1A and/or a functional restorer gene allele for wheat G-type cytoplasmic male sterility located on chromosome 1B, comprising the steps of:
(i) detecting in a wheat plant cell, a wheat plant or part thereof or a wheat seed at least one marker allele linked to the functional restorer gene allele for wheat G-type cytoplasmic male sterility located on chromosome 1A (1A restorer marker allele) and/or at least one marker allele linked to the functional restorer gene allele for wheat G-type cytoplasmic male sterility located on chromosome 1B (IB restorer marker allele); and
(ii) identifying and optionally selecting the plant cell, the plant or part thereof or the seed comprising the at least one 1A restorer marker allele and/or the at least one 1B restorer marker allele.

11. A plant cell, a plant or part thereof or a seed obtained by the method of claim 8, or identified by the method of claim 9 or 10, or comprising the nucleic acids according to any one of claims 1-4, the vector or expression cassette of claim 5 or the recombinant genes of claim 7.

12. A method for determining the presence, absence or zygosity status of a functional restorer gene allele for wheat G-type cytoplasmic male sterility located on chromosome 1A and/or on chromosome 1B in a sample derived from a wheat plant, comprising the steps of
(i) providing genomic DNA from said sample, and
(ii) analyzing said DNA and determining said DNA for the presence, absence or zygosity status of at least one marker allele linked to the functional restorer gene allele for wheat G-type cytoplasmic male sterility located on chromosome 1A (1A restorer marker allele) and/or the presence of at least one marker allele linked to the functional restorer gene allele for wheat G-type cytoplasmic male sterility located on chromosome 1B (1B restorer marker allele).

13. The method of claim 10 or claim 12, wherein
(i) the at least one 1A restorer marker allele localizes within an interval on chromosome 1A comprising and flanked by the markers of SEQ ID NO: 109 and SEQ ID NO: 152 and/or the at least one 1B restorer marker allele localizes within an interval on chromosome 1B comprising and flanked by the markers of SEQ ID NO: 105 and SEQ ID NO: 108; or
(ii) the marker allele linked to the functional restorer gene allele for wheat G-type cytoplasmic male sterility located on chromosome 1A is a SNP selected from the SNPs shown in tables 4-6, and/or wherein the marker allele linked to the functional restorer gene allele for wheat G-type cytoplasmic male sterility located on chromosome 1B is a SNP selected from the SNPs shown in table 3, wherein the nucleotide position of the SNP is shown in column 2 of the respective table, and the nucleotide at this position is shown in column 5 of the respective table.

14. Use of an oligonucleotide comprising a nucleic acid sequence according to any one of SEQ ID NOs: 47-225, wherein the nucleotide position of the SNP is shown in column 2 of the respective tables 3-6, and the nucleotide at this position is shown in column 5 of the respective tables 3-6, or a SNP selected from the group of SNPs shown in tables 3-6, wherein the nucleotide position of the SNP is shown in column 2 of the respective table, and the nucleotide at this position is shown in column 5 of the respective table, in a method for identifying one or more functional restorer gene alleles in a wheat plant.


SEQUENCE LISTING

<110>  KWS SAAT SE
       German Seed Alliance GmbH
       Saatzucht Bauer GmbH & Co. KG
       Saatzucht Streng-Engelen GmbH & Co. KG

<120>  Cytoplasmic Male Sterility Restorer Genes, Molecular Markers and
       Uses thereof

<130>  G08490EP

<160>  225

<170>  PatentIn version 3.5

<210>  1
<211>  2358
<212>  DNA
<213>  Triticum aestivum

<400>  1
ttcaactgtg gctgcttctt ccagaaactg atactttgta gggagcaatt tgatgtgttc      60

tctatatttc cctttccttg aaaacagaga gatcagcagt gaaacagttt tagcttcaag     120

ggagtagctc tttgcatcaa ctttagacat ataatttcca gccttgacca tctctccttt     180

ttccagtaac cttctgatga tatgatttaa catataagag ttggcactac agccgctctt     240

ctccatcgat aaaaaaagat tgtcagcttc ttccactgga ccttctttta taagatttgt     300

catcattatg gtgtaggtaa cagcattagg aaccaagcca ttggctggta tagcagcaaa     360

caaatcctta gcttcttggt ttcgccgaac cctgaagaat gcaccaatca agatattgac     420

aattgcaata ttgaatttga cattcattgt acttaatttc ttggacagcg tgattgcttc     480

accggtgcaa ttatttctac aaagtccacc aagaactata gagtatgtat caatgcacac     540

acttactcca gattcaacca tctcattaaa cttcaccttt gcagcaacag ttcgtccagc     600

cagaaataat ccatccagta tgacgttata attaaaagtt gtaggtttaa gtcccttgcg     660

cagcatctct ttgaatagaa taagcccgtc atcaatcctt ctatttttgc aatagccatt     720

aataagtata ccatacgtat tgttacaagg ttctaaacca tatgacacca tatcatcaaa     780

catttttgtt gcatcctcca tctcgcggac taggcagtat ccaccgatta gtaaattaaa     840

gatgggaaca tcaggcctca tacctatgtg cagcatcaag tcgaggatat cccgtgcttc     900

aattaccctt ccttccgtgc ataggttctg cattattgaa tggaagaact taatatcagg     960

aggacgcata gctttgttcc tcatttcagt aaccaattcc tttgctttca ctaaatcccc    1020

atgtgtacaa aaaccctgga ttagggaatg ataaacagtt gcattcggtc gtactcccat    1080

atcaaccatc tggttaaatt tgtccatagc aatgtccaag ctacccattt tgcaaaatgc    1140

atgtattaca gttgaatagg tgactacatc tggactcact cctcgcttgt gcatttcttt    1200

aaatataagc atagccttat caagcttccc agatttagca tatgcattaa tcagtatgtt    1260

gaagatacga cagttaggta gaatacagtc tgttgccatt gagttgaaga gattgatcat 1320

gtcaactagg catccttcag tggcataccc atgaaggaga atagcatatg aaacggtgtt 1380

aggtttcagg cccttcatgg ccatggagtc aaaaagttct gcagcttctt tgcttcttct 1440

gttgttgcaa agggaggcca tgagcaagtt gcaagtaaaa acatctggta tgataccctt 1500

acttgtcatc tgtttcagca ccctgaccgc ctcctttgaa tggcctgaag aggaatatcc 1560

atggatgagg ctattatatg tgacattatt aggtacgaca ccactatcaa ccatctgacc 1620

aaggaaatac tctgccttgt ccattgctct ggccttgcac agcgcatcaa taaccgagtt 1680

atatgtcacc acattaggcg caacgccctg ctgcgccatt ccatggaaga tatcgcacgc 1740

cttgcctact tggccttccc taaagaagcc atggatgacg gtgttatacg ccaccacgtg 1800

ggtggtgcag ccgagctcag gcatcctgtg cagcagcacg tccagagcct catctgagcg 1860

ctttgcgtgg cagagtccct tgaggagggt gctgaaaacg acttggtctg cctccagacc 1920

cgtcctgagg agacgaccga agaaggcgag tgccatatca gggcggtgtg cgcggcagca 1980

gcagtccatg aggatgctgt aggtgcaagc cgttggatgt gccacacgtc ggagggcgcc 2040

ttgggatatg cggctgaaga gggcaaccgt ggcgacggcc aaggcggcgt ggggatccca 2100

tccctggctg cgtgagggtt gcgaggcgga ggcggagtgg cgggcgtgca ggcggaggcg 2160

gagtggcgac attgacgcgg cataggagaa gccaggcatg ggccttggtg agtaggccgt 2220

gaactctgga gcaagacgag tggggatgta gtgtagtcta gtagtccgga tattcatatc 2280

acatactact actaatttgt aaaagtataa ctttttttga gggttaagta taattttatt 2340

ttgaccaaga aaataaat 2358

<210> 2
<211> 2379
<212> DNA
<213> Artificial Sequence

<220>
<223> cDNA of Rf3 of Primepi

<400> 2
atgcctcgct tctcctccag cacgccaatg tcgccaccac gcctccgcct cctcctcctc 60

ctccgacttg gcgcccgcca ctcctcctcc acctctcatc cctcacgcat ctgggatccc 120

cacgccgcct tcgccgccgc gacgcagcgg gctcgctctg gcacgctcac cacggaggac 180

gcacaccacc tgttcgaaga attgctgcgg caggcaatcc ctgtccagga gcgtcccttg 240

actaactttc tggctgccct tgcccgcgcg cccgcgtccc cattctgcag cgatggcccc 300

gccctggccg tcgccctctt cagccgtttg tcccgaggcg ccggacgacg ggtggcacag 360

ccaaatgtct tcacctatgg agtcctaatg gactgctgct gccgtgcgcg ccgcttggat 420

gtagcgatcg ccttcttcgc ccatctcctc aagacgggac tggaggcagg ccaagtcgtt 480
ttcggcaccc tcctcaaggg actgtgccac gcaaagcact cagatgaggc tttggacgtg 540
gtgcttcaca ggatgcctga gctaggctgc acccccgacg tggtggccta taccacggtc 600
atccacggct tcttgaagga aggccaagta ggcaaggcat gcaatctatt ccatggaatg 660
gcgcagcagg gcgttgcgcc tgatgtggtg acatatagct cagttattga tgcgctgtgc 720
aaggccagag caatggacaa ggcagagtat ttccttcgtg aaatggttga taatggtgtc 780
gtacctaata atgtgacata taatagcctc atccatggat attcctcttt gggccatcag 840
aaggaggctg ttagggtgct gaaagaaatg acaagacagg gtatcatacc tgatgttttt 900
acttgcaact cggtcatggc ctccctttgc aagaacggaa aaagcaaaga agctgcaaaa 960
atgtttgatt caatgtccat gaagggcctg aaacctaaca ccgtttcata tgttattctc 1020
cttcatgggt atgccactga aggatgccta gttgacatga tcaatctctt caattccatg 1080
gcaacagact gtattctacc taactgtcat atattcaaca tactgattta tgcatatgct 1140
aaatctggga agctcgacaa ggctatgctt atatttaaag aaatgcagaa acaaggagtg 1200
agtccagatg cagtcacata ttcaaccgta atacatgcat tttgcaaaaa gggtaggttg 1260
gatgatgctg tgataaagtt taatcagatg attgatacag gagtacgacc ggacgcatct 1320
gtttatcgtt ccctaatcca gggtttttgt acacatggcg atttggtgaa agcaaaggaa 1380
tatgttactg aaatgatgaa gaaaggtatg cttcctcctg atattatgtt cttcagttca 1440
atcatgcaga acctatgcac agaaggaagg gtaatagaag cacgagatat ccttgacttg 1500
atagtgtgca ttggtatgag gcctgatgtt ttcatattta atatactgat cggcggatac 1560
tgcctagtcc gcaagatggc agatgcatca aaaatatttg atgatatggt gtcatatggt 1620
ttagaacctt gtaatattac gtatggtata cttattaatg gctattgcaa aaacggaagg 1680
attgatgacg ggcttattct gttcaaggag atgctgcaca agggacttaa acctacaact 1740
tttaattata acgtcatact ggatggatta tttctggcta gacaaactgt tgctgcaaag 1800
gtgaagtttc atgagatggc tgaatctgga gtaagtgtgt gcattgatac atactctata 1860
gttcttggtg gactttgtag aaataattgc atcggtgaag caatcacgct atttcagaaa 1920
ttaagtacaa tgaatgttaa attcaatatt gcaattgtca atatcatgat tggtgcattc 1980
ttcagggttc ggcgaaacca ggaagctaag gatttgtttg ctgcagtatc agccaatggt 2040
ttggtagcta atgtctttac ctacactgta atgatgataa atcttataga agaagggtca 2100
ctggaagaag ctgacactct ctttttatca atgaagaaga gcggctgtac tgccaactct 2160
tgtatgttaa atcatattat cagaaggtta ctggaaagag gagagatagt caaggctgga 2220
aattatatgt cgaaagttga tgcaaagagc tactcacttg aagctaaaac tgttttgctg 2280
ctgatctctc tcttttcagg gaatgggaaa tatagagaac acataaaatt gctccctatg 2340

aagtatcagt ttctcgaaga agcagccaca gttgaatag 2379

<210> 3
<211> 792
<212> PRT
<213> Triticum aestivum

<400> 3

Met Pro Arg Phe Ser Ser Ser Thr Pro Met Ser Pro Pro Arg Leu Arg
1               5                   10                  15

Leu Leu Leu Leu Leu Arg Leu Gly Ala Arg His Ser Ser Ser Thr Ser
            20                  25                  30

His Pro Ser Arg Ile Trp Asp Pro His Ala Ala Phe Ala Ala Ala Thr
        35                  40                  45

Gln Arg Ala Arg Ser Gly Thr Leu Thr Thr Glu Asp Ala His His Leu
    50                  55                  60

Phe Glu Glu Leu Leu Arg Gln Gly Asn Pro Val Gln Glu Arg Pro Leu
65                  70                  75                  80

Thr Asn Phe Leu Ala Ala Leu Ala Arg Ala Pro Ala Ser Pro Phe Cys
                85                  90                  95

Ser Asp Gly Pro Ala Leu Ala Val Ala Leu Phe Ser Arg Leu Ser Arg
            100                 105                 110

Gly Ala Gly Arg Arg Val Ala Gln Pro Asn Val Phe Thr Tyr Gly Val
        115                 120                 125

Leu Met Asp Cys Cys Cys Arg Ala Arg Arg Leu Asp Val Ala Ile Ala
    130                 135                 140

Phe Phe Ala His Leu Leu Lys Thr Gly Leu Glu Ala Gly Gln Val Val
145                 150                 155                 160

Phe Gly Thr Leu Leu Lys Gly Leu Cys His Ala Lys His Ser Asp Glu
                165                 170                 175

Ala Leu Asp Val Val Leu His Arg Met Pro Glu Leu Gly Cys Thr Pro
            180                 185                 190

Asp Val Val Ala Tyr Thr Thr Val Ile His Gly Phe Leu Lys Glu Gly
        195                 200                 205

Gln Val Gly Lys Ala Cys Asn Leu Phe His Gly Met Ala Gln Gln Gly
    210                 215                 220

Val Ala Pro Asp Val Val Thr Tyr Ser Ser Val Ile Asp Ala Leu Cys
225                 230                 235                 240

Lys Ala Arg Ala Met Asp Lys Ala Glu Tyr Phe Leu Arg Glu Met Val
                245                 250                 255

Asp Asn Gly Val Val Pro Asn Asn Val Thr Tyr Asn Ser Leu Ile His
            260                 265                 270

Gly Tyr Ser Ser Leu Gly His Gln Lys Glu Ala Val Arg Val Leu Lys
        275                 280                 285

Glu Met Thr Arg Gln Gly Ile Ile Pro Asp Val Phe Thr Cys Asn Ser
    290                 295                 300

Val Met Ala Ser Leu Cys Lys Asn Gly Lys Ser Lys Glu Ala Ala Lys
305                 310                 315                 320

Met Phe Asp Ser Met Ser Met Lys Gly Leu Lys Pro Asn Thr Val Ser
                325                 330                 335

Tyr Val Ile Leu Leu His Gly Tyr Ala Thr Glu Gly Cys Leu Val Asp
            340                 345                 350

Met Ile Asn Leu Phe Asn Ser Met Ala Thr Asp Cys Ile Leu Pro Asn
        355                 360                 365

Cys His Ile Phe Asn Ile Leu Ile Tyr Ala Tyr Ala Lys Ser Gly Lys
    370                 375                 380

Leu Asp Lys Ala Met Leu Ile Phe Lys Glu Met Gln Lys Gln Gly Val
385                 390                 395                 400

Ser Pro Asp Ala Val Thr Tyr Ser Thr Val Ile His Ala Phe Cys Lys
                405                 410                 415

Lys Gly Arg Leu Asp Asp Ala Val Ile Lys Phe Asn Gln Met Ile Asp
            420                 425                 430

Thr Gly Val Arg Pro Asp Ala Ser Val Tyr Arg Ser Leu Ile Gln Gly
        435                 440                 445

Phe Cys Thr His Gly Asp Leu Val Lys Ala Lys Glu Tyr Val Thr Glu
    450                 455                 460

Met Met Lys Lys Gly Met Leu Pro Pro Asp Ile Met Phe Phe Ser Ser
465                 470                 475                 480

Ile Met Gln Asn Leu Cys Thr Glu Gly Arg Val Ile Glu Ala Arg Asp
                485                 490                 495

Ile Leu Asp Leu Ile Val Cys Ile Gly Met Arg Pro Asp Val Phe Ile
            500                 505                 510

Phe Asn Ile Leu Ile Gly Gly Tyr Cys Leu Val Arg Lys Met Ala Asp
        515                 520                 525

Ala Ser Lys Ile Phe Asp Asp Met Val Ser Tyr Gly Leu Glu Pro Cys
    530                 535                 540

Asn Ile Thr Tyr Gly Ile Leu Ile Asn Gly Tyr Cys Lys Asn Gly Arg
545                 550                 555                 560

Ile Asp Asp Gly Leu Ile Leu Phe Lys Glu Met Leu His Lys Gly Leu
                565                 570                 575

Lys Pro Thr Thr Phe Asn Tyr Asn Val Ile Leu Asp Gly Leu Phe Leu
            580                 585                 590

Ala Arg Gln Thr Val Ala Ala Lys Val Lys Phe His Glu Met Ala Glu
        595                 600                 605

Ser Gly Val Ser Val Cys Ile Asp Thr Tyr Ser Ile Val Leu Gly Gly
    610                 615                 620

Leu Cys Arg Asn Asn Cys Ile Gly Glu Ala Ile Thr Leu Phe Gln Lys
625                 630                 635                 640

Leu Ser Thr Met Asn Val Lys Phe Asn Ile Ala Ile Val Asn Ile Met
                645                 650                 655

Ile Gly Ala Phe Phe Arg Val Arg Arg Asn Gln Glu Ala Lys Asp Leu
            660                 665                 670

Phe Ala Ala Val Ser Ala Asn Gly Leu Val Ala Asn Val Phe Thr Tyr
        675                 680                 685

Thr Val Met Met Ile Asn Leu Ile Glu Glu Gly Ser Leu Glu Glu Ala
    690                 695                 700

Asp Thr Leu Phe Leu Ser Met Lys Lys Ser Gly Cys Thr Ala Asn Ser
705                 710                 715                 720

Cys Met Leu Asn His Ile Ile Arg Arg Leu Leu Glu Arg Gly Glu Ile
                725                 730                 735

Val Lys Ala Gly Asn Tyr Met Ser Lys Val Asp Ala Lys Ser Tyr Ser
            740                 745                 750

Leu Glu Ala Lys Thr Val Leu Leu Leu Ile Ser Leu Phe Ser Gly Asn
        755                 760                 765

Gly Lys Tyr Arg Glu His Ile Lys Leu Leu Pro Met Lys Tyr Gln Phe
    770                 775                 780

Leu Glu Glu Ala Ala Thr Val Glu
785                 790

<210>  4
<211>  1740
<212>  DNA
<213>  Artificial Sequence

<220>
<223>  cDNA of rf3 of Sperber

<400>  4
atgcaatcta ttccatggaa tggcgcagca gtcgttgcgc ctgatgtggt gacatatagc      60

tcagttattg atgcgctgtg caaggccaga gcaatggaca aggcagagta tttccttcgt     120

gaaatggttg ataatggtgt cgtacctaat aatgtgacat ataatagcct catccatgga     180

tattcctctt tgggccatca gaaggaggct gttagggtgc tgaaagaaat gacaagacag     240

ggtatcatac ctgatgtttt tacttgcaac tcggtcatgg cctccctttg caagaacgga     300

aaaagcaaag aagctgcaaa aatgtttgat tcaatgtcca tgaagggcct gaaacctaac     360

accgtttcat atgttattct ccttcatggg tatgccactg aaggatgcct agttgacatg     420

atcaatctct tcaattccat ggcaacagac tgtattctac ctaactgtca tatattcaac     480

atactgattt atgcatatgc taaatctggg aagctcgaca aggctatgct tatatttaaa     540

gaaatgcaga aacaaggagt gagtccagat gcagtcacat attcaaccgt aatacatgca     600

ttttgcaaaa agggtaggtt ggatgatgct gtgataaagt ttaatcagat gattgataca     660

ggagtacgac tggacgcatc tgtttatcgt tccctaatcc agggtttttg tacacatggc     720

gatttggtga aagcaaagga atatgttact gaaatgatga agaaaggtat gcttcctcct     780

gatattatgt tcttcagttc aatcatgcag aacctatgca cagaaggaag ggtaatagaa     840

gcacgagata tccttgactt gatagtgtgc attggtatga ggcctgatgt tttcatattt     900

aatatactga tcggcggata ctgcctagtc cgcaagatgg cagatgcatc aaaaatattt     960

gatgatatgg tgtcatatgg tttagaacct tgtaatatta cgtatggtat acttattaat 1020

ggctattgca aaaacggaag gattgatgac gggcttattc tgttcaagga gatgctgcac 1080

aagggactta aacctacaac ttttaattat aacgtcatac tggatggatt atttctggct 1140

agacaaactg ttgctgcaaa ggtgaagttt catgagatgg ctgaatctgg agtaagtgtg 1200

tgcattgata catactctat agttcttggt ggactttgta gaaataattg catcggtgaa 1260

gcaatcacgc catttcagaa attaagtaca atgaatgtta aattcaatat tgcaattgtc 1320

aatatcatga ttggtgcatt cttcagggtt tggcgaaacc aggaagctaa ggatttgttt 1380

gctgcagtat cagccaatgg tttggtagct aatgtcttta cctacactgt aatggtgata 1440

aatcttatag aagaagggtc actggaagaa gctgacactc tctttttatc aatgaagaag 1500

agcggctgta ctgccaactc ttgtatgtta aatcatatta tcagaaggtt actggaaaga 1560

ggagagatag tcaaggctgg aaattatatg tcgaaagttg atgcaaagag ctactcactt 1620

gaagctaaaa ctgttttgct gctgatctct ctcttttcag ggaatgggaa atatagagaa 1680

cacataaaat tgctccctat gaagtatcag tttctcgaag aagcagccac agttgaatag 1740

<210> 5
<211> 579
<212> PRT
<213> Triticum aestivum

<400> 5

Met Gln Ser Ile Pro Trp Asn Gly Ala Ala Val Val Ala Pro Asp Val
1               5                   10                  15

Val Thr Tyr Ser Ser Val Ile Asp Ala Leu Cys Lys Ala Arg Ala Met
            20                  25                  30

Asp Lys Ala Glu Tyr Phe Leu Arg Glu Met Val Asp Asn Gly Val Val
        35                  40                  45

Pro Asn Asn Val Thr Tyr Asn Ser Leu Ile His Gly Tyr Ser Ser Leu
    50                  55                  60

Gly His Gln Lys Glu Ala Val Arg Val Leu Lys Glu Met Thr Arg Gln
65                  70                  75                  80

Gly Ile Ile Pro Asp Val Phe Thr Cys Asn Ser Val Met Ala Ser Leu
                85                  90                  95

Cys Lys Asn Gly Lys Ser Lys Glu Ala Ala Lys Met Phe Asp Ser Met
            100                 105                 110

Ser Met Lys Gly Leu Lys Pro Asn Thr Val Ser Tyr Val Ile Leu Leu

115                 120                 125

His Gly Tyr Ala Thr Glu Gly Cys Leu Val Asp Met Ile Asn Leu Phe
    130                 135                 140

Asn Ser Met Ala Thr Asp Cys Ile Leu Pro Asn Cys His Ile Phe Asn
145                 150                 155                 160

Ile Leu Ile Tyr Ala Tyr Ala Lys Ser Gly Lys Leu Asp Lys Ala Met
                165                 170                 175

Leu Ile Phe Lys Glu Met Gln Lys Gln Gly Val Ser Pro Asp Ala Val
            180                 185                 190

Thr Tyr Ser Thr Val Ile His Ala Phe Cys Lys Lys Gly Arg Leu Asp
        195                 200                 205

Asp Ala Val Ile Lys Phe Asn Gln Met Ile Asp Thr Gly Val Arg Leu
    210                 215                 220

Asp Ala Ser Val Tyr Arg Ser Leu Ile Gln Gly Phe Cys Thr His Gly
225                 230                 235                 240

Asp Leu Val Lys Ala Lys Glu Tyr Val Thr Glu Met Met Lys Lys Gly
                245                 250                 255

Met Leu Pro Pro Asp Ile Met Phe Phe Ser Ser Ile Met Gln Asn Leu
            260                 265                 270

Cys Thr Glu Gly Arg Val Ile Glu Ala Arg Asp Ile Leu Asp Leu Ile
        275                 280                 285

Val Cys Ile Gly Met Arg Pro Asp Val Phe Ile Phe Asn Ile Leu Ile
    290                 295                 300

Gly Gly Tyr Cys Leu Val Arg Lys Met Ala Asp Ala Ser Lys Ile Phe
305                 310                 315                 320

Asp Asp Met Val Ser Tyr Gly Leu Glu Pro Cys Asn Ile Thr Tyr Gly
                325                 330                 335

Ile Leu Ile Asn Gly Tyr Cys Lys Asn Gly Arg Ile Asp Asp Gly Leu
            340                 345                 350

Ile Leu Phe Lys Glu Met Leu His Lys Gly Leu Lys Pro Thr Thr Phe
        355                 360                 365

Asn Tyr Asn Val Ile Leu Asp Gly Leu Phe Leu Ala Arg Gln Thr Val
    370                 375                 380

Ala Ala Lys Val Lys Phe His Glu Met Ala Glu Ser Gly Val Ser Val
385                 390                 395                 400

Cys Ile Asp Thr Tyr Ser Ile Val Leu Gly Gly Leu Cys Arg Asn Asn
                405                 410                 415

Cys Ile Gly Glu Ala Ile Thr Pro Phe Gln Lys Leu Ser Thr Met Asn
            420                 425                 430

Val Lys Phe Asn Ile Ala Ile Val Asn Ile Met Ile Gly Ala Phe Phe
        435                 440                 445

Arg Val Trp Arg Asn Gln Glu Ala Lys Asp Leu Phe Ala Ala Val Ser
    450                 455                 460

Ala Asn Gly Leu Val Ala Asn Val Phe Thr Tyr Thr Val Met Val Ile
465                 470                 475                 480

Asn Leu Ile Glu Glu Gly Ser Leu Glu Glu Ala Asp Thr Leu Phe Leu
                485                 490                 495

Ser Met Lys Lys Ser Gly Cys Thr Ala Asn Ser Cys Met Leu Asn His
            500                 505                 510

Ile Ile Arg Arg Leu Leu Glu Arg Gly Glu Ile Val Lys Ala Gly Asn
        515                 520                 525

Tyr Met Ser Lys Val Asp Ala Lys Ser Tyr Ser Leu Glu Ala Lys Thr
    530                 535                 540

Val Leu Leu Leu Ile Ser Leu Phe Ser Gly Asn Gly Lys Tyr Arg Glu
545                 550                 555                 560

His Ile Lys Leu Leu Pro Met Lys Tyr Gln Phe Leu Glu Glu Ala Ala
                565                 570                 575

Thr Val Glu

<210>  6
<211>  1908
<212>  DNA
<213>  Artificial Sequence

<220>
<223>  cDNA of pseudo Rf3 pof Primepi

<400> 6
aagacgggac tggaggcagg ccaagtcgtt ttcggcaccc tcctcaaggg actctgccac 60

gcaaagcact cagatgaggc tttggacgtg gtgcttcaca ggatgcctga gctaggctgc 120

acccccgacg tggtggccta taccacggtc atccacggct tcttgaagga aggccaagta 180

ggcaaggcat gcaatctatt ccatggaatg gcgcagcagg gcgttgcgcc tgatgtggtg 240

acatatagct cagttattga tgcgctgtgc aaggccagag caattgacaa ggcagagtat 300

ttccttcgtg aaatggttga taatggtgtc gtacctaata atgtgacata taatagcctc 360

atccatggat attcctcttt gggccatcag aaggaggctg ttagggtgct gaaagaaatg 420

acaagacagg gtatcatacc tgatgttttt acttgcaact cggtcatggc ctccctttgc 480

aagaacggaa aaagcaaaga agctgcaaaa atgtttgatt caatgtccat gaagggcctg 540

aaacctaaca ccgtttcata tgttattctc cttcatgggt atgccactga aggatgccta 600

gttgacatga tcaatctctt caattccatg gcaacagact gtattcyacc taactgtcat 660

atattcaaca tactgattta tgcatatgct aaatctggga agctcgacaa ggctatgctt 720

atatttaaag aaatgcagaa acaaggagtg agtccagatg cagtcacata ttcaaccgta 780

atacatgcat tttgcaaaaa gggtaggttg gatgatgctg tgataaagtt taatcagatg 840

attgatacag gagtacgacc ggacgcatct gtttatcgtt ccctaatcca gggtgaaagc 900

aaaggaatat gttactgaaa tgatgaagaa aggtatgctt cctcctgata ttatgttctt 960

cagttcaatc atgcagaacc tatgcacaga aggaagggta atagaagcac gagatatcct 1020

tgacttgata gtgtgcattg gtatgaggcc tgatgttttc atatttaata tactgatcgg 1080

cggatactgc ctagtccgca agatggcaga tgcatcaaaa atatttgatg atatggtgtc 1140

atatggttta gaaccttgta atattacgta tggtatactt attaatggyt attgcaaaaa 1200

cggaaggatt gatgacgggc ttattctgtt caaggagatg ctgcacaagg gacttaaacc 1260

tacaactttt aattataacg tcattctgga tggattattt ctggctagac aaactgttgc 1320

ttcaaaggtg aagtttcatg agatggctga atctggagta agtgtgtgca ttgatacata 1380

ctctatagtt cttggtggac tttgtagaaa taattgcatc ggtgaagcaa tcacgctatt 1440

tcagaaatta agtacaatga atgttaaatt caatattgca attgtcaata tcatgattgg 1500

tgcattcttc agggttcggc gaaaccagga agctaaggat ttgtttgctg cagtatcagc 1560

caatggtttg gtagctaatg tctttaccta cactgtaatg atgataaatc ttatagaaga 1620

agggtcactg gaagaagctg acactctctt tttatcaatg aagaagagcg gctgtactgc 1680

caactcttgt atgttaaatc atattatcag aaggttactg gaaagaggag agatagtcaa 1740

ggctggaaat tatatgtcga aagttgatgc aaagagctac tcacttgaag ctaaaactgt 1800

tttgctgctg atctctctct tttcagggaa tgggaaatat agagaacaca taaaattgct 1860

ccctatgaag tatcagtttc tcgaagaagc agccacagtt gaatagtt                    1908

<210>  7
<211>  633
<212>  PRT
<213>  Triticum aestivum

<220>
<221>  misc_feature
<222>  (216)..(216)
<223>  Xaa can be any naturally occurring amino acid

<220>
<221>  misc_feature
<222>  (395)..(395)
<223>  Xaa can be any naturally occurring amino acid

<400>  7

Lys Thr Gly Leu Glu Ala Gly Gln Val Val Phe Gly Thr Leu Leu Lys
1               5                   10                  15

Gly Leu Cys His Ala Lys His Ser Asp Glu Ala Leu Asp Val Val Leu
            20                  25                  30

His Arg Met Pro Glu Leu Gly Cys Thr Pro Asp Val Val Ala Tyr Thr
        35                  40                  45

Thr Val Ile His Gly Phe Leu Lys Glu Gly Gln Val Gly Lys Ala Cys
    50                  55                  60

Asn Leu Phe His Gly Met Ala Gln Gln Gly Val Ala Pro Asp Val Val
65                  70                  75                  80

Thr Tyr Ser Ser Val Ile Asp Ala Leu Cys Lys Ala Arg Ala Ile Asp
                85                  90                  95

Lys Ala Glu Tyr Phe Leu Arg Glu Met Val Asp Asn Gly Val Val Pro
            100                 105                 110

Asn Asn Val Thr Tyr Asn Ser Leu Ile His Gly Tyr Ser Ser Leu Gly
        115                 120                 125

His Gln Lys Glu Ala Val Arg Val Leu Lys Glu Met Thr Arg Gln Gly
    130                 135                 140

Ile Ile Pro Asp Val Phe Thr Cys Asn Ser Val Met Ala Ser Leu Cys
145                 150                 155                 160

Lys Asn Gly Lys Ser Lys Glu Ala Ala Lys Met Phe Asp Ser Met Ser

                165                 170                 175

Met Lys Gly Leu Lys Pro Asn Thr Val Ser Tyr Val Ile Leu Leu His
            180                 185                 190

Gly Tyr Ala Thr Glu Gly Cys Leu Val Asp Met Ile Asn Leu Phe Asn
        195                 200                 205

Ser Met Ala Thr Asp Cys Ile Xaa Pro Asn Cys His Ile Phe Asn Ile
    210                 215                 220

Leu Ile Tyr Ala Tyr Ala Lys Ser Gly Lys Leu Asp Lys Ala Met Leu
225                 230                 235                 240

Ile Phe Lys Glu Met Gln Lys Gln Gly Val Ser Pro Asp Ala Val Thr
                245                 250                 255

Tyr Ser Thr Val Ile His Ala Phe Cys Lys Lys Gly Arg Leu Asp Asp
            260                 265                 270

Ala Val Ile Lys Phe Asn Gln Met Ile Asp Thr Gly Val Arg Pro Asp
        275                 280                 285

Ala Ser Val Tyr Arg Ser Leu Ile Gln Gly Glu Ser Lys Gly Ile Cys
    290                 295                 300

Tyr Met Met Lys Lys Gly Met Leu Pro Pro Asp Ile Met Phe Phe Ser
305                 310                 315                 320

Ser Ile Met Gln Asn Leu Cys Thr Glu Gly Arg Val Ile Glu Ala Arg
                325                 330                 335

Asp Ile Leu Asp Leu Ile Val Cys Ile Gly Met Arg Pro Asp Val Phe
            340                 345                 350

Ile Phe Asn Ile Leu Ile Gly Gly Tyr Cys Leu Val Arg Lys Met Ala
        355                 360                 365

Asp Ala Ser Lys Ile Phe Asp Asp Met Val Ser Tyr Gly Leu Glu Pro
    370                 375                 380

Cys Asn Ile Thr Tyr Gly Ile Leu Ile Asn Xaa Tyr Cys Lys Asn Gly
385                 390                 395                 400

Arg Ile Asp Asp Gly Leu Ile Leu Phe Lys Glu Met Leu His Lys Gly
                405                 410                 415

Leu Lys Pro Thr Thr Phe Asn Tyr Asn Val Ile Leu Asp Gly Leu Phe
            420                 425                 430

Leu Ala Arg Gln Thr Val Ala Ser Lys Val Lys Phe His Glu Met Ala
        435                 440                 445

Glu Ser Gly Val Ser Val Cys Ile Asp Thr Tyr Ser Ile Val Leu Gly
    450                 455                 460

Gly Leu Cys Arg Asn Asn Cys Ile Gly Glu Ala Ile Thr Leu Phe Gln
465                 470                 475                 480

Lys Leu Ser Thr Met Asn Val Lys Phe Asn Ile Ala Ile Val Asn Ile
                485                 490                 495

Met Ile Gly Ala Phe Phe Arg Val Arg Arg Asn Gln Glu Ala Lys Asp
            500                 505                 510

Leu Phe Ala Ala Val Ser Ala Asn Gly Leu Val Ala Asn Val Phe Thr
        515                 520                 525

Tyr Thr Val Met Met Ile Asn Leu Ile Glu Glu Gly Ser Leu Glu Glu
    530                 535                 540

Ala Asp Thr Leu Phe Leu Ser Met Lys Lys Ser Gly Cys Thr Ala Asn
545                 550                 555                 560

Ser Cys Met Leu Asn His Ile Ile Arg Arg Leu Leu Glu Arg Gly Glu
                565                 570                 575

Ile Val Lys Ala Gly Asn Tyr Met Ser Lys Val Asp Ala Lys Ser Tyr
            580                 585                 590

Ser Leu Glu Ala Lys Thr Val Leu Leu Leu Ile Ser Leu Phe Ser Gly
        595                 600                 605

Asn Gly Lys Tyr Arg Glu His Ile Lys Leu Leu Pro Met Lys Tyr Gln
    610                 615                 620

Phe Leu Glu Glu Ala Ala Thr Val Glu
625                 630

<210>  8
<211>  2331
<212>  DNA
<213>  Artificial Sequence

<220>
<223>  cDNA of pseudo Rf3 of Chinese Spring

<400> 8
atgcaaaaaa tgagtactac tagtgtactg gtactagata gcagaaagtt gccacctagg 60
tacaacttgt cttgctccgg acctacctac acaccaaggc ccatgcctcg cttctcctcc 120
accacgtcaa tgtcgtcact ccgcctccgc ctccccctcc gcctgcacgc ccgccactcc 180
tcctccgcct ctcaaccctc acacagccag ggctgggatc cccacgccgc cttcgccgcc 240
gccacggagc gtgcgcgctc tggcagcctc accccggcgg acgcacacca cctgttcgac 300
gaattgctgc ggcaggccaa tcctgtccag gagcgccccc tcaacaactt tctcgccgcc 360
ctcgcccgcg cgccggccct cacggtcgcc ctcttcagcc tcatatccaa aggcgcccgc 420
cgacgggtgg cgcagccaaa tgtcttcacc tatggcgtcc ttatggactg ctgctgccgt 480
gcacaccgcc cggatctggc actagcgttc ttcggccgtt tcctcaggac gggcctggag 540
gcagaccaag tcgtcttcaa caccctcctc aagggccttt gccatgcaaa gcaggcagat 600
gaggccgtga acgtgcttct tcacaggatg cctgacctgg gctgcactcc caatgtggtg 660
gcgtacaaca ccgtcatcca tggcttcttt agggaaggcc aagtaggcaa ggcatgcgat 720
atgttccatg gaatggcaca taagggcgtt atgcctaatg tggtgacata taactcggtt 780
attgatgcac tgtgcaaggc cagggccatg gacaaagcag agacggtcct tggtcaaatg 840
gtaaatgatg gtgtcggacc taataatgtc acatataata gcctcatcca tggatattcc 900
tctttgggta atttgaagga ggcggttagg gtattcaaag agatgagtcg gaggtttaca 960
ccagatgtcg ttacctgcac ctcactcatg gccttccttt gcaagaatgg aaaaagcaaa 1020
gaagctgcag aaatttttga ttcaatggcc accaagggcc tcaaacgtga caccgtttca 1080
tatgctattc tccttcatgg gtatgccact gaaggatgct tggttgacat gattaatctc 1140
ttcaattcaa tggcaacaga ctgtattcta cctaactgtc atatcttcaa tatactgatt 1200
aatgcatatg ctaaatctgg gaagcttgat aaggctatgc ttatcttcaa tgaaatgcag 1260
aaacaaggag tgagtccaga tttaaccagt ccggggtttt tgtacacatg gggatttagt 1320
gaaagcaaag gaattggtta ctgaatgagg aacaaagcta tgcgtcctcc tcatattaag 1380
ttcttccatt caataatgca gaacctatgc acggaaggaa gggtaataga agcacgggat 1440
atcctcgact tgatgctgca cataggtatg aggcctgatg ttcccatctt taatttacta 1500
atcggtggat actgcctagt ccgcgagatg gaggatgcaa caaaaatgtt tgatgatatg 1560
gtgtcatatg gtttagaacc ttgtaacaat acgtatggta tacttattaa tggctattgc 1620
aaaaatagaa ggattgatga cgggcttatt ctattcaaag agatgctgcg caagggactt 1680
aaacctacaa cttttaatta taacgtcata ctggatggat tatttctggc tggacgaact 1740
gttgctgcaa aggtgaagtt taatgagatg gttgaatctg gagtaagtgt gtgcattgat 1800
acatactcta tagttcttgg tggactttgt agaaataatt gcaccggtga agcaatcacg 1860

ctgtccaaga aattaagtac aatgaatgtc aaattcaata ttgcaattgt caatatcttg 1920

attggtgcat tcttcagggt tcggcgaaac caagaagcta aggatttgtt tgctgctata 1980

ccagccaatg gcttggttcc taatgctgtt acctacacca taatgatgac aaatcttata 2040

aaagaaggtt cagtggaaga agctgacact ctctttttat caatggagaa gagcgggtgt 2100

actgccaact cttgtatgtt aaatcatatt atcagaaggt tactggaaag aggagagata 2160

gtcaaggctg gaaattatat gtcgaaagtt gatgcaaaga gctactcact tgaagctaaa 2220

actgtttcgc tgctgatctc cctcttgtca gggaaaggga aatatagaga acacataaaa 2280

ttgctcccta tgaagtatca gtttctcgaa gaagcagcca cagttgaata g 2331

<210> 9
<211> 808
<212> PRT
<213> Triticum aestivum

<400> 9

Met Gln Lys Met Ser Thr Thr Ser Val Leu Val Leu Asp Ser Arg Lys
1               5                   10                  15

Leu Pro Pro Arg Tyr Asn Leu Ser Cys Ser Gly Pro Thr Tyr Thr Pro
            20                  25                  30

Arg Pro Met Pro Arg Phe Ser Ser Thr Thr Ser Met Ser Ser Leu Arg
        35                  40                  45

Leu Arg Leu Pro Leu Arg Leu His Ala Arg His Ser Ser Ser Ala Ser
    50                  55                  60

Gln Pro Ser His Ser Gln Gly Trp Asp Pro His Ala Ala Phe Ala Ala
65                  70                  75                  80

Ala Thr Glu Arg Ala Arg Ser Gly Ser Leu Thr Pro Ala Asp Ala His
                85                  90                  95

His Leu Phe Asp Glu Leu Leu Arg Gln Ala Asn Pro Val Gln Glu Arg
            100                 105                 110

Pro Leu Asn Asn Phe Leu Ala Ala Leu Ala Arg Ala Pro Ala Leu Thr
        115                 120                 125

Val Ala Leu Phe Ser Leu Ile Ser Lys Gly Ala Arg Arg Arg Val Ala
    130                 135                 140

Gln Pro Asn Val Phe Thr Tyr Gly Val Leu Met Asp Cys Cys Cys Arg
145                 150                 155                 160

Ala His Arg Pro Asp Leu Ala Leu Ala Phe Phe Gly Arg Phe Leu Arg
                165                 170                 175

Thr Gly Leu Glu Ala Asp Gln Val Val Phe Asn Thr Leu Leu Lys Gly
            180                 185                 190

Leu Cys His Ala Lys Gln Ala Asp Glu Ala Val Asn Val Leu Leu His
        195                 200                 205

Arg Met Pro Asp Leu Gly Cys Thr Pro Asn Val Val Ala Tyr Asn Thr
    210                 215                 220

Val Ile His Gly Phe Phe Arg Glu Gly Gln Val Gly Lys Ala Cys Asp
225                 230                 235                 240

Met Phe His Gly Met Ala His Lys Gly Val Met Pro Asn Val Val Thr
                245                 250                 255

Tyr Asn Ser Val Ile Asp Ala Leu Cys Lys Ala Arg Ala Met Asp Lys
            260                 265                 270

Ala Glu Thr Val Leu Gly Gln Met Val Asn Asp Gly Val Gly Pro Asn
        275                 280                 285

Asn Val Thr Tyr Asn Ser Leu Ile His Gly Tyr Ser Ser Leu Gly Asn
    290                 295                 300

Leu Lys Glu Ala Val Arg Val Phe Lys Glu Met Ser Arg Arg Phe Thr
305                 310                 315                 320

Pro Asp Val Val Thr Cys Thr Ser Leu Met Ala Phe Leu Cys Lys Asn
                325                 330                 335

Gly Lys Ser Lys Glu Ala Ala Glu Ile Phe Asp Ser Met Ala Thr Lys
            340                 345                 350

Gly Leu Lys Arg Asp Thr Val Ser Tyr Ala Ile Leu Leu His Gly Tyr
        355                 360                 365

Ala Thr Glu Gly Cys Leu Val Asp Met Ile Asn Leu Phe Asn Ser Met
    370                 375                 380

Ala Thr Asp Cys Ile Leu Pro Asn Cys His Ile Phe Asn Ile Leu Ile
385                 390                 395                 400

Asn Ala Tyr Ala Lys Ser Gly Lys Leu Asp Lys Ala Met Leu Ile Phe

405                 410                 415

Asn Glu Met Gln Lys Gln Gly Val Ser Pro Asp Leu Thr Arg Trp Leu
            420                 425                 430

Ile Trp Glu Tyr Asp Arg Met Gln Leu Phe Ile Ile Pro Met Val Asp
        435                 440                 445

Met Gly Val Arg Pro Asn Ala Ile Val Tyr His Ser Leu Ile Arg Gly
    450                 455                 460

Phe Cys Thr His Gly Asp Leu Val Lys Ala Lys Glu Leu Val Thr Glu
465                 470                 475                 480

Met Arg Asn Lys Ala Met Arg Pro Pro His Ile Lys Phe Phe His Ser
                485                 490                 495

Ile Met Gln Asn Leu Cys Thr Glu Gly Arg Val Ile Glu Ala Arg Asp
            500                 505                 510

Ile Leu Asp Leu Met Leu His Ile Gly Met Arg Pro Asp Val Pro Ile
        515                 520                 525

Phe Asn Leu Leu Ile Gly Gly Tyr Cys Leu Val Arg Glu Met Glu Asp
    530                 535                 540

Ala Thr Lys Met Phe Asp Asp Met Val Ser Tyr Gly Leu Glu Pro Cys
545                 550                 555                 560

Asn Asn Thr Tyr Gly Ile Leu Ile Asn Gly Tyr Cys Lys Asn Arg Arg
                565                 570                 575

Ile Asp Asp Gly Leu Ile Leu Phe Lys Glu Met Leu Arg Lys Gly Leu
            580                 585                 590

Lys Pro Thr Thr Phe Asn Tyr Asn Val Ile Leu Asp Gly Leu Phe Leu
        595                 600                 605

Ala Gly Arg Thr Val Ala Ala Lys Val Lys Phe Asn Glu Met Val Glu
    610                 615                 620

Ser Gly Val Ser Val Cys Ile Asp Thr Tyr Ser Ile Val Leu Gly Gly
625                 630                 635                 640

Leu Cys Arg Asn Asn Cys Thr Gly Glu Ala Ile Thr Leu Ser Lys Lys
                645                 650                 655

Leu Ser Thr Met Asn Val Lys Phe Asn Ile Ala Ile Val Asn Ile Leu
            660                 665                 670

Ile Gly Ala Phe Phe Arg Val Arg Arg Asn Gln Glu Ala Lys Asp Leu
        675                 680                 685

Phe Ala Ala Ile Pro Ala Asn Gly Leu Val Pro Asn Ala Val Thr Tyr
    690                 695                 700

Thr Ile Met Met Thr Asn Leu Ile Lys Glu Gly Ser Val Glu Glu Ala
705                 710                 715                 720

Asp Thr Leu Phe Leu Ser Met Glu Lys Ser Gly Cys Thr Ala Asn Ser
                725                 730                 735

Cys Met Leu Asn His Ile Ile Arg Arg Leu Leu Glu Arg Gly Glu Ile
            740                 745                 750

Val Lys Ala Gly Asn Tyr Met Ser Lys Val Asp Ala Lys Ser Tyr Ser
        755                 760                 765

Leu Glu Ala Lys Thr Val Ser Leu Leu Ile Ser Leu Leu Ser Gly Lys
    770                 775                 780

Gly Lys Tyr Arg Glu His Ile Lys Leu Leu Pro Met Lys Tyr Gln Phe
785                 790                 795                 800

Leu Glu Glu Ala Ala Thr Val Glu
                805

<210>  10
<211>  2358
<212>  DNA
<213>  Triticum aestivum

<400>  10
ttcaactgtg gctgcttctt ccagaaactg atactttgta gggagcaatt tgatgtgttc      60

tctatatttc cctttccttg aaaacagaga gatcagcagt gaaacagttt tagcttcaag     120

ggagtagctc tttgcatcaa ctttagacat ataatttcca gccttgacca tctctccttt     180

ttccagtaac cttctgatga tatgatttaa catataagag ttggcactac agccgctctt     240

ctccatcgat aaaaaaagat tgtcagcttc ttccactgga ccttctttta taagatttgt     300

catcattatg gtgtaggtaa cagcattagg aaccaagcca ttggctggta tagcagcaaa     360

caaatcctta gcttcttggt ttcgccgaac cctgaagaat gcaccaatca agatattgac     420

aattgcaata ttgaatttga cattcattgt acttaatttc ttggacagcg tgattgcttc     480

accggtgcaa ttatttctac aaagtccacc aagaactata gagtatgtat caatgcacac     540

acttactcca gattcaacca tctcattaaa cttcaccttt gcagcaacag ttcgtccagc 600
cagaaataat ccatccagta tgacgttata attaaaagtt gtaggtttaa gtcccttgcg 660
cagcatctct ttgaatagaa taagcccgtc atcaatcctt ctatttttgc aatagccatt 720
aataagtata ccatacgtat tgttacaagg ttctaaacca tatgacacca tatcatcaaa 780
catttttgtt gcatcctcca tctcgcggac taggcagtat ccaccgatta gtaaattaaa 840
gatgggaaca tcaggcctca tacctatgtg cagcatcaag tcgaggatat cccgtgcttc 900
aattaccctt ccttccgtgc ataggttctg cattattgaa tggaagaact taatatcagg 960
aggacgcata gctttgttcc tcatttcagt aaccaattcc tttgctttca ctaaatcccc 1020
atgtgtacaa aaaccctgga ttagggaatg ataaacagtt gcattcggtc gtactcccat 1080
atcaaccatc tggttaaatt tgtccatagc aatgtccaag ctacccattt tgcaaaatgc 1140
atgtattaca gttgaatagg tgactacatc tggactcact cctcgcttgt gcatttcttt 1200
aaatataagc atagccttat caagcttccc agatttagca tatgcattaa tcagtatgtt 1260
gaagatacga cagttaggta gaatacagtc tgttgccatt gagttgaaga gattgatcat 1320
gtcaactagg catccttcag tggcataccc atgaaggaga atagcatatg aaacggtgtt 1380
aggtttcagg cccttcatgg ccatggagtc aaaaagttct gcagcttctt tgcttcttct 1440
gttgttgcaa agggaggcca tgagcaagtt gcaagtaaaa acatctggta tgataccctt 1500
acttgtcatc tgtttcagca ccctgaccgc ctcctttgaa tggcctgaag aggaatatcc 1560
atggatgagg ctattatatg tgacattatt aggtacgaca ccactatcaa ccatctgacc 1620
aaggaaatac tctgccttgt ccattgctct ggccttgcac agcgcatcaa taaccgagtt 1680
atatgtcacc acattaggcg caacgccctg ctgcgccatt ccatggaaga tatcgcacgc 1740
cttgcctact tggccttccc taaagaagcc atggatgacg gtgttatacg ccaccacgtg 1800
ggtggtgcag ccgagctcag gcatcctgtg cagcagcacg tccagagcct catctgagcg 1860
ctttgcgtgg cagagtccct tgaggagggt gctgaaaacg acttggtctg cctccagacc 1920
cgtcctgagg agacgaccga agaaggcgag tgccatatca gggcggtgtg cgcggcagca 1980
gcagtccatg aggatgctgt aggtgcaagc cgttggatgt gccacacgtc ggagggcgcc 2040
ttgggatatg cggctgaaga gggcaaccgt ggcgacggcc aaggcggcgt ggggatccca 2100
tccctggctg cgtgagggtt gcgaggcgga ggcggagtgg cgggcgtgca ggcggaggcg 2160
gagtggcgac attgacgcgg cataggagaa gccaggcatg ggccttggtg agtaggccgt 2220
gaactctgga gcaagacgag tggggatgta gtgtagtcta gtagtccgga tattcatatc 2280
acatactact actaatttgt aaaagtataa ctttttttga gggttaagta taattttatt 2340
ttgaccaaga aaataaat 2358

<210>  11
<211>  759
<212>  PRT
<213>  Triticum aestivum

<400>  11

Met Asn Ile Arg Thr Thr Arg Leu His Tyr Ile Pro Thr Arg Leu Ala
1               5                   10                  15

Pro Glu Phe Thr Ala Tyr Ser Pro Arg Pro Met Pro Gly Phe Ser Tyr
            20                  25                  30

Ala Ala Ser Met Ser Pro Leu Arg Leu Arg Leu His Ala Arg His Ser
        35                  40                  45

Ala Ser Ala Ser Gln Pro Ser Arg Ser Gln Gly Trp Asp Pro His Ala
    50                  55                  60

Ala Leu Ala Val Ala Thr Val Ala Leu Phe Ser Arg Ile Ser Gln Gly
65                  70                  75                  80

Ala Leu Arg Arg Val Ala His Pro Thr Ala Cys Thr Tyr Ser Ile Leu
                85                  90                  95

Met Asp Cys Cys Cys Arg Ala His Arg Pro Asp Met Ala Leu Ala Phe
            100                 105                 110

Phe Gly Arg Leu Leu Arg Thr Gly Leu Glu Ala Asp Gln Val Val Phe
        115                 120                 125

Ser Thr Leu Leu Lys Gly Leu Cys His Ala Lys Arg Ser Asp Glu Ala
    130                 135                 140

Leu Asp Val Leu Leu His Arg Met Pro Glu Leu Gly Cys Thr Thr His
145                 150                 155                 160

Val Val Ala Tyr Asn Thr Val Ile His Gly Phe Phe Arg Glu Gly Gln
                165                 170                 175

Val Gly Lys Ala Cys Asp Ile Phe His Gly Met Ala Gln Gln Gly Val
            180                 185                 190

Ala Pro Asn Val Val Thr Tyr Asn Ser Val Ile Asp Ala Leu Cys Lys
        195                 200                 205

Ala Arg Ala Met Asp Lys Ala Glu Tyr Phe Leu Gly Gln Met Val Asp
    210                 215                 220

Ser Gly Val Val Pro Asn Asn Val Thr Tyr Asn Ser Leu Ile His Gly
225                 230                 235                 240

Tyr Ser Ser Ser Gly His Ser Lys Glu Ala Val Arg Val Leu Lys Gln
                245                 250                 255

Met Thr Ser Lys Gly Ile Ile Pro Asp Val Phe Thr Cys Asn Leu Leu
            260                 265                 270

Met Ala Ser Leu Cys Asn Asn Arg Arg Ser Lys Glu Ala Ala Glu Leu
        275                 280                 285

Phe Asp Ser Met Ala Met Lys Gly Leu Lys Pro Asn Thr Val Ser Tyr
    290                 295                 300

Ala Ile Leu Leu His Gly Tyr Ala Thr Glu Gly Cys Leu Val Asp Met
305                 310                 315                 320

Ile Asn Leu Phe Asn Ser Met Ala Thr Asp Cys Ile Leu Pro Asn Cys
                325                 330                 335

Arg Ile Phe Asn Ile Leu Ile Asn Ala Tyr Ala Lys Ser Gly Lys Leu
            340                 345                 350

Asp Lys Ala Met Leu Ile Phe Lys Glu Met His Lys Arg Gly Val Ser
        355                 360                 365

Pro Asp Val Val Thr Tyr Ser Thr Val Ile His Ala Phe Cys Lys Met
    370                 375                 380

Gly Ser Leu Asp Ile Ala Met Asp Lys Phe Asn Gln Met Val Asp Met
385                 390                 395                 400

Gly Val Arg Pro Asn Ala Thr Val Tyr His Ser Leu Ile Gln Gly Phe
                405                 410                 415

Cys Thr His Gly Asp Leu Val Lys Ala Lys Glu Leu Val Thr Glu Met
            420                 425                 430

Arg Asn Lys Ala Met Arg Pro Pro Asp Ile Lys Phe Phe His Ser Ile
        435                 440                 445

Met Gln Asn Leu Cys Thr Glu Gly Arg Val Ile Glu Ala Arg Asp Ile
    450                 455                 460

Leu Asp Leu Met Leu His Ile Gly Met Arg Pro Asp Val Pro Ile Phe
465                 470                 475                 480

Asn Leu Leu Ile Gly Gly Tyr Cys Leu Val Arg Glu Met Glu Asp Ala
                485                 490                 495

Thr Lys Met Phe Asp Asp Met Val Ser Tyr Gly Leu Glu Pro Cys Asn
            500                 505                 510

Asn Thr Tyr Gly Ile Leu Ile Asn Gly Tyr Cys Lys Asn Arg Arg Ile
        515                 520                 525

Asp Asp Gly Leu Ile Leu Phe Lys Glu Met Leu Arg Lys Gly Leu Lys
    530                 535                 540

Pro Thr Thr Phe Asn Tyr Asn Val Ile Leu Asp Gly Leu Phe Leu Ala
545                 550                 555                 560

Gly Arg Thr Val Ala Ala Lys Val Lys Phe Asn Glu Met Val Glu Ser
                565                 570                 575

Gly Val Ser Val Cys Ile Asp Thr Tyr Ser Ile Val Leu Gly Gly Leu
            580                 585                 590

Cys Arg Asn Asn Cys Thr Gly Glu Ala Ile Thr Leu Ser Lys Lys Leu
        595                 600                 605

Ser Thr Met Asn Val Lys Phe Asn Ile Ala Ile Val Asn Ile Leu Ile
    610                 615                 620

Gly Ala Phe Phe Arg Val Arg Arg Asn Gln Glu Ala Lys Asp Leu Phe
625                 630                 635                 640

Ala Ala Ile Pro Ala Asn Gly Leu Val Pro Asn Ala Val Thr Tyr Thr
                645                 650                 655

Ile Met Met Thr Asn Leu Ile Lys Glu Gly Pro Val Glu Glu Ala Asp
            660                 665                 670

Asn Leu Phe Leu Ser Met Glu Lys Ser Gly Cys Ser Ala Asn Ser Tyr
        675                 680                 685

Met Leu Asn His Ile Ile Arg Arg Leu Leu Glu Lys Gly Glu Met Val
    690                 695                 700

Lys Ala Gly Asn Tyr Met Ser Lys Val Asp Ala Lys Ser Tyr Ser Leu
705                 710                 715                 720

Glu Ala Lys Thr Val Ser Leu Leu Ile Ser Leu Phe Ser Arg Lys Gly

725                 730                 735

Lys Tyr Arg Glu His Ile Lys Leu Leu Pro Thr Lys Tyr Gln Phe Leu
            740                 745                 750

Glu Glu Ala Ala Thr Val Glu
        755

<210>  12
<211>  2271
<212>  DNA
<213>  Triticum aestivum

<400>  12
actttcgaca tataatttcc agccttgact atctctcctc ttcccagtaa ccttctgata      60

atatgattta acatccaaga gtttgcagta cagccgctct tctccattga taaaaagagt     120

gtcagcttct tccactgaac cttcttttat aagatttttc atcattatgc tgtaggtaac     180

aacattagct accaagccat tggatggtat tgcagcaaac aaatccttag cttcttggtt     240

tcgccgaacc ttgtagaagg catcaatcat gatagtgaca attctaatat tgaatttcat     300

gttcattgcc cttaatttct ggaacagcat gattacttcg ctagagcaat tatttctgca     360

aagtccagca agaactatcg aatatgtatc agtgcacaca cttactccag attcaaccat     420

ctcatcaaac ttttcctttg cagcaacagt tcgtccagcc agaaataacc catccagtat     480

gacattataa ttaaaagttg taggtttaag tcccttgtgc agcatctctt ggaacagaat     540

aagcccatca tcaatccttc tatttttgca atagccatta ataagtatac catacgtaat     600

attacaaggt tctaaaccaa atgacaccat atcctcaaac atttttgttg catcctccat     660

cttgcagact aggcagtacc caccgatcag tatattaaac atgaaaacat taggcctcat     720

accaatgcgc actatcaagt caaggatatc tcgtgcttct attacccttc cttctgtgca     780

tagtttctgc atgattgaac tgaagaacat aatatcagga ggaagcatac ctttcttcat     840

catttcagta acatattcct ttgctttcac caaatcgcca tgtgtacaaa aaccctggat     900

tagggaacga taagcagatg cttccggtcg tactcctgta tcaatcatct gattaaactt     960

tatcacagca tcatccaacc tacccttttt gcaaaatgca tgtattacgg ttgaatatgt    1020

gactgcatct ggactcactc cttgtttctg catatcttta aatataagca tagccttatc    1080

aagcttccca gatttagcat atgcataaat cagtatgttg aagatatgac agttaggtag    1140

aatacagtct gtagccattg aattgaagag attgatcatg tcaaccaagc atccttcagt    1200

ggcataccca tgaaggagaa tagcatatga aatggtgtta ggtttcaggc ccttcatgga    1260

cattgaatta aaaatttctg cagcttcttt gcttcttcca ttcttgcaaa gggaggccat    1320

gagcgagttg caagtaaaaa catctggtat gataccctta cttgtcatct ctttcagcac    1380

cctgaccgcc tcctttgaat ggcctgaaga ggaatatcca tggatgaggc tattatatgt 1440

cacattgtta ggtacgacac cactatcaac catctgacca aggaaatact ccgccttgtc 1500

cattgctctg gccttgcaca gtgcatcaat aaccgagtta tatgtcacca cgtcaggcgc 1560

aacgccctgc tgcgccattc catggaacag attgcatgcc ttgcctactt ggccttcctt 1620

gaagaagcca tggatgaccg tgctatacgc caccacgtca ggggtgcagc ccagctcagg 1680

catcctgtga agcagcacgt ccagagcctc atctgagcgc tttgcgtggc agagtccctt 1740

gaggagggtg ctgaaaacga cttggtctgc ctccaggccc gtcttgagga gacggccgaa 1800

gaaggcgatc gctagatcca ggcggcgcgc acggcagtag cagtccatga ggacgccata 1860

ggtgaagaca tttggctgcg ccacccgtcg tccggcgcct cgggacaaac ggccgaagag 1920

ggcgacggcc agggcagggc cattgctgaa gaatgcggac gcggacgcgc gggcgagggc 1980

agccagaaag ttagtcaagg gacgctcctg gacaggattg ccctgccgca gcaattcatc 2040

aaacaggtgg tgtgcgtcct ccgtggtgag cgtgccagag cgcgcccgct gcgtcgcggc 2100

gacgaaggcg gcgtggggat cccagatgcg tgagggatga gaggtggagg aggagtggcg 2160

ggcgcagagg aggaggagga ggcggggtgg cgacatcggc gtggtggagg agaagcgagg 2220

catggccttg gtgtgcagtg taggtagatc ctgaactggg agacgacggg g 2271

<210> 13
<211> 2226
<212> DNA
<213> Artificial Sequence

<220>
<223> cDNA of Rf1-2 of R3

<400> 13
atgcctcgct tctccaccac cacgccaatg tcgccacccc gcctcctcct ccgactcggc 60

gcccgccact cctcctccac ctctcatccc tcacgcatct gggatcccca cgccgccttc 120

gccgctgcga cgcagcgggc gcgctctggc acgctcacca cggaggacgc acaccacctg 180

tttgatgaat tgctgcggca gggcaatcct gtccaggagc gtcccttgac taactttctg 240

gctgccctcg cccgcgcgcc cgcgtccgca ttctgcagcg atggccctgc cctggccgtc 300

gccctcttcg gccgtttgtc ccgaggcgcc ggacgacggg tggcgcagcc aaatgtcttc 360

acctatggcg tcctcatgga ctgctgctgc cgtgcgcgcc gcctggatct agcgatcgcc 420

ttcttcgccc gtctcctcaa gacgggactg gaggcaaacc aagtcatctt ctgcaccctc 480

ctcaagggac tctgccacgc aaagcgctca gatgaggctt tggacgtggt gcttcacagg 540

atgcctgagc taggctgcac ccccaacgtg gtggcctata ccacggtcat ccacggcttc 600

ttgaaggaag gccaagtagg caaggcatgc aatctattcc atggaatggc gcagcagggc 660

gttgcgcctg atgtggtgac atataactcg gttatcgatg cgttgtgcaa ggccagagca 720

atggacaagg cagagtattt cctttgtgaa atggttgata atggtgtcgt acctaataat 780

gtgacatata atagcctcat ccatggatat tcctctttgg gccatcagaa ggaggctgtt 840

agggtgctga aagaaatgac aagacagggt atcataccag atgtcattac ctgcacctca 900

ctcatgacct tcctttgcaa gaatggaaaa agcaaggaag ctgcagaaat ttttgattca 960

atgcccacga agggcctgaa acatgacgcc gtttcatatg ctattctcct tcatgggtat 1020

gccactgaag gatgcttggt tgatatgatt aatctcttca attcgatgga cagagactgt 1080

attctaccta actgtcatat cttcaacata ctgatttatg catatgctaa atctgggaag 1140

cttgataagg ctatgcttat atttagagat atgcagaaac aaggagtgag tccagatgca 1200

gtcacatatt caaccgtaat acatgcattt tgcaaaaagg gtaggttgga tgatgctgtg 1260

ataaagttta atcagatgat tgatacagga gtacgaccgg aagcatctgc ttatcgttcc 1320

ctaatccagg gtttttgtac acatggcgat ttggtgaaag caaaggaata tgttactgaa 1380

atgatgaaga aaggtatgct tcctcctgat attatgttct tcagttcaat catgcagaaa 1440

ctatgcacag aaggaagggt aatagaagca cgagatatcc ttgacttgat agtgcgcatt 1500

ggtatgaggc ctaatgtttt catatttaat atactgatcg gtgggtactg cctagtctgc 1560

aagatggagg atgcaacaaa aatgtttgag gatatggtgt catttggttt agaaccttgt 1620

aatattacgt atggtatact tattaatggc tattgcaaaa atagaaggat tgatgatggg 1680

cttattctgt tccaagagat gctgcacaag ggacttaaac ctacaacttt taattataat 1740

gtcatactgg atgggttatt tctggctgga cgaactgttg ctgcaaagga aaagtttgat 1800

gagatggttg aatctggagt aagtgtgtgc agtgatacat attcgatagt tcttgctgga 1860

ctttgcagaa ataattgctc tagcgaagta atcatgctgt tccagaaatt aagggcaatg 1920

aacatgaaat tcaatattag aattgtcact atcatgattg atgccttcta caaggttcgg 1980

cgaaaccaag aagctaagga tttgtttgct gcaataccat ccaatggctt ggtagctaat 2040

gttgttacct acagcataat gatgaaaagt cttataaaag aaggttcagt ggaagaagct 2100

gacactcttt ttatcgatgg agaagagcgg ctgtactgca aactcttgga tgttaaatca 2160

tattatcaga aggttactgg aaagaggaga gatagtcaag gctggaaatt atatgtcgaa 2220

agttga 2226

<210> 14
<211> 741
<212> PRT
<213> Triticum aestivum

<400> 14
   Met Pro Arg Phe Ser Thr Thr Thr Pro Met Ser Pro Pro Arg Leu Leu
   1 5 10 15
   Leu Arg Leu Gly Ala Arg His Ser Ser Ser Thr Ser His Pro Ser Arg
               20                  25                  30
   Ile Trp Asp Pro His Ala Ala Phe Ala Ala Ala Thr Gln Arg Ala Arg
           35                  40                  45
   Ser Gly Thr Leu Thr Thr Glu Asp Ala His His Leu Phe Asp Glu Leu
       50                  55                  60
   Leu Arg Gln Gly Asn Pro Val Gln Glu Arg Pro Leu Thr Asn Phe Leu
   65                  70                  75                  80
   Ala Ala Leu Ala Arg Ala Pro Ala Ser Ala Phe Cys Ser Asp Gly Pro
                   85                  90                  95
   Ala Leu Ala Val Ala Leu Phe Gly Arg Leu Ser Arg Gly Ala Gly Arg
               100                 105                 110
   Arg Val Ala Gln Pro Asn Val Phe Thr Tyr Gly Val Leu Met Asp Cys
           115                 120                 125
   Cys Cys Arg Ala Arg Arg Leu Asp Leu Ala Ile Ala Phe Phe Ala Arg
       130                 135                 140
   Leu Leu Lys Thr Gly Leu Glu Ala Asn Gln Val Ile Phe Cys Thr Leu
   145                 150                 155                 160
   Leu Lys Gly Leu Cys His Ala Lys Arg Ser Asp Glu Ala Leu Asp Val
                   165                 170                 175
   Val Leu His Arg Met Pro Glu Leu Gly Cys Thr Pro Asn Val Val Ala
               180                 185                 190
   Tyr Thr Thr Val Ile His Gly Phe Leu Lys Glu Gly Gln Val Gly Lys
           195                 200                 205
   Ala Cys Asn Leu Phe His Gly Met Ala Gln Gln Gly Val Ala Pro Asp
       210                 215                 220
   Val Val Thr Tyr Asn Ser Val Ile Asp Ala Leu Cys Lys Ala Arg Ala
   225                 230                 235                 240
   Met Asp Lys Ala Glu Tyr Phe Leu Cys Glu Met Val Asp Asn Gly Val
                   245                 250                 255
   Val Pro Asn Asn Val Thr Tyr Asn Ser Leu Ile His Gly Tyr Ser Ser
   260 265 270
   Leu Gly His Gln Lys Glu Ala Val Arg Val Leu Lys Glu Met Thr Arg
   275 280 285
   Gln Gly Ile Ile Pro Asp Val Ile Thr Cys Thr Ser Leu Met Thr Phe
   290 295 300
   Leu Cys Lys Asn Gly Lys Ser Lys Glu Ala Ala Glu Ile Phe Asp Ser
   305 310 315 320
   Met Pro Thr Lys Gly Leu Lys His Asp Ala Val Ser Tyr Ala Ile Leu
   325 330 335
   Leu His Gly Tyr Ala Thr Glu Gly Cys Leu Val Asp Met Ile Asn Leu
   340 345 350
   Phe Asn Ser Met Asp Arg Asp Cys Ile Leu Pro Asn Cys His Ile Phe
   355 360 365
   Asn Ile Leu Ile Tyr Ala Tyr Ala Lys Ser Gly Lys Leu Asp Lys Ala
   370 375 380
   Met Leu Ile Phe Arg Asp Met Gln Lys Gln Gly Val Ser Pro Asp Ala
   385 390 395 400
   Val Thr Tyr Ser Thr Val Ile His Ala Phe Cys Lys Lys Gly Arg Leu
   405 410 415
   Asp Asp Ala Val Ile Lys Phe Asn Gln Met Ile Asp Thr Gly Val Arg
   420 425 430
   Pro Glu Ala Ser Ala Tyr Arg Ser Leu Ile Gln Gly Phe Cys Thr His
   435 440 445
   Gly Asp Leu Val Lys Ala Lys Glu Tyr Val Thr Glu Met Met Lys Lys
   450 455 460
   Gly Met Leu Pro Pro Asp Ile Met Phe Phe Ser Ser Ile Met Gln Lys
   465 470 475 480
   Leu Cys Thr Glu Gly Arg Val Ile Glu Ala Arg Asp Ile Leu Asp Leu
   485 490 495
   Ile Val Arg Ile Gly Met Arg Pro Asn Val Phe Ile Phe Asn Ile Leu
   500 505 510
   Ile Gly Gly Tyr Cys Leu Val Cys Lys Met Glu Asp Ala Thr Lys Met
           515                 520                 525
   Phe Glu Asp Met Val Ser Phe Gly Leu Glu Pro Cys Asn Ile Thr Tyr
       530                 535                 540
   Gly Ile Leu Ile Asn Gly Tyr Cys Lys Asn Arg Arg Ile Asp Asp Gly
   545                 550                 555                 560
   Leu Ile Leu Phe Gln Glu Met Leu His Lys Gly Leu Lys Pro Thr Thr
                   565                 570                 575
   Phe Asn Tyr Asn Val Ile Leu Asp Gly Leu Phe Leu Ala Gly Arg Thr
               580                 585                 590
   Val Ala Ala Lys Glu Lys Phe Asp Glu Met Val Glu Ser Gly Val Ser
           595                 600                 605
   Val Cys Ser Asp Thr Tyr Ser Ile Val Leu Ala Gly Leu Cys Arg Asn
       610                 615                 620
   Asn Cys Ser Ser Glu Val Ile Met Leu Phe Gln Lys Leu Arg Ala Met
   625                 630                 635                 640
   Asn Met Lys Phe Asn Ile Arg Ile Val Thr Ile Met Ile Asp Ala Phe
                   645                 650                 655
   Tyr Lys Val Arg Arg Asn Gln Glu Ala Lys Asp Leu Phe Ala Ala Ile
               660                 665                 670
   Pro Ser Asn Gly Leu Val Ala Asn Val Val Thr Tyr Ser Ile Met Met
           675                 680                 685
   Lys Ser Leu Ile Lys Glu Gly Ser Val Glu Glu Ala Asp Thr Leu Phe
       690                 695                 700
   Ile Asp Gly Glu Glu Arg Leu Tyr Cys Lys Leu Leu Asp Val Lys Ser
   705                 710                 715                 720
   Tyr Tyr Gln Lys Val Thr Gly Lys Arg Arg Asp Ser Gln Gly Trp Lys
                   725                 730                 735
   Leu Tyr Val Glu Ser
               740

<210>  15
<211>  2226
<212>  DNA

<213>  Artificial Sequence

<220>
<223>  cDNA of Rf1-2 of R113

<400>  15
atgcctcgct tctccaccac cacgccaatg tcgccacccc gcctcctcct ccgactcggc      60

gcccgccact cctcctccac ctctcatccc tcacgcatct gggatcccca cgccgccttc     120

gccgctgcga cgcagcgggc gcgctctggc acgctcacca cggaggacgc acaccacctg     180

tttgatgaat tgctgcggca gggcaatcct gtccaggagc gtcccttgac taactttctg     240

gctgccctcg cccgcgcgcc cgcgtccgca ttctgcagcg atggccctgc cctggccgtc     300

gccctcttcg gccgtttgtc ccgaggcgcc ggacgacggg tggcgcagcc aaatgtcttc     360

acctatggcg tcctcatgga ctgctgctgc cgtgcgcgcc gcctggatct agcgatcgcc     420

ttcttcgccc gtctcctcaa gacgggactg gaggcaaacc aagtcatctt ctgcaccctc     480

ctcaagggac tctgccacgc aaagcgctca gatgaggctt tggacgtggt gcttcacagg     540

atgcctgagc taggctgcac ccccaacgtg gtggcctata ccacggtcat ccacggcttc     600

ttgaaggaag gccaagtagg caaggcatgc aatctattcc atggaatggc gcagcagggc     660

gttgcgcctg atgtggtgac atataactcg gttatcgatg cgttgtgcaa ggccagagca     720

atggacaagg cagagtattt cctttgtgaa atggttgata atggtgtcgt acctaataat     780

gtgacatata atagcctcat ccatggatat tcctctttgg gccatcagaa ggaggctgtt     840

agggtgctga aagaaatgac aagacagggt atcataccag atgtcattac ctgcacctca     900

ctcatgacct tcctttgcaa gaatggaaaa agcaaggaag ctgcagaaat ttttgattca     960

atgcccacga agggcctgaa acatgacgcc gtttcatatg ctattctcct tcatgggtat    1020

gccactgaag gatgcttggt tgatatgatt aatctcttca attcgatgga cagagactgt    1080

attctaccta actgtcatat cttcaacata ctgatttatg catatgctaa atctgggaag    1140

cttgataagg ctatgcttat atttagagat atgcagaaac aaggagtgag tccagatgca    1200

gtcacatatt caaccgtaat acatgcattt tgcaaaaagg gtaggttgga tgatgctgtg    1260

ataaagttta atcagatgat tgatacagga gtacgaccgg aagcatctgc ttatcgttcc    1320

ctaatccagg gtttttgtac acatggcgat ttggtgaaag caaaggaata tgttactgaa    1380

atgatgaaga aaggtatgct tcctcctgat attatgttct tcagttcaat catgcagaaa    1440

ctatgcacag aaggaagggt aatagaagca cgagatatcc ttgacttgat agtgcgcatt    1500

ggtatgaggc ctaatgtttt catatttaat atactgatcg gtgggtactg cctagtctgc    1560

aagatggagg atgcaacaaa aatgtttgag gatatggtgt catttggttt agaaccttgt    1620

aatattacgt atggtatact tattaatggc tattgcaaaa atagaaggat tgatgatggg    1680

cttattctgt tccaagagat gctgcacaag ggacttaaac ctacaacttt taattataat    1740

gtcatactgg atgggttatt tctggctgga cgaactgttg ctgcaaagga aaagtttgat 1800

gagatggttg aatctggagt aagtgtgtgc agtgatacat attcgatagt tcttgctgga 1860

ctttgcagaa ataattgctc tagcgaagta atcatgctgt tccagaaatt aagggcaatg 1920

aacatgaaat tcaatattag aattgtcact atcatgattg atgccttcta caaggttcgg 1980

cgaaaccaag aagctaagga tttgtttgct gcaataccat ccaatggctt ggtagctaat 2040

gttgttacct acagcataat gatgaaaagt cttataaaag aaggttcagt ggaagaagct 2100

gacactcttt ttatcgatgg agaagagcgg ctgtactgca aactcttgga tgttaaatca 2160

tattatcaga aggttactgg aaagaggaga gatagtcaag gctggaaatt atatgtcgaa 2220

agttga 2226

<210> 16
<211> 741
<212> PRT
<213> Triticum aestivum

<400> 16

Met Pro Arg Phe Ser Thr Thr Thr Pro Met Ser Pro Pro Arg Leu Leu
1               5                   10                  15

Leu Arg Leu Gly Ala Arg His Ser Ser Ser Thr Ser His Pro Ser Arg
            20                  25                  30

Ile Trp Asp Pro His Ala Ala Phe Ala Ala Ala Thr Gln Arg Ala Arg
        35                  40                  45

Ser Gly Thr Leu Thr Thr Glu Asp Ala His His Leu Phe Asp Glu Leu
    50                  55                  60

Leu Arg Gln Gly Asn Pro Val Gln Glu Arg Pro Leu Thr Asn Phe Leu
65                  70                  75                  80

Ala Ala Leu Ala Arg Ala Pro Ala Ser Ala Phe Cys Ser Asp Gly Pro
                85                  90                  95

Ala Leu Ala Val Ala Leu Phe Gly Arg Leu Ser Arg Gly Ala Gly Arg
            100                 105                 110

Arg Val Ala Gln Pro Asn Val Phe Thr Tyr Gly Val Leu Met Asp Cys
        115                 120                 125

Cys Cys Arg Ala Arg Arg Leu Asp Leu Ala Ile Ala Phe Phe Ala Arg
    130                 135                 140

Leu Leu Lys Thr Gly Leu Glu Ala Asn Gln Val Ile Phe Cys Thr Leu
145                 150                 155                 160

Leu Lys Gly Leu Cys His Ala Lys Arg Ser Asp Glu Ala Leu Asp Val
                165                 170                 175

Val Leu His Arg Met Pro Glu Leu Gly Cys Thr Pro Asn Val Val Ala
            180                 185                 190

Tyr Thr Thr Val Ile His Gly Phe Leu Lys Glu Gly Gln Val Gly Lys
        195                 200                 205

Ala Cys Asn Leu Phe His Gly Met Ala Gln Gln Gly Val Ala Pro Asp
    210                 215                 220

Val Val Thr Tyr Asn Ser Val Ile Asp Ala Leu Cys Lys Ala Arg Ala
225                 230                 235                 240

Met Asp Lys Ala Glu Tyr Phe Leu Cys Glu Met Val Asp Asn Gly Val
                245                 250                 255

Val Pro Asn Asn Val Thr Tyr Asn Ser Leu Ile His Gly Tyr Ser Ser
            260                 265                 270

Leu Gly His Gln Lys Glu Ala Val Arg Val Leu Lys Glu Met Thr Arg
        275                 280                 285

Gln Gly Ile Ile Pro Asp Val Ile Thr Cys Thr Ser Leu Met Thr Phe
    290                 295                 300

Leu Cys Lys Asn Gly Lys Ser Lys Glu Ala Ala Glu Ile Phe Asp Ser
305                 310                 315                 320

Met Pro Thr Lys Gly Leu Lys His Asp Ala Val Ser Tyr Ala Ile Leu
                325                 330                 335

Leu His Gly Tyr Ala Thr Glu Gly Cys Leu Val Asp Met Ile Asn Leu
            340                 345                 350

Phe Asn Ser Met Asp Arg Asp Cys Ile Leu Pro Asn Cys His Ile Phe
        355                 360                 365

Asn Ile Leu Ile Tyr Ala Tyr Ala Lys Ser Gly Lys Leu Asp Lys Ala
    370                 375                 380

Met Leu Ile Phe Arg Asp Met Gln Lys Gln Gly Val Ser Pro Asp Ala
385                 390                 395                 400

Val Thr Tyr Ser Thr Val Ile His Ala Phe Cys Lys Lys Gly Arg Leu
                405                 410                 415

Asp Asp Ala Val Ile Lys Phe Asn Gln Met Ile Asp Thr Gly Val Arg
            420                 425                 430

Pro Glu Ala Ser Ala Tyr Arg Ser Leu Ile Gln Gly Phe Cys Thr His
        435                 440                 445

Gly Asp Leu Val Lys Ala Lys Glu Tyr Val Thr Glu Met Met Lys Lys
    450                 455                 460

Gly Met Leu Pro Pro Asp Ile Met Phe Phe Ser Ser Ile Met Gln Lys
465                 470                 475                 480

Leu Cys Thr Glu Gly Arg Val Ile Glu Ala Arg Asp Ile Leu Asp Leu
                485                 490                 495

Ile Val Arg Ile Gly Met Arg Pro Asn Val Phe Ile Phe Asn Ile Leu
            500                 505                 510

Ile Gly Gly Tyr Cys Leu Val Cys Lys Met Glu Asp Ala Thr Lys Met
        515                 520                 525

Phe Glu Asp Met Val Ser Phe Gly Leu Glu Pro Cys Asn Ile Thr Tyr
    530                 535                 540

Gly Ile Leu Ile Asn Gly Tyr Cys Lys Asn Arg Arg Ile Asp Asp Gly
545                 550                 555                 560

Leu Ile Leu Phe Gln Glu Met Leu His Lys Gly Leu Lys Pro Thr Thr
                565                 570                 575

Phe Asn Tyr Asn Val Ile Leu Asp Gly Leu Phe Leu Ala Gly Arg Thr
            580                 585                 590

Val Ala Ala Lys Glu Lys Phe Asp Glu Met Val Glu Ser Gly Val Ser
        595                 600                 605

Val Cys Ser Asp Thr Tyr Ser Ile Val Leu Ala Gly Leu Cys Arg Asn
    610                 615                 620

Asn Cys Ser Ser Glu Val Ile Met Leu Phe Gln Lys Leu Arg Ala Met
625                 630                 635                 640

Asn Met Lys Phe Asn Ile Arg Ile Val Thr Ile Met Ile Asp Ala Phe
                645                 650                 655

Tyr Lys Val Arg Arg Asn Gln Glu Ala Lys Asp Leu Phe Ala Ala Ile
            660                 665                 670

Pro Ser Asn Gly Leu Val Ala Asn Val Val Thr Tyr Ser Ile Met Met
        675                 680                 685

Lys Ser Leu Ile Lys Glu Gly Ser Val Glu Glu Ala Asp Thr Leu Phe
    690                 695                 700

Ile Asp Gly Glu Glu Arg Leu Tyr Cys Lys Leu Leu Asp Val Lys Ser
705                 710                 715                 720

Tyr Tyr Gln Lys Val Thr Gly Lys Arg Arg Asp Ser Gln Gly Trp Lys
                725                 730                 735

Leu Tyr Val Glu Ser
            740

<210>  17
<211>  2226
<212>  DNA
<213>  Artificial Sequence

<220>
<223>  cDNA of Rf1-2 of L19

<400>  17
atgcctcgct tctccaccac cacgccaatg tcgccacccc gcctcctcct ccgactcggc      60

gcccgccact cctcctccac ctctcatccc tcacgcatct gggatcccca cgccgccttc     120

gccgctgcga cgcagcgggc gcgctctggc acgctcacca cggaggacgc acaccacctg     180

tttgatgaat tgctgcggca gggcaatcct gtccaggagc gtcccttgac taactttctg     240

gctgccctcg cccgcgcgcc cgcgtccgca ttctgcagcg atggccctgc cctggccgtc     300

gccctcttcg gccgtttgtc ccgaggcgcc ggacgacggg tggcgcagcc aaatgtcttc     360

acctatggcg tcctcatgga ctgctgctgc cgtgcgcgcc gcctggatct agcgatcgcc     420

ttcttcgccc gtctcctcaa gacgggactg gaggcaaacc aagtcatctt ctgcaccctc     480

ctcaagggac tctgccacgc aaagcgctca gatgaggctt tggacgtggt gcttcacagg     540

atgcctgagc taggctgcac ccccaacgtg gtggcctata ccacggtcat ccacggcttc     600

ttgaaggaag gccaagtagg caaggcatgc aatctattcc atggaatggc gcagcagggc     660

gttgcgcctg atgtggtgac atataactcg gttatcgatg cgttgtgcaa ggccagagca     720

atggacaagg cagagtattt cctttgtgaa atggttgata atggtgtcgt acctaataat     780

gtgacatata atagcctcat ccatggatat tcctctttgg gccatcagaa ggaggctgtt     840

agggtgctga aagaaatgac aagacagggt atcataccag atgtcattac ctgcacctca 900

ctcatgacct tcctttgcaa gaatggaaaa agcaaggaag ctgcagaaat ttttgattca 960

atgcccacga agggcctgaa acatgacgcc gtttcatatg ctattctcct tcatgggtat 1020

gccactgaag gatgcttggt tgatatgatt aatctcttca attcgatgga cagagactgt 1080

attctaccta actgtcatat cttcaacata ctgatttatg catatgctaa atctgggaag 1140

cttgataagg ctatgcttat atttagagat atgcagaaac aaggagtgag tccagatgca 1200

gtcacatatt caaccgtaat acatgcattt tgcaaaaagg gtaggttgga tgatgctgtg 1260

ataaagttta atcagatgat tgatacagga gtacgaccgg aagcatctgc ttatcgttcc 1320

ctaatccagg gtttttgtac acatggcgat ttggtgaaag caaaggaata tgttactgaa 1380

atgatgaaga aaggtatgct tcctcctgat attatgttct tcagttcaat catgcagaaa 1440

ctatgcacag aaggaagggt aatagaagca cgagatatcc ttgacttgat agtgcgcatt 1500

ggtatgaggc ctaatgtttt catatttaat atactgatcg gtgggtactg cctagtctgc 1560

aagatggagg atgcaacaaa aatgtttgag gatatggtgt catttggttt agaaccttgt 1620

aatattacgt atggtatact tattaatggc tattgcaaaa atagaaggat tgatgatggg 1680

cttattctgt tccaagagat gctgcacaag ggacttaaac ctacaacttt taattataat 1740

gtcatactgg atgggttatt tctggctgga cgaactgttg ctgcaaagga aaagtttgat 1800

gagatggttg aatctggagt aagtgtgtgc agtgatacat attcgatagt tcttgctgga 1860

ctttgcagaa ataattgctc tagcgaagta atcatgctgt tccagaaatt aagggcaatg 1920

aacatgaaat tcaatattag aattgtcact atcatgattg atgccttcta caaggttcgg 1980

cgaaaccaag aagctaagga tttgtttgct gcaataccat ccaatggctt ggtagctaat 2040

gttgttacct acagcataat gatgaaaagt cttataaaag aaggttcagt ggaagaagct 2100

gacactcttt ttatcgatgg agaagagcgg ctgtactgca aactcttgga tgttaaatca 2160

tattatcaga aggttactgg aaagaggaga gatagtcaag gctggaaatt atatgtcgaa 2220

agttga 2226

<210> 18
<211> 741
<212> PRT
<213> Triticum aestivum

<400> 18

Met Pro Arg Phe Ser Thr Thr Thr Pro Met Ser Pro Pro Arg Leu Leu
1               5                   10                  15

Leu Arg Leu Gly Ala Arg His Ser Ser Ser Thr Ser His Pro Ser Arg
            20                  25                  30

Ile Trp Asp Pro His Ala Ala Phe Ala Ala Ala Thr Gln Arg Ala Arg
        35                  40                  45

Ser Gly Thr Leu Thr Thr Glu Asp Ala His His Leu Phe Asp Glu Leu
    50                  55                  60

Leu Arg Gln Gly Asn Pro Val Gln Glu Arg Pro Leu Thr Asn Phe Leu
65                  70                  75                  80

Ala Ala Leu Ala Arg Ala Pro Ala Ser Ala Phe Cys Ser Asp Gly Pro
                85                  90                  95

Ala Leu Ala Val Ala Leu Phe Gly Arg Leu Ser Arg Gly Ala Gly Arg
            100                 105                 110

Arg Val Ala Gln Pro Asn Val Phe Thr Tyr Gly Val Leu Met Asp Cys
        115                 120                 125

Cys Cys Arg Ala Arg Arg Leu Asp Leu Ala Ile Ala Phe Phe Ala Arg
    130                 135                 140

Leu Leu Lys Thr Gly Leu Glu Ala Asn Gln Val Ile Phe Cys Thr Leu
145                 150                 155                 160

Leu Lys Gly Leu Cys His Ala Lys Arg Ser Asp Glu Ala Leu Asp Val
                165                 170                 175

Val Leu His Arg Met Pro Glu Leu Gly Cys Thr Pro Asn Val Val Ala
            180                 185                 190

Tyr Thr Thr Val Ile His Gly Phe Leu Lys Glu Gly Gln Val Gly Lys
        195                 200                 205

Ala Cys Asn Leu Phe His Gly Met Ala Gln Gln Gly Val Ala Pro Asp
    210                 215                 220

Val Val Thr Tyr Asn Ser Val Ile Asp Ala Leu Cys Lys Ala Arg Ala
225                 230                 235                 240

Met Asp Lys Ala Glu Tyr Phe Leu Cys Glu Met Val Asp Asn Gly Val
                245                 250                 255

Val Pro Asn Asn Val Thr Tyr Asn Ser Leu Ile His Gly Tyr Ser Ser
            260                 265                 270

Leu Gly His Gln Lys Glu Ala Val Arg Val Leu Lys Glu Met Thr Arg
        275                 280                 285

Gln Gly Ile Ile Pro Asp Val Ile Thr Cys Thr Ser Leu Met Thr Phe
    290                 295                 300

Leu Cys Lys Asn Gly Lys Ser Lys Glu Ala Ala Glu Ile Phe Asp Ser
305                 310                 315                 320

Met Pro Thr Lys Gly Leu Lys His Asp Ala Val Ser Tyr Ala Ile Leu
                325                 330                 335

Leu His Gly Tyr Ala Thr Glu Gly Cys Leu Val Asp Met Ile Asn Leu
            340                 345                 350

Phe Asn Ser Met Asp Arg Asp Cys Ile Leu Pro Asn Cys His Ile Phe
        355                 360                 365

Asn Ile Leu Ile Tyr Ala Tyr Ala Lys Ser Gly Lys Leu Asp Lys Ala
    370                 375                 380

Met Leu Ile Phe Arg Asp Met Gln Lys Gln Gly Val Ser Pro Asp Ala
385                 390                 395                 400

Val Thr Tyr Ser Thr Val Ile His Ala Phe Cys Lys Lys Gly Arg Leu
                405                 410                 415

Asp Asp Ala Val Ile Lys Phe Asn Gln Met Ile Asp Thr Gly Val Arg
            420                 425                 430

Pro Glu Ala Ser Ala Tyr Arg Ser Leu Ile Gln Gly Phe Cys Thr His
        435                 440                 445

Gly Asp Leu Val Lys Ala Lys Glu Tyr Val Thr Glu Met Met Lys Lys
    450                 455                 460

Gly Met Leu Pro Pro Asp Ile Met Phe Phe Ser Ser Ile Met Gln Lys
465                 470                 475                 480

Leu Cys Thr Glu Gly Arg Val Ile Glu Ala Arg Asp Ile Leu Asp Leu
                485                 490                 495

Ile Val Arg Ile Gly Met Arg Pro Asn Val Phe Ile Phe Asn Ile Leu
            500                 505                 510

Ile Gly Gly Tyr Cys Leu Val Cys Lys Met Glu Asp Ala Thr Lys Met
        515                 520                 525

Phe Glu Asp Met Val Ser Phe Gly Leu Glu Pro Cys Asn Ile Thr Tyr

530                 535                 540

Gly Ile Leu Ile Asn Gly Tyr Cys Lys Asn Arg Arg Ile Asp Asp Gly
545                 550                 555                 560

Leu Ile Leu Phe Gln Glu Met Leu His Lys Gly Leu Lys Pro Thr Thr
                565                 570                 575

Phe Asn Tyr Asn Val Ile Leu Asp Gly Leu Phe Leu Ala Gly Arg Thr
            580                 585                 590

Val Ala Ala Lys Glu Lys Phe Asp Glu Met Val Glu Ser Gly Val Ser
        595                 600                 605

Val Cys Ser Asp Thr Tyr Ser Ile Val Leu Ala Gly Leu Cys Arg Asn
    610                 615                 620

Asn Cys Ser Ser Glu Val Ile Met Leu Phe Gln Lys Leu Arg Ala Met
625                 630                 635                 640

Asn Met Lys Phe Asn Ile Arg Ile Val Thr Ile Met Ile Asp Ala Phe
                645                 650                 655

Tyr Lys Val Arg Arg Asn Gln Glu Ala Lys Asp Leu Phe Ala Ala Ile
            660                 665                 670

Pro Ser Asn Gly Leu Val Ala Asn Val Val Thr Tyr Ser Ile Met Met
        675                 680                 685

Lys Ser Leu Ile Lys Glu Gly Ser Val Glu Glu Ala Asp Thr Leu Phe
    690                 695                 700

Ile Asp Gly Glu Glu Arg Leu Tyr Cys Lys Leu Leu Asp Val Lys Ser
705                 710                 715                 720

Tyr Tyr Gln Lys Val Thr Gly Lys Arg Arg Asp Ser Gln Gly Trp Lys
                725                 730                 735

Leu Tyr Val Glu Ser
            740

<210>  19
<211>  2217
<212>  DNA
<213>  Artificial Sequence

<220>
<223>  cDNA of Rf1-1 of R3

<400> 19
atgcctcgct tctcctccac cacgccaatg tcgccacccc gcctcctcct ccggctcggc 60

gcccgccact cctcctccac ctctcatccc tcacgcatct gggatcccca cgccgccttc 120

gccgctgcga cgcagcgggc gcgctctggc acgctcacca cggaggacgc acaccacctg 180

tttgatgaat tgctgcggca gggcaatcct gtccaggagc gtcccttgac taactttctg 240

gctgccctcg cccgcgcgcc cgcgtccgca ttctgcagcg atggccctgc cctggccgtc 300

gccctcttcg gccgtttgtc ccgaggcgcc ggacgacggg tggcgcagcc aaatgtcttc 360

acctatggcg tcctcatgga ctgctgctgc cgtgcgcgcc gcctggatct agcgatcgcc 420

ttcttcgccc gtctcctcaa gacgggactg gaggcaaacc aagtcatctt ctgcaccctc 480

ctcaagggac tctgccacgc aaagcgctca gatgaggctt tggacgtggt gcttcacagg 540

atgcctgagc taggctgcac ccccaacgtg gtggcctata ccacggtcat ccacggcttc 600

ttgaaggaag gccaagtagg caaggcatgc aatctattcc atggaatggc gcagcagggc 660

gttgcgcctg atgtggtgac atataactcg gttatcgatg cgttgtgcaa ggccagagca 720

atggacaagg cagagtattt ccttcgtgaa atggttgata atggtgtcgt acctaataat 780

gtgacatata atagcctcat ccatggatat tcctctttgg gccatcagaa ggaggctgtt 840

agggtgctga aagaaatgac aagacagggt atcataccag atgtcattac ctgcacctca 900

ctcatgacct tcctttgcaa gaatggaaaa agcaaggaag ctgcagaaat ttttgattca 960

atggccacga agggcctgaa acatgacgcc gtttcatatg ctattctcct tcatgggtat 1020

gccactgaag gatgcttggt tgatatgatt aatctcttca attcgatgga cagagactgt 1080

attctaccta actgtcatat cttcaacata ctgatttatg catatgctaa atctgggaag 1140

cttgataagg ctatgcttat atttagagat atgcagaaac aaggagtgag cccagatgca 1200

ttcacatatt caaccttaat acatgcattt tgtaaaaagg gtcggttgga cgatgctatg 1260

ataaagttta atcagatggt tgatacagga gtacgacagg gcacagctgt ttatggttct 1320

ctaatccagg gtttttgtac acacggcgat ttggtgaaag gaaaggaatt ggttactgaa 1380

atgatgaaca aaggtatacc tcctcctgac attatgttct tccattcaat catgcagaac 1440

ctatgcacag aaggaagggt agtagaagca cgggatatcc ttggcttgat agcacacata 1500

ggtatgaggc ctaatgtttg cacatttaat atactgattg gtggatactg cctagtccgc 1560

aagatggagg atgcctcaaa aatatttcat gatatgatgt catatggttt agaaccttct 1620

aatgttacgt atggtattct tattaatggc tattgcaaaa acagaaggat tgatgacggg 1680

ctgattctgt tcaaagaaat gttgcgcaag ggacttaaac ctacaacttt taattacaac 1740

atcatactgg atggattatt tctggctgga cgaactgttg ctgcaaagga aaagtttgat 1800

gagatggttg aatctggagt aagtatgtgc atcagtactt actctatagt tcttcgtgga 1860

ctttgtagaa ataattgtag cgaagccatc acgctattcc agacattaag cgcaatggat 1920

gtgaaattca atattagaat tgtcaatatc atgattgatg ccttcttcag ggttcagcga 1980

aagcaagaag ctaaggattt gtttgctgca ataacagcca atgggttggt tgctaatgtt 2040

tttacctaca gcctaatgat gacaaatctt ataaaagaag ggtcagtgga agaggctgac 2100

actcttttta tcgatggaga tgagcggctg tacttcgaac tcgtggatgt taaatcttat 2160

tatcagaggg ttgctggaaa aaggagagat agtcaaggct ggatgttata tgtctaa 2217

<210> 20
<211> 738
<212> PRT
<213> Triticum aestivum

<400> 20

Met Pro Arg Phe Ser Ser Thr Thr Pro Met Ser Pro Pro Arg Leu Leu
1               5                   10                  15

Leu Arg Leu Gly Ala Arg His Ser Ser Ser Thr Ser His Pro Ser Arg
            20                  25                  30

Ile Trp Asp Pro His Ala Ala Phe Ala Ala Ala Thr Gln Arg Ala Arg
        35                  40                  45

Ser Gly Thr Leu Thr Thr Glu Asp Ala His His Leu Phe Asp Glu Leu
    50                  55                  60

Leu Arg Gln Gly Asn Pro Val Gln Glu Arg Pro Leu Thr Asn Phe Leu
65                  70                  75                  80

Ala Ala Leu Ala Arg Ala Pro Ala Ser Ala Phe Cys Ser Asp Gly Pro
                85                  90                  95

Ala Leu Ala Val Ala Leu Phe Gly Arg Leu Ser Arg Gly Ala Gly Arg
            100                 105                 110

Arg Val Ala Gln Pro Asn Val Phe Thr Tyr Gly Val Leu Met Asp Cys
        115                 120                 125

Cys Cys Arg Ala Arg Arg Leu Asp Leu Ala Ile Ala Phe Phe Ala Arg
    130                 135                 140

Leu Leu Lys Thr Gly Leu Glu Ala Asn Gln Val Ile Phe Cys Thr Leu
145                 150                 155                 160

Leu Lys Gly Leu Cys His Ala Lys Arg Ser Asp Glu Ala Leu Asp Val
                165                 170                 175

Val Leu His Arg Met Pro Glu Leu Gly Cys Thr Pro Asn Val Val Ala
            180                 185                 190

Tyr Thr Thr Val Ile His Gly Phe Leu Lys Glu Gly Gln Val Gly Lys
        195                 200                 205

Ala Cys Asn Leu Phe His Gly Met Ala Gln Gln Gly Val Ala Pro Asp
    210                 215                 220

Val Val Thr Tyr Asn Ser Val Ile Asp Ala Leu Cys Lys Ala Arg Ala
225                 230                 235                 240

Met Asp Lys Ala Glu Tyr Phe Leu Arg Glu Met Val Asp Asn Gly Val
                245                 250                 255

Val Pro Asn Asn Val Thr Tyr Asn Ser Leu Ile His Gly Tyr Ser Ser
            260                 265                 270

Leu Gly His Gln Lys Glu Ala Val Arg Val Leu Lys Glu Met Thr Arg
        275                 280                 285

Gln Gly Ile Ile Pro Asp Val Ile Thr Cys Thr Ser Leu Met Thr Phe
    290                 295                 300

Leu Cys Lys Asn Gly Lys Ser Lys Glu Ala Ala Glu Ile Phe Asp Ser
305                 310                 315                 320

Met Ala Thr Lys Gly Leu Lys His Asp Ala Val Ser Tyr Ala Ile Leu
                325                 330                 335

Leu His Gly Tyr Ala Thr Glu Gly Cys Leu Val Asp Met Ile Asn Leu
            340                 345                 350

Phe Asn Ser Met Asp Arg Asp Cys Ile Leu Pro Asn Cys His Ile Phe
        355                 360                 365

Asn Ile Leu Ile Tyr Ala Tyr Ala Lys Ser Gly Lys Leu Asp Lys Ala
    370                 375                 380

Met Leu Ile Phe Arg Asp Met Gln Lys Gln Gly Val Ser Pro Asp Ala
385                 390                 395                 400

Phe Thr Tyr Ser Thr Leu Ile His Ala Phe Cys Lys Lys Gly Arg Leu
                405                 410                 415

Asp Asp Ala Met Ile Lys Phe Asn Gln Met Val Asp Thr Gly Val Arg
            420                 425                 430

Gln Gly Thr Ala Val Tyr Gly Ser Leu Ile Gln Gly Phe Cys Thr His
        435                 440                 445

Gly Asp Leu Val Lys Gly Lys Glu Leu Val Thr Glu Met Met Asn Lys
    450                 455                 460

Gly Ile Pro Pro Pro Asp Ile Met Phe Phe His Ser Ile Met Gln Asn
465                 470                 475                 480

Leu Cys Thr Glu Gly Arg Val Val Glu Ala Arg Asp Ile Leu Gly Leu
                485                 490                 495

Ile Ala His Ile Gly Met Arg Pro Asn Val Cys Thr Phe Asn Ile Leu
            500                 505                 510

Ile Gly Gly Tyr Cys Leu Val Arg Lys Met Glu Asp Ala Ser Lys Ile
        515                 520                 525

Phe His Asp Met Met Ser Tyr Gly Leu Glu Pro Ser Asn Val Thr Tyr
    530                 535                 540

Gly Ile Leu Ile Asn Gly Tyr Cys Lys Asn Arg Arg Ile Asp Asp Gly
545                 550                 555                 560

Leu Ile Leu Phe Lys Glu Met Leu Arg Lys Gly Leu Lys Pro Thr Thr
                565                 570                 575

Phe Asn Tyr Asn Ile Ile Leu Asp Gly Leu Phe Leu Ala Gly Arg Thr
            580                 585                 590

Val Ala Ala Lys Glu Lys Phe Asp Glu Met Val Glu Ser Gly Val Ser
        595                 600                 605

Met Cys Ile Ser Thr Tyr Ser Ile Val Leu Arg Gly Leu Cys Arg Asn
    610                 615                 620

Asn Cys Ser Glu Ala Ile Thr Leu Phe Gln Thr Leu Ser Ala Met Asp
625                 630                 635                 640

Val Lys Phe Asn Ile Arg Ile Val Asn Ile Met Ile Asp Ala Phe Phe
                645                 650                 655

Arg Val Gln Arg Lys Gln Glu Ala Lys Asp Leu Phe Ala Ala Ile Thr
            660                 665                 670

Ala Asn Gly Leu Val Ala Asn Val Phe Thr Tyr Ser Leu Met Met Thr

675                 680                 685

Asn Leu Ile Lys Glu Gly Ser Val Glu Glu Ala Asp Thr Leu Phe Ile
    690                 695                 700

Asp Gly Asp Glu Arg Leu Tyr Phe Glu Leu Val Asp Val Lys Ser Tyr
705                 710                 715                 720

Tyr Gln Arg Val Ala Gly Lys Arg Arg Asp Ser Gln Gly Trp Met Leu
                725                 730                 735

Tyr Val

<210>  21
<211>  2217
<212>  DNA
<213>  Artificial Sequence

<220>
<223>  cDNA of Rf1-1 of R113

<400>  21
atgcctcgct tctcctccac cacgccaatg tcgccacccc gcctcctcct ccggctcggc      60

gcccgccact cctcctccac ctctcatccc tcacgcatct gggatcccca cgccgccttc     120

gccgctgcga cgcagcgggc gcgctctggc acgctcacca cggaggacgc acaccacctg     180

tttgatgaat tgctgcggca gggcaatcct gtccaggagc gtcccttgac taactttctg     240

gctgccctcg cccgcgcgcc cgcgtccgca ttctgcagcg atggccctgc cctggccgtc     300

gccctcttcg gccgtttgtc ccgaggcgcc ggacgacggg tggcgcagcc aaatgtcttc     360

acctatggcg tcctcatgga ctgctgctgc cgtgcgcgcc gcctggatct agcgatcgcc     420

ttcttcgccc gtctcctcaa gacgggactg gaggcaaacc aagtcatctt ctgcaccctc     480

ctcaagggac tctgccacgc aaagcgctca gatgaggctt tggacgtggt gcttcacagg     540

atgcctgagc taggctgcac ccccaacgtg gtggcctata ccacggtcat ccacggcttc     600

ttgaaggaag gccaagtagg caaggcatgc aatctattcc atggaatggc gcagcagggc     660

gttgcgcctg atgtggtgac atataactcg gttatcgatg cgttgtgcaa ggccagagca     720

atggacaagr cagagtattt ccttcgtgaa atggttgata atggtgtcgt acctaataat     780

gtgacatata atagcctcat ccatggatat tcctctttgg gccatcagaa ggaggctgtt     840

agggtgctga aagaaatgac aagacagggt atcataccag atgtcattac ctgcacctca     900

ctcatgacct tcctttgcaa gaatggaaaa agcaaggaag ctgcagaaat ttttgattca     960

atggccacga agggcctgaa acatgacgcc gtttcatatg ctattctcct tcatgggtat    1020

gccactgaag gatgcttggt tgatatgatt aatctcttca attcgatgga cagagactgt    1080

attctaccta actgtcatat cttcaacata ctgatttatg catatgctaa atctgggaag 1140

cttgataagg ctatgcttat atttagagat atgcagaaac aaggagtgag cccagatgca 1200

ttcacatatt caaccttaat acatgcattt tgtaaaaagg gtcggttgga cgatgctatg 1260

ataaagttta atcagatggt tgatacagga gtacgacagg gcacagctgt ttatggttct 1320

ctaatccagg gtttttgtac acacggcgat ttggtgaaag gaaaggaatt ggttactgaa 1380

atgatgaaca aaggtatacc tcctcctgac attatgttct tccattcaat catgcagaac 1440

ctatgcacag aaggaagggt agtagaagca cgggatatcc ttggcttgat agcacacata 1500

ggtatgaggc ctaatgtttg cacatttaat atactgattg gtggatactg cctagtccgc 1560

aagatggagg atgcctcaaa aatatttcat gatatgatgt catatggttt agaaccttct 1620

aatgttacgt atggtattct tattaatggc tattgcaaaa acagaaggat tgatgacggg 1680

ctgattctgt tcaaagaaat gttgcgcaag ggacttaaac ctacaacttt taattacaac 1740

atcatactgg atggattatt tctggctgga cgaactgttg ctgcaaagga aaagtttgat 1800

gagatggttg aatctggagt aagtatgtgc atcagtactt actctatagt tcttcgtgga 1860

ctttgtagaa ataattgtag cgaagccatc acgctattcc agacattaag cgcaatggat 1920

gtgaaattca atattagaat tgtcaatatc atgattgatg ccttcttcag ggttcagcga 1980

aagcaagaag ctaaggattt gtttgctgca ataacagcca atgggttggt tgctaatgtt 2040

tttacctaca gcctaatgat gacaaatctt ataaaagaag ggtcagtgga agaggctgac 2100

actcttttta tcgatggaga tgagcggctg tacttcgaac tcgtggatgt taaatcttat 2160

tatcagaggg ttgctggaaa aaggagagat agtcaaggct ggatgttata tgtctaa 2217

<210> 22
<211> 738
<212> PRT
<213> Triticum aestivum

<220>
<221> misc_feature
<222> (244)..(244)
<223> Xaa can be any naturally occurring amino acid

<400> 22

Met Pro Arg Phe Ser Ser Thr Thr Pro Met Ser Pro Pro Arg Leu Leu
1               5                   10                  15

Leu Arg Leu Gly Ala Arg His Ser Ser Ser Thr Ser His Pro Ser Arg
            20                  25                  30

Ile Trp Asp Pro His Ala Ala Phe Ala Ala Ala Thr Gln Arg Ala Arg
        35                  40                  45

Ser Gly Thr Leu Thr Thr Glu Asp Ala His His Leu Phe Asp Glu Leu
    50                  55                  60

Leu Arg Gln Gly Asn Pro Val Gln Glu Arg Pro Leu Thr Asn Phe Leu
65                  70                  75                  80

Ala Ala Leu Ala Arg Ala Pro Ala Ser Ala Phe Cys Ser Asp Gly Pro
                85                  90                  95

Ala Leu Ala Val Ala Leu Phe Gly Arg Leu Ser Arg Gly Ala Gly Arg
            100                 105                 110

Arg Val Ala Gln Pro Asn Val Phe Thr Tyr Gly Val Leu Met Asp Cys
        115                 120                 125

Cys Cys Arg Ala Arg Arg Leu Asp Leu Ala Ile Ala Phe Phe Ala Arg
    130                 135                 140

Leu Leu Lys Thr Gly Leu Glu Ala Asn Gln Val Ile Phe Cys Thr Leu
145                 150                 155                 160

Leu Lys Gly Leu Cys His Ala Lys Arg Ser Asp Glu Ala Leu Asp Val
                165                 170                 175

Val Leu His Arg Met Pro Glu Leu Gly Cys Thr Pro Asn Val Val Ala
            180                 185                 190

Tyr Thr Thr Val Ile His Gly Phe Leu Lys Glu Gly Gln Val Gly Lys
        195                 200                 205

Ala Cys Asn Leu Phe His Gly Met Ala Gln Gln Gly Val Ala Pro Asp
    210                 215                 220

Val Val Thr Tyr Asn Ser Val Ile Asp Ala Leu Cys Lys Ala Arg Ala
225                 230                 235                 240

Met Asp Lys Xaa Glu Tyr Phe Leu Arg Glu Met Val Asp Asn Gly Val
                245                 250                 255

Val Pro Asn Asn Val Thr Tyr Asn Ser Leu Ile His Gly Tyr Ser Ser
            260                 265                 270

Leu Gly His Gln Lys Glu Ala Val Arg Val Leu Lys Glu Met Thr Arg
        275                 280                 285

Gln Gly Ile Ile Pro Asp Val Ile Thr Cys Thr Ser Leu Met Thr Phe

290                 295                 300

Leu Cys Lys Asn Gly Lys Ser Lys Glu Ala Ala Glu Ile Phe Asp Ser
305                 310                 315                 320

Met Ala Thr Lys Gly Leu Lys His Asp Ala Val Ser Tyr Ala Ile Leu
                325                 330                 335

Leu His Gly Tyr Ala Thr Glu Gly Cys Leu Val Asp Met Ile Asn Leu
            340                 345                 350

Phe Asn Ser Met Asp Arg Asp Cys Ile Leu Pro Asn Cys His Ile Phe
        355                 360                 365

Asn Ile Leu Ile Tyr Ala Tyr Ala Lys Ser Gly Lys Leu Asp Lys Ala
    370                 375                 380

Met Leu Ile Phe Arg Asp Met Gln Lys Gln Gly Val Ser Pro Asp Ala
385                 390                 395                 400

Phe Thr Tyr Ser Thr Leu Ile His Ala Phe Cys Lys Lys Gly Arg Leu
                405                 410                 415

Asp Asp Ala Met Ile Lys Phe Asn Gln Met Val Asp Thr Gly Val Arg
            420                 425                 430

Gln Gly Thr Ala Val Tyr Gly Ser Leu Ile Gln Gly Phe Cys Thr His
        435                 440                 445

Gly Asp Leu Val Lys Gly Lys Glu Leu Val Thr Glu Met Met Asn Lys
    450                 455                 460

Gly Ile Pro Pro Pro Asp Ile Met Phe Phe His Ser Ile Met Gln Asn
465                 470                 475                 480

Leu Cys Thr Glu Gly Arg Val Val Glu Ala Arg Asp Ile Leu Gly Leu
                485                 490                 495

Ile Ala His Ile Gly Met Arg Pro Asn Val Cys Thr Phe Asn Ile Leu
            500                 505                 510

Ile Gly Gly Tyr Cys Leu Val Arg Lys Met Glu Asp Ala Ser Lys Ile
        515                 520                 525

Phe His Asp Met Met Ser Tyr Gly Leu Glu Pro Ser Asn Val Thr Tyr
    530                 535                 540

Gly Ile Leu Ile Asn Gly Tyr Cys Lys Asn Arg Arg Ile Asp Asp Gly
545                 550                 555                 560

Leu Ile Leu Phe Lys Glu Met Leu Arg Lys Gly Leu Lys Pro Thr Thr
                565                 570                 575

Phe Asn Tyr Asn Ile Ile Leu Asp Gly Leu Phe Leu Ala Gly Arg Thr
            580                 585                 590

Val Ala Ala Lys Glu Lys Phe Asp Glu Met Val Glu Ser Gly Val Ser
        595                 600                 605

Met Cys Ile Ser Thr Tyr Ser Ile Val Leu Arg Gly Leu Cys Arg Asn
    610                 615                 620

Asn Cys Ser Glu Ala Ile Thr Leu Phe Gln Thr Leu Ser Ala Met Asp
625                 630                 635                 640

Val Lys Phe Asn Ile Arg Ile Val Asn Ile Met Ile Asp Ala Phe Phe
                645                 650                 655

Arg Val Gln Arg Lys Gln Glu Ala Lys Asp Leu Phe Ala Ala Ile Thr
            660                 665                 670

Ala Asn Gly Leu Val Ala Asn Val Phe Thr Tyr Ser Leu Met Met Thr
        675                 680                 685

Asn Leu Ile Lys Glu Gly Ser Val Glu Glu Ala Asp Thr Leu Phe Ile
    690                 695                 700

Asp Gly Asp Glu Arg Leu Tyr Phe Glu Leu Val Asp Val Lys Ser Tyr
705                 710                 715                 720

Tyr Gln Arg Val Ala Gly Lys Arg Arg Asp Ser Gln Gly Trp Met Leu
                725                 730                 735

Tyr Val

<210>  23
<211>  2223
<212>  DNA
<213>  Triticum aestivum

<400>  23
atgcctcgct tctcctccac cacgccgatg tcgccacccc gcctcctcct cctcctctgc      60

gcccgccact cctcctccac ctctcatccc tcacgcatct gggatcccca cgccgccttc     120

gtcgccgcga cgcagcgggc gcgctctggc acgctcacca cggaggacgc acaccacctg     180

tttgatgaat tgctgcggca gggcaatcct gtccaggagc gtcccttgac taactttctg 240

gctgccctcg cccgcgcgtc cgcgtccgca ttcttcagca atggccctgc cctggccgtc 300

gccctcttcg gccgtttgtc ccgaggcgcc ggacgacggg tggcgcagcc aaatgtcttc 360

acctatggcg tcctcatgga ctgctactgc cgtgcgcgcc gcctggatct agcgatcgcc 420

ttcttcggcc gtctcctcaa gacgggcctg gaggcagacc aagtcgtttt cagcaccctc 480

ctcaagggac tctgccacgc aaagcgctca gatgaggctc tggacgtgct gcttcacagg 540

atgcctgagc tgggctgcac ccctgacgtg gtggcgtata gcacggtcat ccatggcttc 600

ttcaaggaag gccaagtagg caaggcatgc aatctgttcc atggaatggc gcagcagggc 660

gttgcgcctg acgtggtgac atataactcg gttattgatg cactgtgcaa ggccagagca 720

atggacaagg cggagtattt ccttggtcag atggttgata gtggtgtcgt acctaacaat 780

gtgacatata atagcctcat ccatggatat tcctcttcag gccattcaaa ggaggcggtc 840

agggtgctga aagagatgac aagtaagggt atcataccag atgtttttac ttgcaactcg 900

ctcatggcct ccctttgcaa gaatggaaga agcaaagaag ctgcagaaat ttttaattca 960

atgtccatga agggcctgaa acctaacacc atttcatatg ctattctcct tcatgggtat 1020

gccactgaag gatgcttggt tgacatgatc aatctcttca attcaatggc tacagactgt 1080

attctaccta actgtcatat cttcaacata ctgatttatg catatgctaa atctgggaag 1140

cttgataagg ctatgcttat atttaaagat atgcagaaac aaggagtgag tccagatgca 1200

gtcacatatt caaccgtaat acatgcattt tgcaaaaagg gtaggttgga tgatgctgtg 1260

ataaagttta atcagatgat tgatacagga gtacgaccgg aagcatctgc ttatcgttcc 1320

ctaatccagg gtttttgtac acatggcgat ttggtgaaag caaaggaata tgttactgaa 1380

atgatgaaga aaggtatgct tcctcctgat attatgttct tcagttcaat catgcagaaa 1440

ctatgcacag aaggaagggt aatagaagca cgagatatcc ttgacttgat agtgcgcatt 1500

ggtatgaggc ctaatgtttt catgtttaat atactgatcg gtgggtactg cctagtctgc 1560

aagatggagg atgcaacaaa aatgtttgag gatatggtgt catttggttt agaaccttgt 1620

aatattacgt atggtatact tattaatggc tattgcaaaa atagaaggat tgatgatggg 1680

cttattctgt tccaagagat gctgcacaag ggacttaaac ctacaacttt taattataat 1740

gtcatactgg atgggttatt tctggctgga cgaactgttg ctgcaaagga aaagtttgat 1800

gagatggttg aatctggagt aagtgtgtgc actgatacat attcgatagt tcttgctgga 1860

ctttgcagaa ataattgctc tagcgaagta atcatgctgt tccagaaatt aagggcaatg 1920

aacatgaaat tcaatattag aattgtcact atcatgattg atgccttcta caaggttcgg 1980

cgaaaccaag aagctaagga tttgtttgct gcaataccat ccaatggctt ggtagctaat 2040

gttgttacct acagcataat gatgaaaaat cttataaaag aaggttcagt ggaagaagct 2100

gacactcttt ttatcaatgg agaagagcgg ctgtactgca aactcttgga tgttaaatca 2160

tattatcaga aggttactgg gaagaggaga gatagtcaag gctggaaatt atatgtcgaa 2220

agt 2223

<210> 24
<211> 741
<212> PRT
<213> Triticum aestivum

<400> 24

Met Pro Arg Phe Ser Ser Thr Thr Pro Met Ser Pro Pro Arg Leu Leu
1               5                   10                  15

Leu Leu Leu Cys Ala Arg His Ser Ser Ser Thr Ser His Pro Ser Arg
            20                  25                  30

Ile Trp Asp Pro His Ala Ala Phe Val Ala Ala Thr Gln Arg Ala Arg
        35                  40                  45

Ser Gly Thr Leu Thr Thr Glu Asp Ala His His Leu Phe Asp Glu Leu
    50                  55                  60

Leu Arg Gln Gly Asn Pro Val Gln Glu Arg Pro Leu Thr Asn Phe Leu
65                  70                  75                  80

Ala Ala Leu Ala Arg Ala Ser Ala Ser Ala Phe Phe Ser Asn Gly Pro
                85                  90                  95

Ala Leu Ala Val Ala Leu Phe Gly Arg Leu Ser Arg Gly Ala Gly Arg
            100                 105                 110

Arg Val Ala Gln Pro Asn Val Phe Thr Tyr Gly Val Leu Met Asp Cys
        115                 120                 125

Tyr Cys Arg Ala Arg Arg Leu Asp Leu Ala Ile Ala Phe Phe Gly Arg
    130                 135                 140

Leu Leu Lys Thr Gly Leu Glu Ala Asp Gln Val Val Phe Ser Thr Leu
145                 150                 155                 160

Leu Lys Gly Leu Cys His Ala Lys Arg Ser Asp Glu Ala Leu Asp Val
                165                 170                 175

Leu Leu His Arg Met Pro Glu Leu Gly Cys Thr Pro Asp Val Val Ala
            180                 185                 190

Tyr Ser Thr Val Ile His Gly Phe Phe Lys Glu Gly Gln Val Gly Lys
        195                 200                 205

Ala Cys Asn Leu Phe His Gly Met Ala Gln Gln Gly Val Ala Pro Asp
    210                 215                 220

Val Val Thr Tyr Asn Ser Val Ile Asp Ala Leu Cys Lys Ala Arg Ala
225                 230                 235                 240

Met Asp Lys Ala Glu Tyr Phe Leu Gly Gln Met Val Asp Ser Gly Val
                245                 250                 255

Val Pro Asn Asn Val Thr Tyr Asn Ser Leu Ile His Gly Tyr Ser Ser
            260                 265                 270

Ser Gly His Ser Lys Glu Ala Val Arg Val Leu Lys Glu Met Thr Ser
        275                 280                 285

Lys Gly Ile Ile Pro Asp Val Phe Thr Cys Asn Ser Leu Met Ala Ser
    290                 295                 300

Leu Cys Lys Asn Gly Arg Ser Lys Glu Ala Ala Glu Ile Phe Asn Ser
305                 310                 315                 320

Met Ser Met Lys Gly Leu Lys Pro Asn Thr Ile Ser Tyr Ala Ile Leu
                325                 330                 335

Leu His Gly Tyr Ala Thr Glu Gly Cys Leu Val Asp Met Ile Asn Leu
            340                 345                 350

Phe Asn Ser Met Ala Thr Asp Cys Ile Leu Pro Asn Cys His Ile Phe
        355                 360                 365

Asn Ile Leu Ile Tyr Ala Tyr Ala Lys Ser Gly Lys Leu Asp Lys Ala
    370                 375                 380

Met Leu Ile Phe Lys Asp Met Gln Lys Gln Gly Val Ser Pro Asp Ala
385                 390                 395                 400

Val Thr Tyr Ser Thr Val Ile His Ala Phe Cys Lys Lys Gly Arg Leu
                405                 410                 415

Asp Asp Ala Val Ile Lys Phe Asn Gln Met Ile Asp Thr Gly Val Arg
            420                 425                 430

Pro Glu Ala Ser Ala Tyr Arg Ser Leu Ile Gln Gly Phe Cys Thr His
        435                 440                 445

Gly Asp Leu Val Lys Ala Lys Glu Tyr Val Thr Glu Met Met Lys Lys
    450                 455                 460

Gly Met Leu Pro Pro Asp Ile Met Phe Phe Ser Ser Ile Met Gln Lys
465                 470                 475                 480

Leu Cys Thr Glu Gly Arg Val Ile Glu Ala Arg Asp Ile Leu Asp Leu
                485                 490                 495

Ile Val Arg Ile Gly Met Arg Pro Asn Val Phe Met Phe Asn Ile Leu
            500                 505                 510

Ile Gly Gly Tyr Cys Leu Val Cys Lys Met Glu Asp Ala Thr Lys Met
        515                 520                 525

Phe Glu Asp Met Val Ser Phe Gly Leu Glu Pro Cys Asn Ile Thr Tyr
    530                 535                 540

Gly Ile Leu Ile Asn Gly Tyr Cys Lys Asn Arg Arg Ile Asp Asp Gly
545                 550                 555                 560

Leu Ile Leu Phe Gln Glu Met Leu His Lys Gly Leu Lys Pro Thr Thr
                565                 570                 575

Phe Asn Tyr Asn Val Ile Leu Asp Gly Leu Phe Leu Ala Gly Arg Thr
            580                 585                 590

Val Ala Ala Lys Glu Lys Phe Asp Glu Met Val Glu Ser Gly Val Ser
        595                 600                 605

Val Cys Thr Asp Thr Tyr Ser Ile Val Leu Ala Gly Leu Cys Arg Asn
    610                 615                 620

Asn Cys Ser Ser Glu Val Ile Met Leu Phe Gln Lys Leu Arg Ala Met
625                 630                 635                 640

Asn Met Lys Phe Asn Ile Arg Ile Val Thr Ile Met Ile Asp Ala Phe
                645                 650                 655

Tyr Lys Val Arg Arg Asn Gln Glu Ala Lys Asp Leu Phe Ala Ala Ile
            660                 665                 670

Pro Ser Asn Gly Leu Val Ala Asn Val Val Thr Tyr Ser Ile Met Met
        675                 680                 685

Lys Asn Leu Ile Lys Glu Gly Ser Val Glu Glu Ala Asp Thr Leu Phe

690                 695                 700

Ile Asn Gly Glu Glu Arg Leu Tyr Cys Lys Leu Leu Asp Val Lys Ser
705                 710                 715                 720

Tyr Tyr Gln Lys Val Thr Gly Lys Arg Arg Asp Ser Gln Gly Trp Lys
                725                 730                 735

Leu Tyr Val Glu Ser
            740

<210>  25
<211>  2352
<212>  DNA
<213>  Triticum aestivum

<400>  25
atgtcgccac cacgcctccg cctcctcctc ctcctcctcc tcctcctctg cgcccgccac      60

tcctcctcca cctctcatcc ctcacgcatc tgggatcccc acgccgcctt cgccgccgcg     120

acggagcggg cgcgctctgg taggctcacc acggaggacg cacaccacct gttcgacgaa     180

ttgctgcggc agggcaatcc tgtccaggag cgtcccttga ataactttct ggctgccctc     240

gcccgcgcgc ccgcgtctgc atcctgcagc gatggccccg ccctggccgt cgccctcttc     300

ggccgtttgt cccgaggcgc cgggcaacgg gtggcgcagc caaatgtgtt cacctatggc     360

gtcctcatgg actgctgctg ccgcgcttgc cgcacagatc tggcgctcgc cttctttggc     420

cgtcttctca ggacgggcct gcaggcaggc caagtcgtct tcaacaccct cctcaagggc     480

ctttgccaca caaagcgggc ggatgaggct ctggacgtgc ttcttcacag gatgcctgag     540

ctgggctgca cccccgatgt ggtggcgtat aacaccgtca tccatggctt ctttagggaa     600

ggccaagtag gcaaggcatg cgatatgttc catggaatgg cacagaaggg tgttatgcct     660

aatgtggtga catataactc ggttattgat gggctgtgca aggccagggc catggacaaa     720

gcagagacgg tccttagtca aatggtaaat gatggtgtcc gacctgataa cgtgacatat     780

aatagtctca ttcatggata ttcctctttg ggcaattgga aggaggcggt tagggtattc     840

aaagagatga gtcggaggtt tacaccagat gtcattacct gcacctcact catggccttc     900

ctttgcaaga atggaaaaag caaagaagct gcagaaattt ttgattcaat ggctatgaag     960

ggcctgaaac gtgacaccgt ttcatatgct attctccttc atgggtatgc cactgaagga    1020

tgcttggttg acatgattaa tctcttcaat tcaatggcaa cagactgtat tctacctaac    1080

tgtcatatct tcaatatact gattaatgca tatgctaaat ctggcatgat gggtgaggcc    1140

atgcttatat tcaaaaaaat gcacgaacaa ggagtgagtc cagatgtagt cacctattca    1200

actgtaatac atgcattttg caaaatgggt agcttggaca ttgctatgga caaatttaac    1260

cagatggttg atatgggagt acgaccgaat gcaattgttt atcattccct aatccagggt 1320

ttttgtacac atggggattt agtgaaagca aaggaattgg ttactgaaat gaggaacaaa 1380

gctatgcgtc ctcctgatat taagatcttc cattcaataa tgaagaacct atgcacggaa 1440

ggaagggtaa tagaagcacg ggatatcctc gacttgatgg tgcacatagg tatgaggcct 1500

gatgttccca tctttaattt actgattggt ggatactgcc tagtccacaa gatggaggat 1560

gcaacaaaaa tgtttgatga tatggtgtca tatggtttag aaccttgtaa cagtacgtat 1620

ggtatactta ttaatggcta ttgcaaaaac ggaaggattg atgacgggct gaatctgttc 1680

aaagaaatgt tgcgcaaggg acttaaacct acaactttta attacaacgt catactggat 1740

ggattatttc tggctggacg aactgttgct gcaaaggaaa agtttgatga gatggttgaa 1800

tctggagtaa gtgtgtgcat tgatacatat tcgatagttc ttggtggact ttgtagaaat 1860

aattgcaccg gtgaagcaat cacgctgtcc aagaaattaa gtacaatgaa tgtcaaattc 1920

aatattgcaa ttgtcaatat cttgattggt gcattcttca gggttcgacg aaaccaagaa 1980

gctaaggatt tgtttgccgc aataccagcc aatggcttgg tagctaatgt tgttacctac 2040

agcatgatga tgataaatct tataaaagaa gggtcactgg aagaagctga cgatctcttt 2100

ttatcaatgg agaagagcgg ctgtaccgct gactcttgta tgttaaatca tatcatcaga 2160

aggctactgg aaagaggaga gatagtcaag gctggaaatt atatgtcgaa agttgatgca 2220

aagagctact cacttgaagc taaaactgtt tcgctgctga tctccctctt tttagggaaa 2280

gggagatata gagaacacat gaaattgctc cctatgaagt atcagtttct cgaagaagca 2340

gccacagttg aa 2352

<210> 26
<211> 784
<212> PRT
<213> Triticum aestivum

<400> 26

Met Ser Pro Pro Arg Leu Arg Leu Leu Leu Leu Leu Leu Leu Leu Leu
1               5                   10                  15

Cys Ala Arg His Ser Ser Ser Thr Ser His Pro Ser Arg Ile Trp Asp
            20                  25                  30

Pro His Ala Ala Phe Ala Ala Ala Thr Glu Arg Ala Arg Ser Gly Arg
        35                  40                  45

Leu Thr Thr Glu Asp Ala His His Leu Phe Asp Glu Leu Leu Arg Gln
    50                  55                  60

Gly Asn Pro Val Gln Glu Arg Pro Leu Asn Asn Phe Leu Ala Ala Leu

65                  70                  75                  80

Ala Arg Ala Pro Ala Ser Ala Ser Cys Ser Asp Gly Pro Ala Leu Ala
                85                  90                  95

Val Ala Leu Phe Gly Arg Leu Ser Arg Gly Ala Gly Gln Arg Val Ala
            100                 105                 110

Gln Pro Asn Val Phe Thr Tyr Gly Val Leu Met Asp Cys Cys Cys Arg
        115                 120                 125

Ala Cys Arg Thr Asp Leu Ala Leu Ala Phe Phe Gly Arg Leu Leu Arg
    130                 135                 140

Thr Gly Leu Gln Ala Gly Gln Val Val Phe Asn Thr Leu Leu Lys Gly
145                 150                 155                 160

Leu Cys His Thr Lys Arg Ala Asp Glu Ala Leu Asp Val Leu Leu His
                165                 170                 175

Arg Met Pro Glu Leu Gly Cys Thr Pro Asp Val Val Ala Tyr Asn Thr
            180                 185                 190

Val Ile His Gly Phe Phe Arg Glu Gly Gln Val Gly Lys Ala Cys Asp
        195                 200                 205

Met Phe His Gly Met Ala Gln Lys Gly Val Met Pro Asn Val Val Thr
    210                 215                 220

Tyr Asn Ser Val Ile Asp Gly Leu Cys Lys Ala Arg Ala Met Asp Lys
225                 230                 235                 240

Ala Glu Thr Val Leu Ser Gln Met Val Asn Asp Gly Val Arg Pro Asp
                245                 250                 255

Asn Val Thr Tyr Asn Ser Leu Ile His Gly Tyr Ser Ser Leu Gly Asn
            260                 265                 270

Trp Lys Glu Ala Val Arg Val Phe Lys Glu Met Ser Arg Arg Phe Thr
        275                 280                 285

Pro Asp Val Ile Thr Cys Thr Ser Leu Met Ala Phe Leu Cys Lys Asn
    290                 295                 300

Gly Lys Ser Lys Glu Ala Ala Glu Ile Phe Asp Ser Met Ala Met Lys
305                 310                 315                 320

Gly Leu Lys Arg Asp Thr Val Ser Tyr Ala Ile Leu Leu His Gly Tyr
                325                 330                 335

Ala Thr Glu Gly Cys Leu Val Asp Met Ile Asn Leu Phe Asn Ser Met
            340                 345                 350

Ala Thr Asp Cys Ile Leu Pro Asn Cys His Ile Phe Asn Ile Leu Ile
        355                 360                 365

Asn Ala Tyr Ala Lys Ser Gly Met Met Gly Glu Ala Met Leu Ile Phe
    370                 375                 380

Lys Lys Met His Glu Gln Gly Val Ser Pro Asp Val Val Thr Tyr Ser
385                 390                 395                 400

Thr Val Ile His Ala Phe Cys Lys Met Gly Ser Leu Asp Ile Ala Met
                405                 410                 415

Asp Lys Phe Asn Gln Met Val Asp Met Gly Val Arg Pro Asn Ala Ile
            420                 425                 430

Val Tyr His Ser Leu Ile Gln Gly Phe Cys Thr His Gly Asp Leu Val
        435                 440                 445

Lys Ala Lys Glu Leu Val Thr Glu Met Arg Asn Lys Ala Met Arg Pro
    450                 455                 460

Pro Asp Ile Lys Ile Phe His Ser Ile Met Lys Asn Leu Cys Thr Glu
465                 470                 475                 480

Gly Arg Val Ile Glu Ala Arg Asp Ile Leu Asp Leu Met Val His Ile
                485                 490                 495

Gly Met Arg Pro Asp Val Pro Ile Phe Asn Leu Leu Ile Gly Gly Tyr
            500                 505                 510

Cys Leu Val His Lys Met Glu Asp Ala Thr Lys Met Phe Asp Asp Met
        515                 520                 525

Val Ser Tyr Gly Leu Glu Pro Cys Asn Ser Thr Tyr Gly Ile Leu Ile
    530                 535                 540

Asn Gly Tyr Cys Lys Asn Gly Arg Ile Asp Asp Gly Leu Asn Leu Phe
545                 550                 555                 560

Lys Glu Met Leu Arg Lys Gly Leu Lys Pro Thr Thr Phe Asn Tyr Asn
                565                 570                 575

Val Ile Leu Asp Gly Leu Phe Leu Ala Gly Arg Thr Val Ala Ala Lys
            580                 585                 590

Glu Lys Phe Asp Glu Met Val Glu Ser Gly Val Ser Val Cys Ile Asp
        595                 600                 605

Thr Tyr Ser Ile Val Leu Gly Gly Leu Cys Arg Asn Asn Cys Thr Gly
    610                 615                 620

Glu Ala Ile Thr Leu Ser Lys Lys Leu Ser Thr Met Asn Val Lys Phe
625                 630                 635                 640

Asn Ile Ala Ile Val Asn Ile Leu Ile Gly Ala Phe Phe Arg Val Arg
                645                 650                 655

Arg Asn Gln Glu Ala Lys Asp Leu Phe Ala Ala Ile Pro Ala Asn Gly
            660                 665                 670

Leu Val Ala Asn Val Val Thr Tyr Ser Met Met Met Ile Asn Leu Ile
        675                 680                 685

Lys Glu Gly Ser Leu Glu Glu Ala Asp Asp Leu Phe Leu Ser Met Glu
    690                 695                 700

Lys Ser Gly Cys Thr Ala Asp Ser Cys Met Leu Asn His Ile Ile Arg
705                 710                 715                 720

Arg Leu Leu Glu Arg Gly Glu Ile Val Lys Ala Gly Asn Tyr Met Ser
                725                 730                 735

Lys Val Asp Ala Lys Ser Tyr Ser Leu Glu Ala Lys Thr Val Ser Leu
            740                 745                 750

Leu Ile Ser Leu Phe Leu Gly Lys Gly Arg Tyr Arg Glu His Met Lys
        755                 760                 765

Leu Leu Pro Met Lys Tyr Gln Phe Leu Glu Glu Ala Ala Thr Val Glu
    770                 775                 780

<210>  27
<211>  1863
<212>  DNA
<213>  Artificial Sequence

<220>
<223>  cDNA of Rf1-3 of R3

<400>  27
acctacggca tcctcatgga ctgctgctgc cgtgcacatc gcctggatct ggcgtttgcc        60

ttcttcggcc gtctcctcag gacgggactg aaggcaggcg tcattctctg cactcttctc 120

aagggccttt gccgcgcaga gcgggctgat gaggccatgg acgtgctgct tcataggatg 180

cctgagctgg gctgcacccc caacgtggtg gcctatacca cggtcatcca cggcttcttg 240

aaggaaggcc aagtaggcaa ggcatgcaat ctattccatg gaatggcgca gcagggcgtt 300

gcacctaatg tggtgacata taactcgktt atcgatgcgc tatgcaaggc cagggccatg 360

ggaaaggcag agatggtcct tggtcaaatg gttgttaatg gtgttggacc kagtaacgtg 420

acatataata ctctcatcca tggatattcc tcttcaggtc atcagaagga ggcagttagg 480

gtgctgaaag aaatgagaag tcagggtatc ataccagatg tttttacttg cagctcgctc 540

atggcctccc tttgcaacaa cggaaaaagc aaagaagctg aagaaattct tcactccatg 600

gtcatgaagg gcctgaaacc tgacatcgtc tcgtacagta ctctgcttca cgggtatgcc 660

accgaaggat gcttagttgg tatgactaat ctcttcaatt caatgactag atatggcatt 720

cgccctaaca gtcatgcttt taacatactg attaatgtat gtgctaaatc tgrcatgatg 780

gatgaggcta tgcttatatt taaagaaatg cagaagcgag gagtgagtcc aaatctagtc 840

acctattcaa ctgtaataca tgcattttgc aaaatgggta gcttggacat tgctatggac 900

aaatttaacc agatggttga tataggagta cgaccggatg caattgttta tcgttcccta 960

atccatggtt tttgtacaca tggggattta gtgaaaccaa aggaattggt tactgaaatg 1020

aggaacaaag gtatgcgtcc tcctgatatt aagttcttcc attcaataat gcagaaccta 1080

tgcacggaag gaagggtaat agaagcacgg gatatcctcg acttgatagt gcgcattggt 1140

atgaggcctg atgttttcat atttaatata ctgatcggtg gatactgcct agtccgcaag 1200

atggcagatg catcaaaaat atttgatgat atggtgtcat atggtttaga accttcaaat 1260

attacgtatg gtatacttat taatggctat tgcaaaaaca gaaggattga tgagggcctg 1320

attctattca aagaaatgtt gtgcaaggga cttaaaccta caacttttaa ctataacgtc 1380

atactggatg gactatttct ggctggacga actgttgctg cgaaggakaa gtttgatgag 1440

atggttgaat ctggagtaag tgtgtgcatc gatacttact ctatatttct tgctggactt 1500

tgcagaaata attgctttag tgaagtaatc atgctgttcc agaaattaag cgcaatgaac 1560

atgaaattca atattagaat tgtcaatatc atgattgatg ccttctacaa ggttcggcga 1620

aaccawgaag ctaaggattt gtttgccgca ataccatcca atggcttggt agctaatgtt 1680

gttacctaca gcataatgat gacaaatctg ataaaagaag gttcagtgga agaagctgac 1740

actcttttta tcaatggaga agagyggctg tactgyaaac tcttggatgt taaatcatat 1800

tatcagaagg ttactggaaa gaggagagat agtcaaggct ggaaattata tgtcgaaagt 1860

tga 1863

<210>  28
<211>  620
<212>  PRT
<213>  Triticum aestivum

<220>
<221>  misc_feature
<222>  (110)..(110)
<223>  Xaa can be any naturally occurring amino acid

<220>
<221>  misc_feature
<222>  (137)..(137)
<223>  Xaa can be any naturally occurring amino acid

<220>
<221>  misc_feature
<222>  (258)..(258)
<223>  Xaa can be any naturally occurring amino acid

<220>
<221>  misc_feature
<222>  (476)..(476)
<223>  Xaa can be any naturally occurring amino acid

<220>
<221>  misc_feature
<222>  (542)..(542)
<223>  Xaa can be any naturally occurring amino acid

<220>
<221>  misc_feature
<222>  (589)..(589)
<223>  Xaa can be any naturally occurring amino acid

<220>
<221>  misc_feature
<222>  (592)..(592)
<223>  Xaa can be any naturally occurring amino acid

<400>  28

Thr Tyr Gly Ile Leu Met Asp Cys Cys Cys Arg Ala His Arg Leu Asp
1               5                   10                  15

Leu Ala Phe Ala Phe Phe Gly Arg Leu Leu Arg Thr Gly Leu Lys Ala
            20                  25                  30

Gly Val Ile Leu Cys Thr Leu Leu Lys Gly Leu Cys Arg Ala Glu Arg
        35                  40                  45

Ala Asp Glu Ala Met Asp Val Leu Leu His Arg Met Pro Glu Leu Gly
    50                  55                  60

Cys Thr Pro Asn Val Val Ala Tyr Thr Thr Val Ile His Gly Phe Leu
65                  70                  75                  80

Lys Glu Gly Gln Val Gly Lys Ala Cys Asn Leu Phe His Gly Met Ala
                85                  90                  95

Gln Gln Gly Val Ala Pro Asn Val Val Thr Tyr Asn Ser Xaa Ile Asp
            100                 105                 110

Ala Leu Cys Lys Ala Arg Ala Met Gly Lys Ala Glu Met Val Leu Gly
        115                 120                 125

Gln Met Val Val Asn Gly Val Gly Xaa Ser Asn Val Thr Tyr Asn Thr
    130                 135                 140

Leu Ile His Gly Tyr Ser Ser Ser Gly His Gln Lys Glu Ala Val Arg
145                 150                 155                 160

Val Leu Lys Glu Met Arg Ser Gln Gly Ile Ile Pro Asp Val Phe Thr
                165                 170                 175

Cys Ser Ser Leu Met Ala Ser Leu Cys Asn Asn Gly Lys Ser Lys Glu
            180                 185                 190

Ala Glu Glu Ile Leu His Ser Met Val Met Lys Gly Leu Lys Pro Asp
        195                 200                 205

Ile Val Ser Tyr Ser Thr Leu Leu His Gly Tyr Ala Thr Glu Gly Cys
    210                 215                 220

Leu Val Gly Met Thr Asn Leu Phe Asn Ser Met Thr Arg Tyr Gly Ile
225                 230                 235                 240

Arg Pro Asn Ser His Ala Phe Asn Ile Leu Ile Asn Val Cys Ala Lys
                245                 250                 255

Ser Xaa Met Met Asp Glu Ala Met Leu Ile Phe Lys Glu Met Gln Lys
            260                 265                 270

Arg Gly Val Ser Pro Asn Leu Val Thr Tyr Ser Thr Val Ile His Ala
        275                 280                 285

Phe Cys Lys Met Gly Ser Leu Asp Ile Ala Met Asp Lys Phe Asn Gln
    290                 295                 300

Met Val Asp Ile Gly Val Arg Pro Asp Ala Ile Val Tyr Arg Ser Leu
305                 310                 315                 320

Ile His Gly Phe Cys Thr His Gly Asp Leu Val Lys Pro Lys Glu Leu
                325                 330                 335

Val Thr Glu Met Arg Asn Lys Gly Met Arg Pro Pro Asp Ile Lys Phe
            340                 345                 350

Phe His Ser Ile Met Gln Asn Leu Cys Thr Glu Gly Arg Val Ile Glu
        355                 360                 365

Ala Arg Asp Ile Leu Asp Leu Ile Val Arg Ile Gly Met Arg Pro Asp
    370                 375                 380

Val Phe Ile Phe Asn Ile Leu Ile Gly Gly Tyr Cys Leu Val Arg Lys
385                 390                 395                 400

Met Ala Asp Ala Ser Lys Ile Phe Asp Asp Met Val Ser Tyr Gly Leu
                405                 410                 415

Glu Pro Ser Asn Ile Thr Tyr Gly Ile Leu Ile Asn Gly Tyr Cys Lys
            420                 425                 430

Asn Arg Arg Ile Asp Glu Gly Leu Ile Leu Phe Lys Glu Met Leu Cys
        435                 440                 445

Lys Gly Leu Lys Pro Thr Thr Phe Asn Tyr Asn Val Ile Leu Asp Gly
    450                 455                 460

Leu Phe Leu Ala Gly Arg Thr Val Ala Ala Lys Xaa Lys Phe Asp Glu
465                 470                 475                 480

Met Val Glu Ser Gly Val Ser Val Cys Ile Asp Thr Tyr Ser Ile Phe
                485                 490                 495

Leu Ala Gly Leu Cys Arg Asn Asn Cys Phe Ser Glu Val Ile Met Leu
            500                 505                 510

Phe Gln Lys Leu Ser Ala Met Asn Met Lys Phe Asn Ile Arg Ile Val
        515                 520                 525

Asn Ile Met Ile Asp Ala Phe Tyr Lys Val Arg Arg Asn Xaa Glu Ala
    530                 535                 540

Lys Asp Leu Phe Ala Ala Ile Pro Ser Asn Gly Leu Val Ala Asn Val
545                 550                 555                 560

Val Thr Tyr Ser Ile Met Met Thr Asn Leu Ile Lys Glu Gly Ser Val
                565                 570                 575

Glu Glu Ala Asp Thr Leu Phe Ile Asn Gly Glu Glu Xaa Leu Tyr Xaa

580                 585                 590

Lys Leu Leu Asp Val Lys Ser Tyr Tyr Gln Lys Val Thr Gly Lys Arg
        595                 600                 605

Arg Asp Ser Gln Gly Trp Lys Leu Tyr Val Glu Ser
    610                 615                 620

<210>  29
<211>  2217
<212>  DNA
<213>  Artificial Sequence

<220>
<223>  cDNA of pseudo1 Rf1 of R3

<400>  29
cacaccaagg ccatgcctcg cttctcctcc accacgccaa tggcgccacc ccgcctccgc      60

ctccgactcg gctcccgcca ctcctcctcc acctctcatc cctcacgcat ctgggatccc     120

cacgccgcct tcgccgccgc cgcagcgcgc gcgctctggc aacctcaccg cggaggacgc     180

acaccacctg ttcgacgaat tgctgcggca gggcaatcct gtccaggacc gccccctcac     240

aaactttctc cgccctcgcc cgtgctccgg cgtccgcggc ctgcggcgac ggcccgccct     300

cgcggtcgcc ctcttcagcc gcatatccca aggtgcccgc cgacgggtgg cagagccaac     360

ggcctgcact tacggtatcc tcatggactg ctgcagccgt gcgtgctgcc cggaactggc     420

gcttgccttc ttcgcccgtc tactgaggtc ggggctgagg gtaggtgtca gcaccctcct     480

caagggcctc tgccatgcaa agcgggcaga ggaggccatg gacgtgctgc ttcacaggat     540

gcccgagctg ggctgcaccc cgggcgtggt ggcatataac actgtcatca acggcttctt     600

taaggaaggc caagtaggca aggcgtgcag tctattccat ggaatgccgc agcggggcgt     660

tttgcctgat gtagtgacat atacctctgt tgtcgatggg ctgtgcaagg ccagagccat     720

ggacaaggca gagtatttcc ttcgtcagat ggttgatagt ggtgtcgtac ctgataatgt     780

gacatataat agcctcatcc atggatattc ctctttgggc catcagaagg aggctgttag     840

ggtgctgaaa gagatgaaag ggttacacca gatgtcatta cctgcacctc actcatggcc     900

ttcctttgca agaatggaaa aagcaaggaa gctgcagaaa tttttgattc aatggccacg     960

aagggcctga aacatgacgc cgtttcatat gctattctcc ttcatgggta tgccactgaa    1020

ggatgcttgg ttgatatgat taatctcttc aattcgatgg acagagactg tattctacct    1080

gacggtcgta tcttcaacat actgattaat gcatatgcta aatctgggaa ccttgataag    1140

gctatgctta tatttaaaga aatgcagaaa caaggagtga gtccagatgc agtcacatat    1200

tcaaccgtaa tacatgcttt ttgcaaaaag ggtaggttgg atgatgctgt gataaagttt    1260

aatcagatga ttgatgcagg agtacgaccg gacgcatctg tttatcgttc cctaatccag    1320

ggtttttgta cacatggcga tttggtgaaa gcaaaggaat atgttactga aatgatgaag 1380

aaaggtatgc ttcctcctga tattatgttc ttcagttcaa tcatgcagaa actatgcaca 1440

gaaggaaggg taatagaagc acgagatatc cttgacttga tagtgcgcat tggtatcagg 1500

cctgatgttt tcatatttaa tatactgatc ggtggatact gcctagtccg caagatggca 1560

gatgcatcaa aaatatttga tgatatggtg tcatatggtt tagaaccttg taatagtacg 1620

tatggtatac ttattaatgg ctattgcaaa aacggaagga ttgatgacgg gctgattctg 1680

ttcaaagaaa tgttgcccaa gggacttaaa cctacaactt ttaattacaa cgtcatactg 1740

gatggattat ttctggctgg acaaactgtt gctgcaaagg agaagtttga tgagatggtt 1800

gaatctggag taagtgtgtg catcgatact tactctatag ttcttggtgg actttgtaga 1860

aataattgtg ccggtgaagc aatcacgcta tttcagaaat taagcacaac gaatgtgaaa 1920

ttcgatatta gaattgtcaa tatcatgatt gatgccttct accgggttcg gcgaaaccag 1980

gaagctaagg atttgtttgc tgcagtatca gccaatggtt tggtagctaa tgtctttacc 2040

tacaccgtaa tgatgataaa tcttatagaa gaagggtcac tggaagaagc tgacactctt 2100

tttatcaatg gagaggagcg gctgtactgc caactcttat atgttaaatc atattatcag 2160

aaggttactg gaaagaggag agatagtcaa ggctggaaat tatatgtcga aagttga 2217

<210> 30
<211> 628
<212> PRT
<213> Triticum aestivum

<400> 30

Met Pro Arg Phe Ser Ser Thr Thr Pro Met Ala Pro Pro Arg Leu Arg
1               5                   10                  15

Leu Arg Leu Gly Ser Arg His Ser Ser Ser Thr Ser His Pro Ser Arg
            20                  25                  30

Ile Trp Asp Pro His Ala Ala Phe Ala Ala Ala Ala Ala Arg Ala Leu
        35                  40                  45

Trp Gln Pro His Arg Gly Gly Arg Thr Pro Pro Val Arg Arg Ile Ala
    50                  55                  60

Ala Ala Gly Gln Ser Cys Pro Gly Pro Pro Pro His Lys Leu Ser Pro
65                  70                  75                  80

Pro Ser Pro Val Leu Arg Arg Pro Arg Pro Ala Ala Thr Ala Arg Pro
                85                  90                  95

Arg Gly Arg Pro Leu Gln Pro His Ile Pro Arg Cys Pro Pro Thr Gly
            100                 105                 110

Gly Arg Ala Asn Gly Leu His Leu Arg Tyr Pro His Gly Leu Leu Gln
        115                 120                 125

Pro Cys Val Leu Pro Gly Thr Gly Ala Cys Leu Leu Arg Pro Ser Thr
    130                 135                 140

Glu Val Gly Ala Glu Gly Arg Cys Gln His Pro Pro Gln Gly Pro Leu
145                 150                 155                 160

Pro Cys Lys Ala Gly Arg Gly Gly His Gly Arg Ala Ala Ser Gln Asp
                165                 170                 175

Ala Arg Ala Gly Leu His Pro Gly Arg Gly Gly Ile Met Ala Phe Leu
            180                 185                 190

Cys Lys Asn Gly Lys Ser Lys Glu Ala Ala Glu Ile Phe Asp Ser Met
        195                 200                 205

Ala Thr Lys Gly Leu Lys His Asp Ala Val Ser Tyr Ala Ile Leu Leu
    210                 215                 220

His Gly Tyr Ala Thr Glu Gly Cys Leu Val Asp Met Ile Asn Leu Phe
225                 230                 235                 240

Asn Ser Met Asp Arg Asp Cys Ile Leu Pro Asp Gly Arg Ile Phe Asn
                245                 250                 255

Ile Leu Ile Asn Ala Tyr Ala Lys Ser Gly Asn Leu Asp Lys Ala Met
            260                 265                 270

Leu Ile Phe Lys Glu Met Gln Lys Gln Gly Val Ser Pro Asp Ala Val
        275                 280                 285

Thr Tyr Ser Thr Val Ile His Ala Phe Cys Lys Lys Gly Arg Leu Asp
    290                 295                 300

Asp Ala Val Ile Lys Phe Asn Gln Met Ile Asp Ala Gly Val Arg Pro
305                 310                 315                 320

Asp Ala Ser Val Tyr Arg Ser Leu Ile Gln Gly Phe Cys Thr His Gly
                325                 330                 335

Asp Leu Val Lys Ala Lys Glu Tyr Val Thr Glu Met Met Lys Lys Gly
            340                 345                 350

Met Leu Pro Pro Asp Ile Met Phe Phe Ser Ser Ile Met Gln Lys Leu
        355                 360                 365

Cys Thr Glu Gly Arg Val Ile Glu Ala Arg Asp Ile Leu Asp Leu Ile
    370                 375                 380

Val Arg Ile Gly Ile Arg Pro Asp Val Phe Ile Phe Asn Ile Leu Ile
385                 390                 395                 400

Gly Gly Tyr Cys Leu Val Arg Lys Met Ala Asp Ala Ser Lys Ile Phe
                405                 410                 415

Asp Asp Met Val Ser Tyr Gly Leu Glu Pro Cys Asn Ser Thr Tyr Gly
            420                 425                 430

Ile Leu Ile Asn Gly Tyr Cys Lys Asn Gly Arg Ile Asp Asp Gly Leu
        435                 440                 445

Ile Leu Phe Lys Glu Met Leu Pro Lys Gly Leu Lys Pro Thr Thr Phe
    450                 455                 460

Asn Tyr Asn Val Ile Leu Asp Gly Leu Phe Leu Ala Gly Gln Thr Val
465                 470                 475                 480

Ala Ala Lys Glu Lys Phe Asp Glu Met Val Glu Ser Gly Val Ser Val
                485                 490                 495

Cys Ile Asp Thr Tyr Ser Ile Val Leu Gly Gly Leu Cys Arg Asn Asn
            500                 505                 510

Cys Ala Gly Glu Ala Ile Thr Leu Phe Gln Lys Leu Ser Thr Thr Asn
        515                 520                 525

Val Lys Phe Asp Ile Arg Ile Val Asn Ile Met Ile Asp Ala Phe Tyr
    530                 535                 540

Arg Val Arg Arg Asn Gln Glu Ala Lys Asp Leu Phe Ala Ala Val Ser
545                 550                 555                 560

Ala Asn Gly Leu Val Ala Asn Val Phe Thr Tyr Thr Val Met Met Ile
                565                 570                 575

Asn Leu Ile Glu Glu Gly Ser Leu Glu Glu Ala Asp Thr Leu Phe Ile
            580                 585                 590

Asn Gly Glu Glu Arg Leu Tyr Cys Gln Leu Leu Tyr Val Lys Ser Tyr
        595                 600                 605

Tyr Gln Lys Val Thr Gly Lys Arg Arg Asp Ser Gln Gly Trp Lys Leu
    610                 615                 620

Tyr Val Glu Ser
625

<210>  31
<211>  1746
<212>  DNA
<213>  Artificial Sequence

<220>
<223>  cDNA of pseudo2 Rf1 of R3

<400>  31
ctcaagggac tctgccacgc aaagcgctca gatgaggctt tggacgtggt gctycacagg      60

atgcctgagc taggctgcac ccccaacgtg gtggcctata ccacggtcat ccacggcttc     120

ttgaaggaag gccaagtagg caaggcatgc aatctattcc atggaatggc gcagcagggc     180

gttgcgcctg atgtggtgac atataactcg gttatcgatg cgttgtgcaa ggccagagca     240

atggacaagg cagagtattt cctttgtgaa atggttgata atggtgtcgt acctaataat     300

gtgacatata atagcctcat ccatggatat tcctctttgg gccatcagaa ggaggctgtt     360

agggtgctga aagaaatgac aagacagggt atcataccag atgtcattac ctgcacctca     420

ctcatgacct tcctttgcaa gaatggaaaa agcaaggaag ctgcagaaat ttttgattaa     480

atgcccacga agggcctgaa acatgacgcc gtttcatatg ctattctcct tcatgggtat     540

gccactgaag gatgcttggt tgatatgatt aatctcttca attcgatgga cagagactgt     600

attctaccta actgtcatat cttcaacata ctgatttatg catatgctaa atctgggaag     660

cttgataagg ctatgcttat atttagagat atgcagaaac aaggagtgag tccagatgca     720

gtcacatatt caaccgtaat acatgcattt tgcaaaaagg gtaggttgga tgatgctgtg     780

ataaagttta atcagatgat tgatacagga gtacgaccgg aagcatctgc ttatcgttcc     840

ctaatccagg gtttttgtac acatggcgat ttggtgaaag caaaggaata tgttactgaa     900

atgatgaaga aaggtatgct tcctcctgat attatgttct tcagttcaat catgcagaaa     960

ctatgcacag aaggaagggt aatagaagca cgagatatcc ttgacttgat agtgcgcatt    1020

ggtatgaggc ctaatgtttt catatttaat atactgatcg gtgggtactg cctagtctgc    1080

aagatggagg atgcaacaaa aatgtttgag gatatggtgt catttggttt agaaccttgt    1140

aatattacgt atggtatact tattaatggc tattgcaaaa atagaaggat tgatgatggg    1200

cttattctgt tccaagagat gctgcacaag ggacttaaac ctacaacttt taattataat    1260

gtcatactgg atgggttatt tctggctgga cgaactgttg ctgcaaagga aaagtttgat    1320

gagatggttg aatctggagt aagtgtgtgc agtgatacat attcgatagt tcttgctgga 1380

ctttgcagaa ataattgctc tagcgaagta atcatgctgt tccagaaatt aagggcaatg 1440

aacatgaaat tcaatattag aattgtcact atcatgattg atgccttata caaggttcgg 1500

cgaaaccaag aagctaagga tttgtttgct gcaataccat ccaatggctt ggtagctaat 1560

gttgttacct acagcataat gatgaaaagt cttataaaag aaggttcagt ggaagaagct 1620

gacactcttt ttatcgatgg agaagagcgg ctgtactgca aactcttgga tgttaaatca 1680

tattatcaga aggttactgg aaagaggaga gatagtcaag gctggaaatt atatgtcgaa 1740

agttga 1746

<210> 32
<211> 580
<212> PRT
<213> Triticum aestivum

<220>
<221> misc_feature
<222> (18)..(18)
<223> Xaa can be any naturally occurring amino acid

<400> 32

Leu Lys Gly Leu Cys His Ala Lys Arg Ser Asp Glu Ala Leu Asp Val
1               5                   10                  15

Val Xaa His Arg Met Pro Glu Leu Gly Cys Thr Pro Asn Val Val Ala
            20                  25                  30

Tyr Thr Thr Val Ile His Gly Phe Leu Lys Glu Gly Gln Val Gly Lys
        35                  40                  45

Ala Cys Asn Leu Phe His Gly Met Ala Gln Gln Gly Val Ala Pro Asp
    50                  55                  60

Val Val Thr Tyr Asn Ser Val Ile Asp Ala Leu Cys Lys Ala Arg Ala
65                  70                  75                  80

Met Asp Lys Ala Glu Tyr Phe Leu Cys Glu Met Val Asp Asn Gly Val
                85                  90                  95

Val Pro Asn Asn Val Thr Tyr Asn Ser Leu Ile His Gly Tyr Ser Ser
            100                 105                 110

Leu Gly His Gln Lys Glu Ala Val Arg Val Leu Lys Glu Met Thr Arg
        115                 120                 125

Gln Gly Ile Ile Pro Asp Val Ile Thr Cys Thr Ser Leu Met Thr Phe

130                 135                 140

Leu Cys Lys Asn Gly Lys Ser Lys Glu Ala Ala Glu Ile Phe Asp Met
145                 150                 155                 160

Pro Thr Lys Gly Leu Lys His Asp Ala Val Ser Tyr Ala Ile Leu Leu
                165                 170                 175

His Gly Tyr Ala Thr Glu Gly Cys Leu Val Asp Met Ile Asn Leu Phe
            180                 185                 190

Asn Ser Met Asp Arg Asp Cys Ile Leu Pro Asn Cys His Ile Phe Asn
        195                 200                 205

Ile Leu Ile Tyr Ala Tyr Ala Lys Ser Gly Lys Leu Asp Lys Ala Met
    210                 215                 220

Leu Ile Phe Arg Asp Met Gln Lys Gln Gly Val Ser Pro Asp Ala Val
225                 230                 235                 240

Thr Tyr Ser Thr Val Ile His Ala Phe Cys Lys Lys Gly Arg Leu Asp
                245                 250                 255

Asp Ala Val Ile Lys Phe Asn Gln Met Ile Asp Thr Gly Val Arg Pro
            260                 265                 270

Glu Ala Ser Ala Tyr Arg Ser Leu Ile Gln Gly Phe Cys Thr His Gly
        275                 280                 285

Asp Leu Val Lys Ala Lys Glu Tyr Val Thr Glu Met Met Lys Lys Gly
    290                 295                 300

Met Leu Pro Pro Asp Ile Met Phe Phe Ser Ser Ile Met Gln Lys Leu
305                 310                 315                 320

Cys Thr Glu Gly Arg Val Ile Glu Ala Arg Asp Ile Leu Asp Leu Ile
                325                 330                 335

Val Arg Ile Gly Met Arg Pro Asn Val Phe Ile Phe Asn Ile Leu Ile
            340                 345                 350

Gly Gly Tyr Cys Leu Val Cys Lys Met Glu Asp Ala Thr Lys Met Phe
        355                 360                 365

Glu Asp Met Val Ser Phe Gly Leu Glu Pro Cys Asn Ile Thr Tyr Gly
    370                 375                 380

Ile Leu Ile Asn Gly Tyr Cys Lys Asn Arg Arg Ile Asp Asp Gly Leu
385                 390                 395                 400

Ile Leu Phe Gln Glu Met Leu His Lys Gly Leu Lys Pro Thr Thr Phe
                405                 410                 415

Asn Tyr Asn Val Ile Leu Asp Gly Leu Phe Leu Ala Gly Arg Thr Val
            420                 425                 430

Ala Ala Lys Glu Lys Phe Asp Glu Met Val Glu Ser Gly Val Ser Val
        435                 440                 445

Cys Ser Asp Thr Tyr Ser Ile Val Leu Ala Gly Leu Cys Arg Asn Asn
    450                 455                 460

Cys Ser Ser Glu Val Ile Met Leu Phe Gln Lys Leu Arg Ala Met Asn
465                 470                 475                 480

Met Lys Phe Asn Ile Arg Ile Val Thr Ile Met Ile Asp Ala Leu Tyr
                485                 490                 495

Lys Val Arg Arg Asn Gln Glu Ala Lys Asp Leu Phe Ala Ala Ile Pro
            500                 505                 510

Ser Asn Gly Leu Val Ala Asn Val Val Thr Tyr Ser Ile Met Met Lys
        515                 520                 525

Ser Leu Ile Lys Glu Gly Ser Val Glu Glu Ala Asp Thr Leu Phe Ile
    530                 535                 540

Asp Gly Glu Glu Arg Leu Tyr Cys Lys Leu Leu Asp Val Lys Ser Tyr
545                 550                 555                 560

Tyr Gln Lys Val Thr Gly Lys Arg Arg Asp Ser Gln Gly Trp Lys Leu
                565                 570                 575

Tyr Val Glu Ser
            580

<210>  33
<211>  2225
<212>  DNA
<213>  Artificial Sequence

<220>
<223>  cDNA of rf1-1 of Sperber

<400>  33
atgcctcgct tctcctccag cacgccaatg tcgccacccc gcctcctcct cctcctccgc        60

gcccgtcact cctcctccac ctctcatccc tcacgcatct gggatcccca cgccgccttc 120

gccgccgcga cgcagcgggc tcgctctggc acgctcacca cggaggacgc acaccacctg 180

ttcgacgaat tgctgcggca gggcaatcct gtccaggagc gtcccttgac taactttctg 240

tctgcccttg cccgcgcgcc cgcgtcccca ttctgcagcg atggccccgc cctggccgtc 300

gccctcttca gccgtttgtc ccgaggcgcc ggacgacggg tggcacagcc aaatgtcttc 360

acctatggag tcctaatgga ctgctgctgc cgtgcgcgcc gcttggatct agcgttcgcc 420

ttcttcgccc atctcctcaa gacgggactg gaggcaggcc aagtcgtttt cggcaccctc 480

ctcaagggac tctgccacgc aaagcgctca gatgaggctt tggacgtggt gcttcacagg 540

atgcctgagc taggctgcac ccccgacatg gtggcctata ccacggtcat ccacggcttc 600

ttgaaggaag gccaagtagg caaggcatgc aatctattcc atggaatggc gcagcagtcg 660

ttgcgcctga tgtggtgaca tatagctcag ttattgatgc gctgtgcaag gccagagcaa 720

tggacaaggc agagtatttc cttcgtgaaa tggttgataa tggtgtcgta cctaataatg 780

tgacatataa tagcctcatc catggatatt cctctttggg ccatcagaag gaggctgtta 840

gggtgctgaa agaaatgaca agacagggta tcatacctga tgtttttact tgcaactcgg 900

tcatggcctc cctttgcaag aacggaaaaa gcaaagaagc tgcaaaaatg tttgattcaa 960

tgtccatgaa gggcctgaaa cctaacaccg tttcatatgt tattctcctt catgggtatg 1020

ccactgaagg atgcctagtt gacatgatca atctcttcaa ttccatggca acagactgta 1080

ttctacctaa ctgtcatata ttcaacatac tgatttatgc atatgctaaa tctgggaagc 1140

tcgacaaggc tatgcttata tttaaagaaa tgcagaaaca aggagtgagt ccagatgcag 1200

tcacatattc aaccgtaata catgcatttt gcaaaaaggg taggttggat gatgctgtga 1260

taaagtttaa tcagatgatt gatacaggag tacgactgga cgcatctgtt tatcgttccc 1320

taatccaggg tttttgtaca catggcgatt tggtgaaagc aaaggaatat gttactgaaa 1380

tgatgaagaa aggtatgctt cctcctgata ttatgttctt cagttcaatc atgcagaacc 1440

tatgcacaga aggaagggta atagaagcac gagatatcct tgacttgata gtgtgcattg 1500

gtatgaggcc tgatgttttc atatttaata tactgatcgg cggatactgc ctagtccgca 1560

agatggcaga tgcatcaaaa atatttgatg atatggtgtc atatggttta gaaccttgta 1620

atattacgta tggtatactt attaatggct attgcaaaaa cggaaggatt gatgacgggc 1680

ttattctgtt caaggagatg ctgcacaagg gacttaaacc tacaactttt aattataacg 1740

tcatactgga tggattattt ctggctagac aaactgttgc tgcaaaggtg aagtttcatg 1800

agatggctga atctggagta agtgtgtgca ttgatacata ctctatagtt cttggtggac 1860

tttgtagaaa taattgcatc ggtgaagcaa tcacgccatt tcagaaatta agtacaatga 1920

atgttaaatt caatattgca attgtcaata tcatgattgg tgcattcttc agggtttggc 1980

gaaaccagga agctaaggat ttgtttgctg cagtatcagc caatggtttg gtagctaatg 2040

tctttaccta cactgtaatg gtgataaatc ttatagaaga agggtcactg gaagaagctg 2100

acactctttt tatcaatgaa gaagagcggc tgtactgcca actcttgtat gttaaatcat 2160

attatcagaa ggttactgga aagaggagag atagtcaagg ctggaaatta tatgtcgaaa 2220

gttga 2225

<210> 34
<211> 225
<212> PRT
<213> Triticum aestivum

<400> 34

Met Pro Arg Phe Ser Ser Ser Thr Pro Met Ser Pro Pro Arg Leu Leu
1               5                   10                  15

Leu Leu Leu Arg Ala Arg His Ser Ser Ser Thr Ser His Pro Ser Arg
            20                  25                  30

Ile Trp Asp Pro His Ala Ala Phe Ala Ala Ala Thr Gln Arg Ala Arg
        35                  40                  45

Ser Gly Thr Leu Thr Thr Glu Asp Ala His His Leu Phe Asp Glu Leu
    50                  55                  60

Leu Arg Gln Gly Asn Pro Val Gln Glu Arg Pro Leu Thr Asn Phe Leu
65                  70                  75                  80

Ser Ala Leu Ala Arg Ala Pro Ala Ser Pro Phe Cys Ser Asp Gly Pro
                85                  90                  95

Ala Leu Ala Val Ala Leu Phe Ser Arg Leu Ser Arg Gly Ala Gly Arg
            100                 105                 110

Arg Val Ala Gln Pro Asn Val Phe Thr Tyr Gly Val Leu Met Asp Cys
        115                 120                 125

Cys Cys Arg Ala Arg Arg Leu Asp Leu Ala Phe Ala Phe Phe Ala His
    130                 135                 140

Leu Leu Lys Thr Gly Leu Glu Ala Gly Gln Val Val Phe Gly Thr Leu
145                 150                 155                 160

Leu Lys Gly Leu Cys His Ala Lys Arg Ser Asp Glu Ala Leu Asp Val
                165                 170                 175

Val Leu His Arg Met Pro Glu Leu Gly Cys Thr Pro Asp Met Val Ala
            180                 185                 190

Tyr Thr Thr Val Ile His Gly Phe Leu Lys Glu Gly Gln Val Gly Lys
        195                 200                 205

Ala Cys Asn Leu Phe His Gly Met Ala Gln Gln Ser Leu Arg Leu Met
    210                 215                 220

Trp
225

<210>  35
<211>  1476
<212>  DNA
<213>  Artificial Sequence

<220>
<223>  cDNA of rf1-2 of Sperber

<400>  35
accaaggcca tgcctcgcct ctcctccacc acgccaatgt cgccactccg cctccgactc      60

cccgccggcc actcgtcctc cacctctcat ccctcacgca tctgggatcc ccacgccgcc     120

ttcgccgctg cgacgcagcg ggcgcgctct ggcacgctca ccacggagga cgcacaccac     180

ctgttcgacg aattgctgcg gcagggcaat cctgtccagg agcgtccctt gaagaacttt     240

ctggctgccc tcgcccgcgc gcccgcgtcc gcatcctgca gcgatggccc cgccctggcc     300

gtcgccctct tcggccgttt gtcccgaggc gccaggcaac gggtggcgca gccaaatgtg     360

ttcacctatg gcgtcctcat ggactgctac tgccgtgcgc gccgcctgga tctagcgatc     420

gccttcttcg gccgtctcct caagacgggc ctggaggcag accaagtcgt tttcagcacc     480

ctcctcaagg gactctgaca tgcaaagcgc tcagatgagg ctctggacgt gctgcttcac     540

aggatgcctg agctgggctg cacccctgac gtggtggcgt atagcacggt catccatggc     600

ttcttcaagg aaggccaagt aggcaaggca tgcaatctgt tccatggaat ggcacagcag     660

ggcgttgcgc ctgacgtggt gacatataac tcggttattg atgcactgtg caaggccaga     720

gcaatggaca aggcggagta tttccttggt cagatggttg atagtggtgt cgtacctaac     780

aatgtgacat ataatagcct catccatgga tattcctctt caggccattc aaaggaggcg     840

gtcagggtgc tgaaagatat gacaagtaag ggtatcatac cagatgtttt tacttgcaac     900

tcgctcatgg cctccctttg caagaatgga agaagcaaag aagctgcaga aatttttaat     960

tcaatgtcca tgaagggcct gaaacctaac accatttcat atgctattct ccttcatggg    1020

tatgccactg aaggatgctt ggttgacatg atcaatctct tcaattcaat ggctacagac    1080

tgtattctac ctaactgtca tatcttcaac atactgatta atgcatatgc taaatatggg    1140

aagcttgata aggctatgct tatatttaaa gatatgcaga aacaaggagt gagtccagat 1200

gcagtcacat attcaaccgt aatacatgca ttttgcaaaa agggtaggtt ggatgatgct 1260

gtgataaagt ttaatcagat gattgataca ggagtactac tggaagcatc tgtttatcgt 1320

tccctaatcc agggtttttg tacacatggc gatttggtga aagcaacgga atatgttact 1380

gaaatgatga agaaaggtat gcttcctcct gatattatgt tcttcagttc aatcatgcag 1440

aacctatgca cagaaggaag ggtaatagaa gcatga 1476

<210> 36
<211> 162
<212> PRT
<213> Triticum aestivum

<400> 36

Met Pro Arg Leu Ser Ser Thr Thr Pro Met Ser Pro Leu Arg Leu Arg
1               5                   10                  15

Leu Pro Ala Gly His Ser Ser Ser Thr Ser His Pro Ser Arg Ile Trp
            20                  25                  30

Asp Pro His Ala Ala Phe Ala Ala Ala Thr Gln Arg Ala Arg Ser Gly
        35                  40                  45

Thr Leu Thr Thr Glu Asp Ala His His Leu Phe Asp Glu Leu Leu Arg
    50                  55                  60

Gln Gly Asn Pro Val Gln Glu Arg Pro Leu Lys Asn Phe Leu Ala Ala
65                  70                  75                  80

Leu Ala Arg Ala Pro Ala Ser Ala Ser Cys Ser Asp Gly Pro Ala Leu
                85                  90                  95

Ala Val Ala Leu Phe Gly Arg Leu Ser Arg Gly Ala Arg Gln Arg Val
            100                 105                 110

Ala Gln Pro Asn Val Phe Thr Tyr Gly Val Leu Met Asp Cys Tyr Cys
        115                 120                 125

Arg Ala Arg Arg Leu Asp Leu Ala Ile Ala Phe Phe Gly Arg Leu Leu
    130                 135                 140

Lys Thr Gly Leu Glu Ala Asp Gln Val Val Phe Ser Thr Leu Leu Lys
145                 150                 155                 160

Gly Leu

<210>  37
<211>  2487
<212>  DNA
<213>  Triticum aestivum

<400>  37
ttcaactgtg gctggttttt ccagaaactg atactttata gggagcaatt tgatgtggtc      60

tctatattcc ccttcccttg aaaacagaga gatcagcagt gaaacagttt tagcttcaag     120

tgagtagctc tttacatcaa ctttagacat ataatttcca gccttgacta tctctcctct     180

ttccagtaac cttctgataa tatgatttaa catccaagag tttgcagtag agccgctctt     240

ctccattgat aaaaaaagat tgtcggcttc ttccactgaa ccttctttta taagatttgt     300

catcattatg gtgtaggtaa cagcattagg aaccaagcca ttggctggta tagcagcaaa     360

caaatcctta gcctcttggt ttctcttgac cctgtagaat gcaccaataa tgatattgac     420

aattttaata tcaaatttca cattcattgc acttaatttt cggaacaggg tgatcgcttc     480

gctactgcag ctatttctac aaagtccacc aagaactata gagtatgtat caatgcacac     540

acttactcca gattcaacca tctcatcaaa cttctctttt gcagcaacag tttgtccagc     600

cagaaataat ccatccagta tgacgttata attaacagtt gtaggtttaa gtcccttgtg     660

cagcatctct ttgaacagaa taagcccgtc atcaatcctt ctatttttgc aatagccatt     720

aataagtata ccatacgtaa agttacaagg ttctaaacca tatgacacca tatcatcaaa     780

tactttcgat gcatctgcca tcttgcggac taggcagtat ccaccaatca gtatattaaa     840

tgtgcaaaca ttaggcctca tacctatgtg tgctatcaag ccaaggatat cccgtgcttc     900

tattaccctt ccttctgtgc ataggttctg catgattgaa tggaagaact taatatcagg     960

aggaggtata cctttgttca tcatttcagt aagcaattcc tttgctttca ccaaatcgcc    1020

gtgtgtacaa aatccctgga ttagggaacg ataaacagct gtgccctgtc gtactcctgt    1080

atcaatcatc tgattaaact ttatcatagc atcgtccaac tgaccctttt tgcaaaatgc    1140

atctattacg gttgaatatg tgactgcatt tggactcact ccttgtttct gcatttcatt    1200

gaagataagc atagccttat caagcttccc agatttagca tatgcataaa tcagtatgct    1260

gaagatacga cagtcaggaa gaatgcagtc tctttccatt gaattgaaga gattaatcat    1320

atcaactaag catccttcgg tggcataccc atgaaggcga atagcatatg aaacgttgtc    1380

aggtttcagg cccttcatag ccatggaatc aaaaattcct gcagcttctt tgcttcttcc    1440

atggttgcaa agaaaggtca taaacatgtt gtaagtatgc aaatctggtg taaccctccg    1500

acttgtcatc tctttgaata ccctaacagc ctccttccag tggcctgaag aggaatatcc    1560

atggatgagg ctattatatg tcacattatc agatacgaca ccatcatcaa ccatctgacc    1620

aaggaaatac tctgccttgt ccattgcttt ggccttgcac aacgcatcaa taaccgagtt    1680

atatgtcaca acatcaggtg taacgccctg ctgcctcatt tcatggaata gattgcacgc 1740

cttgcctact tggccttccc taaagaagcc atggatgacc gtgttatatg ccaccacgtt 1800

gggggtgcag cctagctcag gcatcctgtg aagcagaatg tccagagcct catcagcccg 1860

ctttgcgcgg cagagtccct tgagaagagt gttgacttct atgacgcctg ccttgagtcc 1920

cgtcctgagg agacgggcga agaaggcgag cgccagatcc tggcggtttg cgcggcagca 1980

gcagtccatg aggatgccgt aggtgcaggc cgttgggtgc gccacacgtc ggcgggcgcc 2040

ttgggatatg cggctgaaga ggttgactgc gagtgcaggg ccatcgctgc acgccgcgga 2100

cgccggagca cgggcgaggg cggcaagaag gttgttgagg gggcgctcct ggacaggatt 2160

ggcctgccgc agcaattcgt cgaacaggtg gtgtgcgtcc tccggggtga ggttgcctga 2220

acgcgcacgc tccgtggcgg tggcgaaggc ggtgtgggga tcccagatgc gtgagggatg 2280

agaggtggag gaggagtggc gggcgcggag gcggagtggc gacatgggcg tggtggagga 2340

gaggcgaggc atggccttgg tgtgaagtgg gagcaagacg agtgggagac gacatgggcg 2400

agtagcattt tttttacagt gagtaggtcg tgaactatgg caagacgagt gggagattac 2460

tgttgcactt ttagttattt tctttct 2487

<210> 38
<211> 784
<212> PRT
<213> Triticum aestivum

<400> 38

Met Pro Arg Leu Ser Ser Thr Thr Pro Met Ser Pro Leu Arg Leu Arg
1               5                   10                  15

Ala Arg His Ser Ser Ser Thr Ser His Pro Ser Arg Ile Trp Asp Pro
            20                  25                  30

His Thr Ala Phe Ala Thr Ala Thr Glu Arg Ala Arg Ser Gly Asn Leu
        35                  40                  45

Thr Pro Glu Asp Ala His His Leu Phe Asp Glu Leu Leu Arg Gln Ala
    50                  55                  60

Asn Pro Val Gln Glu Arg Pro Leu Asn Asn Leu Leu Ala Ala Leu Ala
65                  70                  75                  80

Arg Ala Pro Ala Ser Ala Ala Cys Ser Asp Gly Pro Ala Leu Ala Val
                85                  90                  95

Asn Leu Phe Ser Arg Ile Ser Gln Gly Ala Arg Arg Arg Val Ala His
            100                 105                 110

Pro Thr Ala Cys Thr Tyr Gly Ile Leu Met Asp Cys Cys Cys Arg Ala
        115                 120                 125

Asn Arg Gln Asp Leu Ala Leu Ala Phe Phe Ala Arg Leu Leu Arg Thr
    130                 135                 140

Gly Leu Lys Ala Gly Val Ile Glu Val Asn Thr Leu Leu Lys Gly Leu
145                 150                 155                 160

Cys Arg Ala Lys Arg Ala Asp Glu Ala Leu Asp Ile Leu Leu His Arg
                165                 170                 175

Met Pro Glu Leu Gly Cys Thr Pro Asn Val Val Ala Tyr Asn Thr Val
            180                 185                 190

Ile His Gly Phe Phe Arg Glu Gly Gln Val Gly Lys Ala Cys Asn Leu
        195                 200                 205

Phe His Glu Met Arg Gln Gln Gly Val Thr Pro Asp Val Val Thr Tyr
    210                 215                 220

Asn Ser Val Ile Asp Ala Leu Cys Lys Ala Lys Ala Met Asp Lys Ala
225                 230                 235                 240

Glu Tyr Phe Leu Gly Gln Met Val Asp Asp Gly Val Val Ser Asp Asn
                245                 250                 255

Val Thr Tyr Asn Ser Leu Ile His Gly Tyr Ser Ser Ser Gly His Trp
            260                 265                 270

Lys Glu Ala Val Arg Val Phe Lys Glu Met Thr Ser Arg Arg Val Thr
        275                 280                 285

Pro Asp Leu His Thr Tyr Asn Met Phe Met Thr Phe Leu Cys Asn His
    290                 295                 300

Gly Arg Ser Lys Glu Ala Ala Gly Ile Phe Asp Ser Met Ala Met Lys
305                 310                 315                 320

Gly Leu Lys Pro Asp Asn Val Ser Tyr Ala Ile Arg Leu His Gly Tyr
                325                 330                 335

Ala Thr Glu Gly Cys Leu Val Asp Met Ile Asn Leu Phe Asn Ser Met
            340                 345                 350

Glu Arg Asp Cys Ile Leu Pro Asp Cys Arg Ile Phe Ser Ile Leu Ile
        355                 360                 365

Tyr Ala Tyr Ala Lys Ser Gly Lys Leu Asp Lys Ala Met Leu Ile Phe
    370                 375                 380

Asn Glu Met Gln Lys Gln Gly Val Ser Pro Asn Ala Val Thr Tyr Ser
385                 390                 395                 400

Thr Val Ile Asp Ala Phe Cys Lys Lys Gly Gln Leu Asp Asp Ala Met
                405                 410                 415

Ile Lys Phe Asn Gln Met Ile Asp Thr Gly Val Arg Gln Gly Thr Ala
            420                 425                 430

Val Tyr Arg Ser Leu Ile Gln Gly Phe Cys Thr His Gly Asp Leu Val
        435                 440                 445

Lys Ala Lys Glu Leu Leu Thr Glu Met Met Asn Lys Gly Ile Pro Pro
    450                 455                 460

Pro Asp Ile Lys Phe Phe His Ser Ile Met Gln Asn Leu Cys Thr Glu
465                 470                 475                 480

Gly Arg Val Ile Glu Ala Arg Asp Ile Leu Gly Leu Ile Ala His Ile
                485                 490                 495

Gly Met Arg Pro Asn Val Cys Thr Phe Asn Ile Leu Ile Gly Gly Tyr
            500                 505                 510

Cys Leu Val Arg Lys Met Ala Asp Ala Ser Lys Val Phe Asp Asp Met
        515                 520                 525

Val Ser Tyr Gly Leu Glu Pro Cys Asn Phe Thr Tyr Gly Ile Leu Ile
    530                 535                 540

Asn Gly Tyr Cys Lys Asn Arg Arg Ile Asp Asp Gly Leu Ile Leu Phe
545                 550                 555                 560

Lys Glu Met Leu His Lys Gly Leu Lys Pro Thr Thr Val Asn Tyr Asn
                565                 570                 575

Val Ile Leu Asp Gly Leu Phe Leu Ala Gly Gln Thr Val Ala Ala Lys
            580                 585                 590

Glu Lys Phe Asp Glu Met Val Glu Ser Gly Val Ser Val Cys Ile Asp
        595                 600                 605

Thr Tyr Ser Ile Val Leu Gly Gly Leu Cys Arg Asn Ser Cys Ser Ser

610                 615                 620

Glu Ala Ile Thr Leu Phe Arg Lys Leu Ser Ala Met Asn Val Lys Phe
625                 630                 635                 640

Asp Ile Lys Ile Val Asn Ile Ile Ile Gly Ala Phe Tyr Arg Val Lys
                645                 650                 655

Arg Asn Gln Glu Ala Lys Asp Leu Phe Ala Ala Ile Pro Ala Asn Gly
            660                 665                 670

Leu Val Pro Asn Ala Val Thr Tyr Thr Ile Met Met Thr Asn Leu Ile
        675                 680                 685

Lys Glu Gly Ser Val Glu Glu Ala Asp Asn Leu Phe Leu Ser Met Glu
    690                 695                 700

Lys Ser Gly Ser Thr Ala Asn Ser Trp Met Leu Asn His Ile Ile Arg
705                 710                 715                 720

Arg Leu Leu Glu Arg Gly Glu Ile Val Lys Ala Gly Asn Tyr Met Ser
                725                 730                 735

Lys Val Asp Val Lys Ser Tyr Ser Leu Glu Ala Lys Thr Val Ser Leu
            740                 745                 750

Leu Ile Ser Leu Phe Ser Arg Glu Gly Glu Tyr Arg Asp His Ile Lys
        755                 760                 765

Leu Leu Pro Ile Lys Tyr Gln Phe Leu Glu Lys Pro Ala Thr Val Glu
    770                 775                 780

<210>  39
<211>  2400
<212>  DNA
<213>  Triticum aestivum

<400>  39
cacaactgta gctgcttctt cacgaaaccg atactttgca gggagcaatc ctatgtgttc      60

tctatatttc cctttccctg aaaagagaga gataagcagc gaaacagttt tagcttcaag     120

tgagtagctc tttgcatcaa ttttagatat ataattccca gccttgacta tctctccttt     180

tcccagtaac attctgacga taagatttaa catataagag tcagcagtac agctgctctt     240

ctccattgat aaaaagagat cgtcagcttc ttccactgac ccttctttta taagattttt     300

catcattatg gtgtaggtaa agacattagc taccaaacca ttggctgata ctgcagcaaa     360

caaatccttg gcttcttggt ttcgctgaac cttgtagaaa gcatcaatca ttatattgac     420

aattataata ttgaatttca catccattgt gcttaatttc tggaatagcg tgattgcttc 480

gccgctacaa ttatttctac aaagtccacc aagaactatc gaatatgtac caatgcacac 540

acttactcca gattcaacca tctcatcaaa cttttccttt gcagcaacag ttcgtccagc 600

cagaaataat ccatccagta tgacgttata attaaaagtt gtaggtttaa gtcccttgtg 660

cagcatctct ttgaacagaa taagcccgtc atcaatcctt ctatttttgc aatagccatt 720

aataagtata ccatacgtaa tattagaagg ttctaagcca tatgacatca tatcatcaaa 780

tatttttgag gcatgttcca tcttgccgac taggcagtat ccaccgatca gtatattaaa 840

tgtgcaaaca tcaggcctca tacctatgtg cgctatcaag ccaaggacat cccgtgcttc 900

tattaccctt ccttctgtgc aaaggttctg catgattgaa ttgaagaatg caatatcagg 960

aggaagcata cctttgttca tcatttcagt aagcaattcc tttgctttca ccaaatcgcc 1020

gtgtgtacaa aaaccctgga ttagggaacg ataaacagct gtgccctgtc gtactcctgt 1080

atcaatcatc tgattaaact ttatcacagc atcgtccaac ctaccctttt tgcaaaatgc 1140

atgtattatg gttgaatatg tgactgcatt tggactcact ccttgtttct gcatttcatt 1200

gaagataagc atggccttat caagcttccc agatttagca tgtgcattaa tcagtatgtt 1260

gaagatacga cagtcaggta gaatgcagta tctttccatt gaattgaaga gattaatcat 1320

atcaactaag catccttcgg tggcataccc gtgaaggaga atagcatatg aaacgttgtt 1380

atgtttcagg cccttcatag ccatggtatc aaaaattcct gcagcttctt tgcttcttcc 1440

atgtttgcaa agaaaggtca taaacatgtt gtgagtatgc acatttggtt taaccctccg 1500

acttgtcatc tctttgaata ccctaactgc ctccttccag tggcctgaag aggaatatcc 1560

atggatgagg ctattatatg tcacattatc aggtacgaca ccatcatcaa ccatctgacg 1620

aaggaaatac tctgccttgt ccatggctct ggccttgcac agcgcatcaa taaccgagtt 1680

atatgtcacc aaactaggcg caacgccctg ctgcgccatt tcatggaaca agctgcacgc 1740

cttgcctgct tggccttctt ggaagaagcc atggatgacc gtgttatatg ccaccacgtt 1800

gggggtgcag cctagctcag gcatcctgtg aagcagaatg tccagagcct catcagcccg 1860

ctttgcgcgg cagagtccct tgagaagagt gttgacttct atgacgcctg ccttgagtcc 1920

cgtcctgagg agacgggcga agaaggcgag cgccagatcc tggcggtttg cgcggcagca 1980

gcagtccatg aggatgccgt aggtgcaggc cgttggctgc accacccgtt ggcgggcacc 2040

ttgggacaaa cggctgaaga gggcgagggc aagggcgggg ccgttgctgc aggctgcgga 2100

cgcaggcgcg cgagccaggg cggtgagaag gtcgttggga aggcgcatgt tgaccggatt 2160

agcctgcagg agcaattggt cgaacagctt gtgtgcgtcc tccgaggtga ggttgccaga 2220

tcgagctcgc tccgtggcag cagcgaaggc ggcgtgggga tcccagccct ggctgcgtga 2280

gggatgcgag gtggagtagg agtggcgggc atggaggcgg ggccggaggc ggaggcggag 2340

tggcgacatt agctgcgtgg tggtctggtg gaggagaggc aagttcgtcc caggttccgc     2400

<210>  40
<211>  783
<212>  PRT
<213>  Triticum aestivum

<400>  40

Met Ser Pro Leu Arg Leu Arg Leu Arg Pro Arg Leu His Ala Arg His
1               5                   10                  15

Ser Tyr Ser Thr Ser His Pro Ser Arg Ser Gln Gly Trp Asp Pro His
            20                  25                  30

Ala Ala Phe Ala Ala Ala Thr Glu Arg Ala Arg Ser Gly Asn Leu Thr
        35                  40                  45

Ser Glu Asp Ala His Lys Leu Phe Asp Gln Leu Leu Leu Gln Ala Asn
    50                  55                  60

Pro Val Asn Met Arg Leu Pro Asn Asp Leu Leu Thr Ala Leu Ala Arg
65                  70                  75                  80

Ala Pro Ala Ser Ala Ala Cys Ser Asn Gly Pro Ala Leu Ala Leu Ala
                85                  90                  95

Leu Phe Ser Arg Leu Ser Gln Gly Ala Arg Gln Arg Val Val Gln Pro
            100                 105                 110

Thr Ala Cys Thr Tyr Gly Ile Leu Met Asp Cys Cys Cys Arg Ala Asn
        115                 120                 125

Arg Gln Asp Leu Ala Leu Ala Phe Phe Ala Arg Leu Leu Arg Thr Gly
    130                 135                 140

Leu Lys Ala Gly Val Ile Glu Val Asn Thr Leu Leu Lys Gly Leu Cys
145                 150                 155                 160

Arg Ala Lys Arg Ala Asp Glu Ala Leu Asp Ile Leu Leu His Arg Met
                165                 170                 175

Pro Glu Leu Gly Cys Thr Pro Asn Val Val Ala Tyr Asn Thr Val Ile
            180                 185                 190

His Gly Phe Phe Gln Glu Gly Gln Ala Gly Lys Ala Cys Ser Leu Phe
        195                 200                 205

His Glu Met Ala Gln Gln Gly Val Ala Pro Ser Leu Val Thr Tyr Asn
    210                 215                 220

Ser Val Ile Asp Ala Leu Cys Lys Ala Arg Ala Met Asp Lys Ala Glu
225                 230                 235                 240

Tyr Phe Leu Arg Gln Met Val Asp Asp Gly Val Val Pro Asp Asn Val
                245                 250                 255

Thr Tyr Asn Ser Leu Ile His Gly Tyr Ser Ser Ser Gly His Trp Lys
            260                 265                 270

Glu Ala Val Arg Val Phe Lys Glu Met Thr Ser Arg Arg Val Lys Pro
        275                 280                 285

Asn Val His Thr His Asn Met Phe Met Thr Phe Leu Cys Lys His Gly
    290                 295                 300

Arg Ser Lys Glu Ala Ala Gly Ile Phe Asp Thr Met Ala Met Lys Gly
305                 310                 315                 320

Leu Lys His Asn Asn Val Ser Tyr Ala Ile Leu Leu His Gly Tyr Ala
                325                 330                 335

Thr Glu Gly Cys Leu Val Asp Met Ile Asn Leu Phe Asn Ser Met Glu
            340                 345                 350

Arg Tyr Cys Ile Leu Pro Asp Cys Arg Ile Phe Asn Ile Leu Ile Asn
        355                 360                 365

Ala His Ala Lys Ser Gly Lys Leu Asp Lys Ala Met Leu Ile Phe Asn
    370                 375                 380

Glu Met Gln Lys Gln Gly Val Ser Pro Asn Ala Val Thr Tyr Ser Thr
385                 390                 395                 400

Ile Ile His Ala Phe Cys Lys Lys Gly Arg Leu Asp Asp Ala Val Ile
                405                 410                 415

Lys Phe Asn Gln Met Ile Asp Thr Gly Val Arg Gln Gly Thr Ala Val
            420                 425                 430

Tyr Arg Ser Leu Ile Gln Gly Phe Cys Thr His Gly Asp Leu Val Lys
        435                 440                 445

Ala Lys Glu Leu Leu Thr Glu Met Met Asn Lys Gly Met Leu Pro Pro
    450                 455                 460

Asp Ile Ala Phe Phe Asn Ser Ile Met Gln Asn Leu Cys Thr Glu Gly
465                 470                 475                 480

Arg Val Ile Glu Ala Arg Asp Val Leu Gly Leu Ile Ala His Ile Gly
                485                 490                 495

Met Arg Pro Asp Val Cys Thr Phe Asn Ile Leu Ile Gly Gly Tyr Cys
            500                 505                 510

Leu Val Gly Lys Met Glu His Ala Ser Lys Ile Phe Asp Asp Met Met
        515                 520                 525

Ser Tyr Gly Leu Glu Pro Ser Asn Ile Thr Tyr Gly Ile Leu Ile Asn
    530                 535                 540

Gly Tyr Cys Lys Asn Arg Arg Ile Asp Asp Gly Leu Ile Leu Phe Lys
545                 550                 555                 560

Glu Met Leu His Lys Gly Leu Lys Pro Thr Thr Phe Asn Tyr Asn Val
                565                 570                 575

Ile Leu Asp Gly Leu Phe Leu Ala Gly Arg Thr Val Ala Ala Lys Glu
            580                 585                 590

Lys Phe Asp Glu Met Val Glu Ser Gly Val Ser Val Cys Ile Gly Thr
        595                 600                 605

Tyr Ser Ile Val Leu Gly Gly Leu Cys Arg Asn Asn Cys Ser Gly Glu
    610                 615                 620

Ala Ile Thr Leu Phe Gln Lys Leu Ser Thr Met Asp Val Lys Phe Asn
625                 630                 635                 640

Ile Ile Ile Val Asn Ile Met Ile Asp Ala Phe Tyr Lys Val Gln Arg
                645                 650                 655

Asn Gln Glu Ala Lys Asp Leu Phe Ala Ala Val Ser Ala Asn Gly Leu
            660                 665                 670

Val Ala Asn Val Phe Thr Tyr Thr Ile Met Met Lys Asn Leu Ile Lys
        675                 680                 685

Glu Gly Ser Val Glu Glu Ala Asp Asp Leu Phe Leu Ser Met Glu Lys
    690                 695                 700

Ser Ser Cys Thr Ala Asp Ser Tyr Met Leu Asn Leu Ile Val Arg Met
705                 710                 715                 720

Leu Leu Gly Lys Gly Glu Ile Val Lys Ala Gly Asn Tyr Ile Ser Lys
                725                 730                 735

Ile Asp Ala Lys Ser Tyr Ser Leu Glu Ala Lys Thr Val Ser Leu Leu
            740                 745                 750

Ile Ser Leu Phe Ser Gly Lys Gly Lys Tyr Arg Glu His Ile Gly Leu
        755                 760                 765

Leu Pro Ala Lys Tyr Arg Phe Arg Glu Glu Ala Ala Thr Val Val
    770                 775                 780

<210>  41
<211>  2571
<212>  DNA
<213>  Triticum aestivum

<400>  41
attcactcat tttgttccgt atgtagtcat ttgttgaaat ctctagagag acaattattt      60

aggaacggag ggagtaagat aactacccta aaaaaaaaga taactgaagg ttgccaccta     120

gcacattcac attggtacaa cttggaaaag cacagccccg tcgtcctgct cccagttgag     180

ttcgcgacct acacaccggc catgccccgc ttctcctcca ccacgccaat gtcgccaccc     240

cgcctccgcc tccgcctccg actctgcgcc cgccactcct cctccacctc tcatccctca     300

cgcatctggg atccccacgc cgccttcgcc gccgcggcac agcgggcgag ctctggcacg     360

ctcactacgg aggacgcaca ccacctgttt gacgaattgc tgcggcgggg caatcctgtc     420

caggagcgtc ccttgaataa atttctggct gccctcgccc gcgcgcccgc gtccgcatcc     480

tgctgcgatg gccccgccct ggcagtcacc ctcttcggcc gtttgtcccg agacgtcgga     540

cgacgggtgg cgcagccaaa tgtcttcacc tatggcgtcc tcatggactg ctgctgccgc     600

gcttgccgca cagatctggt gctcgccttc tttggccgtc tcctcaagac gggcctggag     660

gcaaaccaag tcgtcttcaa caccctcctc aagggccttt gccacacaaa gcgggcggat     720

gaggctctgg acgtgctgct tcacaggatg cctgagctgg gctgcactcc taatgtggtg     780

gcgtataaca ccgttatcca tggcttcttt aaggaaggcc atgtaagcaa ggcctgcaat     840

ctgttccatg aaatggcgca gcagggcgtt aagcctaatg tggtgacata taactcagtt     900

attgatgcgc tgtgcaaggc cagagccatg gacaaggcag aggtggtcct tcgtcagatg     960

attgatgatg gtgttggacc tgataatgtg acgtatagta gcctcatcca tggatattcc    1020

tcttcaggcc actggaagga ggcagttagg gtattcaaag agatgacaag tcggagggtt    1080

acagcagatg tgcatactta caacatgttt atgacctttc tttgcaaaca tggaagaagc    1140

aaagaagctg caggaatttt tgataccatg gctatcaagg gcctgaaacc tgacaacgtt    1200

tcatatgcta ttcgccttca tgggtatgcc accgaaggat gcctagttga catgatcaat 1260

ctcttcaatt ccatggcaac acactgcatt ctacctaact gtcatatatt caacatactg 1320

attaatgcat atgctaaatc tgggaagctt gataaggcta tgcttatctt caatgaaatg 1380

cagaaacaag gagtgagtcc aaatgcagtc acatattcaa ccgtaataca tgcattttgc 1440

aagaagggta ggttggatga tgctgtgata aagtttaatc agatgattga tacaggagta 1500

cgaccggacg catctgttta tcgtccccta atccagggtt tttgtacaca tggcgatttg 1560

gtgaaagcaa aggaatatgt tactgaaatg atgaagaaag gtatgcctcc tcctgatatt 1620

atgttcttca gttcaatcat gcagaaccta tgcacagaag gaagggtaac agaagcacgg 1680

gatatccttg acttgatagt gcacattggt atgaggccta atgttatcat atttaatttg 1740

ctgatcggtg gatactgcct agtccgcaag atggcagatg cattgaaagt atttgatgat 1800

atggtgtcat atggtttaga accttgtaac tttacgtatg gtatacttat taatggctat 1860

tgcaaaaata gaaggattga tgacgggctt attctgttca aagagatgct gcacaaggga 1920

cttaaaccta caacttttaa ttataacgtc atactggatg gattatttct ggctggacaa 1980

actgttgctg caaaagagaa gtttgatgag atggttgaat ctggagtaag tgtgtgcatt 2040

gatacatact ctataattct tggtggactt tgtagaaata gctgcagtag cgaagcgatc 2100

acccttttcc ggaaattaag cgcaatgaat gtgaaatttg atattacaat tgtcaatatc 2160

attattggtg ccttatacag ggtcgagaga aaccaagagg ctaaggattt gtttgctgct 2220

atgccagcca atggcttggt tcctaatgct gttacctaca ccgtaatgat gacaaatctt 2280

ataaaagaag gttcagtgga agaagctgac aatcttttct tatccatgga gaagagcggc 2340

tgtactgcca actcttgcct gttaaatcat atcatcagaa ggttactgga aaaaggagag 2400

atagtcaagg ctggaaatta tatgtctaaa gttgatgcaa agagctactc acttgaagct 2460

aaaactgttt cgctgctgat ctctctgttt tcaaggaaag ggaaatatag agaacacatc 2520

aaattgcttc ctacaaagta tcagtttctg gaagaagcag ccacagttga a 2571

<210> 42
<211> 790
<212> PRT
<213> Triticum aestivum

<400> 42

Met Pro Arg Phe Ser Ser Thr Thr Pro Met Ser Pro Pro Arg Leu Arg
1               5                   10                  15

Leu Arg Leu Arg Leu Cys Ala Arg His Ser Ser Ser Thr Ser His Pro
            20                  25                  30

Ser Arg Ile Trp Asp Pro His Ala Ala Phe Ala Ala Ala Ala Gln Arg
        35                  40                  45

Ala Ser Ser Gly Thr Leu Thr Thr Glu Asp Ala His His Leu Phe Asp
    50                  55                  60

Glu Leu Leu Arg Arg Gly Asn Pro Val Gln Glu Arg Pro Leu Asn Lys
65                  70                  75                  80

Phe Leu Ala Ala Leu Ala Arg Ala Pro Ala Ser Ala Ser Cys Cys Asp
                85                  90                  95

Gly Pro Ala Leu Ala Val Thr Leu Phe Gly Arg Leu Ser Arg Asp Val
            100                 105                 110

Gly Arg Arg Val Ala Gln Pro Asn Val Phe Thr Tyr Gly Val Leu Met
        115                 120                 125

Asp Cys Cys Cys Arg Ala Cys Arg Thr Asp Leu Val Leu Ala Phe Phe
    130                 135                 140

Gly Arg Leu Leu Lys Thr Gly Leu Glu Ala Asn Gln Val Val Phe Asn
145                 150                 155                 160

Thr Leu Leu Lys Gly Leu Cys His Thr Lys Arg Ala Asp Glu Ala Leu
                165                 170                 175

Asp Val Leu Leu His Arg Met Pro Glu Leu Gly Cys Thr Pro Asn Val
            180                 185                 190

Val Ala Tyr Asn Thr Val Ile His Gly Phe Phe Lys Glu Gly His Val
        195                 200                 205

Ser Lys Ala Cys Asn Leu Phe His Glu Met Ala Gln Gln Gly Val Lys
    210                 215                 220

Pro Asn Val Val Thr Tyr Asn Ser Val Ile Asp Ala Leu Cys Lys Ala
225                 230                 235                 240

Arg Ala Met Asp Lys Ala Glu Val Val Leu Arg Gln Met Ile Asp Asp
                245                 250                 255

Gly Val Gly Pro Asp Asn Val Thr Tyr Ser Ser Leu Ile His Gly Tyr
            260                 265                 270

Ser Ser Ser Gly His Trp Lys Glu Ala Val Arg Val Phe Lys Glu Met
        275                 280                 285

Thr Ser Arg Arg Val Thr Ala Asp Val His Thr Tyr Asn Met Phe Met
    290                 295                 300

Thr Phe Leu Cys Lys His Gly Arg Ser Lys Glu Ala Ala Gly Ile Phe
305                 310                 315                 320

Asp Thr Met Ala Ile Lys Gly Leu Lys Pro Asp Asn Val Ser Tyr Ala
                325                 330                 335

Ile Arg Leu His Gly Tyr Ala Thr Glu Gly Cys Leu Val Asp Met Ile
            340                 345                 350

Asn Leu Phe Asn Ser Met Ala Thr His Cys Ile Leu Pro Asn Cys His
        355                 360                 365

Ile Phe Asn Ile Leu Ile Asn Ala Tyr Ala Lys Ser Gly Lys Leu Asp
    370                 375                 380

Lys Ala Met Leu Ile Phe Asn Glu Met Gln Lys Gln Gly Val Ser Pro
385                 390                 395                 400

Asn Ala Val Thr Tyr Ser Thr Val Ile His Ala Phe Cys Lys Lys Gly
                405                 410                 415

Arg Leu Asp Asp Ala Val Ile Lys Phe Asn Gln Met Ile Asp Thr Gly
            420                 425                 430

Val Arg Pro Asp Ala Ser Val Tyr Arg Pro Leu Ile Gln Gly Phe Cys
        435                 440                 445

Thr His Gly Asp Leu Val Lys Ala Lys Glu Tyr Val Thr Glu Met Met
    450                 455                 460

Lys Lys Gly Met Pro Pro Pro Asp Ile Met Phe Phe Ser Ser Ile Met
465                 470                 475                 480

Gln Asn Leu Cys Thr Glu Gly Arg Val Thr Glu Ala Arg Asp Ile Leu
                485                 490                 495

Asp Leu Ile Val His Ile Gly Met Arg Pro Asn Val Ile Ile Phe Asn
            500                 505                 510

Leu Leu Ile Gly Gly Tyr Cys Leu Val Arg Lys Met Ala Asp Ala Leu
        515                 520                 525

Lys Val Phe Asp Asp Met Val Ser Tyr Gly Leu Glu Pro Cys Asn Phe
    530                 535                 540

Thr Tyr Gly Ile Leu Ile Asn Gly Tyr Cys Lys Asn Arg Arg Ile Asp
545                 550                 555                 560

Asp Gly Leu Ile Leu Phe Lys Glu Met Leu His Lys Gly Leu Lys Pro
                565                 570                 575

Thr Thr Phe Asn Tyr Asn Val Ile Leu Asp Gly Leu Phe Leu Ala Gly
            580                 585                 590

Gln Thr Val Ala Ala Lys Glu Lys Phe Asp Glu Met Val Glu Ser Gly
        595                 600                 605

Val Ser Val Cys Ile Asp Thr Tyr Ser Ile Ile Leu Gly Gly Leu Cys
    610                 615                 620

Arg Asn Ser Cys Ser Ser Glu Ala Ile Thr Leu Phe Arg Lys Leu Ser
625                 630                 635                 640

Ala Met Asn Val Lys Phe Asp Ile Thr Ile Val Asn Ile Ile Ile Gly
                645                 650                 655

Ala Leu Tyr Arg Val Glu Arg Asn Gln Glu Ala Lys Asp Leu Phe Ala
            660                 665                 670

Ala Met Pro Ala Asn Gly Leu Val Pro Asn Ala Val Thr Tyr Thr Val
        675                 680                 685

Met Met Thr Asn Leu Ile Lys Glu Gly Ser Val Glu Glu Ala Asp Asn
    690                 695                 700

Leu Phe Leu Ser Met Glu Lys Ser Gly Cys Thr Ala Asn Ser Cys Leu
705                 710                 715                 720

Leu Asn His Ile Ile Arg Arg Leu Leu Glu Lys Gly Glu Ile Val Lys
                725                 730                 735

Ala Gly Asn Tyr Met Ser Lys Val Asp Ala Lys Ser Tyr Ser Leu Glu
            740                 745                 750

Ala Lys Thr Val Ser Leu Leu Ile Ser Leu Phe Ser Arg Lys Gly Lys
        755                 760                 765

Tyr Arg Glu His Ile Lys Leu Leu Pro Thr Lys Tyr Gln Phe Leu Glu
    770                 775                 780

Glu Ala Ala Thr Val Glu

785                790

<210>  43
<211>  1956
<212>  DNA
<213>  Triticum aestivum

<400>  43
cttctgcacc aagtcatctt ctgcaccctc ctcaagggcc tggaggcaaa ccaagtcatc      60

ttctgcaccc tcctcaaggg actctgccac gcaaagcgca cagatgaggc tctggacgtg     120

ctgcttcaca ggatgcctga gctgggctgc acccccaacg tggtggcata taccacggtc     180

atccacggct tctttaagga aggccaagta ggcaaggcat gcaatctgtt ccatggaatg     240

gcgcagcagg gcgttgcgcc taatttggtg acatataact cggttattga tgcgttgtgc     300

aaggccaaag caatggacaa ggcagagtat ttccttggtc agatggttga tgatggtgtc     360

gtacctgata atgtgacata taatagcctc atccatggat attcctcttc aggccactgg     420

aaggaggcag ttagggtatt caaagagatg acaagtcgga gggttacagc agatgtgcat     480

acttacaaca tgtttatgac ctttctttgc aaacatggaa gaagcaaaga agctgcagga     540

atttttgata ccatggctat gaagggcctg aaacctgaca acgtttcata tgctattctc     600

cttcatgggt atgccaccga aggatgctta gttgatatga ttaatctctt caattcaatg     660

gaaagagatt gtattctacc tgactgtcgt atcttcaaca tactgattaa tgcatatgct     720

aaatctggga agcttgataa ggctatgctt atcttcaatg aaatgcagaa acaaggagtg     780

agtccaaatg cagtcacata ttcaactgta atagatgcat tttgcaaaaa gggtcagttg     840

gacgatgcta tgataaagtt taatcagatg attgatacag gagtacgaca gggcacagct     900

gtttatggtt ctctaatcca gggtttttgt acacacggcg atttggtgaa agcaaaggaa     960

ttgcttactg aaatgatgaa caaaggtatg cttcctcctg atatcaagtt cttccattca    1020

atcatgcaga acctttgcac agaaggaagg gtaatagaag cacgggatgt ccttggcttg    1080

atagcgcaca taggtatgag gcctgatgtt tgcacattta atatactgat cggtggatac    1140

tgcctagtcg gcaagatgga ggatgcctca aaaatatttg atgatatgat gtcatatggc    1200

ttagaacctt ctaatattac gtatggtata cttattaatg gctattgcaa aaatagaagg    1260

attgatgacg ggcttattct gttcaaagag atgctgcaca agggacttaa acctacaact    1320

tttaattata acatcatact ggatgggtta cttctggctg gacgaactgt tgctgcaaag    1380

gaaaagttta atgagatggt tgaatctgga gtaagtgtgt gcactgatac atattcaata    1440

gttcttggtg gacttcgcag aaataattgc tttagcgaag caatcatgct gttccagaaa    1500

ttaagggcaa tgaacatgaa attcaatatt agaattgtca atatcatgat tgatgccttc    1560

tttagggttc agagaaagca agaagctaaa gatttgtttg ccgcaatacc agccaatggc    1620

ttggtagcta atgttgttac ctacagcata acgatgacaa atcttataaa agaaggttca 1680

gtggaagaag ctgacgatct ctttttatca atggagaaga gcggctgtac tgctgactct 1740

tatatgttaa atcttatcgt cagaatgtta ctggaaaaag gagagctagt caaggctggg 1800

aattatatgt ctaaagttga tgcaatgagc tactcacttg aagctgaaac cgtttcgctg 1860

cttatctctc tcttttcagg gaaagggaaa tatagagaac acataggatt gctccctgca 1920

aagtatcggt ttcgtgaaga agcagccaca gttgtg 1956

<210> 44
<211> 608
<212> PRT
<213> Triticum aestivum

<400> 44

Met Pro Glu Leu Gly Cys Thr Pro Asn Val Val Ala Tyr Thr Thr Val
1               5                   10                  15

Ile His Gly Phe Phe Lys Glu Gly Gln Val Gly Lys Ala Cys Asn Leu
            20                  25                  30

Phe His Gly Met Ala Gln Gln Gly Val Ala Pro Asn Leu Val Thr Tyr
        35                  40                  45

Asn Ser Val Ile Asp Ala Leu Cys Lys Ala Lys Ala Met Asp Lys Ala
    50                  55                  60

Glu Tyr Phe Leu Gly Gln Met Val Asp Asp Gly Val Val Pro Asp Asn
65                  70                  75                  80

Val Thr Tyr Asn Ser Leu Ile His Gly Tyr Ser Ser Ser Gly His Trp
                85                  90                  95

Lys Glu Ala Val Arg Val Phe Lys Glu Met Thr Ser Arg Arg Val Thr
            100                 105                 110

Ala Asp Val His Thr Tyr Asn Met Phe Met Thr Phe Leu Cys Lys His
        115                 120                 125

Gly Arg Ser Lys Glu Ala Ala Gly Ile Phe Asp Thr Met Ala Met Lys
    130                 135                 140

Gly Leu Lys Pro Asp Asn Val Ser Tyr Ala Ile Leu Leu His Gly Tyr
145                 150                 155                 160

Ala Thr Glu Gly Cys Leu Val Asp Met Ile Asn Leu Phe Asn Ser Met
                165                 170                 175

Glu Arg Asp Cys Ile Leu Pro Asp Cys Arg Ile Phe Asn Ile Leu Ile
        180                 185                 190

Asn Ala Tyr Ala Lys Ser Gly Lys Leu Asp Lys Ala Met Leu Ile Phe
    195                 200                 205

Asn Glu Met Gln Lys Gln Gly Val Ser Pro Asn Ala Val Thr Tyr Ser
    210                 215                 220

Thr Val Ile Asp Ala Phe Cys Lys Lys Gly Gln Leu Asp Asp Ala Met
225                 230                 235                 240

Ile Lys Phe Asn Gln Met Ile Asp Thr Gly Val Arg Gln Gly Thr Ala
                245                 250                 255

Val Tyr Gly Ser Leu Ile Gln Gly Phe Cys Thr His Gly Asp Leu Val
            260                 265                 270

Lys Ala Lys Glu Leu Leu Thr Glu Met Met Asn Lys Gly Met Leu Pro
        275                 280                 285

Pro Asp Ile Lys Phe Phe His Ser Ile Met Gln Asn Leu Cys Thr Glu
    290                 295                 300

Gly Arg Val Ile Glu Ala Arg Asp Val Leu Gly Leu Ile Ala His Ile
305                 310                 315                 320

Gly Met Arg Pro Asp Val Cys Thr Phe Asn Ile Leu Ile Gly Gly Tyr
                325                 330                 335

Cys Leu Val Gly Lys Met Glu Asp Ala Ser Lys Ile Phe Asp Asp Met
            340                 345                 350

Met Ser Tyr Gly Leu Glu Pro Ser Asn Ile Thr Tyr Gly Ile Leu Ile
        355                 360                 365

Asn Gly Tyr Cys Lys Asn Arg Arg Ile Asp Asp Gly Leu Ile Leu Phe
    370                 375                 380

Lys Glu Met Leu His Lys Gly Leu Lys Pro Thr Thr Phe Asn Tyr Asn
385                 390                 395                 400

Ile Ile Leu Asp Gly Leu Leu Leu Ala Gly Arg Thr Val Ala Ala Lys
                405                 410                 415

Glu Lys Phe Asn Glu Met Val Glu Ser Gly Val Ser Val Cys Thr Asp
            420                 425                 430

Thr Tyr Ser Ile Val Leu Gly Gly Leu Arg Arg Asn Asn Cys Phe Ser
        435                 440                 445

Glu Ala Ile Met Leu Phe Gln Lys Leu Arg Ala Met Asn Met Lys Phe
    450                 455                 460

Asn Ile Arg Ile Val Asn Ile Met Ile Asp Ala Phe Phe Arg Val Gln
465                 470                 475                 480

Arg Lys Gln Glu Ala Lys Asp Leu Phe Ala Ala Ile Pro Ala Asn Gly
                485                 490                 495

Leu Val Ala Asn Val Val Thr Tyr Ser Ile Thr Met Thr Asn Leu Ile
            500                 505                 510

Lys Glu Gly Ser Val Glu Glu Ala Asp Asp Leu Phe Leu Ser Met Glu
        515                 520                 525

Lys Ser Gly Cys Thr Ala Asp Ser Tyr Met Leu Asn Leu Ile Val Arg
    530                 535                 540

Met Leu Leu Glu Lys Gly Glu Leu Val Lys Ala Gly Asn Tyr Met Ser
545                 550                 555                 560

Lys Val Asp Ala Met Ser Tyr Ser Leu Glu Ala Glu Thr Val Ser Leu
                565                 570                 575

Leu Ile Ser Leu Phe Ser Gly Lys Gly Lys Tyr Arg Glu His Ile Gly
            580                 585                 590

Leu Leu Pro Ala Lys Tyr Arg Phe Arg Glu Glu Ala Ala Thr Val Val
        595                 600                 605

<210>  45
<211>  2478
<212>  DNA
<213>  Triticum aestivum

<400>  45
gatgtttgtg cagaaaatac ggagggagta caacttggaa aagcacctct gccgcatcgt      60

ctcctcccac tcgtcttgct cagcgttcac gacccacaca ccaaggccat gcctcgcttc     120

tcctccacca cgcccatgtc gccactccgc ctccgcctcc gcctccgact cggcgcccgc     180

cactcctcct ccacctccca ttcctcacgc agctgggatc cccactccgc cttcgccgcc     240

gccgcggagc gtgcgcgctc tggcatcctc accccggagg acgcacacca cctgttcgac     300

gaattgctcc ggcagggcaa tcctgtccag gaccgcccct tgaacaaact tctctccgcc     360

ctcgcccgtg ctctggcgtc cgcgacctgc agaaacggcc ctgcactcgc ggtcgccctc 420

ttcagccgca tatcccaagg tgcccgccga cgtgtggcag agccaacggc ctgcacctac 480

ggcatcctca tggactgctg ctgccgcgca caccgcctcg atctggtgtt cgctttcttc 540

ggccgtctcc tcaggacggg acttaaggca ggcgtcattc aagtttgcac tcttctcaag 600

ggcctctgcc gcgcaaagcg ggctgatgag gctctcgacg tgctgcttca caggatgcct 660

gagctgggct gcacccccaa cgtggtggca tataccacgg tcatccacgg cttctttaag 720

gaaggccaag taggcaaggc atgcaatctg ttccatggaa tggcgcagca gggcgttgcg 780

cctaatttgg tgacatataa ctcggttatt gatgcgttgt gcaaggccaa agcaatggac 840

aaggcagagt atttccttgg tcagatggtt gatgatggtg tcgtacctga taatgtgaca 900

tataatagcc tcatccatgg atattcctct tcaggccact ggaaggaggc agttagggta 960

ttcaaagaga tgacaagtcg gagggttaca gcagatgtgc atacttacaa catgtttatg 1020

acctttcttt gcaaacatgg aagaagcaaa gaagctgcag gaatttttga taccatggct 1080

atcaagggcc tgaaacctga caacgtttca tatgctattc tccttcatgg gtatgccacc 1140

gaaggatgct tagttgatat gattaatctc ttcaattcaa tggaaagaga ttgtattcta 1200

cctgactgtc gtatcttcaa catactgatt aatgcatatg ctaaatctgg gaagcttgat 1260

aaggctatgc ttatcttcaa tgaaatgcag aaacaaggag cgagtccaga tgcagtcacg 1320

tattcaactg taatagatgc attttgcaaa aagggtcagt tggacgatgc tatgataaag 1380

tttaatcaga tgattgatac aggagtacca cagggcacag ctgtttatgg ttctctaatc 1440

cagggttttt gtacacacgg cgatttggtg aaagcaaagg aattggttac tgaaatgatg 1500

aacaaaggta tacctcctcc tgatattaag ttcttccatt caatcatgca gaacctttgc 1560

acagaaggaa gggtaataga agcacgggat gtccttggct tgatagcgca catagggatg 1620

aagcctaatg tttgcacatt taatatactg ggcggtggat attgcctagt cggcaagatg 1680

gaggatgcct caaaaatatt tgatgatatg atgtcatatg gcttagaacc ttctaatatt 1740

acgtatggta tacttattaa tggctattgc aaaaacgaaa ggattaacga tgggttgatt 1800

ctgttcaaag aaatgttgcg caagggactt aaacctacaa cttttaatta caacgtcata 1860

ctggatggat tatttctaac tggacgaact gttgctgcaa aggaaaagtt tgatgagatg 1920

gttgaatctg gagtaagtgt gtgcattgat acatattcga tagttcttgg tggactttgt 1980

agaaataatt gtagcggcga agcaatcatg ctattccaga aattaagcac aatggatgtg 2040

aaattcaata ttacaattgt caatataatg attgatgcct tctacagggt tcagcgaaac 2100

caagaagcca aggatttgtt tgctgcagta tcagccaatg gtttggtagg taatgtcttt 2160

acctacacca taatgatgaa aaatcttata aaagaagggt cagtggaaga agctgacgat 2220

ctctttttat caatggagaa gagcggctgt actgctgact cttatatgtt aaatcttatc 2280

gtcagaatgt tactggaaaa aggagagcta gtcaaggctg ggaattatat gtctaaagtt 2340

gatgcaatga gctactcact tgaagctgaa accgtttcgc tgcttatctc tctcttttca 2400

gggaaaggga aatatagaga acacatagga ttgctccctg caaagtatcg gtttcgtgaa 2460

gaagcagcca cagttgtg 2478

<210> 46
<211> 790
<212> PRT
<213> Triticum aestivum

<400> 46

Met Pro Arg Phe Ser Ser Thr Thr Pro Met Ser Pro Leu Arg Leu Arg
1               5                   10                  15

Leu Arg Leu Arg Leu Gly Ala Arg His Ser Ser Ser Thr Ser His Ser
            20                  25                  30

Ser Arg Ser Trp Asp Pro His Ser Ala Phe Ala Ala Ala Ala Glu Arg
        35                  40                  45

Ala Arg Ser Gly Ile Leu Thr Pro Glu Asp Ala His His Leu Phe Asp
    50                  55                  60

Glu Leu Leu Arg Gln Gly Asn Pro Val Gln Asp Arg Pro Leu Asn Lys
65                  70                  75                  80

Leu Leu Ser Ala Leu Ala Arg Ala Leu Ala Ser Ala Thr Cys Arg Asn
                85                  90                  95

Gly Pro Ala Leu Ala Val Ala Leu Phe Ser Arg Ile Ser Gln Gly Ala
            100                 105                 110

Arg Arg Arg Val Ala Glu Pro Thr Ala Cys Thr Tyr Gly Ile Leu Met
        115                 120                 125

Asp Cys Cys Cys Arg Ala His Arg Leu Asp Leu Val Phe Ala Phe Phe
    130                 135                 140

Gly Arg Leu Leu Arg Thr Gly Leu Lys Ala Gly Val Ile Gln Val Cys
145                 150                 155                 160

Thr Leu Leu Lys Gly Leu Cys Arg Ala Lys Arg Ala Asp Glu Ala Leu
                165                 170                 175

Asp Val Leu Leu His Arg Met Pro Glu Leu Gly Cys Thr Pro Asn Val

180                 185                 190

Val Ala Tyr Thr Thr Val Ile His Gly Phe Phe Lys Glu Gly Gln Val
        195                 200                 205

Gly Lys Ala Cys Asn Leu Phe His Gly Met Ala Gln Gln Gly Val Ala
    210                 215                 220

Pro Asn Leu Val Thr Tyr Asn Ser Val Ile Asp Ala Leu Cys Lys Ala
225                 230                 235                 240

Lys Ala Met Asp Lys Ala Glu Tyr Phe Leu Gly Gln Met Val Asp Asp
                245                 250                 255

Gly Val Val Pro Asp Asn Val Thr Tyr Asn Ser Leu Ile His Gly Tyr
            260                 265                 270

Ser Ser Ser Gly His Trp Lys Glu Ala Val Arg Val Phe Lys Glu Met
        275                 280                 285

Thr Ser Arg Arg Val Thr Ala Asp Val His Thr Tyr Asn Met Phe Met
    290                 295                 300

Thr Phe Leu Cys Lys His Gly Arg Ser Lys Glu Ala Ala Gly Ile Phe
305                 310                 315                 320

Asp Thr Met Ala Ile Lys Gly Leu Lys Pro Asp Asn Val Ser Tyr Ala
                325                 330                 335

Ile Leu Leu His Gly Tyr Ala Thr Glu Gly Cys Leu Val Asp Met Ile
            340                 345                 350

Asn Leu Phe Asn Ser Met Glu Arg Asp Cys Ile Leu Pro Asp Cys Arg
        355                 360                 365

Ile Phe Asn Ile Leu Ile Asn Ala Tyr Ala Lys Ser Gly Lys Leu Asp
    370                 375                 380

Lys Ala Met Leu Ile Phe Asn Glu Met Gln Lys Gln Gly Ala Ser Pro
385                 390                 395                 400

Asp Ala Val Thr Tyr Ser Thr Val Ile Asp Ala Phe Cys Lys Lys Gly
                405                 410                 415

Gln Leu Asp Asp Ala Met Ile Lys Phe Asn Gln Met Ile Asp Thr Gly
            420                 425                 430

Val Pro Gln Gly Thr Ala Val Tyr Gly Ser Leu Ile Gln Gly Phe Cys
        435                 440                 445

Thr His Gly Asp Leu Val Lys Ala Lys Glu Leu Val Thr Glu Met Met
    450                 455                 460

Asn Lys Gly Ile Pro Pro Pro Asp Ile Lys Phe Phe His Ser Ile Met
465                 470                 475                 480

Gln Asn Leu Cys Thr Glu Gly Arg Val Ile Glu Ala Arg Asp Val Leu
                485                 490                 495

Gly Leu Ile Ala His Ile Gly Met Lys Pro Asn Val Cys Thr Phe Asn
            500                 505                 510

Ile Leu Gly Gly Gly Tyr Cys Leu Val Gly Lys Met Glu Asp Ala Ser
        515                 520                 525

Lys Ile Phe Asp Asp Met Met Ser Tyr Gly Leu Glu Pro Ser Asn Ile
    530                 535                 540

Thr Tyr Gly Ile Leu Ile Asn Gly Tyr Cys Lys Asn Glu Arg Ile Asn
545                 550                 555                 560

Asp Gly Leu Ile Leu Phe Lys Glu Met Leu Arg Lys Gly Leu Lys Pro
                565                 570                 575

Thr Thr Phe Asn Tyr Asn Val Ile Leu Asp Gly Leu Phe Leu Thr Gly
            580                 585                 590

Arg Thr Val Ala Ala Lys Glu Lys Phe Asp Glu Met Val Glu Ser Gly
        595                 600                 605

Val Ser Val Cys Ile Asp Thr Tyr Ser Ile Val Leu Gly Gly Leu Cys
    610                 615                 620

Arg Asn Asn Cys Ser Gly Glu Ala Ile Met Leu Phe Gln Lys Leu Ser
625                 630                 635                 640

Thr Met Asp Val Lys Phe Asn Ile Thr Ile Val Asn Ile Met Ile Asp
                645                 650                 655

Ala Phe Tyr Arg Val Gln Arg Asn Gln Glu Ala Lys Asp Leu Phe Ala
            660                 665                 670

Ala Val Ser Ala Asn Gly Leu Val Gly Asn Val Phe Thr Tyr Thr Ile
        675                 680                 685

Met Met Lys Asn Leu Ile Lys Glu Gly Ser Val Glu Glu Ala Asp Asp
    690                 695                 700

Leu Phe Leu Ser Met Glu Lys Ser Gly Cys Thr Ala Asp Ser Tyr Met
705                 710                 715                 720

Leu Asn Leu Ile Val Arg Met Leu Leu Glu Lys Gly Glu Leu Val Lys
                725                 730                 735

Ala Gly Asn Tyr Met Ser Lys Val Asp Ala Met Ser Tyr Ser Leu Glu
            740                 745                 750

Ala Glu Thr Val Ser Leu Leu Ile Ser Leu Phe Ser Gly Lys Gly Lys
        755                 760                 765

Tyr Arg Glu His Ile Gly Leu Leu Pro Ala Lys Tyr Arg Phe Arg Glu
    770                 775                 780

Glu Ala Ala Thr Val Val
785                 790

<210>  47
<211>  201
<212>  DNA
<213>  Triticum aestivum

<400>  47
tattcaactg tggctggttt ttccagaaac tgatacttta tagggagcaa tttgatgtgg      60

tctctatatt ccccttccct tgaaaacaga gagatcagca rtgaaacagt tttagcttca     120

agtgagtagc tctttacatc aactttagac atataatttc cagccttgac tatctctcct     180

ctttccagta accttctgat a                                               201

<210>  48
<211>  201
<212>  DNA
<213>  Triticum aestivum

<400>  48
ctttataggg agcaatttga tgtggtctct atattcccct tcccttgaaa acagagagat      60

cagcagtgaa acagttttag cttcaagtga gtagctcttt rcatcaactt tagacatata     120

atttccagcc ttgactatct ctcctctttc cagtaacctt ctgataatat gatttaacat     180

ccaagagttt gcagtagagc c                                               201

<210>  49
<211>  201
<212>  DNA
<213>  Triticum aestivum

<400>  49
tgaaacagtt ttagcttcaa gtgagtagct ctttacatca actttagaca tataatttcc      60

agccttgact atctctcctc tttccagtaa ccttctgata rtatgattta acatccaaga     120

gtttgcagta gagccgctct tctccattga taaaaaaaga ttgtcggctt cttccactga     180

accttctttt ataagatttg t                                               201


<210>  50
<211>  201
<212>  DNA
<213>  Triticum aestivum

<400>  50
agtgagtagc tctttacatc aactttagac atataatttc cagccttgac tatctctcct      60

ctttccagta accttctgat aatatgattt aacatccaag mgtttgcagt agagccgctc     120

ttctccattg ataaaaaaag attgtcggct tcttccactg aaccttcttt tataagattt     180

gtcatcatta tggtgtaggt a                                               201


<210>  51
<211>  201
<212>  DNA
<213>  Triticum aestivum

<400>  51
ctttacatca actttagaca tataatttcc agccttgact atctctcctc tttccagtaa      60

ccttctgata atatgattta acatccaaga gtttgcagta sagccgctct tctccattga     120

taaaaaaaga ttgtcggctt cttccactga accttctttt ataagatttg tcatcattat     180

ggtgtaggta acagcattag g                                               201


<210>  52
<211>  201
<212>  DNA
<213>  Triticum aestivum

<400>  52
agcccgtcat caatccttct atttttgcaa tagccattaa taagtatacc atacgtaaag      60

ttacaaggtt ctaaaccata tgacaccata tcatcaaata ytttcgatgc atctgccatc     120

ttgcggacta ggcagtatcc accaatcagt atattaaatg tgcaaacatt aggcctcata     180

cctatgtgtg ctatcaagcc a                                               201


<210>  53
<211>  201
<212>  DNA
<213>  Triticum aestivum

<400>  53
cgtcatcaat ccttctattt ttgcaatagc cattaataag tataccatac gtaaagttac      60

aaggttctaa accatatgac accatatcat caaatacttt ygatgcatct gccatcttgc 120

ggactaggca gtatccacca atcagtatat taaatgtgca aacattaggc ctcataccta 180

tgtgtgctat caagccaagg a 201

<210> 54
<211> 201
<212> DNA
<213> Triticum aestivum

<400> 54
caatgcacac acttactcca gattcaacca tctcatcaaa cttttccttt gcagcaacag 60

ttcgtccagc cagaaataat ccatccagta tgacgttata rttaaaagtt gtaggtttaa 120

gtcccttgtg cagcatctct ttgaacagaa taagcccgtc atcaatcctt ctatttttgc 180

aatagccatt aataagtata c 201

<210> 55
<211> 201
<212> DNA
<213> Triticum aestivum

<400> 55
agcatctctt tgaacagaat aagcccgtca tcaatccttc tatttttgca atagccatta 60

ataagtatac catacgtaat attagaaggt tctaagccat rtgacatcat atcatcaaat 120

atttttgagg catgttccat cttgccgact aggcagtatc caccgatcag tatattaaat 180

gtgcaaacat caggcctcat a 201

<210> 56
<211> 201
<212> DNA
<213> Triticum aestivum

<400> 56
tatgacatca tatcatcaaa tatttttgag gcatgttcca tcttgccgac taggcagtat 60

ccaccgatca gtatattaaa tgtgcaaaca tcaggcctca yacctatgtg cgctatcaag 120

ccaaggacat cccgtgcttc tattaccctt ccttctgtgc aaaggttctg catgattgaa 180

ttgaagaatg caatatcagg a 201

<210> 57
<211> 201
<212> DNA
<213> Triticum aestivum

<400> 57
gcgctatcaa gccaaggaca tcccgtgctt ctattaccct tccttctgtg caaaggttct 60

gcatgattga attgaagaat gcaatatcag gaggaagcat rcctttgttc atcatttcag 120

taagcaattc ctttgctttc accaaatcgc cgtgtgtaca aaaaccctgg attagggaac 180

gataaacagc tgtgccctgt c 201

<210> 58
<211> 201
<212> DNA
<213> Triticum aestivum

<400> 58
cccgtgcttc tattaccctt ccttctgtgc aaaggttctg catgattgaa ttgaagaatg 60

caatatcagg aggaagcata cctttgttca tcatttcagt ragcaattcc tttgctttca 120

ccaaatcgcc gtgtgtacaa aaaccctgga ttagggaacg ataaacagct gtgccctgtc 180

gtactcctgt atcaatcatc t 201

<210> 59
<211> 201
<212> DNA
<213> Triticum aestivum

<400> 59
ttctgtgcaa aggttctgca tgattgaatt gaagaatgca atatcaggag gaagcatacc 60

tttgttcatc atttcagtaa gcaattcctt tgctttcacc raatcgccgt gtgtacaaaa 120

accctggatt agggaacgat aaacagctgt gccctgtcgt actcctgtat caatcatctg 180

attaaacttt atcacagcat c 201

<210> 60
<211> 201
<212> DNA
<213> Triticum aestivum

<400> 60
agaatgcaat atcaggagga agcatacctt tgttcatcat ttcagtaagc aattcctttg 60

ctttcaccaa atcgccgtgt gtacaaaaac cctggattag rgaacgataa acagctgtgc 120

cctgtcgtac tcctgtatca atcatctgat taaactttat cacagcatcg tccaacctac 180

ccttttgca aaatgcatgt a 201

<210> 61
<211> 201
<212> DNA
<213> Triticum aestivum

<400> 61
gcaatatcag gaggaagcat acctttgttc atcatttcag taagcaattc ctttgctttc 60

accaaatcgc cgtgtgtaca aaaaccctgg attagggaac sataaacagc tgtgccctgt 120

cgtactcctg tatcaatcat ctgattaaac tttatcacag catcgtccaa cctacccttt 180

ttgcaaaatg catgtattat g 201

<210>  62
<211>  201
<212>  DNA
<213>  Triticum aestivum

<400>  62
tgtattatgg ttgaatatgt gactgcattt ggactcactc cttgtttctg catttcattg      60

aagataagca tggccttatc aagcttccca gatttagcat rtgcattaat cagtatgttg     120

aagatacgac agtcaggtag aatgcagtat ctttccattg aattgaagag attaatcata     180

tcaactaagc atccttcggt g                                               201


<210>  63
<211>  201
<212>  DNA
<213>  Triticum aestivum

<400>  63
tgtgactgca tttggactca ctccttgttt ctgcatttca ttgaagataa gcatggcctt      60

atcaagcttc ccagatttag catgtgcatt aatcagtatg ytgaagatac gacagtcagg     120

tagaatgcag tatctttcca ttgaattgaa gagattaatc atatcaacta agcatccttc     180

ggtggcatac ccgtgaagga g                                               201


<210>  64
<211>  201
<212>  DNA
<213>  Triticum aestivum

<400>  64
tgcatttcat tgaagataag catggcctta tcaagcttcc cagatttagc atgtgcatta      60

atcagtatgt tgaagatacg acagtcaggt agaatgcagt mtctttccat tgaattgaag     120

agattaatca tatcaactaa gcatccttcg gtggcatacc cgtgaaggag aatagcatat     180

gaaacgttgt tatgtttcag g                                               201


<210>  65
<211>  201
<212>  DNA
<213>  Triticum aestivum

<400>  65
ctttgcttct tccatgtttg caaagaaagg tcataaacat gttgtgagta tgcacatttg      60

gtttaaccct ccgacttgtc atctctttga ataccctaac wgcctccttc cagtggcctg     120

aagaggaata tccatggatg aggctattat atgtcacatt atcaggtacg acaccatcat     180

caaccatctg acgaaggaaa t                                               201


<210>  66

<211>  201
<212>  DNA
<213>  Triticum aestivum

<400>  66
accctaactg cctccttcca gtggcctgaa gaggaatatc catggatgag gctattatat      60

gtcacattat caggtacgac accatcatca accatctgac saaggaaata ctctgccttg     120

tccatggctc tggccttgca cagcgcatca ataaccgagt tatatgtcac caaactaggc     180

gcaacgccct gctgcgccat t                                               201


<210>  67
<211>  201
<212>  DNA
<213>  Triticum aestivum

<400>  67
tggaagaagc catggatgac cgtgttatat gccaccacgt tgggggtgca gcctagctca      60

ggcatcctgt gaagcagaat gtccagagcc tcatcagccc rctttgcgcg gcagagtccc     120

ttgagaagag tgttgacttc tatgacgcct gccttgagtc ccgtcctgag gagacgggcg     180

aagaaggcga gcgccagatc c                                               201


<210>  68
<211>  201
<212>  DNA
<213>  Triticum aestivum

<400>  68
agccatggat gaccgtgtta tatgccacca cgttgggggt gcagcctagc tcaggcatcc      60

tgtgaagcag aatgtccaga gcctcatcag cccgctttgc rcggcagagt cccttgagaa     120

gagtgttgac ttctatgacg cctgccttga gtcccgtcct gaggagacgg gcgaagaagg     180

cgagcgccag atcctggcgg t                                               201


<210>  69
<211>  201
<212>  DNA
<213>  Triticum aestivum

<400>  69
aatgtccaga gcctcatcag cccgctttgc gcggcagagt cccttgagaa gagtgttgac      60

ttctatgacg cctgccttga gtcccgtcct gaggagacgg scgaagaagg cgagcgccag     120

atcctggcgg tttgcgcggc agcagcagtc catgaggatg ccgtaggtgc aggccgttgg     180

ctgcaccacc cgttggcggg c                                               201


<210>  70
<211>  201
<212>  DNA
<213>  Triticum aestivum

<400>  70
gctttgcgcg gcagagtccc ttgagaagag tgttgacttc tatgacgcct gccttgagtc      60

ccgtcctgag gagacgggcg aagaaggcga gcgccagatc ytggcggttt gcgcggcagc     120

agcagtccat gaggatgccg taggtgcagg ccgttggctg caccacccgt tggcgggcac     180

cttgggacaa acggctgaag a                                               201


<210>  71
<211>  201
<212>  DNA
<213>  Triticum aestivum

<400>  71
ggcagagtcc cttgagaaga gtgttgactt ctatgacgcc tgccttgagt cccgtcctga      60

ggagacgggc gaagaaggcg agcgccagat cctggcggtt ygcgcggcag cagcagtcca     120

tgaggatgcc gtaggtgcag gccgttggct gcaccacccg ttggcgggca ccttgggaca     180

aacggctgaa gagggcgagg g                                               201


<210>  72
<211>  201
<212>  DNA
<213>  Triticum aestivum

<400>  72
atgacgcctg ccttgagtcc cgtcctgagg agacgggcga agaaggcgag cgccagatcc      60

tggcggtttg cgcggcagca gcagtccatg aggatgccgt rggtgcaggc cgttggctgc     120

accacccgtt ggcgggcacc ttgggacaaa cggctgaaga gggcgagggc aagggcgggg     180

ccgttgctgc aggctgcgga c                                               201


<210>  73
<211>  201
<212>  DNA
<213>  Triticum aestivum

<400>  73
aggctctgga cgtgctgctt cacaggatgc ctgagctggg ctgcactcct aatgtggtgg      60

cgtataacac cgttatccat ggcttcttta aggaaggcca wgtaagcaag gcctgcaatc     120

tgttccatga aatggcgcag cagggcgtta agcctaatgt ggtgacatat aactcagtta     180

ttgatgcgct gtgcaaggcc a                                               201


<210>  74
<211>  201
<212>  DNA
<213>  Triticum aestivum

<400>  74
tgctgcttca caggatgcct gagctgggct gcactcctaa tgtggtggcg tataacaccg      60

ttatccatgg cttctttaag gaaggccatg taagcaaggc mtgcaatctg ttccatgaaa 120

tggcgcagca gggcgttaag cctaatgtgg tgacatataa ctcagttatt gatgcgctgt 180

gcaaggccag agccatggac a 201

<210> 75
<211> 201
<212> DNA
<213> Triticum aestivum

<400> 75
gtaagcaagg cctgcaatct gttccatgaa atggcgcagc agggcgttaa gcctaatgtg 60

gtgacatata actcagttat tgatgcgctg tgcaaggcca ragccatgga caaggcagag 120

gtggtccttc gtcagatgat tgatgatggt gttggacctg ataatgtgac gtatagtagc 180

ctcatccatg gatattcctc t 201

<210> 76
<211> 201
<212> DNA
<213> Triticum aestivum

<400> 76
cgcagcaggg cgttaagcct aatgtggtga catataactc agttattgat gcgctgtgca 60

aggccagagc catggacaag gcagaggtgg tccttcgtca ratgattgat gatggtgttg 120

gacctgataa tgtgacgtat agtagcctca tccatggata ttcctcttca ggccactgga 180

aggaggcagt tagggtattc a 201

<210> 77
<211> 201
<212> DNA
<213> Triticum aestivum

<400> 77
taagcctaat gtggtgacat ataactcagt tattgatgcg ctgtgcaagg ccagagccat 60

ggacaaggca gaggtggtcc ttcgtcagat gattgatgat rgtgttggac ctgataatgt 120

gacgtatagt agcctcatcc atggatattc ctcttcaggc cactggaagg aggcagttag 180

ggtattcaaa gagatgacaa g 201

<210> 78
<211> 201
<212> DNA
<213> Triticum aestivum

<400> 78
ggtgacatat aactcagtta ttgatgcgct gtgcaaggcc agagccatgg acaaggcaga 60

ggtggtcctt cgtcagatga ttgatgatgg tgttggacct rataatgtga cgtatagtag 120

cctcatccat ggatattcct cttcaggcca ctggaaggag gcagttaggg tattcaaaga 180

gatgacaagt cggagggtta c 201

<210> 79
<211> 201
<212> DNA
<213> Triticum aestivum

<400> 79
acatataact cagttattga tgcgctgtgc aaggccagag ccatggacaa ggcagaggtg 60

gtccttcgtc agatgattga tgatggtgtt ggacctgata rtgtgacgta tagtagcctc 120

atccatggat attcctcttc aggccactgg aaggaggcag ttagggtatt caaagagatg 180

acaagtcgga gggttacagc a 201

<210> 80
<211> 201
<212> DNA
<213> Triticum aestivum

<400> 80
catataactc agttattgat gcgctgtgca aggccagagc catggacaag gcagaggtgg 60

tccttcgtca gatgattgat gatggtgttg gacctgataa ygtgacgtat agtagcctca 120

tccatggata ttcctcttca ggccactgga aggaggcagt tagggtattc aaagagatga 180

caagtcggag ggttacagca g 201

<210> 81
<211> 201
<212> DNA
<213> Triticum aestivum

<400> 81
gccactggaa ggaggcagtt agggtattca aagagatgac aagtcggagg gttacagcag 60

atgtgcatac ttacaacatg tttatgacct ttctttgcaa rcatggaaga agcaaagaag 120

ctgcaggaat ttttgatacc atggctatca agggcctgaa acctgacaac gtttcatatg 180

ctattcgcct tcatgggtat g 201

<210> 82
<211> 201
<212> DNA
<213> Triticum aestivum

<400> 82
ccactggaag gaggcagtta gggtattcaa agagatgaca agtcggaggg ttacagcaga 60

tgtgcatact tacaacatgt ttatgacctt tctttgcaaa matggaagaa gcaaagaagc 120

tgcaggaatt tttgatacca tggctatcaa gggcctgaaa cctgacaacg tttcatatgc 180

tattcgcctt catgggtatg c 201

<210>  83
<211>  201
<212>  DNA
<213>  Triticum aestivum

<400>  83
aaggaggcag ttagggtatt caaagagatg acaagtcgga gggttacagc agatgtgcat      60

acttacaaca tgtttatgac ctttctttgc aaacatggaa raagcaaaga agctgcagga     120

atttttgata ccatggctat caagggcctg aaacctgaca acgtttcata tgctattcgc     180

cttcatgggt atgccaccga a                                               201


<210>  84
<211>  201
<212>  DNA
<213>  Triticum aestivum

<400>  84
gcagtcacat attcaaccgt aatacatgca ttttgcaaga agggtaggtt ggatgatgct      60

gtgataaagt ttaatcagat gattgataca ggagtacgac yggacgcatc tgtttatcgt     120

cccctaatcc agggtttttg tacacatggc gatttggtga aagcaaagga atatgttact     180

gaaatgatga agaaaggtat g                                               201


<210>  85
<211>  201
<212>  DNA
<213>  Triticum aestivum

<400>  85
atttaatttg ctgatcggtg gatactgcct agtccgcaag atggcagatg cattgaaagt      60

atttgatgat atggtgtcat atggtttaga accttgtaac wttacgtatg gtatacttat     120

taatggctat tgcaaaaata gaaggattga tgacgggctt attctgttca aagagatgct     180

gcacaaggga cttaaaccta c                                               201


<210>  86
<211>  201
<212>  DNA
<213>  Triticum aestivum

<400>  86
ggatggatta tttctggctg gacaaactgt tgctgcaaaa gagaagtttg atgagatggt      60

tgaatctgga gtaagtgtgt gcattgatac atactctata rttcttggtg gactttgtag     120

aaatagctgc agtagcgaag cgatcaccct tttccggaaa ttaagcgcaa tgaatgtgaa     180

atttgatatt acaattgtca a                                               201


<210>  87

<211>  201
<212>  DNA
<213>  Triticum aestivum

<400>  87
atgagatggt tgaatctgga gtaagtgtgt gcattgatac atactctata attcttggtg      60

gactttgtag aaatagctgc agtagcgaag cgatcaccct kttccggaaa ttaagcgcaa     120

tgaatgtgaa atttgatatt acaattgtca atatcattat tggtgcctta tacagggtcg     180

agagaaacca agaggctaag g                                               201


<210>  88
<211>  201
<212>  DNA
<213>  Triticum aestivum

<400>  88
atggttgaat ctggagtaag tgtgtgcatt gatacatact ctataattct tggtggactt      60

tgtagaaata gctgcagtag cgaagcgatc acccttttcc rgaaattaag cgcaatgaat     120

gtgaaatttg atattacaat tgtcaatatc attattggtg ccttatacag ggtcgagaga     180

aaccaagagg ctaaggattt g                                               201


<210>  89
<211>  201
<212>  DNA
<213>  Triticum aestivum

<400>  89
gaaatagctg cagtagcgaa gcgatcaccc ttttccggaa attaagcgca atgaatgtga      60

aatttgatat tacaattgtc aatatcatta ttggtgcctt mtacagggtc gagagaaacc     120

aagaggctaa ggatttgttt gctgctatgc cagccaatgg cttggttcct aatgctgtta     180

cctacaccgt aatgatgaca a                                               201


<210>  90
<211>  201
<212>  DNA
<213>  Triticum aestivum

<400>  90
caatgaatgt gaaatttgat attacaattg tcaatatcat tattggtgcc ttatacaggg      60

tcgagagaaa ccaagaggct aaggatttgt ttgctgctat kccagccaat ggcttggttc     120

ctaatgctgt tacctacacc gtaatgatga caaatcttat aaaagaaggt tcagtggaag     180

aagctgacaa tcttttctta t                                               201


<210>  91
<211>  201
<212>  DNA
<213>  Triticum aestivum

<400>  91
attgtcaata tcattattgg tgccttatac agggtcgaga gaaaccaaga ggctaaggat      60

ttgtttgctg ctatgccagc caatggcttg gttcctaatg ytgttaccta caccgtaatg     120

atgacaaatc ttataaaaga aggttcagtg gaagaagctg acaatctttt cttatccatg     180

gagaagagcg gctgtactgc c                                               201


<210>  92
<211>  201
<212>  DNA
<213>  Triticum aestivum

<400>  92
tattggtgcc ttatacaggg tcgagagaaa ccaagaggct aaggatttgt ttgctgctat      60

gccagccaat ggcttggttc ctaatgctgt tacctacacc rtaatgatga caaatcttat     120

aaaagaaggt tcagtggaag aagctgacaa tcttttctta tccatggaga agagcggctg     180

tactgccaac tcttgcctgt t                                               201


<210>  93
<211>  201
<212>  DNA
<213>  Triticum aestivum

<400>  93
gaaaccaaga ggctaaggat ttgtttgctg ctatgccagc caatggcttg gttcctaatg      60

ctgttaccta caccgtaatg atgacaaatc ttataaaaga rggttcagtg gaagaagctg     120

acaatctttt cttatccatg gagaagagcg gctgtactgc caactcttgc ctgttaaatc     180

atatcatcag aaggttactg g                                               201


<210>  94
<211>  201
<212>  DNA
<213>  Triticum aestivum

<400>  94
cagccaatgg cttggttcct aatgctgtta cctacaccgt aatgatgaca aatcttataa      60

aagaaggttc agtggaagaa gctgacaatc ttttcttatc satggagaag agcggctgta     120

ctgccaactc ttgcctgtta aatcatatca tcagaaggtt actggaaaaa ggagagatag     180

tcaaggctgg aaattatatg t                                               201


<210>  95
<211>  201
<212>  DNA
<213>  Triticum aestivum

<400>  95
aagttgatgc aaagagctac tcacttgaag ctaaaactgt ttcgctgctg atctctctgt      60

tttcaaggaa agggaaatat agagaacaca tcaaattgct ycctacaaag tatcagtttc 120

tggaagaagc agccacagtt gaatagttgg tacatgatat ctgaaattta atttgcatcg 180

cttgccatgg gtccgtctgc t 201

<210> 96
<211> 201
<212> DNA
<213> Triticum aestivum

<400> 96
gaagaagcaa agaagctgca ggaatttttg ataccatggc tatgaagggc ctgaaacctg 60

acaacgtttc atatgctatt ctccttcatg ggtatgccac ygaaggatgc ttagttgata 120

tgattaatct cttcaattca atggaaagag attgtattct acctgactgt cgtatcttca 180

acatactgat taatgcatat g 201

<210> 97
<211> 201
<212> DNA
<213> Triticum aestivum

<400> 97
atattcaact gtaatagatg cattttgcaa aaagggtcag ttggacgatg ctatgataaa 60

gtttaatcag atgattgata caggagtacg acagggcaca kctgtttatg gttctctaat 120

ccagggtttt tgtacacacg gcgatttggt gaaagcaaag gaattgctta ctgaaatgat 180

gaacaaaggt atgcttcctc c 201

<210> 98
<211> 201
<212> DNA
<213> Triticum aestivum

<400> 98
ttcaactgta atagatgcat tttgcaaaaa gggtcagttg gacgatgcta tgataaagtt 60

taatcagatg attgatacag gagtacgaca gggcacagct rtttatggtt ctctaatcca 120

gggtttttgt acacacggcg atttggtgaa agcaaaggaa ttgcttactg aaatgatgaa 180

caaaggtatg cttcctcctg a 201

<210> 99
<211> 201
<212> DNA
<213> Triticum aestivum

<400> 99
gttggacgat gctatgataa agtttaatca gatgattgat acaggagtac gacagggcac 60

agctgtttat ggttctctaa tccagggttt ttgtacacac kgcgatttgg tgaaagcaaa 120

ggaattgctt actgaaatga tgaacaaagg tatgcttcct cctgatatca agttcttcca 180

ttcaatcatg cagaaccttt g 201

<210> 100
<211> 201
<212> DNA
<213> Triticum aestivum

<400> 100
ataaagttta atcagatgat tgatacagga gtacgacagg gcacagctgt ttatggttct 60

ctaatccagg gtttttgtac acacggcgat ttggtgaaag saaaggaatt gcttactgaa 120

atgatgaaca aaggtatgct tcctcctgat atcaagttct tccattcaat catgcagaac 180

ctttgcacag aaggaagggt a 201

<210> 101
<211> 201
<212> DNA
<213> Triticum aestivum

<400> 101
atattcctct tcaggccact ggaaggaggc agttagggta ttcaaagaga tgacaagtcg 60

gagggttaca gcagatgtgc atacttacaa catgtttatg rcctttcttt gcaaacatgg 120

aagaagcaaa gaagctgcag gaatttttga taccatggct atcaagggcc tgaaacctga 180

caacgtttca tatgctattc t 201

<210> 102
<211> 201
<212> DNA
<213> Triticum aestivum

<400> 102
ttcaactgta atagatgcat tttgcaaaaa gggtcagttg gacgatgcta tgataaagtt 60

taatcagatg attgatacag gagtaccaca gggcacagct rtttatggtt ctctaatcca 120

gggtttttgt acacacggcg atttggtgaa agcaaaggaa ttggttactg aaatgatgaa 180

caaaggtata cctcctcctg a 201

<210> 103
<211> 201
<212> DNA
<213> Triticum aestivum

<400> 103
cagatgattg atacaggagt accacagggc acagctgttt atggttctct aatccagggt 60

ttttgtacac acggcgattt ggtgaaagca aaggaattgg ytactgaaat gatgaacaaa 120

ggtatacctc ctcctgatat taagttcttc cattcaatca tgcagaacct ttgcacagaa 180

ggaagggtaa tagaagcacg g 201

<210>  104
<211>  201
<212>  DNA
<213>  Triticum aestivum

<400>  104
acggcgattt ggtgaaagca aaggaattgg ttactgaaat gatgaacaaa ggtatacctc      60

ctcctgatat taagttcttc cattcaatca tgcagaacct wtgcacagaa ggaagggtaa     120

tagaagcacg ggatgtcctt ggcttgatag cgcacatagg tatgaagcct aatgtttgca     180

catttaatat actgggcggt g                                               201


<210>  105
<211>  201
<212>  DNA
<213>  Triticum aestivum

<400>  105
cttaaaccta caacttttaa ttacaacgtc atactggatg gattatttct aactggacga      60

actgttgctg caaaggaaaa gtttgatgag atggttgaat ytggagtaag tgtgtgcatt     120

gatacatatt cgatagttct tggtggactt tgtagaaata attgtagcgg cgaagcaatc     180

atgctattcc agaaattaag c                                               201


<210>  106
<211>  201
<212>  DNA
<213>  Triticum aestivum

<400>  106
ctattcaact gtggctgctt cttccagaaa ctgatacttt gtagggagca atttgatgtg      60

ttctctatat ttccctttcc ttgaaaacag agagatcagc wgtgaaacag ttttagcttc     120

aagggagtag ctctttgcat caactttaga catataattt ccagccttga ccatctctcc     180

tttttccagt aaccttctga t                                               201


<210>  107
<211>  201
<212>  DNA
<213>  Triticum aestivum

<400>  107
ccagaaactg atactttgta gggagcaatt tgatgtgttc tctatatttc cctttccttg      60

aaaacagaga gatcagcagt gaaacagttt tagcttcaag kgagtagctc tttgcatcaa     120

ctttagacat ataatttcca gccttgacca tctctccttt ttccagtaac cttctgatga     180

tatgatttaa catataagag t                                               201


<210>  108

<211>  201
<212>  DNA
<213>  Triticum aestivum

<400>  108
catcctccat ctcgcggact aggcagtatc caccgattag taaattaaag atgggaacat        60

caggcctcat acctatgtgc agcatcaagt cgaggatatc ycgtgcttca attacccttc       120

cttccgtgca taggttctgc attattgaat ggaagaactt aatatcagga ggacgcatag       180

ctttgttcct catttcagta a                                                 201


<210>  109
<211>  201
<212>  DNA
<213>  Triticum aestivum

<400>  109
caagcataag gacccatggc aagcaacaat ccaagttaac ttctaggagt tgagttcaga        60

gatcatgtat caaactattc aactgtggct gcttcttcga kaaactgata cttcataggg       120

agcaatttta tgtgttctct atacttccct ttccctgaaa agagggagat cagcagcgaa       180

acagttttag cttcaagtga g                                                 201


<210>  110
<211>  201
<212>  DNA
<213>  Triticum aestivum

<400>  110
aaagagggag atcagcagcg aaacagtttt agcttcaagt gagtagctct ttgcatcaac        60

tttcgacata taatttccag ccttgactat ctctcctctt yccagtaacc ttctgataat       120

atgatttaac atccaagagt ttgcagtaca gccgctcttc tccattgata aaaagagtgt       180

cagcttcttc cactgaacct t                                                 201


<210>  111
<211>  201
<212>  DNA
<213>  Triticum aestivum

<400>  111
gaatttcatg ttcattgccc ttaatttctg gaacagcatg attacttcgc tagagcaatt        60

atttctgcaa agtccagcaa gaactatcga atatgtatca stgcacacac ttactccaga       120

ttcaaccatc tcatcaaact tttcctttgc agcaacagtt cgtccagcca gaaataaccc       180

atccagtatg acattataat t                                                 201


<210>  112
<211>  201
<212>  DNA
<213>  Triticum aestivum

<400>  112
tttctgcaaa gtccagcaag aactatcgaa tatgtatcag tgcacacact tactccagat      60

tcaaccatct catcaaactt ttcctttgca gcaacagttc rtccagccag aaataaccca     120

tccagtatga cattataatt aaaagttgta ggtttaagtc ccttgtgcag catctcttgg     180

aacagaataa gcccatcatc a                                               201


<210>  113
<211>  201
<212>  DNA
<213>  Triticum aestivum

<400>  113
tattacaagg ttctaaacca aatgacacca tatcctcaaa catttttgtt gcatcctcca      60

tcttgcagac taggcagtac ccaccgatca gtatattaaa yatgaaaaca ttaggcctca     120

taccaatgcg cactatcaag tcaaggatat ctcgtgcttc tattaccctt ccttctgtgc     180

atagtttctg catgattgaa c                                               201


<210>  114
<211>  201
<212>  DNA
<213>  Triticum aestivum

<400>  114
ctttatcaca gcatcatcca acctaccctt tttgcaaaat gcatgtatta cggttgaata      60

tgtgactgca tctggactca ctccttgttt ctgcatatct ytaaatataa gcatagcctt     120

atcaagcttc ccagatttag catatgcata aatcagtatg ttgaagatat gacagttagg     180

tagaatacag tctgtagcca t                                               201


<210>  115
<211>  201
<212>  DNA
<213>  Triticum aestivum

<400>  115
ttaggtagaa tacagtctgt agccattgaa ttgaagagat tgatcatgtc aaccaagcat      60

ccttcagtgg catacccatg aaggagaata gcatatgaaa yggtgttagg tttcaggccc     120

ttcatggaca ttgaattaaa aatttctgca gcttctttgc ttcttccatt cttgcaaagg     180

gaggccatga gcgagttgca a                                               201


<210>  116
<211>  201
<212>  DNA
<213>  Triticum aestivum

<400>  116
agattgatca tgtcaaccaa gcatccttca gtggcatacc catgaaggag aatagcatat      60

gaaatggtgt taggtttcag gcccttcatg gacattgaat yaaaaatttc tgcagcttct 120

ttgcttcttc cattcttgca aagggaggcc atgagcgagt tgcaagtaaa aacatctggt 180

atgataccct tacttgtcat c 201

<210> 117
<211> 201
<212> DNA
<213> Triticum aestivum

<400> 117
tgatcatgtc aaccaagcat ccttcagtgg catacccatg aaggagaata gcatatgaaa 60

tggtgttagg tttcaggccc ttcatggaca ttgaattaaa watttctgca gcttctttgc 120

ttcttccatt cttgcaaagg gaggccatga gcgagttgca agtaaaaaca tctggtatga 180

tacccttact tgtcatctct t 201

<210> 118
<211> 201
<212> DNA
<213> Triticum aestivum

<400> 118
taaaaatttc tgcagcttct ttgcttcttc cattcttgca aagggaggcc atgagcgagt 60

tgcaagtaaa aacatctggt atgataccct tacttgtcat mtctttcagc accctgaccg 120

cctcctttga atggcctgaa gaggaatatc catggatgag gctattatat gtcacattgt 180

taggtacgac accactatca a 201

<210> 119
<211> 201
<212> DNA
<213> Triticum aestivum

<400> 119
agttgcaagt aaaaacatct ggtatgatac ccttacttgt catctctttc agcaccctga 60

ccgcctcctt tgaatggcct gaagaggaat atccatggat raggctatta tatgtcacat 120

tgttaggtac gacaccacta tcaaccatct gaccaaggaa atactccgcc ttgtccattg 180

ctctggcctt gcacagtgca t 201

<210> 120
<211> 201
<212> DNA
<213> Triticum aestivum

<400> 120
gtatgatacc cttacttgtc atctctttca gcaccctgac cgcctccttt gaatggcctg 60

aagaggaata tccatggatg aggctattat atgtcacatt rttaggtacg acaccactat 120

caaccatctg accaaggaaa tactccgcct tgtccattgc tctggccttg cacagtgcat 180

caataaccga gttatatgtc a 201

<210> 121
<211> 201
<212> DNA
<213> Triticum aestivum

<400> 121
atgataccct tacttgtcat ctctttcagc accctgaccg cctcctttga atggcctgaa 60

gaggaatatc catggatgag gctattatat gtcacattgt yaggtacgac accactatca 120

accatctgac caaggaaata ctccgccttg tccattgctc tggccttgca cagtgcatca 180

ataaccgagt tatatgtcac c 201

<210> 122
<211> 201
<212> DNA
<213> Triticum aestivum

<400> 122
tgtcatctct ttcagcaccc tgaccgcctc ctttgaatgg cctgaagagg aatatccatg 60

gatgaggcta ttatatgtca cattgttagg tacgacacca ytatcaacca tctgaccaag 120

gaaatactcc gccttgtcca ttgctctggc cttgcacagt gcatcaataa ccgagttata 180

tgtcaccacg tcaggcgcaa c 201

<210> 123
<211> 201
<212> DNA
<213> Triticum aestivum

<400> 123
agccatggat gaccgtgcta tacgccacca cgtcaggggt gcagcccagc tcaggcatcc 60

tgtgaagcag cacgtccaga gcctcatctg agcgctttgc rtggcagagt cccttgagga 120

gggtgctgaa aacgacttgg tctgcctcca ggcccgtctt gaggagacgg ccgaagaagg 180

cgatcgctag atccaggcgg c 201

<210> 124
<211> 201
<212> DNA
<213> Triticum aestivum

<400> 124
catggatgac cgtgctatac gccaccacgt caggggtgca gcccagctca ggcatcctgt 60

gaagcagcac gtccagagcc tcatctgagc gctttgcgtg kcagagtccc ttgaggaggg 120

tgctgaaaac gacttggtct gcctccaggc ccgtcttgag gagacggccg aagaaggcga 180

tcgctagatc caggcggcgc g 201

<210>  125
<211>  201
<212>  DNA
<213>  Triticum aestivum

<400>  125
accacgtcag gggtgcagcc cagctcaggc atcctgtgaa gcagcacgtc cagagcctca      60

tctgagcgct ttgcgtggca gagtcccttg aggagggtgc wgaaaacgac ttggtctgcc      120

tccaggcccg tcttgaggag acggccgaag aaggcgatcg ctagatccag gcggcgcgca      180

cggcagtagc agtccatgag g                                                201


<210>  126
<211>  201
<212>  DNA
<213>  Triticum aestivum

<400>  126
cgtcaggggt gcagcccagc tcaggcatcc tgtgaagcag cacgtccaga gcctcatctg      60

agcgctttgc gtggcagagt cccttgagga gggtgctgaa racgacttgg tctgcctcca      120

ggcccgtctt gaggagacgg ccgaagaagg cgatcgctag atccaggcgg cgcgcacggc      180

agtagcagtc catgaggacg c                                                201


<210>  127
<211>  201
<212>  DNA
<213>  Triticum aestivum

<400>  127
tcaggggtgc agcccagctc aggcatcctg tgaagcagca cgtccagagc ctcatctgag      60

cgctttgcgt ggcagagtcc cttgaggagg gtgctgaaaa ygacttggtc tgcctccagg      120

cccgtcttga ggagacggcc gaagaaggcg atcgctagat ccaggcggcg cgcacggcag      180

tagcagtcca tgaggacgcc a                                                201


<210>  128
<211>  201
<212>  DNA
<213>  Triticum aestivum

<400>  128
cagcccagct caggcatcct gtgaagcagc acgtccagag cctcatctga gcgctttgcg      60

tggcagagtc ccttgaggag ggtgctgaaa acgacttggt ytgcctccag gcccgtcttg      120

aggagacggc cgaagaaggc gatcgctaga tccaggcggc gcgcacggca gtagcagtcc      180

atgaggacgc cataggtgaa g                                                201


<210>  129

<211>  201
<212>  DNA
<213>  Triticum aestivum

<400>  129
caggcatcct gtgaagcagc acgtccagag cctcatctga gcgctttgcg tggcagagtc      60

ccttgaggag ggtgctgaaa acgacttggt ctgcctccag kcccgtcttg aggagacggc     120

cgaagaaggc gatcgctaga tccaggcggc gcgcacggca gtagcagtcc atgaggacgc     180

cataggtgaa gacatttggc t                                               201


<210>  130
<211>  201
<212>  DNA
<213>  Triticum aestivum

<400>  130
cacgtccaga gcctcatctg agcgctttgc gtggcagagt cccttgagga gggtgctgaa      60

aacgacttgg tctgcctcca ggcccgtctt gaggagacgg scgaagaagg cgatcgctag     120

atccaggcgg cgcgcacggc agtagcagtc catgaggacg ccataggtga agacatttgg     180

ctgcgccacc cgtcgtccgg c                                               201


<210>  131
<211>  201
<212>  DNA
<213>  Triticum aestivum

<400>  131
cttgaggagg gtgctgaaaa cgacttggtc tgcctccagg cccgtcttga ggagacggcc      60

gaagaaggcg atcgctagat ccaggcggcg cgcacggcag yagcagtcca tgaggacgcc     120

ataggtgaag acatttggct gcgccacccg tcgtccggcg cctcgggaca aacggccgaa     180

gagggcgacg gccagggcag g                                               201


<210>  132
<211>  201
<212>  DNA
<213>  Triticum aestivum

<400>  132
ctgcctccag gcccgtcttg aggagacggc cgaagaaggc gatcgctaga tccaggcggc      60

gcgcacggca gtagcagtcc atgaggacgc cataggtgaa sacatttggc tgcgccaccc     120

gtcgtccggc gcctcgggac aaacggccga agagggcgac ggccagggca gggccattgc     180

tgaagaatgc ggacgcggac g                                               201


<210>  133
<211>  201
<212>  DNA
<213>  Triticum aestivum

<400>  133
gagacggccg aagaaggcga tcgctagatc caggcggcgc gcacggcagt agcagtccat      60

gaggacgcca taggtgaaga catttggctg cgccacccgt ygtccggcgc ctcgggacaa     120

acggccgaag agggcgacgg ccagggcagg gccattgctg aagaatgcgg acgcggacgc     180

gcgggcgagg gcagccagaa a                                               201


<210>  134
<211>  201
<212>  DNA
<213>  Triticum aestivum

<400>  134
gacggccgaa gaaggcgatc gctagatcca ggcggcgcgc acggcagtag cagtccatga      60

ggacgccata ggtgaagaca tttggctgcg ccacccgtcg yccggcgcct cgggacaaac     120

ggccgaagag ggcgacggcc agggcagggc cattgctgaa gaatgcggac gcggacgcgc     180

gggcgagggc agccagaaag t                                               201


<210>  135
<211>  201
<212>  DNA
<213>  Triticum aestivum

<400>  135
cggccgaaga aggcgatcgc tagatccagg cggcgcgcac ggcagtagca gtccatgagg      60

acgccatagg tgaagacatt tggctgcgcc acccgtcgtc yggcgcctcg ggacaaacgg     120

ccgaagaggg cgacggccag ggcagggcca ttgctgaaga atgcggacgc ggacgcgcgg     180

gcgagggcag ccagaaagtt a                                               201


<210>  136
<211>  201
<212>  DNA
<213>  Triticum aestivum

<400>  136
agtagcagtc catgaggacg ccataggtga agacatttgg ctgcgccacc cgtcgtccgg      60

cgcctcggga caaacggccg aagagggcga cggccagggc rgggccattg ctgaagaatg     120

cggacgcgga cgcgcgggcg agggcagcca gaaagttagt caagggacgc tcctggacag     180

gattgccctg ccgcagcaat t                                               201


<210>  137
<211>  201
<212>  DNA
<213>  Triticum aestivum

<400>  137
gacgccatag gtgaagacat ttggctgcgc cacccgtcgt ccggcgcctc gggacaaacg      60

gccgaagagg gcgacggcca gggcagggcc attgctgaag ratgcggacg cggacgcgcg 120

ggcgagggca gccagaaagt tagtcaaggg acgctcctgg acaggattgc cctgccgcag 180

caattcatca aacaggtggt g 201

<210> 138
<211> 201
<212> DNA
<213> Triticum aestivum

<400> 138
caattgcaat attgaatttg acattcattg tacttaattt cttggacagc gtgattgctt 60

caccggtgca attatttcta caaagtccac caagaactat mgaatatgta tcaatgcaca 120

cacttactcc agattcaacc atctcatcaa acttttcctt tgcagcaaca gttcgtccag 180

ccagaaataa tccatccagt a 201

<210> 139
<211> 201
<212> DNA
<213> Triticum aestivum

<400> 139
ttgcaatatt gaatttgaca ttcattgtac ttaatttctt ggacagcgtg attgcttcac 60

cggtgcaatt atttctacaa agtccaccaa gaactatcga rtatgtatca atgcacacac 120

ttactccaga ttcaaccatc tcatcaaact tttcctttgc agcaacagtt cgtccagcca 180

gaaataatcc atccagtatg a 201

<210> 140
<211> 201
<212> DNA
<213> Triticum aestivum

<400> 140
gtataccata cgtactgtta caaggttcta aaccatatga caccatatca tcaaacattt 60

ttgttgcatc ctccatcttg tggactaggc agtatccacc ratcagtaaa ttaaagatgg 120

gaacatcagg cctcatacct atgtgcacca tcaagtcgag gatatcccgt gcttctatta 180

cccttccttc cgtgcatagg t 201

<210> 141
<211> 201
<212> DNA
<213> Triticum aestivum

<400> 141
tgttacaagg ttctaaacca tatgacacca tatcatcaaa catttttgtt gcatcctcca 60

tcttgtggac taggcagtat ccaccaatca gtaaattaaa katgggaaca tcaggcctca 120

tacctatgtg caccatcaag tcgaggatat cccgtgcttc tattaccctt ccttccgtgc 180

ataggttctt cattattgaa t 201

<210> 142
<211> 201
<212> DNA
<213> Triticum aestivum

<400> 142
tacaaggttc taaaccatat gacaccatat catcaaacat ttttgttgca tcctccatct 60

tgtggactag gcagtatcca ccaatcagta aattaaagat sggaacatca ggcctcatac 120

ctatgtgcac catcaagtcg aggatatccc gtgcttctat tacccttcct tccgtgcata 180

ggttcttcat tattgaatgg a 201

<210> 143
<211> 201
<212> DNA
<213> Triticum aestivum

<400> 143
acaaggttct aaaccatatg acaccatatc atcaaacatt tttgttgcat cctccatctt 60

gtggactagg cagtatccac caatcagtaa attaaagatg rgaacatcag gcctcatacc 120

tatgtgcacc atcaagtcga ggatatcccg tgcttctatt acccttcctt ccgtgcatag 180

gttcttcatt attgaatgga a 201

<210> 144
<211> 201
<212> DNA
<213> Triticum aestivum

<400> 144
caaggttcta aaccatatga caccatatca tcaaacattt ttgttgcatc ctccatcttg 60

tggactaggc agtatccacc aatcagtaaa ttaaagatgg raacatcagg cctcatacct 120

atgtgcacca tcaagtcgag gatatcccgt gcttctatta cccttccttc cgtgcatagg 180

ttcttcatta ttgaatggaa g 201

<210> 145
<211> 201
<212> DNA
<213> Triticum aestivum

<400> 145
acaccatatc atcaaacatt tttgttgcat cctccatctt gtggactagg cagtatccac 60

caatcagtaa attaaagatg ggaacatcag gcctcatacc watgtgcacc atcaagtcga 120

ggatatcccg tgcttctatt acccttcctt ccgtgcatag gttcttcatt attgaatgga 180

agatcttaat atcaggagga c 201

<210>  146
<211>  201
<212>  DNA
<213>  Triticum aestivum

<400>  146
catatcatca aacatttttg ttgcatcctc catcttgtgg actaggcagt atccaccaat      60

cagtaaatta aagatgggaa catcaggcct catacctatg ygcaccatca agtcgaggat      120

atcccgtgct tctattaccc ttccttccgt gcataggttc ttcattattg aatggaagat      180

cttaatatca ggaggacgca t                                               201


<210>  147
<211>  201
<212>  DNA
<213>  Triticum aestivum

<400>  147
catcaaacat ttttgttgca tcctccatct tgtggactag gcagtatcca ccaatcagta      60

aattaaagat gggaacatca ggcctcatac ctatgtgcac yatcaagtcg aggatatccc      120

gtgcttctat tacccttcct tccgtgcata ggttcttcat tattgaatgg aagatcttaa      180

tatcaggagg acgcatagct t                                               201


<210>  148
<211>  201
<212>  DNA
<213>  Triticum aestivum

<400>  148
catcctccat cttgtggact aggcagtatc caccaatcag taaattaaag atgggaacat      60

caggcctcat acctatgtgc accatcaagt cgaggatatc ycgtgcttct attacccttc      120

cttccgtgca taggttcttc attattgaat ggaagatctt aatatcagga ggacgcatag      180

ctttgttcct catttcagta a                                               201


<210>  149
<211>  201
<212>  DNA
<213>  Triticum aestivum

<400>  149
tcccgtgctt ctattaccct tccttccgtg cataggttct tcattattga atggaagatc      60

ttaatatcag gaggacgcat agctttgttc ctcatttcag yaaccaattc ctttgctttc      120

actaaatccc catgtgtaca aaaaccctgg attagggaat gataaacaat tgcattcggt      180

cgtactccca tatcaaccat c                                               201


<210>  150

<211>  201
<212>  DNA
<213>  Triticum aestivum

<400>  150
ccattgaatt gaagagatta atcatgtcaa ccaagcatcc ttcagtggca tacccatgaa      60

ggagaatagc atatgaaacg gtgtcacgtt tcaggccctt satagccatt gaatcaaaaa     120

tttctgcagc ttctttgctt tttccattct tgcaaaggaa ggccatgagt gaggtgcagg     180

taatgacatc tggtgtaaac c                                               201


<210>  151
<211>  201
<212>  DNA
<213>  Triticum aestivum

<400>  151
ccagaaagtt attcaaggga cgctcctgga caggattgcc ctgccgcagc aattcgtcga      60

acaggtggtg tgcgtcctcc gtggtgagcc taccagagcg mgcccgctcc gtcgcggcgg     120

cgaaggcggc gtggggatcc cagatgcgtg agggatgaga ggtggaggag gagtggcggg     180

cgcagaggag gaggaggagg a                                               201


<210>  152
<211>  201
<212>  DNA
<213>  Triticum aestivum

<400>  152
cctccgtggt gagcctacca gagcgcgccc gctccgtcgc ggcggcgaag gcggcgtggg      60

gatcccagat gcgtgaggga tgagaggtgg aggaggagtg rcgggcgcag aggaggagga     120

ggaggaggag gaggaggcgg aggcgtggtg gcgacattgg cgtgctggag gagaagcgtc     180

aagcgaggca tgggcttggt g                                               201


<210>  153
<211>  201
<212>  DNA
<213>  Triticum aestivum

<400>  153
caagcataag gacccatggc aagcaacaat ccaagttaac ttctaggagt tgagttcaga      60

gatcatgtat caaactattc aactgtggct gcttcttcga kaaactgata cttcataggg     120

agcaatttta tgtgttctct atacttccct ttccctgaaa agagggagat cagcagcgaa     180

acagttttag cttcaagtga g                                               201


<210>  154
<211>  201
<212>  DNA
<213>  Triticum aestivum

<400>  154
aaagagggag atcagcagcg aaacagtttt agcttcaagt gagtagctct ttgcatcaac      60

tttcgacata taatttccag ccttgactat ctctcctctt yccagtaacc ttctgataat     120

atgatttaac atccaagagt ttgcagtaca gccgctcttc tccattgata aaaagagtgt     180

cagcttcttc cactgaacct t                                               201


<210>  155
<211>  201
<212>  DNA
<213>  Triticum aestivum

<400>  155
acatataatt tccagccttg actatctctc ctcttcccag taaccttctg ataatatgat      60

ttaacatcca agagtttgca gtacagccgc tcttctccat ygataaaaag agtgtcagct     120

tcttccactg aaccttcttt tataagattt ttcatcatta tgctgtaggt aacaacatta     180

gctaccaagc cattggatgg t                                               201


<210>  156
<211>  201
<212>  DNA
<213>  Triticum aestivum

<400>  156
ctgataatat gatttaacat ccaagagttt gcagtacagc cgctcttctc cattgataaa      60

aagagtgtca gcttcttcca ctgaaccttc ttttataaga yttttcatca ttatgctgta     120

ggtaacaaca ttagctacca agccattgga tggtattgca gcaaacaaat ccttagcttc     180

ttggtttcgc cgaaccttgt a                                               201


<210>  157
<211>  201
<212>  DNA
<213>  Triticum aestivum

<400>  157
gaatttcatg ttcattgccc ttaatttctg gaacagcatg attacttcgc tagagcaatt      60

atttctgcaa agtccagcaa gaactatcga atatgtatca stgcacacac ttactccaga     120

ttcaaccatc tcatcaaact tttcctttgc agcaacagtt cgtccagcca gaaataaccc     180

atccagtatg acattataat t                                               201


<210>  158
<211>  201
<212>  DNA
<213>  Triticum aestivum

<400>  158
tttctgcaaa gtccagcaag aactatcgaa tatgtatcag tgcacacact tactccagat      60

tcaaccatct catcaaactt ttcctttgca gcaacagttc rtccagccag aaataaccca 120

tccagtatga cattataatt aaaagttgta ggtttaagtc ccttgtgcag catctcttgg 180

aacagaataa gcccatcatc a 201

<210> 159
<211> 201
<212> DNA
<213> Triticum aestivum

<400> 159
tattacaagg ttctaaacca aatgacacca tatcctcaaa catttttgtt gcatcctcca 60

tcttgcagac taggcagtac ccaccgatca gtatattaaa yatgaaaaca ttaggcctca 120

taccaatgcg cactatcaag tcaaggatat ctcgtgcttc tattaccctt ccttctgtgc 180

atagtttctg catgattgaa c 201

<210> 160
<211> 201
<212> DNA
<213> Triticum aestivum

<400> 160
catatcttta aatataagca tagccttatc aagcttccca gatttagcat atgcataaat 60

cagtatgttg aagatatgac agttaggtag aatacagtct stagccattg aattgaagag 120

attgatcatg tcaaccaagc atccttcagt ggcataccca tgaaggagaa tagcatatga 180

aatggtgtta ggtttcaggc c 201

<210> 161
<211> 201
<212> DNA
<213> Triticum aestivum

<400> 161
atctttaaat ataagcatag ccttatcaag cttcccagat ttagcatatg cataaatcag 60

tatgttgaag atatgacagt taggtagaat acagtctgta kccattgaat tgaagagatt 120

gatcatgtca accaagcatc cttcagtggc atacccatga aggagaatag catatgaaat 180

ggtgttaggt ttcaggccct t 201

<210> 162
<211> 201
<212> DNA
<213> Triticum aestivum

<400> 162
taaatataag catagcctta tcaagcttcc cagatttagc atatgcataa atcagtatgt 60

tgaagatatg acagttaggt agaatacagt ctgtagccat ygaattgaag agattgatca 120

tgtcaaccaa gcatccttca gtggcatacc catgaaggag aatagcatat gaaatggtgt 180

taggtttcag gcccttcatg g 201

<210> 163
<211> 201
<212> DNA
<213> Triticum aestivum

<400> 163
ccttatcaag cttcccagat ttagcatatg cataaatcag tatgttgaag atatgacagt 60

taggtagaat acagtctgta gccattgaat tgaagagatt ratcatgtca accaagcatc 120

cttcagtggc atacccatga aggagaatag catatgaaat ggtgttaggt ttcaggccct 180

tcatggacat tgaattaaaa a 201

<210> 164
<211> 201
<212> DNA
<213> Triticum aestivum

<400> 164
caagcttccc agatttagca tatgcataaa tcagtatgtt gaagatatga cagttaggta 60

gaatacagtc tgtagccatt gaattgaaga gattgatcat rtcaaccaag catccttcag 120

tggcataccc atgaaggaga atagcatatg aaatggtgtt aggtttcagg cccttcatgg 180

acattgaatt aaaaatttct g 201

<210> 165
<211> 201
<212> DNA
<213> Triticum aestivum

<400> 165
ttaggtagaa tacagtctgt agccattgaa ttgaagagat tgatcatgtc aaccaagcat 60

ccttcagtgg catacccatg aaggagaata gcatatgaaa yggtgttagg tttcaggccc 120

ttcatggaca ttgaattaaa aatttctgca gcttctttgc ttcttccatt cttgcaaagg 180

gaggccatga gcgagttgca a 201

<210> 166
<211> 201
<212> DNA
<213> Triticum aestivum

<400> 166
agccatggat gaccgtgcta tacgccacca cgtcaggggt gcagcccagc tcaggcatcc 60

tgtgaagcag cacgtccaga gcctcatctg agcgctttgc rtggcagagt cccttgagga 120

gggtgctgaa aacgacttgg tctgcctcca ggcccgtctt gaggagacgg ccgaagaagg 180

cgatcgctag atccaggcgg c 201

<210>  167
<211>  201
<212>  DNA
<213>  Triticum aestivum

<400>  167
catggatgac cgtgctatac gccaccacgt caggggtgca gcccagctca ggcatcctgt      60

gaagcagcac gtccagagcc tcatctgagc gctttgcgtg kcagagtccc ttgaggaggg     120

tgctgaaaac gacttggtct gcctccaggc ccgtcttgag gagacggccg aagaaggcga     180

tcgctagatc caggcggcgc g                                               201


<210>  168
<211>  201
<212>  DNA
<213>  Triticum aestivum

<400>  168
accacgtcag gggtgcagcc cagctcaggc atcctgtgaa gcagcacgtc cagagcctca      60

tctgagcgct ttgcgtggca gagtcccttg aggagggtgc wgaaaacgac ttggtctgcc     120

tccaggcccg tcttgaggag acggccgaag aaggcgatcg ctagatccag gcggcgcgca     180

cggcagtagc agtccatgag g                                               201


<210>  169
<211>  201
<212>  DNA
<213>  Triticum aestivum

<400>  169
cgtcaggggt gcagcccagc tcaggcatcc tgtgaagcag cacgtccaga gcctcatctg      60

agcgctttgc gtggcagagt cccttgagga gggtgctgaa racgacttgg tctgcctcca     120

ggcccgtctt gaggagacgg ccgaagaagg cgatcgctag atccaggcgg cgcgcacggc     180

agtagcagtc catgaggacg c                                               201


<210>  170
<211>  201
<212>  DNA
<213>  Triticum aestivum

<400>  170
tcaggggtgc agcccagctc aggcatcctg tgaagcagca cgtccagagc ctcatctgag      60

cgctttgcgt ggcagagtcc cttgaggagg gtgctgaaaa ygacttggtc tgcctccagg     120

cccgtcttga ggagacggcc gaagaaggcg atcgctagat ccaggcggcg cgcacggcag     180

tagcagtcca tgaggacgcc a                                               201


<210>  171

<211>  201
<212>  DNA
<213>  Triticum aestivum

<400>  171
cagcccagct caggcatcct gtgaagcagc acgtccagag cctcatctga gcgctttgcg      60

tggcagagtc ccttgaggag ggtgctgaaa acgacttggt ytgcctccag gcccgtcttg     120

aggagacggc cgaagaaggc gatcgctaga tccaggcggc gcgcacggca gtagcagtcc     180

atgaggacgc cataggtgaa g                                               201


<210>  172
<211>  201
<212>  DNA
<213>  Triticum aestivum

<400>  172
caggcatcct gtgaagcagc acgtccagag cctcatctga gcgctttgcg tggcagagtc      60

ccttgaggag ggtgctgaaa acgacttggt ctgcctccag kcccgtcttg aggagacggc     120

cgaagaaggc gatcgctaga tccaggcggc gcgcacggca gtagcagtcc atgaggacgc     180

cataggtgaa gacatttggc t                                               201


<210>  173
<211>  201
<212>  DNA
<213>  Triticum aestivum

<400>  173
cacgtccaga gcctcatctg agcgctttgc gtggcagagt cccttgagga gggtgctgaa      60

aacgacttgg tctgcctcca ggcccgtctt gaggagacgg scgaagaagg cgatcgctag     120

atccaggcgg cgcgcacggc agtagcagtc catgaggacg ccataggtga agacatttgg     180

ctgcgccacc cgtcgtccgg c                                               201


<210>  174
<211>  201
<212>  DNA
<213>  Triticum aestivum

<400>  174
cttgaggagg gtgctgaaaa cgacttggtc tgcctccagg cccgtcttga ggagacggcc      60

gaagaaggcg atcgctagat ccaggcggcg cgcacggcag yagcagtcca tgaggacgcc     120

ataggtgaag acatttggct gcgccacccg tcgtccggcg cctcgggaca aacggccgaa     180

gagggcgacg gccagggcag g                                               201


<210>  175
<211>  201
<212>  DNA
<213>  Triticum aestivum

<400>  175
ctgcctccag gcccgtcttg aggagacggc cgaagaaggc gatcgctaga tccaggcggc      60

gcgcacggca gtagcagtcc atgaggacgc cataggtgaa sacatttggc tgcgccaccc     120

gtcgtccggc gcctcgggac aaacggccga agagggcgac ggccagggca gggccattgc     180

tgaagaatgc ggacgcggac g                                               201


<210>  176
<211>  201
<212>  DNA
<213>  Triticum aestivum

<400>  176
gagacggccg aagaaggcga tcgctagatc caggcggcgc gcacggcagt agcagtccat      60

gaggacgcca taggtgaaga catttggctg cgccacccgt ygtccggcgc ctcgggacaa     120

acggccgaag agggcgacgg ccagggcagg gccattgctg aagaatgcgg acgcggacgc     180

gcgggcgagg gcagccagaa a                                               201


<210>  177
<211>  201
<212>  DNA
<213>  Triticum aestivum

<400>  177
gacggccgaa gaaggcgatc gctagatcca ggcggcgcgc acggcagtag cagtccatga      60

ggacgccata ggtgaagaca tttggctgcg ccacccgtcg yccggcgcct cgggacaaac     120

ggccgaagag ggcgacggcc agggcagggc cattgctgaa gaatgcggac gcggacgcgc     180

gggcgagggc agccagaaag t                                               201


<210>  178
<211>  201
<212>  DNA
<213>  Triticum aestivum

<400>  178
cggccgaaga aggcgatcgc tagatccagg cggcgcgcac ggcagtagca gtccatgagg      60

acgccatagg tgaagacatt tggctgcgcc acccgtcgtc yggcgcctcg ggacaaacgg     120

ccgaagaggg cgacggccag ggcagggcca ttgctgaaga atgcggacgc ggacgcgcgg     180

gcgagggcag ccagaaagtt a                                               201


<210>  179
<211>  201
<212>  DNA
<213>  Triticum aestivum

<400>  179
agtagcagtc catgaggacg ccataggtga agacatttgg ctgcgccacc cgtcgtccgg      60

cgcctcggga caaacggccg aagagggcga cggccagggc rgggccattg ctgaagaatg 120

cggacgcgga cgcgcgggcg agggcagcca gaaagttagt caagggacgc tcctggacag 180

gattgccctg ccgcagcaat t 201

<210> 180
<211> 201
<212> DNA
<213> Triticum aestivum

<400> 180
gacgccatag gtgaagacat ttggctgcgc cacccgtcgt ccggcgcctc gggacaaacg 60

gccgaagagg gcgacggcca gggcagggcc attgctgaag ratgcggacg cggacgcgcg 120

ggcgagggca gccagaaagt tagtcaaggg acgctcctgg acaggattgc cctgccgcag 180

caattcatca aacaggtggt g 201

<210> 181
<211> 201
<212> DNA
<213> Triticum aestivum

<400> 181
cagttcgtcc agccagaaat aatccatcca gtatgacgtt gtaattaaaa gttgtaggtt 60

taagtccctt gcgcaacatt tctttgaaca gattcagccc rtcatcaatc cttccgtttt 120

tgcaatagcc attaataagt ataccatacg tactgttaca aggttctaaa ccatatgaca 180

ccatatcatc aaacattttt g 201

<210> 182
<211> 201
<212> DNA
<213> Triticum aestivum

<400> 182
tgtaggttta agtcccttgc gcaacatttc tttgaacaga ttcagcccgt catcaatcct 60

tccgtttttg caatagccat taataagtat accatacgta mtgttacaag gttctaaacc 120

atatgacacc atatcatcaa acatttttgt tgcatcctcc atcttgtgga ctaggcagta 180

tccaccaatc agtaaattaa a 201

<210> 183
<211> 201
<212> DNA
<213> Triticum aestivum

<400> 183
caccatcaag tcgaggatat cccgtgcttc tattaccctt ccttccgtgc ataggttctt 60

cattattgaa tggaagatct taatatcagg aggacgcata sctttgttcc tcatttcagt 120

aaccaattcc tttgctttca ctaaatcccc atgtgtacaa aaaccctgga ttagggaatg 180

ataaacaatt gcattcggtc g 201

<210> 184
<211> 201
<212> DNA
<213> Triticum aestivum

<400> 184
tcccgtgctt ctattaccct tccttccgtg cataggttct tcattattga atggaagatc 60

ttaatatcag gaggacgcat agctttgttc ctcatttcag yaaccaattc ctttgctttc 120

actaaatccc catgtgtaca aaaaccctgg attagggaat gataaacaat tgcattcggt 180

cgtactccca tatcaaccat c 201

<210> 185
<211> 201
<212> DNA
<213> Triticum aestivum

<400> 185
acccttcctt ccgtgcatag gttcttcatt attgaatgga agatcttaat atcaggagga 60

cgcatagctt tgttcctcat ttcagtaacc aattcctttg stttcactaa atccccatgt 120

gtacaaaaac cctggattag ggaatgataa acaattgcat tcggtcgtac tcccatatca 180

accatctggt taaatttgtc c 201

<210> 186
<211> 201
<212> DNA
<213> Triticum aestivum

<400> 186
ttgaatggaa gatcttaata tcaggaggac gcatagcttt gttcctcatt tcagtaacca 60

attcctttgc tttcactaaa tccccatgtg tacaaaaacc mtggattagg gaatgataaa 120

caattgcatt cggtcgtact cccatatcaa ccatctggtt aaatttgtcc atagcaatgt 180

ccaagctacc cattttgcaa a 201

<210> 187
<211> 201
<212> DNA
<213> Triticum aestivum

<400> 187
cttaatatca ggaggacgca tagctttgtt cctcatttca gtaaccaatt cctttgcttt 60

cactaaatcc ccatgtgtac aaaaaccctg gattagggaa ygataaacaa ttgcattcgg 120

tcgtactccc atatcaacca tctggttaaa tttgtccata gcaatgtcca agctacccat 180

tttgcaaaat gcatgtatta c 201

<210>  188
<211>  201
<212>  DNA
<213>  Triticum aestivum

<400>  188
cgcatagctt tgttcctcat ttcagtaacc aattcctttg ctttcactaa atccccatgt      60

gtacaaaaac cctggattag ggaatgataa acaattgcat ycggtcgtac tcccatatca     120

accatctggt taaatttgtc catagcaatg tccaagctac ccattttgca aaatgcatgt     180

attacagttg aataggtgac t                                               201


<210>  189
<211>  201
<212>  DNA
<213>  Triticum aestivum

<400>  189
tcctcatttc agtaaccaat tcctttgctt tcactaaatc cccatgtgta caaaaaccct      60

ggattaggga atgataaaca attgcattcg gtcgtactcc yatatcaacc atctggttaa     120

atttgtccat agcaatgtcc aagctaccca ttttgcaaaa tgcatgtatt acagttgaat     180

aggtgactac atctggactc a                                               201


<210>  190
<211>  201
<212>  DNA
<213>  Triticum aestivum

<400>  190
caagcataag gacccatggc aagcaacaat ccaagttaac ttctaggagt tgagttcaga      60

gatcatgtat caaactattc aactgtggct gcttcttcga kaaactgata cttcataggg     120

agcaatttta tgtgttctct atacttccct ttccctgaaa agagggagat cagcagcgaa     180

acagttttag cttcaagtga g                                               201


<210>  191
<211>  201
<212>  DNA
<213>  Triticum aestivum

<400>  191
aaagagggag atcagcagcg aaacagtttt agcttcaagt gagtagctct ttgcatcaac      60

tttcgacata taatttccag ccttgactat ctctcctctt yccagtaacc ttctgataat     120

atgatttaac atccaagagt ttgcagtaca gccgctcttc tccattgata aaaagagtgt     180

cagcttcttc cactgaacct t                                               201


<210>  192

<211>  201
<212>  DNA
<213>  Triticum aestivum

<400>  192
gaatttcatg ttcattgccc ttaatttctg gaacagcatg attacttcgc tagagcaatt      60

atttctgcaa agtccagcaa gaactatcga atatgtatca stgcacacac ttactccaga      120

ttcaaccatc tcatcaaact tttcctttgc agcaacagtt cgtccagcca gaaataaccc      180

atccagtatg acattataat t                                                201


<210>  193
<211>  201
<212>  DNA
<213>  Triticum aestivum

<400>  193
tttctgcaaa gtccagcaag aactatcgaa tatgtatcag tgcacacact tactccagat      60

tcaaccatct catcaaactt ttcctttgca gcaacagttc rtccagccag aaataaccca      120

tccagtatga cattataatt aaaagttgta ggtttaagtc ccttgtgcag catctcttgg      180

aacagaataa gcccatcatc a                                                201


<210>  194
<211>  201
<212>  DNA
<213>  Triticum aestivum

<400>  194
tattacaagg ttctaaacca aatgacacca tatcctcaaa catttttgtt gcatcctcca      60

tcttgcagac taggcagtac ccaccgatca gtatattaaa yatgaaaaca ttaggcctca      120

taccaatgcg cactatcaag tcaaggatat ctcgtgcttc tattaccctt ccttctgtgc      180

atagtttctg catgattgaa c                                                201


<210>  195
<211>  201
<212>  DNA
<213>  Triticum aestivum

<400>  195
ctttatcaca gcatcatcca acctaccctt tttgcaaaat gcatgtatta cggttgaata      60

tgtgactgca tctggactca ctccttgttt ctgcatatct ytaaatataa gcatagcctt      120

atcaagcttc ccagatttag catatgcata aatcagtatg ttgaagatat gacagttagg      180

tagaatacag tctgtagcca t                                                201


<210>  196
<211>  201
<212>  DNA
<213>  Triticum aestivum

<400>  196
catatcttta aatataagca tagccttatc aagcttccca gatttagcat atgcataaat      60

cagtatgttg aagatatgac agttaggtag aatacagtct stagccattg aattgaagag      120

attgatcatg tcaaccaagc atccttcagt ggcataccca tgaaggagaa tagcatatga      180

aatggtgtta ggtttcaggc c                                                201


<210>  197
<211>  201
<212>  DNA
<213>  Triticum aestivum

<400>  197
atctttaaat ataagcatag ccttatcaag cttcccagat ttagcatatg cataaatcag      60

tatgttgaag atatgacagt taggtagaat acagtctgta kccattgaat tgaagagatt      120

gatcatgtca accaagcatc cttcagtggc atacccatga aggagaatag catatgaaat      180

ggtgttaggt ttcaggccct t                                                201


<210>  198
<211>  201
<212>  DNA
<213>  Triticum aestivum

<400>  198
taaatataag catagcctta tcaagcttcc cagatttagc atatgcataa atcagtatgt      60

tgaagatatg acagttaggt agaatacagt ctgtagccat ygaattgaag agattgatca      120

tgtcaaccaa gcatccttca gtggcatacc catgaaggag aatagcatat gaaatggtgt      180

taggtttcag gcccttcatg g                                                201


<210>  199
<211>  201
<212>  DNA
<213>  Triticum aestivum

<400>  199
ccttatcaag cttcccagat ttagcatatg cataaatcag tatgttgaag atatgacagt      60

taggtagaat acagtctgta gccattgaat tgaagagatt ratcatgtca accaagcatc      120

cttcagtggc atacccatga aggagaatag catatgaaat ggtgttaggt ttcaggccct      180

tcatggacat tgaattaaaa a                                                201


<210>  200
<211>  201
<212>  DNA
<213>  Triticum aestivum

<400>  200
caagcttccc agatttagca tatgcataaa tcagtatgtt gaagatatga cagttaggta      60

gaatacagtc tgtagccatt gaattgaaga gattgatcat rtcaaccaag catccttcag 120

tggcataccc atgaaggaga atagcatatg aaatggtgtt aggtttcagg cccttcatgg 180

acattgaatt aaaaatttct g 201

<210> 201
<211> 201
<212> DNA
<213> Triticum aestivum

<400> 201
ttaggtagaa tacagtctgt agccattgaa ttgaagagat tgatcatgtc aaccaagcat 60

ccttcagtgg catacccatg aaggagaata gcatatgaaa yggtgttagg tttcaggccc 120

ttcatggaca ttgaattaaa aatttctgca gcttctttgc ttcttccatt cttgcaaagg 180

gaggccatga gcgagttgca a 201

<210> 202
<211> 201
<212> DNA
<213> Triticum aestivum

<400> 202
cctacttggc cttccttgaa gaagccatgg atgaccgtgc tatacgccac cacgtcaggg 60

gtgcagccca gctcaggcat cctgtgaagc agcacgtcca ragcctcatc tgagcgcttt 120

gcgtggcaga gtcccttgag gagggtgctg aaaacgactt ggtctgcctc caggcccgtc 180

ttgaggagac ggccgaagaa g 201

<210> 203
<211> 201
<212> DNA
<213> Triticum aestivum

<400> 203
agccatggat gaccgtgcta tacgccacca cgtcaggggt gcagcccagc tcaggcatcc 60

tgtgaagcag cacgtccaga gcctcatctg agcgctttgc rtggcagagt cccttgagga 120

gggtgctgaa aacgacttgg tctgcctcca ggcccgtctt gaggagacgg ccgaagaagg 180

cgatcgctag atccaggcgg c 201

<210> 204
<211> 201
<212> DNA
<213> Triticum aestivum

<400> 204
catggatgac cgtgctatac gccaccacgt caggggtgca gcccagctca ggcatcctgt 60

gaagcagcac gtccagagcc tcatctgagc gctttgcgtg kcagagtccc ttgaggaggg 120

tgctgaaaac gacttggtct gcctccaggc ccgtcttgag gagacggccg aagaaggcga 180

tcgctagatc caggcggcgc g 201

<210> 205
<211> 201
<212> DNA
<213> Triticum aestivum

<400> 205
accacgtcag gggtgcagcc cagctcaggc atcctgtgaa gcagcacgtc cagagcctca 60

tctgagcgct ttgcgtggca gagtcccttg aggagggtgc wgaaaacgac ttggtctgcc 120

tccaggcccg tcttgaggag acggccgaag aaggcgatcg ctagatccag gcggcgcgca 180

cggcagtagc agtccatgag g 201

<210> 206
<211> 201
<212> DNA
<213> Triticum aestivum

<400> 206
cgtcaggggt gcagcccagc tcaggcatcc tgtgaagcag cacgtccaga gcctcatctg 60

agcgctttgc gtggcagagt cccttgagga gggtgctgaa racgacttgg tctgcctcca 120

ggcccgtctt gaggagacgg ccgaagaagg cgatcgctag atccaggcgg cgcgcacggc 180

agtagcagtc catgaggacg c 201

<210> 207
<211> 201
<212> DNA
<213> Triticum aestivum

<400> 207
tcaggggtgc agcccagctc aggcatcctg tgaagcagca cgtccagagc ctcatctgag 60

cgctttgcgt ggcagagtcc cttgaggagg gtgctgaaaa ygacttggtc tgcctccagg 120

cccgtcttga ggagacggcc gaagaaggcg atcgctagat ccaggcggcg cgcacggcag 180

tagcagtcca tgaggacgcc a 201

<210> 208
<211> 201
<212> DNA
<213> Triticum aestivum

<400> 208
cagcccagct caggcatcct gtgaagcagc acgtccagag cctcatctga gcgctttgcg 60

tggcagagtc ccttgaggag ggtgctgaaa acgacttggt ytgcctccag gcccgtcttg 120

aggagacggc cgaagaaggc gatcgctaga tccaggcggc gcgcacggca gtagcagtcc 180

atgaggacgc cataggtgaa g 201

<210>  209
<211>  201
<212>  DNA
<213>  Triticum aestivum

<400>  209
caggcatcct gtgaagcagc acgtccagag cctcatctga gcgctttgcg tggcagagtc      60

ccttgaggag ggtgctgaaa acgacttggt ctgcctccag kcccgtcttg aggagacggc     120

cgaagaaggc gatcgctaga tccaggcggc gcgcacggca gtagcagtcc atgaggacgc     180

cataggtgaa gacatttggc t                                               201


<210>  210
<211>  201
<212>  DNA
<213>  Triticum aestivum

<400>  210
cacgtccaga gcctcatctg agcgctttgc gtggcagagt cccttgagga gggtgctgaa      60

aacgacttgg tctgcctcca ggcccgtctt gaggagacgg scgaagaagg cgatcgctag     120

atccaggcgg cgcgcacggc agtagcagtc catgaggacg ccataggtga agacatttgg     180

ctgcgccacc cgtcgtccgg c                                               201


<210>  211
<211>  201
<212>  DNA
<213>  Triticum aestivum

<400>  211
cttgaggagg gtgctgaaaa cgacttggtc tgcctccagg cccgtcttga ggagacggcc      60

gaagaaggcg atcgctagat ccaggcggcg cgcacggcag yagcagtcca tgaggacgcc     120

ataggtgaag acatttggct gcgccacccg tcgtccggcg cctcgggaca aacggccgaa     180

gagggcgacg gccagggcag g                                               201


<210>  212
<211>  201
<212>  DNA
<213>  Triticum aestivum

<400>  212
ctgcctccag gcccgtcttg aggagacggc cgaagaaggc gatcgctaga tccaggcggc      60

gcgcacggca gtagcagtcc atgaggacgc cataggtgaa sacatttggc tgcgccaccc     120

gtcgtccggc gcctcgggac aaacggccga agagggcgac ggccagggca gggccattgc     180

tgaagaatgc ggacgcggac g                                               201


<210>  213

<211>  201
<212>  DNA
<213>  Triticum aestivum

<400>  213
gagacggccg aagaaggcga tcgctagatc caggcggcgc gcacggcagt agcagtccat      60

gaggacgcca taggtgaaga catttggctg cgccacccgt ygtccggcgc ctcgggacaa      120

acggccgaag agggcgacgg ccagggcagg gccattgctg aagaatgcgg acgcggacgc      180

gcgggcgagg gcagccagaa a                                                201


<210>  214
<211>  201
<212>  DNA
<213>  Triticum aestivum

<400>  214
gacggccgaa gaaggcgatc gctagatcca ggcggcgcgc acggcagtag cagtccatga      60

ggacgccata ggtgaagaca tttggctgcg ccacccgtcg yccggcgcct cgggacaaac      120

ggccgaagag ggcgacggcc agggcagggc cattgctgaa gaatgcggac gcggacgcgc      180

gggcgagggc agccagaaag t                                                201


<210>  215
<211>  201
<212>  DNA
<213>  Triticum aestivum

<400>  215
cggccgaaga aggcgatcgc tagatccagg cggcgcgcac ggcagtagca gtccatgagg      60

acgccatagg tgaagacatt tggctgcgcc acccgtcgtc yggcgcctcg ggacaaacgg      120

ccgaagaggg cgacggccag ggcagggcca ttgctgaaga atgcggacgc ggacgcgcgg      180

gcgagggcag ccagaaagtt a                                                201


<210>  216
<211>  201
<212>  DNA
<213>  Triticum aestivum

<400>  216
agtagcagtc catgaggacg ccataggtga agacatttgg ctgcgccacc cgtcgtccgg      60

cgcctcggga caaacggccg aagagggcga cggccagggc rgggccattg ctgaagaatg      120

cggacgcgga cgcgcgggcg agggcagcca gaaagttagt caagggacgc tcctggacag      180

gattgccctg ccgcagcaat t                                                201


<210>  217
<211>  201
<212>  DNA
<213>  Triticum aestivum

<400>  217
gacgccatag gtgaagacat ttggctgcgc cacccgtcgt ccggcgcctc gggacaaacg      60

gccgaagagg gcgacggcca gggcagggcc attgctgaag ratgcggacg cggacgcgcg      120

ggcgagggca gccagaaagt tagtcaaggg acgctcctgg acaggattgc cctgccgcag      180

caattcatca aacaggtggt g                                                201


<210>  218
<211>  201
<212>  DNA
<213>  Triticum aestivum

<400>  218
catctcatca aacttttcct ttgcagcaac agttcgtcca gccagaaata atccatccag      60

tatgacgttg taattaaaag ttgtaggttt aagtcccttg sgcaacattt ctttgaacag      120

attcagcccg tcatcaatcc ttccgttttt gcaatagcca ttaataagta taccatacgt      180

actgttacaa ggttctaaac c                                                201


<210>  219
<211>  201
<212>  DNA
<213>  Triticum aestivum

<400>  219
caccatcaag tcgaggatat cccgtgcttc tattaccctt ccttccgtgc ataggttctt      60

cattattgaa tggaagatct taatatcagg aggacgcata sctttgttcc tcatttcagt      120

aaccaattcc tttgctttca ctaaatcccc atgtgtacaa aaaccctgga ttagggaatg      180

ataaacaatt gcattcggtc g                                                201


<210>  220
<211>  201
<212>  DNA
<213>  Triticum aestivum

<400>  220
tcccgtgctt ctattaccct tccttccgtg cataggttct tcattattga atggaagatc      60

ttaatatcag gaggacgcat agctttgttc ctcatttcag yaaccaattc ctttgctttc      120

actaaatccc catgtgtaca aaaaccctgg attagggaat gataaacaat tgcattcggt      180

cgtactccca tatcaaccat c                                                201


<210>  221
<211>  201
<212>  DNA
<213>  Triticum aestivum

<400>  221
acccttcctt ccgtgcatag gttcttcatt attgaatgga agatcttaat atcaggagga      60

cgcatagctt tgttcctcat ttcagtaacc aattcctttg stttcactaa atccccatgt 120

gtacaaaaac cctggattag ggaatgataa acaattgcat tcggtcgtac tcccatatca 180

accatctggt taaatttgtc c 201

<210> 222
<211> 201
<212> DNA
<213> Triticum aestivum

<400> 222
ttgaatggaa gatcttaata tcaggaggac gcatagcttt gttcctcatt tcagtaacca 60

attcctttgc tttcactaaa tccccatgtg tacaaaaacc mtggattagg gaatgataaa 120

caattgcatt cggtcgtact cccatatcaa ccatctggtt aaatttgtcc atagcaatgt 180

ccaagctacc cattttgcaa a 201

<210> 223
<211> 201
<212> DNA
<213> Triticum aestivum

<400> 223
cttaatatca ggaggacgca tagctttgtt cctcatttca gtaaccaatt cctttgcttt 60

cactaaatcc ccatgtgtac aaaaaccctg gattagggaa ygataaacaa ttgcattcgg 120

tcgtactccc atatcaacca tctggttaaa tttgtccata gcaatgtcca agctacccat 180

tttgcaaaat gcatgtatta c 201

<210> 224
<211> 201
<212> DNA
<213> Triticum aestivum

<400> 224
cgcatagctt tgttcctcat ttcagtaacc aattcctttg ctttcactaa atccccatgt 60

gtacaaaaac cctggattag ggaatgataa acaattgcat ycggtcgtac tcccatatca 120

accatctggt taaatttgtc catagcaatg tccaagctac ccattttgca aaatgcatgt 180

attacagttg aataggtgac t 201

<210> 225
<211> 201
<212> DNA
<213> Triticum aestivum

<400> 225
tcctcatttc agtaaccaat tcctttgctt tcactaaatc cccatgtgta caaaaaccct 60

ggattaggga atgataaaca attgcattcg gtcgtactcc yatatcaacc atctggttaa 120

atttgtccat agcaatgtcc aagctaccca ttttgcaaaa tgcatgtatt acagttgaat 180

aggtgactac atctggactc a 201